# EUROPEAN PATENT APPLICATION

(11) **EP 2 657 231 A2**
(43) Date of publication of application: **30.10.2013**
(21) Application number: 11844522.0
(22) Date of filing: 28.11.2011
(51) Int. Cl.: C07D 403/04

(54) **SUBSTITUTED AZOLES, ANTI-VIRAL ACTIVE INGREDIENT, PHARMACEUTICAL COMPOSITION, METHOD FOR THE PRODUCTION AND USE THEREOF**

(30) Priority: 30.11.2010 RU 2010148813
(71) Applicant: Ivachtchenko, Alexandre Vasilievich, Encinitas, CA 92024 (US); Alla Chem, LLC., Carson City, NV 89701 (US)
(72) Inventor: BICHKO, Vadim Vasilievich, San Diego, CA 92129 (US); MITKIN, Oleg Dmitrievich, Moskovskaya obl. 141400 (RU)
(74) Representative: Patentanwälte Zellentin & Partner
(86) International application number: PCT/RU2011/000932
(87) International publication number: WO 2012/074437

(57) **Abstract**

The present invention relates to novel azoles, novel antiviral active components of the general formulas **1A** and **1B**, pharmaceutical composition, antiviral medicament, method for prophylaxis and treatment of viral diseases, particularly caused by hepatisis C viruses (HCV). In general formulas **1A** and **1B** 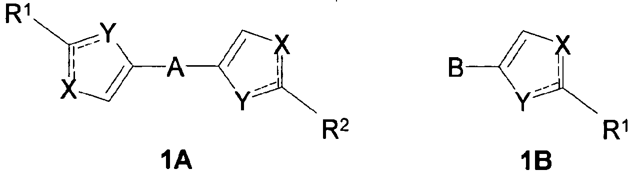 wherein: solid lines with accompanying dotted lines (---) represent ordinary bond or double bond, provided that one of them is an ordinary bond, the other one is double bond; **X** and **Y** accept various meanings, one of them is - nitrogen, the other - oxygen, sulfur or NH group; **R¹** and **R²** - optionally the same radicals selected from 2-(R)- and (S)- substituted N-acyl pyrrolidine derivatives; N-methyl-N-[2-(R) and (S)- substituted 2,2-disubstituted acetamides; methyl[2-(R) and (S)-substituted ((methyl)amino)-(1-oxobutan-2-yl)-2-(R)-] and (S)-iso-propyl)-carbamates. **A** represents aliphatic C₂-C₈ biradical; dioxane, cyclo- and bicycloaliphatic, alkyloxyalkyl, alkyloxyalkylenoxyalkyl, alkenyloxyalkyl, alkynyloxyalkyl biradicals and their thioanaloges; aryl and thiophene alkynylcycloalkyl, alkynyldioxane, alkynylaryl, alkylthiophene, alkenylthiophene and alkynylthiophene, alkyloxyaryl, alkenyloxyaryl, alkynyloxyaryl, alkylthioaryl, alkenylthioaryl, alkynylthioaryl, cycloalkylthiophene, aryldioxane and thiophenyldioxane biradicals. **B** represents: aliphatic C₂-C₈ radical, including 1,2 or 3 triple C≡C bonds; aryl and thiophene, alkynylcycloalkyl, alkynyldioxane, alkynylaryl, alkylthiophene, alkenylthiophene and alkynylthiophene, cycloalkylbenzene, 4-cycloalkylbiphenyl, bicycloalkylbenzene, 4-bicycloalkylbiphenyl, cycloalkylthiophene, aryldioxane and thiophenyldioxane radicals.

## Description

The present invention relates to novel azoles, novel antiviral active component, pharmaceutical composition, antiviral medicament, method for prophylaxis and treatment of viral diseases, particularly caused by hepatitis C viruses (HCV).

Virus infections may cause a great number of diseases that creates a serious threat for health and existence of mankind. For the last 20 years not less than 30 essentially new infectious agents have been discovered such as: HIV, viral hepatitises, acute and long-lasting diarrhea, hemorrhagic fever (Ebola, Venezuelan, Brazilian, Rift valleys) [a) Lednicky J.A., Rayner J.O. Uncommon respiratory pathogens. Curr. Opin. Pulm. Med. 2006, 12(3), 235-239. b) Hayden F.G. Respiratory viral threats. Curr. Opin. Infect. Dis. 2006, 19(2), 169-178]. In particular, special anxiety is caused by the risk of so named avian influenza infection. [a) Liu J.P. Avian influenza - a pandemic waiting to happen? J. Microbiol. Immunol. Infect. 2006, 39(1), 4-10. b) Henter J.I.; Chow C.B.; Leung C.W, Lau Y.L. Cytotoxic therapy for severe avian influenza A (H5N1) infection. Lancet. 2006 367(9513), 870-873. Review]. According to statistical data 60-65% of epidemic infections have viral ethiology. Because of the complexity of interaction in triad "virus - host's organism - drug", most of modern antiviral drugs lead to side effects in the course of therapy and form resistant virus strains [Jain R., Clark N.M., Diaz-Linares M., Grim S.A. Limitations of current antiretroviral agents and opportunities for development. Curr. Pharm. Des. 2006, 12(9), 1065-1074.]. At present, the number of antiviral drugs that may be used in clinical practice is extremely limited - only 43 low molecular weight substances [http://integrity.prous.com/integrity], that is far from satisfying the requirements of prophylaxis and treatment of virus diseases. Moreover, there are a lot of virus infections causing diseases for treatment of which there are no chematherapeutic agents. It concerns, for example, to the diseases caused by viruses of papilloma, adenoviruses, herpes-6, variola, syndrome SARS, hemorrhagic fevers, Western

Nile fever, avian influenza and so on. [De Clercq E. Recent highlights in the development of new antiviral drugs. Curr Opin Microbiol. 2005, 8(5), 552-560].

Hepatitis C virus falls into the category of Flaviviruses (genus Flaviviridae), together with other important human pathogens, such as yellow fever virus, West Nile virus, Dengue virus and hepatitis GBV-C virus. Flaviviruses possess similar genom structure, including genom coding non-structural NS5A protein. Being a structural component of virus replication complex NS5A plays important role in virus RNA-genom replication. As far as this protein has been validated now in clinical trials as a target for design of medicaments for treating long-lasting hepatitis C, NS5A is considered to be a promising target for other listed above clinically important Flaviviruses as well.

Thus, the development of novel antiflavivirus medicaments, especially possessing high activity and low toxicity is of great importance now.

There are some publications in patent literature, dedicated to various derivatives of 2-pyrrolidin-2-yl-1H-imidazoles, which are ligands of non-structural protein NS5A and suppress hepatitis C virus (HCV) [WO 2008021927A2, WO2009020825A1, WO2009020828A1, WO2010065668A1, WO2010065681A1, WO2010096302A1, WO2010096462A1, WO2010096777A1, WO2010111534A1, WO2010111673A1, WO2010117635A1, WO2010117977A1].

However, now searching of novel medicaments exhibiting high antiflavivirus efficiency is still one of the principal directions in the developing of new pharmacological agents for treating vide and diversified types of viral infections, including HCV.

In this context, synthesis of new compounds and putting them to use as antiviral active components, including HCV, for pharmaceutical compositions and medicament is of high priority.

In context of the invention, terms are generally defined as follows:
**"Aliphatic"** radical means a radical derived at removal of hydrogen atom from non aromatic C-H bond. Aliphatic radical may additionally comprise substituents - aliphatic or aromatic radicals defined in this section. Alkyl, alkenyl, alkynyl, cycloalkyl, cycloalkenyl, heterocyclyl, heterocyclenyl, aralkenyl, aralkoxyalkyl, aralkyloxycarbonylalkyl, aralkyl, aralkynyl, aralkyloxyalkenyl, heteroaralkenyl, heteroaralkyl, heteroaralkyloxyalkenyl, heteroaralkyloxyalkyl, heteroaralkenyl, annelated arylcycloalkyl, annelated heteroarylcycloalkyl, annelated arylcycloalkenyl, annelated heteroarylcycloalkenyl, annelated arylheterocyclyl, annelated heteroarylheterocyclyl, annelated arylheterocyclenyl, annelated heteroarylheterocyclenyl are aliphatic radicals.
**"Aliphatic"** biradical means a biradical derived at removal of hydrogen atom from C-H bond of aliphatic radical, specification of which was given above.
**"Alkenyl"** means aliphatic straight or branched hydrocarbon chain, comprising 2-7 carbon atoms and including at least one carbon-carbon double bond. Branched means that straight alkenyl chain contains one or more lower alkyl groups, such as methyl, ethyl or propyl. Alkyl group may have one or more substituents, for example, halogen, alkenyloxy, cycloalkyl, cyano, hydroxy, alkoxy, carboxy, alkynyloxy, aralkoxy, aryloxy, aryloxycarbonyl, alkylthio, heteroaralkyloxy, heterocyclyl, heterocyclylalkyloxy, alkoxycarbonyl, aralkoxycarbonyl, heteroaralkyloxycarbonyl or Rₖ^{a}Rₖ₊₁^{a}N-, Rₖ^{a}Rₖ₊₁^{a}NC(=O)-, Rₖ^{a}Rₖ₊₁^{a}NSO₂-, wherein Rₖ^{a} and Rₖ₊₁^{a} independently of each other represent "amino group substituent" , the meanings of which are defined in this section, for example, hydrogen, alkyl, aryl, aralkyl, heteroaralkyl, heterocyclyl or heteroaryl, or Rₖ^{a} and Rₖ₊₁^{a} together with the nitrogen atom they are attached to form through Rₖ^{a} and Rₖ₊₁^{a} 4 - 7 membered heterocyclyl or heterocyclenyl. Preferred alkyl groups are methyl, trifluoromethyl, cyclopropylmethyl, cyclopentylmethyl, ethyl, *n*-propyl, *iso*-propyl, n-butyl, *tert*.-butyl, *n*-pentyl, 3-pentyl, methoxyethyl, carboxymethyl, methoxycarbonylmethyl, benzyloxycarbonylmethyl and pyridylmethyloxycarbonylmethyl. The preferred alkenyl groups are ethenyl, propenyl, *n*-butenyl, *iso*-butenyl, 3-methylbut-2-enyl, *n*-pentenyl and cyclohexylbutenyl.
**"Alkenyloxy"** means alkenyl-O-group, in which alkenyl is defined in this section. The preferred alkenyloxy- groups are allyloxy- and 3-butenyloxy.
**"Alkenyloxyalkyl"** means alkenyl-O-alkyl group, in which alkyl and alkenyl are defined in this section.
**"Alkyl"** means aliphatic hydrocarbon straight or branched chain with 1-12 carbon atoms. Branched means alkyl chain with one or more "lower alkyl" substituents. Alkyl group may have one or more substituents of the same or different structure ("alkyl substituent") including halogen, alkenyloxy, cycloalkyl, aryl, heteroaryl, heterocyclyl, aroyl, cyano, hydroxy, alkoxy, carboxy, alkynyloxy, aralkoxy, aryloxy, aryloxycarbonyl, alkylthio, heteroarylthio, aralkylthio, arylsulfonyl, alkylsulfonylheteroaralkyloxy, annelated heteroarylcycloalkenyl, annelated heteroarylcycloalkyl, annelated heteroarylheterocyclenyl, annelated heteroarylheterocyclyl, annelated arylcycloalkenyl, annelated arylcycloalkyl, annelated arylheterocyclenyl, annelated arylheterocyclyl, alkoxycarbonyl, aralkoxycarbonyl, heteroaralkyloxycarbonyl or Rₖ^{a}Rₖ₊₁^{a}N-, Rₖ^{a}Rₖ₊₁^{a}NC(=O)-, Rₖ^{a}Rₖ₊₁^{a}NC(=S)-, Rₖ^{a}Rₖ₊₁^{a}NSO₂-, where Rₖ^{a} and Rₖ₊₁^{a} independently of each other represent "amino group substituents", the meanings of which are defined in this section, for example, hydrogen, alkyl, aryl, aralkyl, heteroaralkyl, heterocyclyl or heteroaryl, or Rₖ^{a} and Rₖ₊₁^{a} together with the N-atom, they are attached to, form through Rₖ^{a} and Rₖ₊₁^{a} 4-7-membered heterocyclyl or heterocyclenyl. Preferred alkyl groups are methyl, trifluoromethyl, cyclopropylmethyl, cyclopentylmethyl, ethyl, *n*-propyl, *iso*-propyl, *n*-butyl, *tert-*butyl, *n*-pentyl, 3-pentyl, methoxyethyl, carboxymethyl, methoxycarbonylmethyl, ethoxycarbonylmethyl, benzyloxycarbonylmethyl methoxycarbonylmethyl and pyridylmethyloxycarbonylmethyl. The preferred "alkyl substituents" are cycloalkyl, aryl, heteroaryl, heterocyclyl, hydroxy, alkoxy, alkoxycarbonyl, aralkoxy, aryloxy, alkylthio, heteroarylthio, aralkylthio, alkylsulfonyl, arylsulfonyl, alkoxycarbonyl, aralkoxycarbonyl, heteroaralkyloxycarbonyl or Rₖ^{a}Rₖ₊₁^{a}N-, Rₖ^{a}Rₖ₊₁^{a}NC(=O)-, annelated arylheterocyclenyl, annelated arylheterocyclyl.
**"Alkynyl"** means aliphatic straight or branched hydrocarbon chain comprising 2 - 12 carbon atoms and including at least one carbon-carbon triple bond. Branched means, that straight alkynyl chain contains one or more lower alkyl groups, such as methyl, ethyl or propyl. Alkyl group may have one or more substituents of the same or different structure ("alkyl substituent") including halogen, alkenyloxy, cycloalkyl, cyano, hydroxy, alkoxy, alkynyloxy, aralkoxy, aryloxy, aryloxycarbonyl, alkylthio, heteroaralkyloxy, heterocyclyl, heterocyclylalkyloxy, alkoxycarbonyl, aralkoxycarbonyl, heteroaralkyloxycarbonyl or Rₖ^{a}Rₖ₊₁^{a}N-, Rₖ^{a}Rₖ₊₁^{a}NC(=O)-, Rₖ^{a}Rₖ₊₁^{a}NSO₂-, wherein Rₖ^{a} and Rₖ₊₁^{a} independently of each other represent "amino group substituents", the meanings of which are defined in this section, for example, hydrogen, alkyl, aryl, aralkyl, heteroaralkyl, heterocyclyl or heteroaryl, or Rₖ^{a} and Rₖ₊₁^{a} together with the N-atom, they are attached to, form through Rₖ^{a} and Rₖ₊₁^{a} 4-7-membered heterocyclyl or heterocyclenyl. Preferred alkyl groups are methyl, trifluoromethyl, cyclopropylmethyl, cyclopentylmethyl, ethyl, *n*-propyl, *iso*-propyl, *n*-butyl, *tert*-butyl, *n*-pentyl, 3-pentyl, methoxyethyl, carboxymethyl, methoxycarbonylmethyl, ethoxycarbonylmethyl, benzyloxycarbonylmethyl and pyridylmethyloxycarbonylmethyl. The preferred **alkenyl** groups are ethenyl, propenyl, *n*-butenyl, iso-butenyl, 3-methylbut-2-enyl, *n*-pentenyl, buta-1,3-diyn and hexa-1,3,5-triyn.
**"Alkynykoxyalkyl"** means alkynyl-O-alkyl group, in which alkyl and alkynyl are defined in this section.
**"Alkoxy"** means alkyl-O-group, where alkyl is defined in this section. Preferred alkoxy groups are methoxy, ethoxy, *n*-propoxy, *iso*-propoxy and n-butoxy.
**"Alkyloxyalkyl"** means alkyl-O-alkyl group, in which alkyl groups independent of each other and defined in this section.
**"Alkyloxyalkylenoxyalkyl"** means alkyl-O-(CHR)ₙ-O-alkyl group, where R represents hydrogen or alkyl, n >2, preferably 2, 3 or 4.
**"Alkyloxyaryl"** means alkyl-O-aryl group, where alkyl and aryl are defined in this section.
**"Alkenyloxyaryl"** means alkenyl-O-aryl group, where alkenyl and aryl are defined in this section.
**"Alkynyloxyaryl"** means alkynyl-O-aryl group, where alkynyl and aryl are defined in this section.
**"Alkylthio"** means alkyl-S group, in which alkyl group is defined in this section.
**"Alkylthioalkyl"** means alkyl-S-alkyl group, where alkyl groups are independent of each other and defined in this section.
**"Alkylthioaryl"** means alkyl-S-aryl group, where alkyl and aryl are defined in this section.
**"Alkenylthioaryl"** means alkenyl-S-aryl group, where alkenyl and aryl are defined in this section.
**"Alkynylthioaryl"** means alkynyl-S-aryl group, where alkynyl and aryl are defined in this section.
**"Aryl"** means aromatic mono- or polycyclic system with 6 - 14 carbon atoms, predominantly 6-10 carbon atoms. Aryl may have one or more "cyclic system substituents" of the same or different structure. Phenyl or naphthyl, substituted phenyl or substituted naphthyl are the representatives of aryl groups. Aryl may be annelated with nonaromatic cyclic system or heterocycle.
**"Aryloxy"** means Aryl-O- group, where the meaning of aryl is defined in this section. Phenoxy- and 2-naphthyloxy are the representatives of aryloxy-groups.
**"Arylthio"** means aryl-S- group, where the meaning of aryl is defined in this section. Phenylthio- and 2-naphthylthio- are representatives of arylthio- groups.
**"Biradical"** means a radical derived at removal of two hydrogen atoms from two C-H bonds of the molecule.
**"Substituent"** means a chemical radical, which is attached to a scaffold (fragment), for example, "alkyl substituent", "amino group substituent", "carbamoyl substituent", "cyclic system substituent".
**"Active component"** (drug-substance) means a physiologically active compound of synthetic or other (biotechnological, vegetable, animal, microbe and so on) origins exhibiting pharmacological activity which is an active ingredient of pharmaceutical composition which is employed in production and preparation of medicaments.
**"Medicament"** is a compound (or mixture of compounds in the form of pharmaceutical composition) in the form of tablets, capsules, injections, ointments and other ready forms intended for restoration, improvement or modification of physiological functions at humans and animals, and for treatment and prophylaxis of diseases or diagnostics, anesthesia, contraception, cosmetology and others.
**"Lower alkyl"** means straight or branched alkyl with 1-4 carbon atoms.
**"Therapeutic kit"** is a simultaneously administered combination of two or more drug substances with different mechanism of pharmacological action and aimed at different biotargets taking part in pathogenesis of the disease.
**"Pharmaceutical composition"** means a composition comprising a compound of general formula 1 and at least one of components selected from group consisting of pharmaceutically acceptable and pharmacologicaly compatible excipients, solvents, diluents, carriers, auxiliary, distributing and sensing agents, delivery agents, such as preservatives, stabilizers, excipients, disintegrators, moisteners, emulsifiers, suspending agents, thickeners, sweeteners, flavouring agents, aromatizing agents, antibacterial agents, fungicides, lubricants, and prolonged delivery controllers, choice and suitable proportions of which depend on nature and way of administration and dosage. Examples of suitable suspending agents are ethoxylated isostearyl alcohol, polyoxyethene, sorbitol and sorbitol ether, microcrystalline cellulose, aluminum metahydroxide, bentonite, agar-agar and tragacant and their mixtures as well. Protection against the action of microorganisms can be provided by various antibacterial and antifungal agents, such as, for example, parabens, chlorobutanole, sorbic acid, and similar compounds. Composition may also contain isotonic agents, such as, for example, sugar, sodium chloride, and similar compounds. Prolonged effect of composition may be achieved by agents slowing down absorption of active ingredient, for example, aluminum monostearate and gelatine. Examples of suitable carriers, solvents, diluents and delivery agents include water, ethanol, polyalcohols and their mixtures, natural oils (such as olive oil) and organic esters (such as ethyl oleate) for injections. Examples of excipients are lactose, milk-sugar, sodium citrate, calcium carbonate, calcium phosphate and the like. Examples of disintegrators and distributors are starch, alginic acid and its salts, and silicates. Examples of suitable lubricants are magnesium stearate, sodium lauryl sulfate, talc and high molecular weight polyethylene glycol. Pharmaceutical composition for peroral, sublingval, transdermal, intramuscular, intravenous, subcutaneous, local or rectal administration of active ingredient, alone or in combination with another active compound may be administered to humans and animals in standard administration form, or in mixture with traditional pharmaceutical carriers. Suitable standard administration forms include peroral forms such as tablets, gelatin capsules, pills, powders, granules, chewing-gums and peroral solutions or suspensions, sublingval and transbuccal administration forms; aerosols; implants; local, transdermal, subcutaneous, intramuscular, intravenous, intranasal or intraocular forms and rectal administration forms.
**"Pharmaceutically acceptable salt"** means relatively nontoxic both organic and inorganic salts of acids and bases disclosed in this invention. Salts could be prepared **in situ** in processes of synthesis, isolation or purification of compounds or they could be prepared specially. In particular, bases salts could be prepared starting from purified base of disclosed compound and suitable organic or mineral acid. Examples of salts prepared in this manner include hydrochlorides, hydrobromides, sulfates, bisulfates, phosphates, nitrates, acetates, oxalates, valeriates, oleates, palmitates, stearates, laurates, borates, benzoates, lactates, p-toluenesulfonates, citrates, maleates, fumarates, succinates, tartrates, methane sulphonates, malonates, salicylates, propionates, ethane sulphonates, benzene sulfonates, sulfamates and the like (Detailed description of properties of such salts is given in: Berge S.M., et al., "Pharmaceutical Salts" J.Pharm.Sci., 1977, 66: 1-19). Salts of disclosed acids may be also prepared by reaction of purified acids specifically with suitable base; moreover, metal salts and amine salts may be synthesized too. Metal salts are salts of sodium, potassium, calcium, barium, zinc, magnesium, lithium and aluminum, sodium and potassium salts being preferred. Suitable inorganic bases from which metal salts can be prepared are sodium hydroxide, carbonate, bicarbonate and hydride; potassium hydroxide, carbonate and bicarbonate, lithium hydroxide, calcium hydroxide, magnesium hydroxide, zinc hydroxide. Organic bases suitable for preparation of the disclosed acid salts are amines and amino acids of the sufficient basicity to produce a stable salt and suitable for use for medical purposes (in particular, they are to have low toxicity). Such amines include ammonia, methylamine, dimethylamine, trimethylamine, ethylamine, diethylamine, triethylamine, benzylamine, dibenzylamine, dicyclohexylamine, piperazine, ethylpiperidine, tris(hydroxymethyl)aminomethane and the like. Besides, salts can be prepared using some tetraalkylammonium hydroxides, such as, for example, holine, tetramethylammonium, tetraethylammonium, and the like. Aminoacids may be selected from the main aminoacids - lysine, ornithine and agrinine.

The subject of the present invention is novel substituted azoles of the general formulas **1A** and **1B** and pharmaceutically acceptable salts thereof. 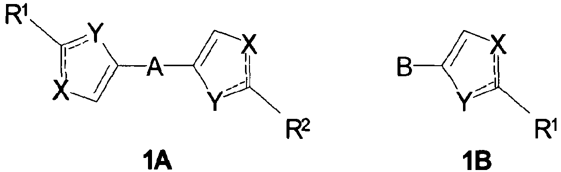 wherein:
solid lines with accompanying dotted lines (---) represent ordinary bond or double bond, provided that one of them is an ordinary bond, the other one is double bond;
**X** and **Y** optionally accept different meanings and represent nitrogen, oxygen, sulfur or NH group;
**R¹** and **R²** - represent optionally identical radicals selected from **2.1-2.20,** where an asterisk (*) denotes the places of azole fragment attachment; 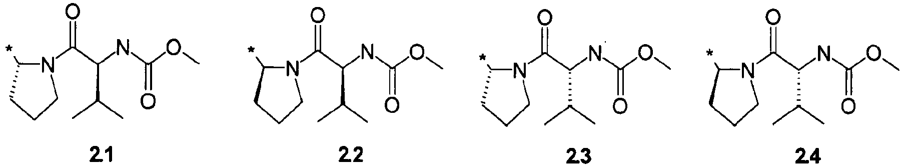 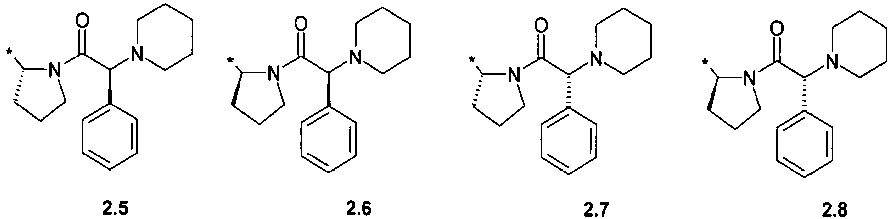 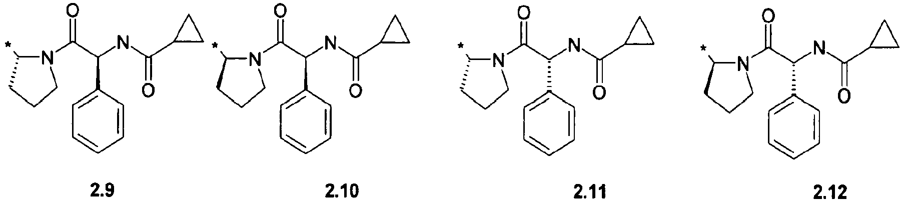 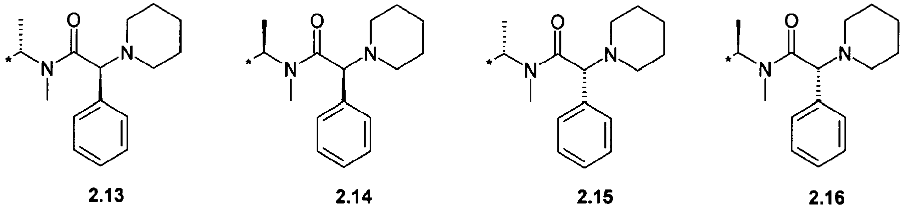 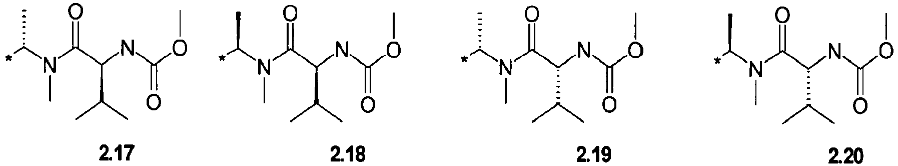
**A** represents:
- aliphatic C₂-C₈ biradical selected from biradicals **3.1-3.36,** where an asterisk (*) denotes the places of azole fragment attachment;

| | | |
|---|---|---|
| *-CH₂-CH₂-* | *-CH₂-CH₂-CH₂-* | *-CH₂-CH₂-CH₂-CH₂-* |
| **3.1** | **3.2** | **3.3** |
| *-CH₂-CH₂-CH₂-CH₂-CH₂-* | *-CH₂-CH₂-CH₂-CH₂-CH₂-CH₂-* | *-CH=CH-* |
| **3.4** | **3.5** | **3.6** |
| *-CH₂-CH=CH-* | *-CH=CH-CH₂-CH₂-* | *-CH₂-CH=CH-CH₂-* |
| **3.7** | **3.8** | **3.9** |
| *-CH=CH-CH₂-CH₂-CH₂-* | *-CH₂-CH=CH-CH₂-CH₂-* | *-CH=CH-CH₂-CH=CH-* |
| **3.10** | **3.11** | **3.12** |
| *-CH₂-CH=C=CH-CH₂-* | *-CH=CH-CH₂-CH₂-CH₂-CH₂-* | *-CH₂-CH=CH-CH₂-CH₂-CH₂-* |
| **3.13** | **3.14** | **3.15** |
| *-CH=CH-CH=CH-CH₂-CH₂-* | *-CH₂-CH=CH-CH=CH-CH₂-* | *-CH₂-CH=C=CH-CH₂-CH₂-* |
| **3.16** | **3.17** | **3.18** |
| *-C≡C-* | *-C≡C-CH₂-* | *-C≡C-CH₂-CH₂-* |
| **3.19** | **3.20** | **3.21** |
| *-CH₂-C≡C-CH₂-* | *-C≡C-C≡C-* | *-C≡C-CH₂-CH₂-CH₂-* |
| **3.22** | **3.23** | **3.24** |
| *-CH₂-C≡C-CH₂-CH₂-* | *-C≡C-C≡C-CH₂-* | *-C≡C-CH₂-C≡C-* |
| **3.25** | **3.26** | **3.27** |
| *-C≡C-CH₂-CH₂-CH₂-CH₂-* | *-CH₂-C≡C-CH₂-CH₂-CH₂-* | *-CH₂-CH₂-C≡C-CH₂-CH₂-* |
| **3.28** | **3.29** | **3.30** |
| *-C≡C-C≡C-CH₂-CH₂-* | *-C≡C-CH₂-CH₂-C≡C-* | *-C≡C-CH₂-CH=CH-CH₂-* |
| **3.31** | **3.33** | **3.34** |
| *-C≡C-CH₂-CH₂-CH=CH-* | *-C≡C-C≡C-C≡C-* | |
| **3.35** | **3.36** | |

- dioxane, cyclo- and bicycloaliphatic biradical, selected from biradicals 3.37-3.47, where an asterisk (*) denotes the places of azole fragment attachment; 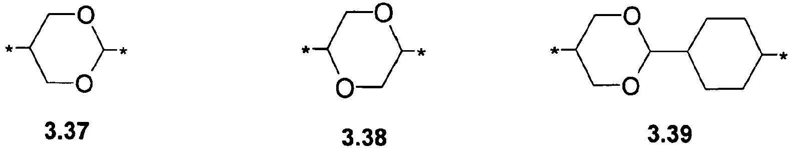 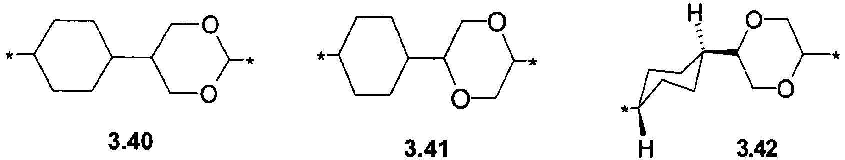 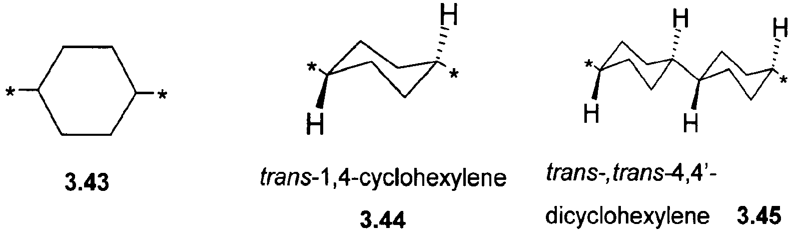 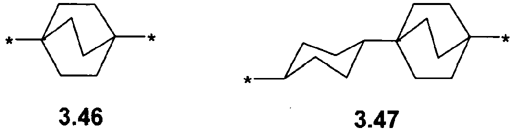
- alkyloxyalkyl, alkenyloxyalkyl, alkynyloxyalkyl biradicals and their thioanalogs, preferably of formula **3.48-3.56,** where an asterisk (*) denotes the places of azole fragment attachment;

| | | |
|---|---|---|
| *-CH₂-CH₂-O-CH₂-CH₂-* | *-CH₂-CH₂-S-CH₂-CH₂-* | *-CH=CH-O-CH₂-CH₂-* |
| **3.48** | **3.49** | **3.50** |
| *-CH₂-CH₂-O-CH₂-CH₂-O-CH₂-CH₂-* | *-CH=CH-CH₂-O-CH₂-CH=CH₂-* | *-C≡C-CH₂-O-CH₂-CH₂- * |
| **3.51** | **3.52** | **3.53** |
| *-C≡C-CH₂-O-CH₂-C≡C-* | *-CH=CH-CH₂-O-CH₂-C≡C-* | *-CH₂-CH₂-O-CH₂-C≡C- * |
| **3.54** | **3.55** | **3.56** |

- aryl and thiophene biradicals selected from biradicals 3.57-3.71, where an asterisk (*) denotes the places of azole fragment attachment; 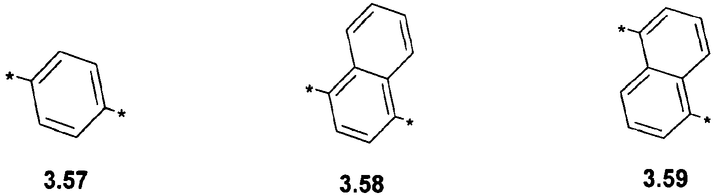 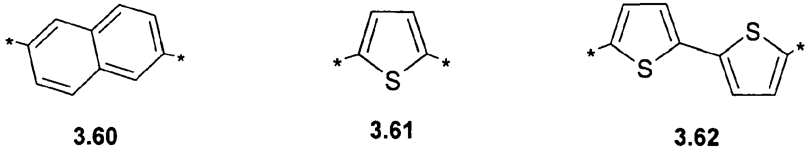 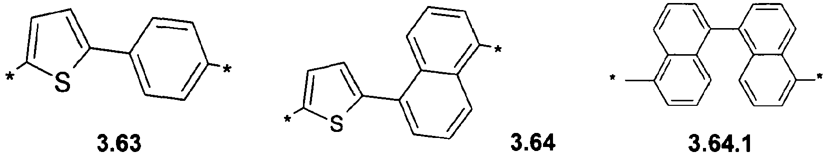 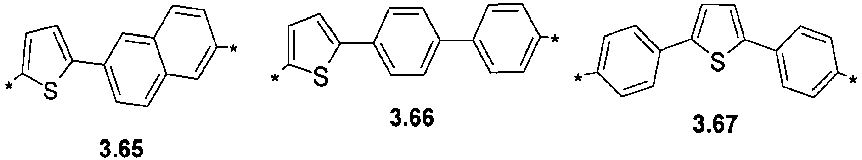 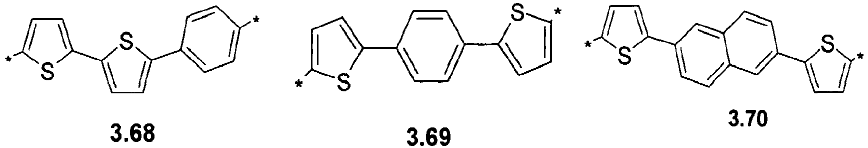 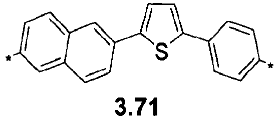
- alkynylcycloalkyl, alkynyldioxane, alkynylaryl, alkylthiophene, alkenylthiophene and alkynylthiophene biradical selected from biradicals **3.72-3.129,** where an asterisk (*) denotes the places of azole fragment attachment; 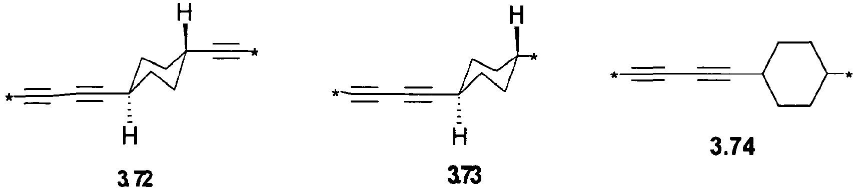 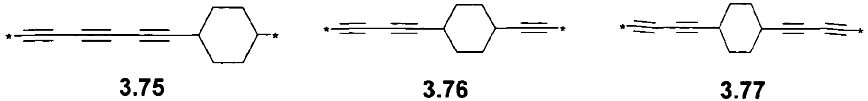 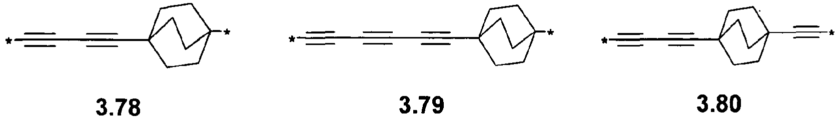 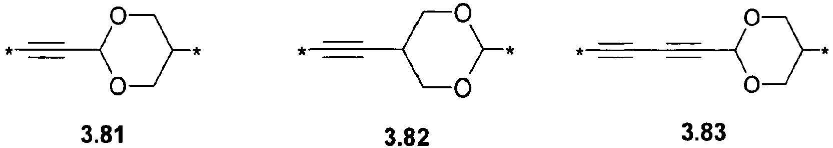 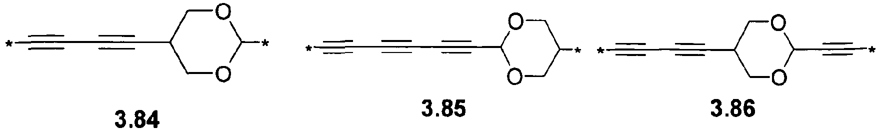 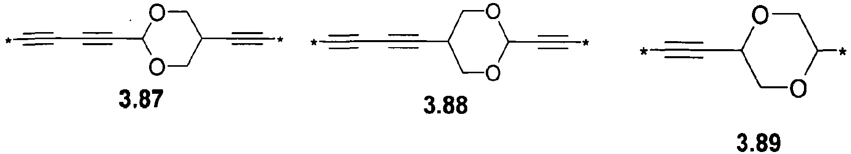 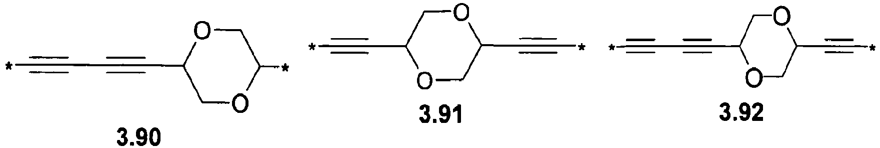 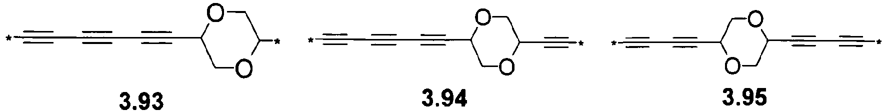 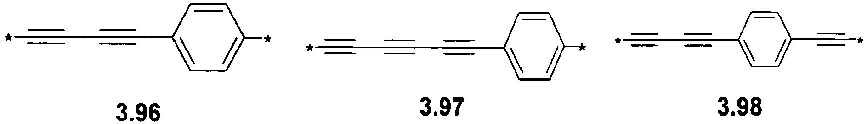 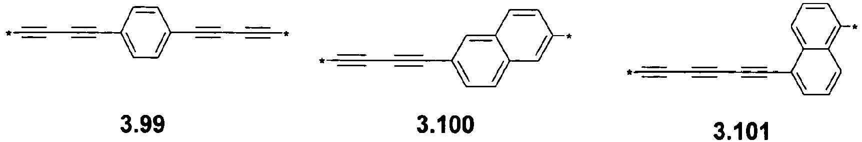 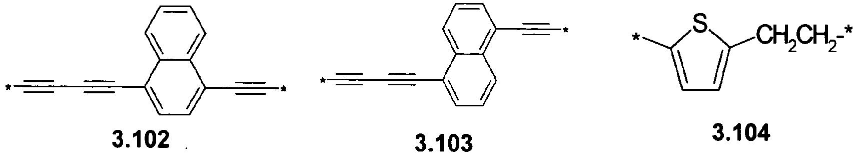 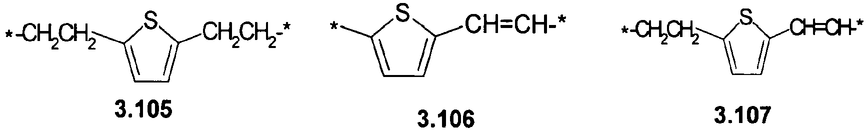 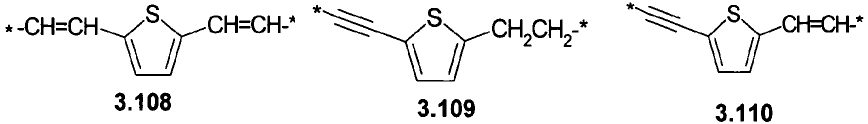 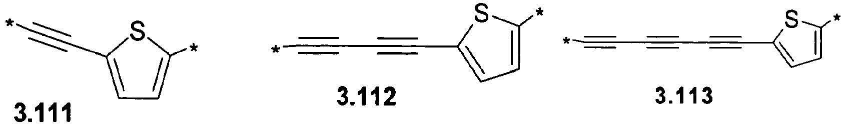 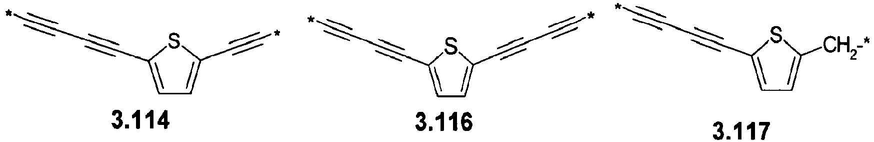 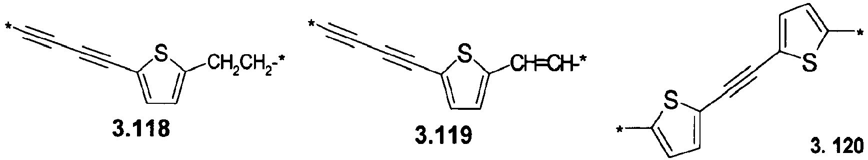 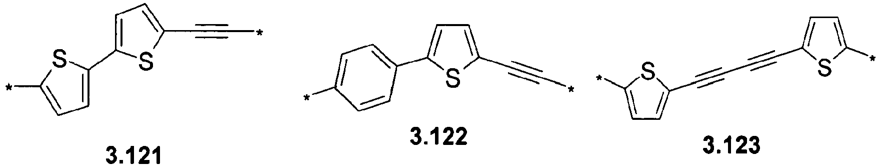 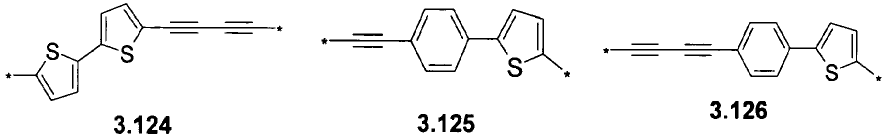 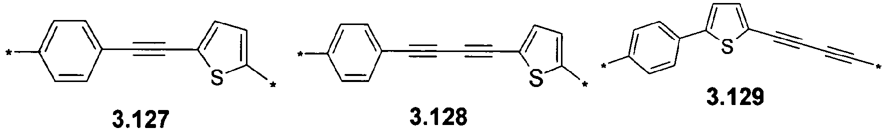
- alkyloxyaryl, alkenyloxyaryl, alkynyloxyaryl biradical selected from biradicals **3.130-3.136,** where an asterisk (*) denotes the places of azole fragment attachment; 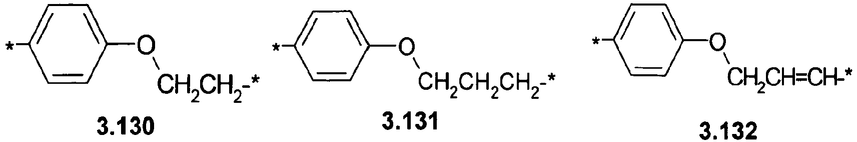 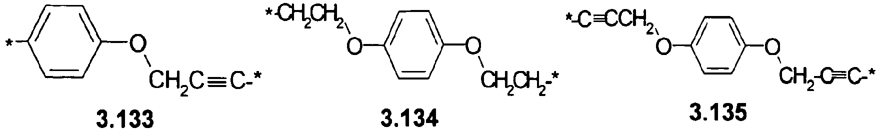 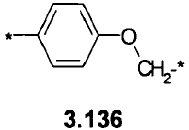
- cycloalkylthiophene, aryldioxane and thiophenedioxane biradical selected from biradicals **3.137-3.154,** where an asterisk (*) denotes the places of azole fragment attachment; 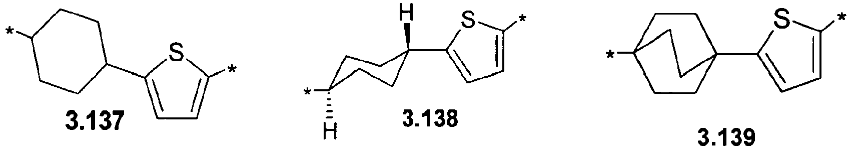 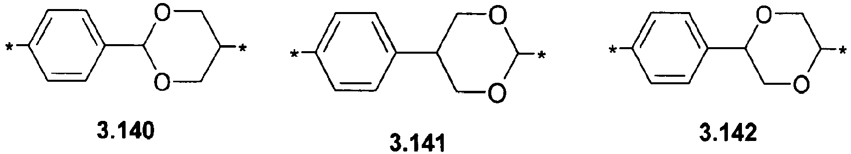 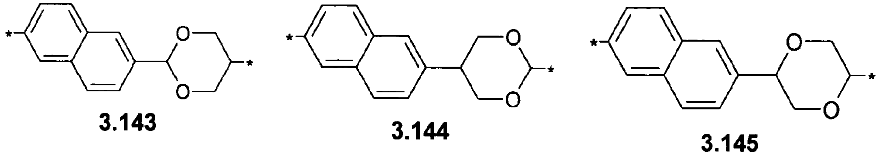 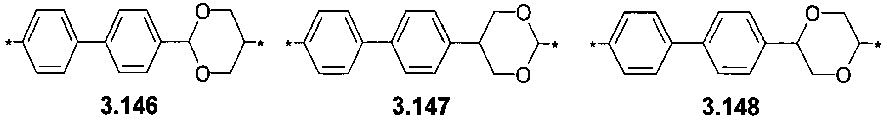 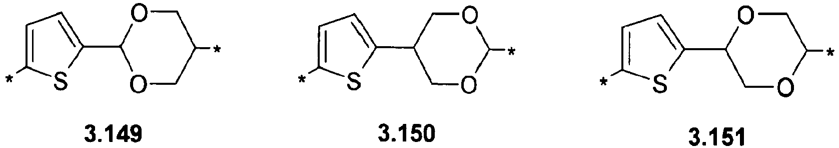 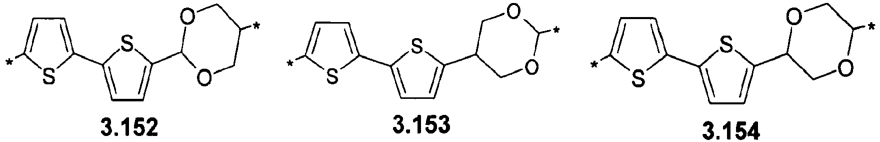 And also 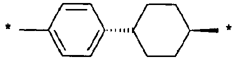 provided that Y= NH in one of the azole rings, and Y = O in the other azole rind, R¹ =R² = **2.3.**
**B** represents:
- aliphatic C₂-C₈ radical selected from radicals **4.1-4.12,** where an asterisk (*) denotes the place of azole fragment attachment;

| | | |
|---|---|---|
| *-C≡CH | HC≡C-CH₂-* | HC≡C-CH₂-CH₂-* |
| **4.1** | **4.2** | **4.3** |
| CH₃-C≡C-CH₂-* | HC≡C-C≡C-* | HC≡C-C≡C-CH₂-* |
| **4.4** | **4.5** | **4.6** |
| *-C≡C-C≡C-CH₃ | HC≡C-CH₂-C≡C-* | HC≡C-C≡C-CH₂-CH₂-* |
| **4.7** | **4.8** | **4.9** |
| *-C≡C-C≡C-CH₂-CH₃ | HC≡C-CH₂-CH₂-C≡C-* | HC≡C-C≡C-C≡C-* |
| **4.10** | **4.11** | **4.12** |

- aryl or thiophenyl radical selected from radicals **4.13-4.30,** where an asterisk (*) denotes the place of azole fragment attachment; 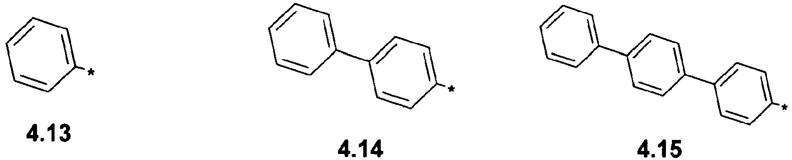 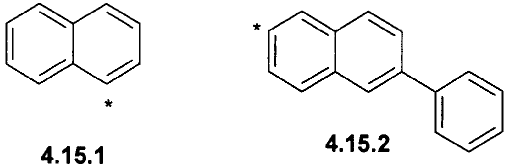 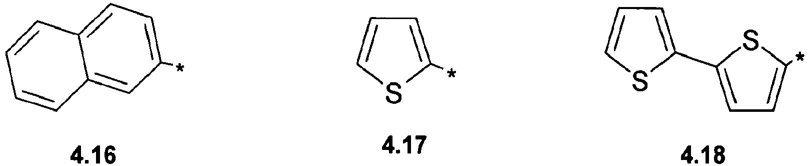 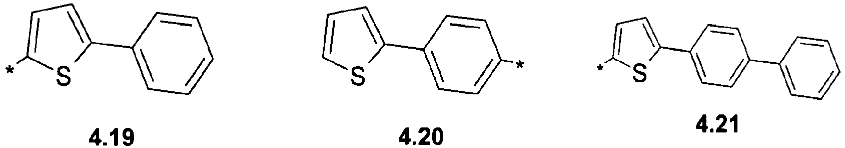 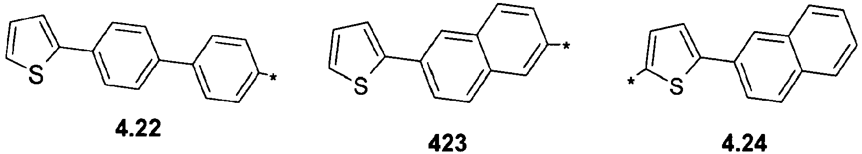 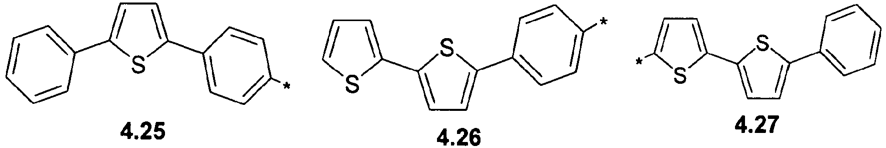 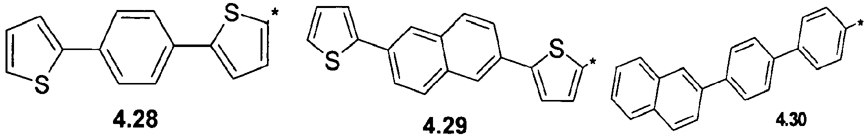
- alkynylcycloalkyl, alkynylaryl, alkylthiophene and alkynylthiophene radical, **4.31-4.47,** where an asterisk (*) denotes the place of azole fragment attachment; 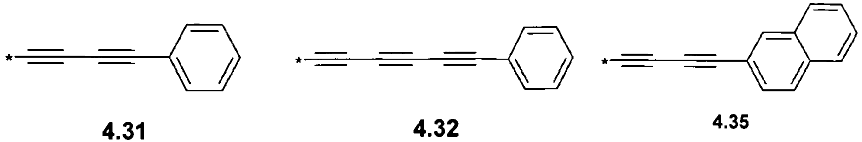 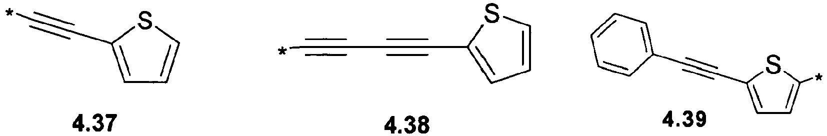 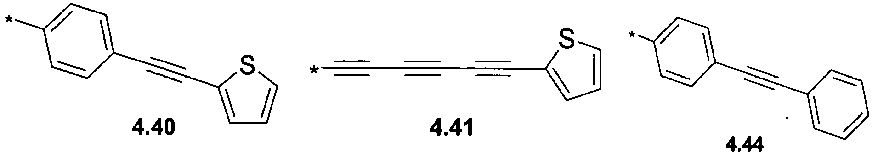 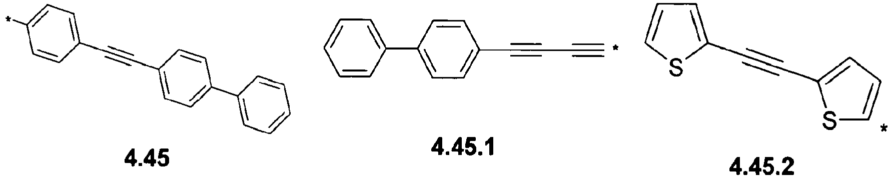 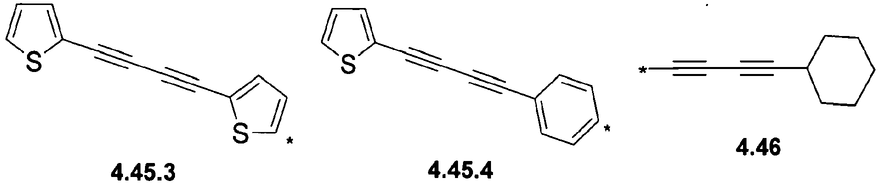 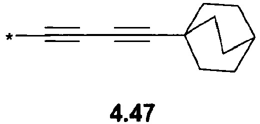
- cycloalkylbenzene, 4-cycloalkylbiphenyl, (bicyclooctane)benzene, 4-(bicyclooctane)biphenyl, aryldioxane and thiophenedioxane radical selected from radicals **4.48-4.72,** where an asterisk (*) denotes the place of azole fragment attachment. 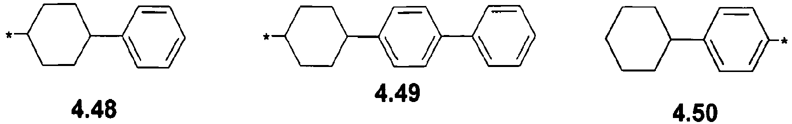 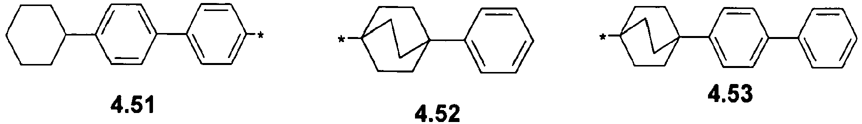 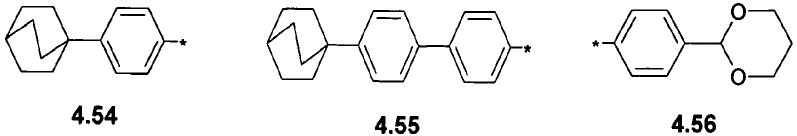 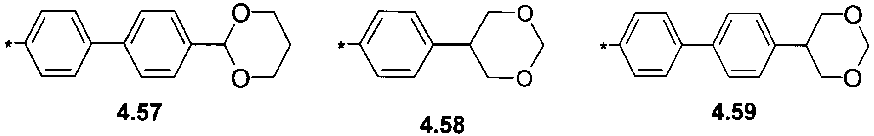 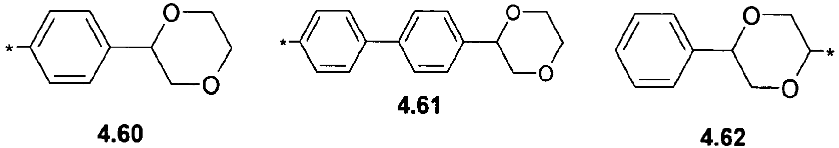 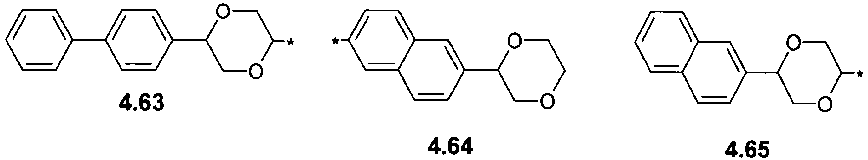 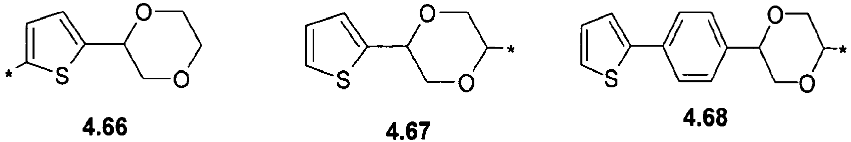 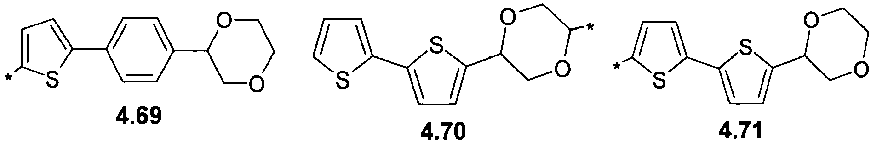 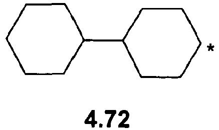 **with the exception of:**
((R)-1-{(R)-2-[5-(4-{2-[(R)-1-((R)-2-methoxycarbonylamino-3-methyl-butyryl)-pyrrolidin-2-yl]-3H-imidazol-4-yl}-buta-1,3-diynyl)-1H-imidazol-2-yl]-pyrrolidine-1-carbonyl}-2-methyl-propyl]-carbamic acid methyl ester;
((S)-1-{(S)-2-[5-(4-{2-[(S)-1-((S)-2-methoxycarbonylamino-3-methyl-butyryl)-pyrrolidin-2-yl]-3H-imidazol-4-yl}-phenyl)-1H-imidazol-2-yl]-pyrrolidine-1-carbonyl}-2-methyl-propyl)-carbamic acid methyl ester dihydrochloride;
((S)-1-{(S)-2-[5-(6-{2-[(S)-1-((S)-2-methoxycarbonylamino-3-methyl-butyryl)-pyrrolidin-2-yl]-3H-imidazol-4-yl}-naphthalen-2-yl)-1H-imidazol-2-yl]-pyrrolidine-1-carbonyl}-2-methyl-propyl)-carbamic acid methyl ester dihydrochloride;
[(S)-1-((S)-2-{5-[5-(4-{2-[(S)-1-((S)-2-methoxycarbonylamino-3-methyl-butyryl)-pyrrolidin-2-yl]-3H-imidazol-4-yl}-phenyl)-thiophen-2-yl]-1H-imidazol-2-yl}-pyrrolidine-1-carbonyl)-2-methyl-propyl]-carbamic acid methyl ester and dimethyl (2S,2'S)-1,1'-((2R,2'R)-2,2'-(5,5'-(4,4'-(thiophene-2,5-diyl)bis(4,1-phenylene))bis(1H-imidazole-5,2-diyl))bis(pyrrolidine-2,1-diyl))bis(3-methyl-1-oxobutane-2,1-diyl)dicarbamate.

The more preferable substituted azoles are compounds of the general formulas **5.1** - **5.70,** where X, Y, R¹, R² and solid lines with accompanying dotted lines (---) have the above meanings.

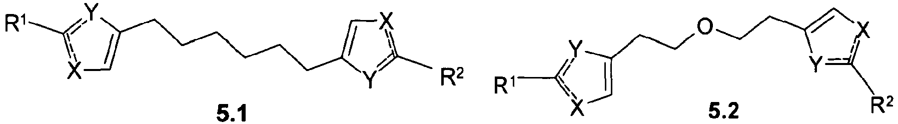

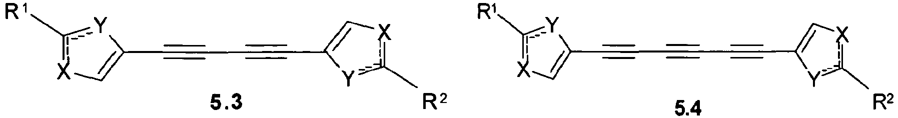

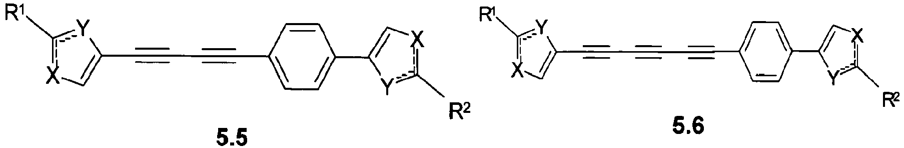

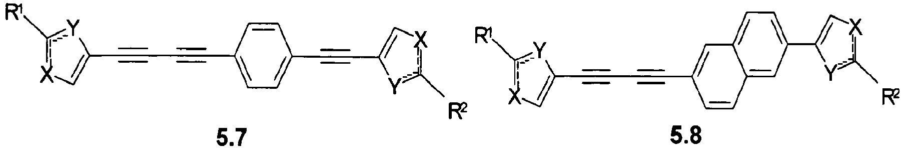

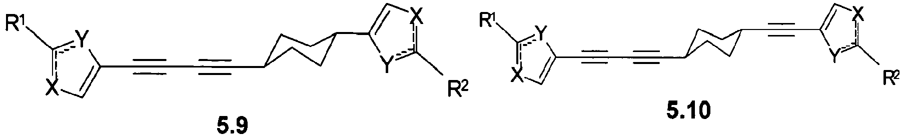

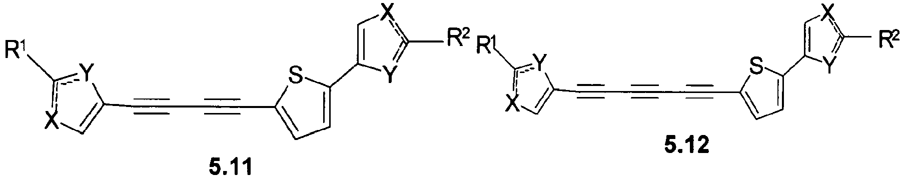

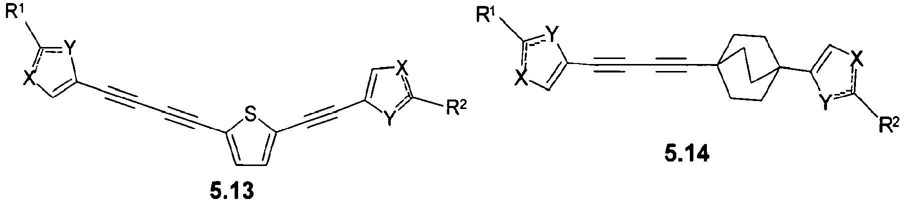

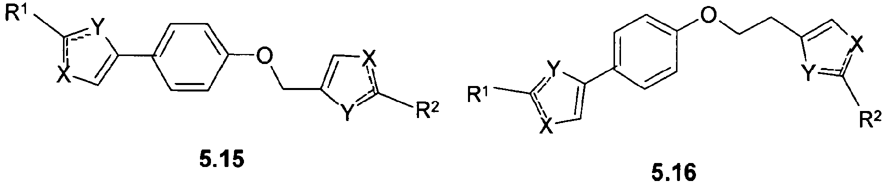

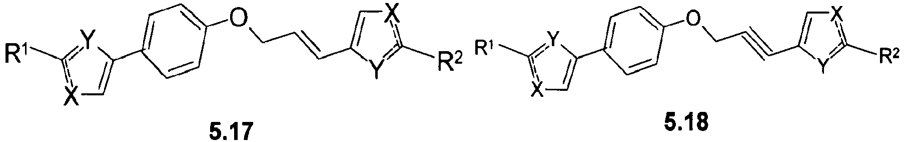

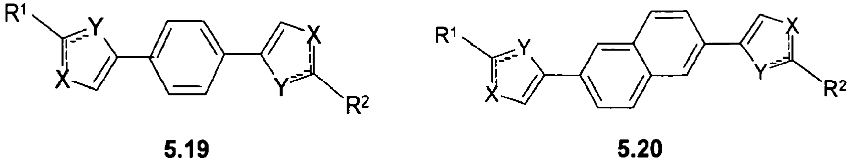

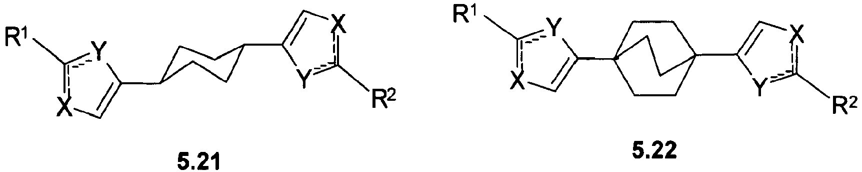

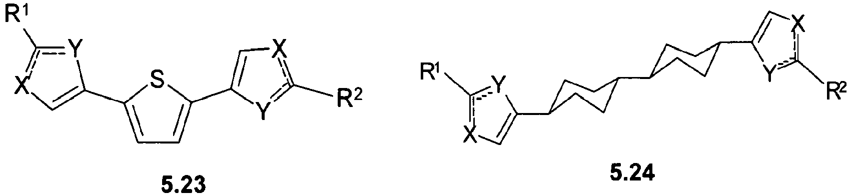

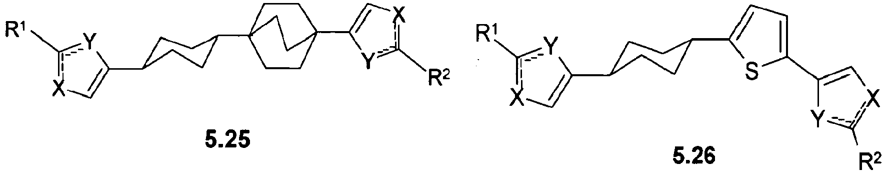

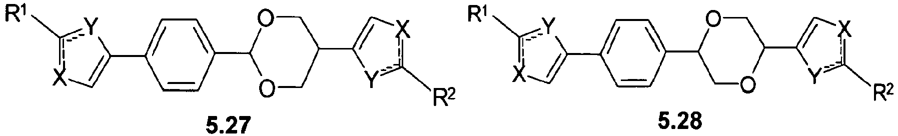

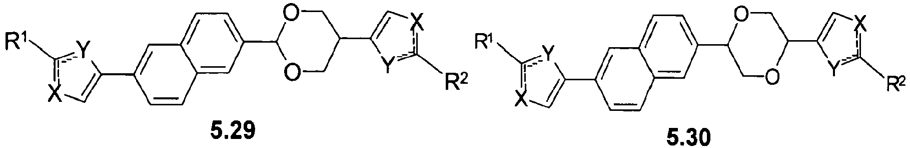

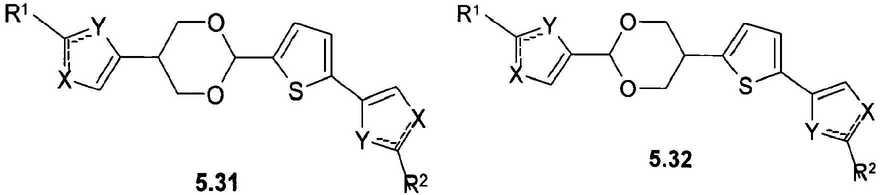

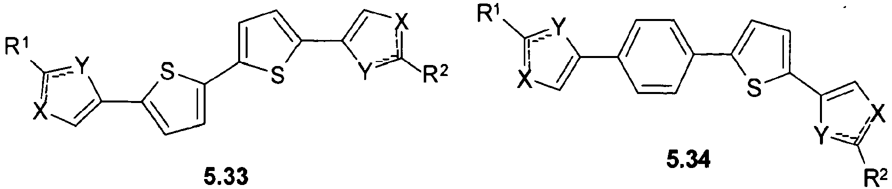

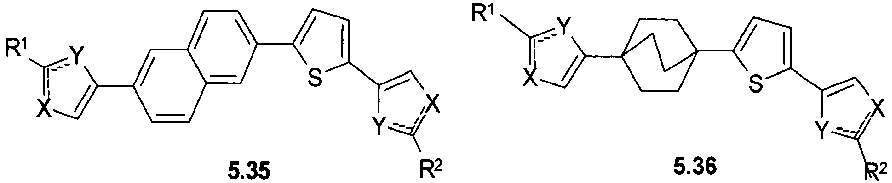

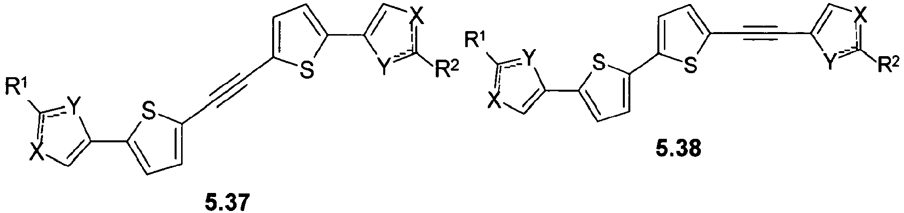

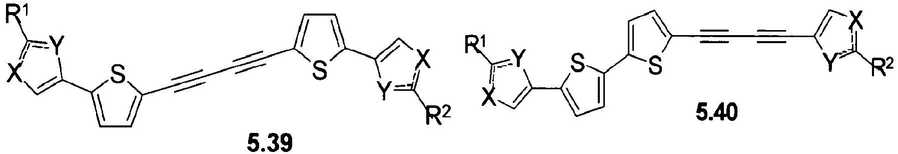

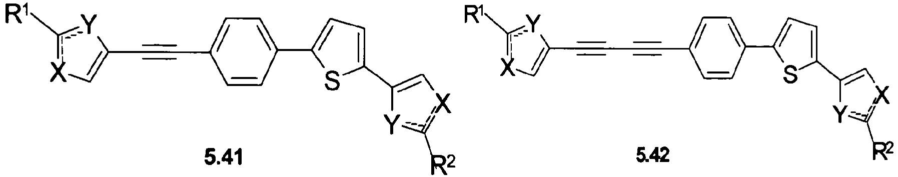

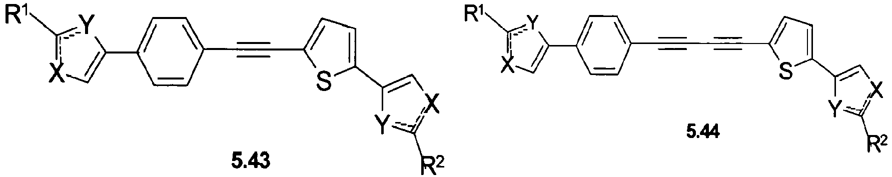

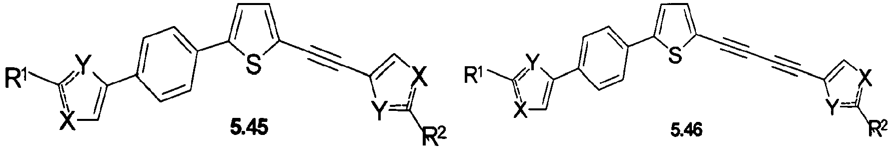

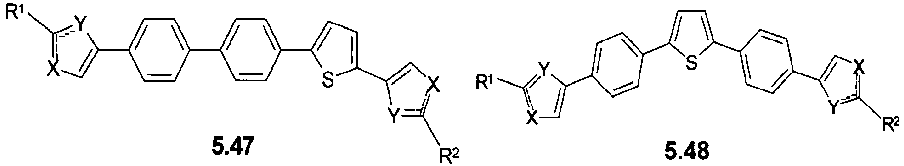

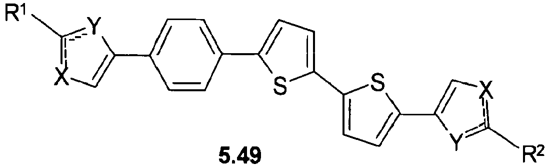

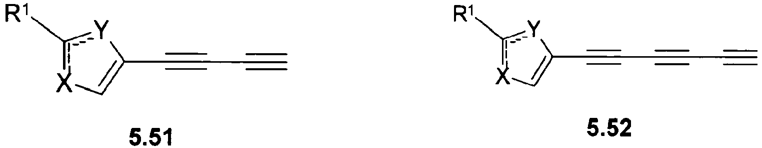

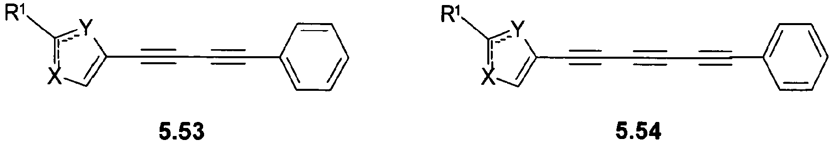

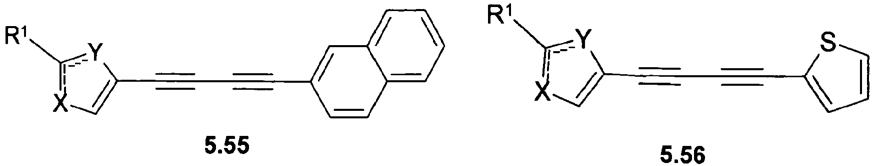

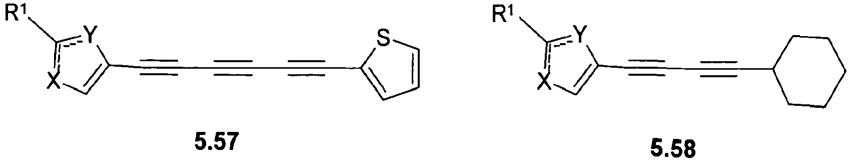

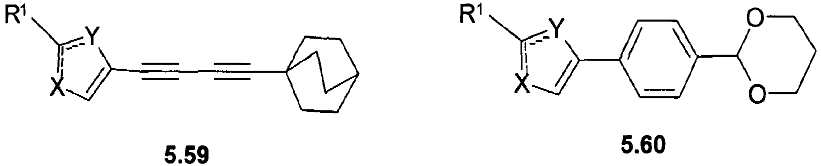

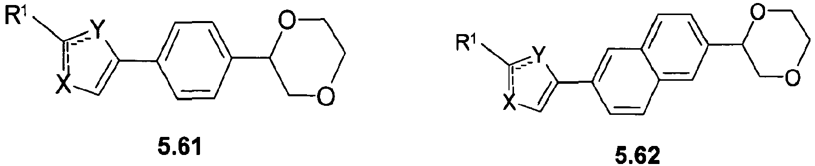

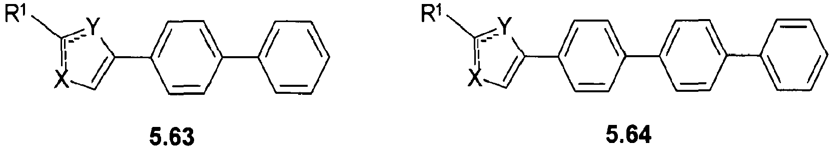

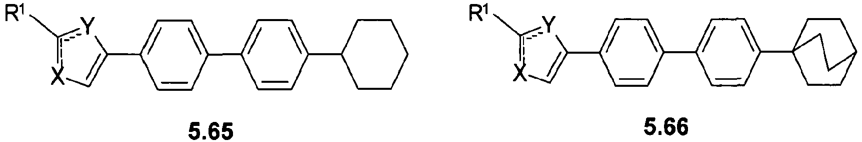

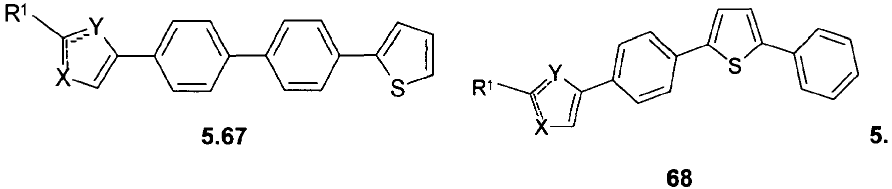

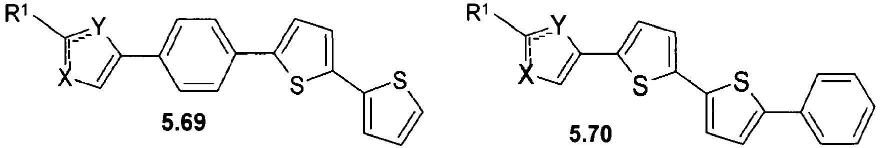

The more preferable substituted azoles are compounds of the general formulas **6.1** - **6.70,** where **A, X, Y,** and solid lines with accompanying dotted lines (---) have the above meanings.

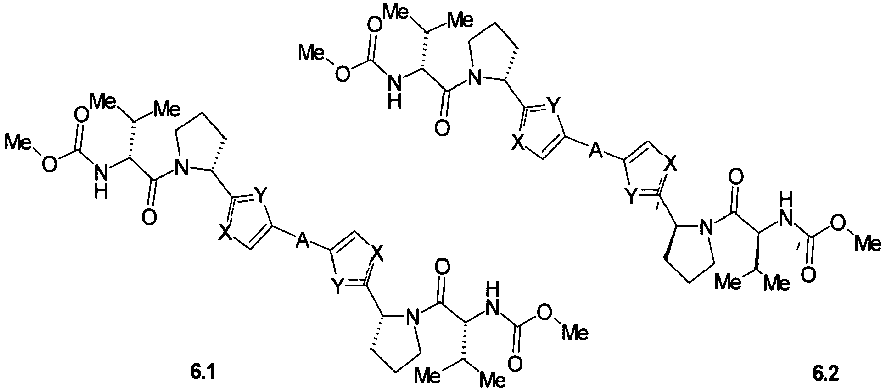

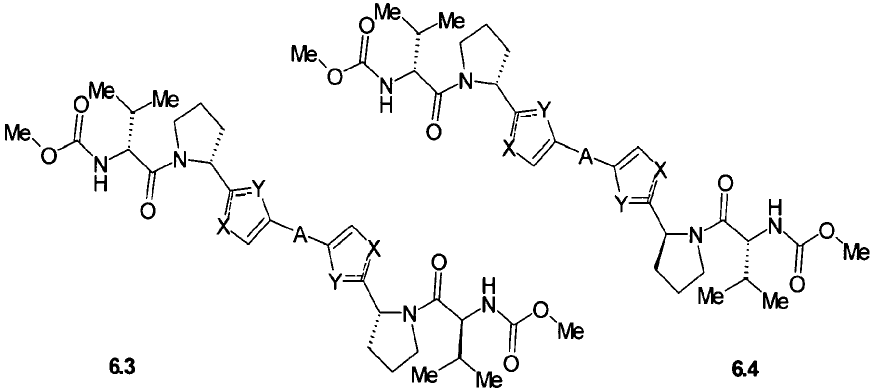

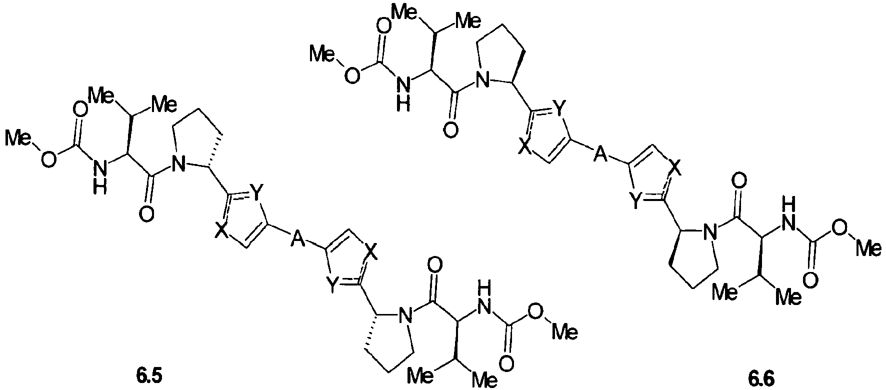

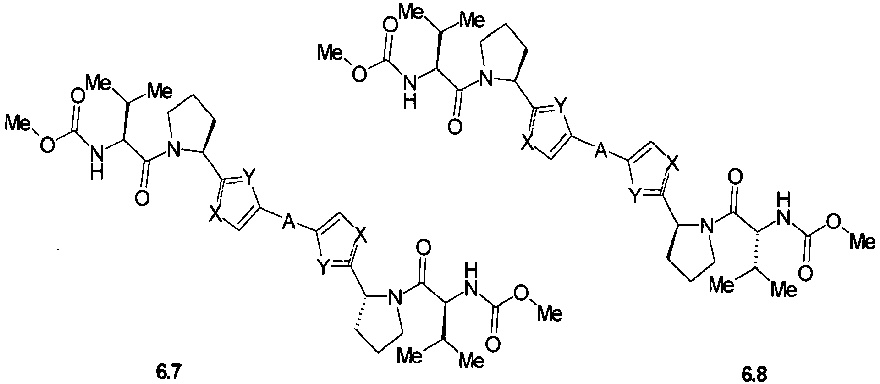

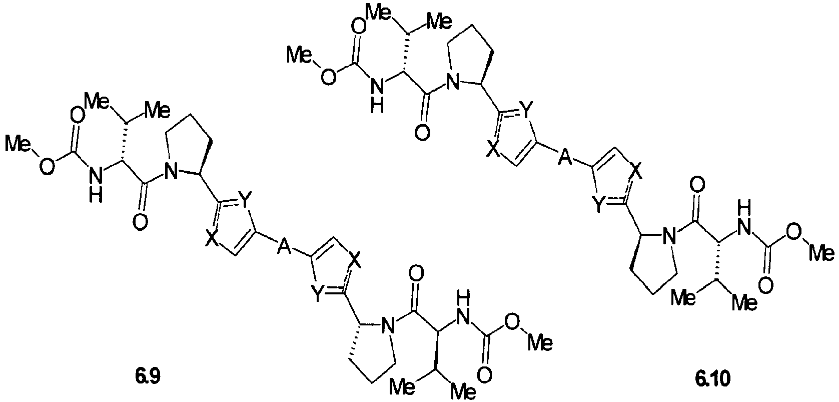

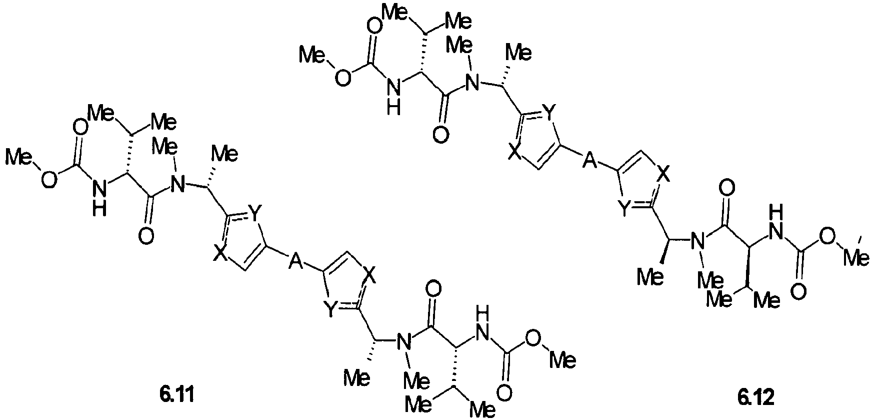

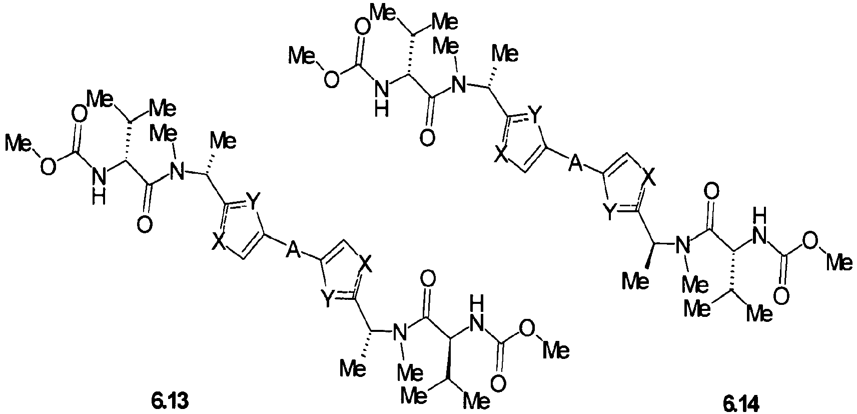

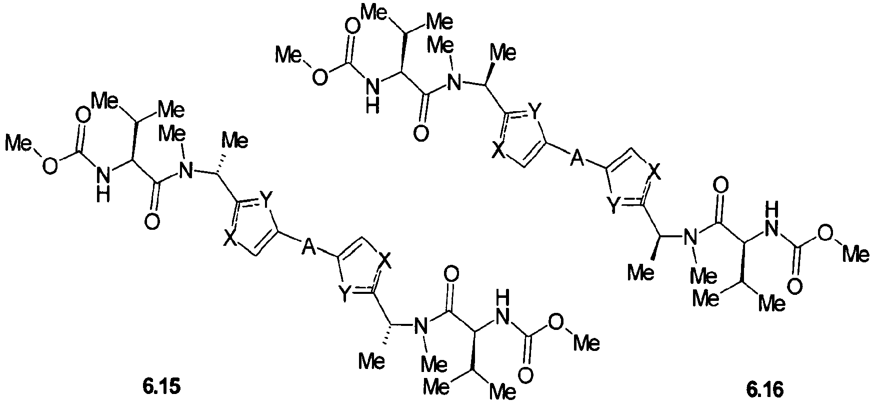

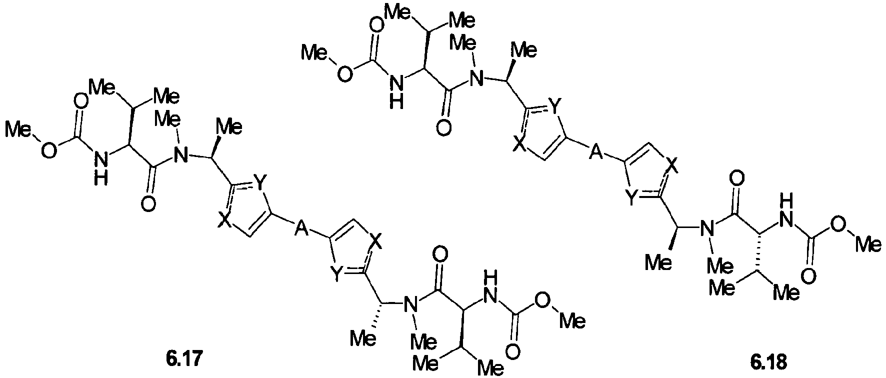

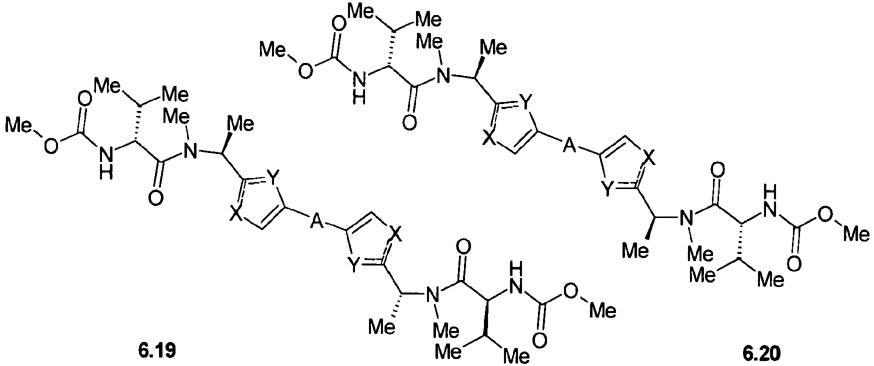

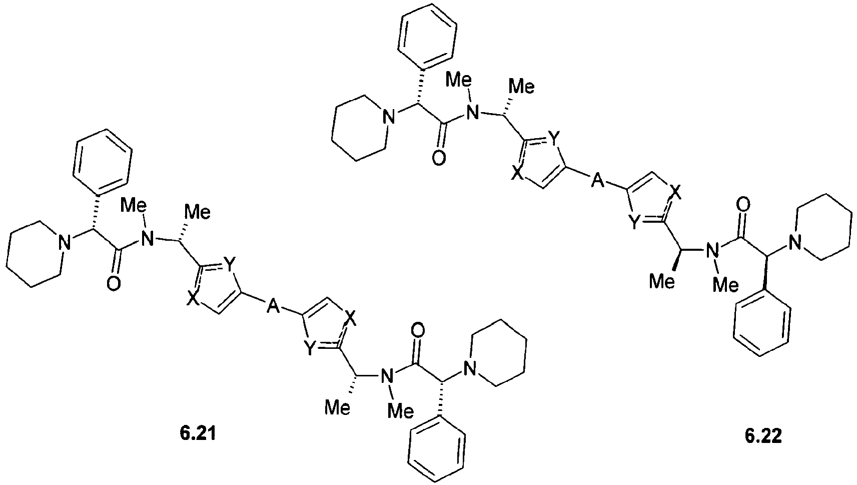

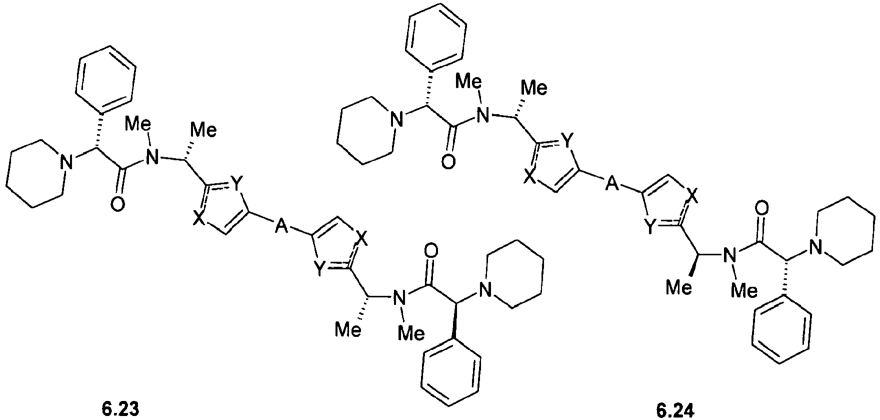

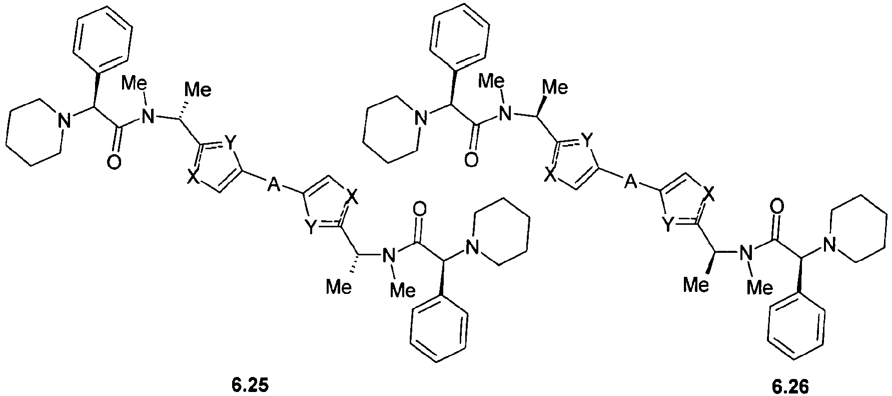

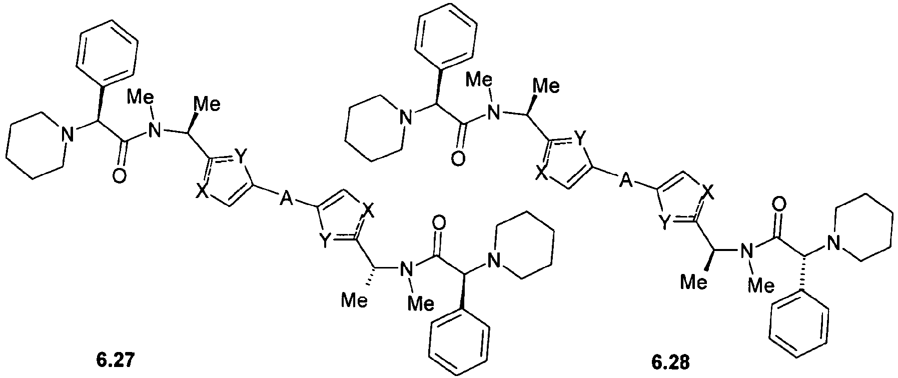

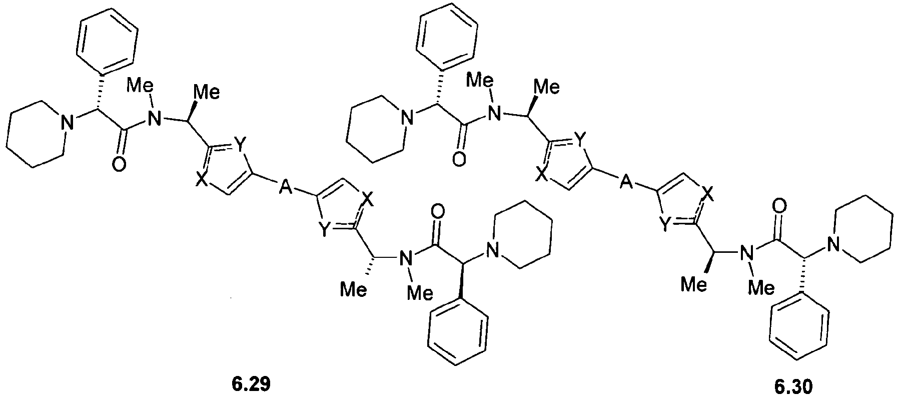

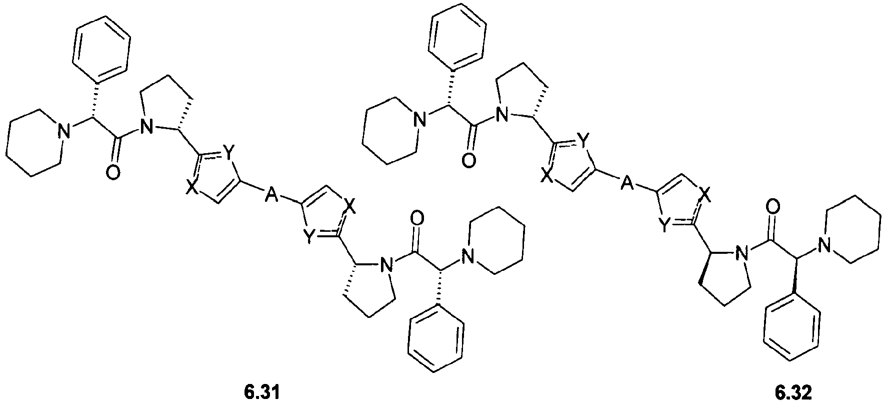

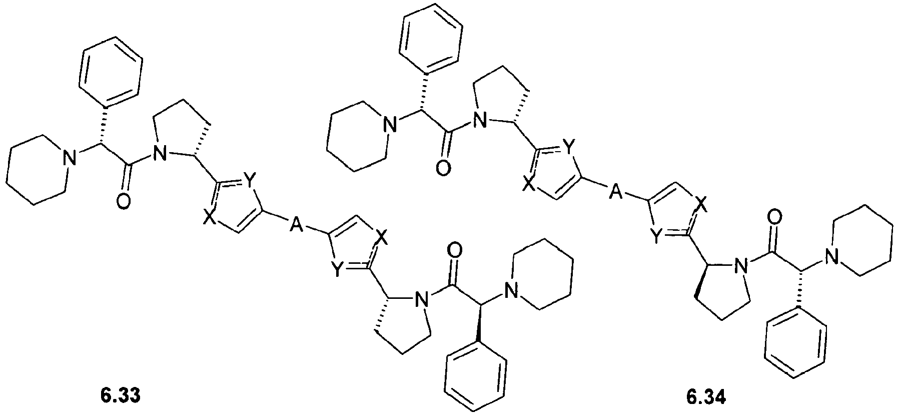

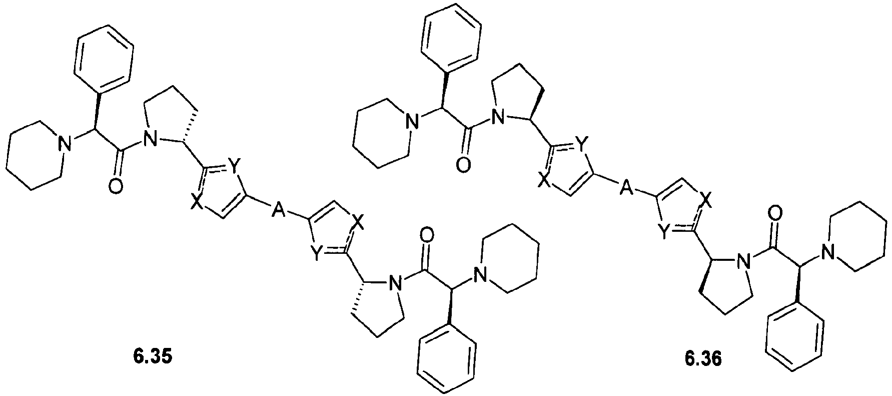

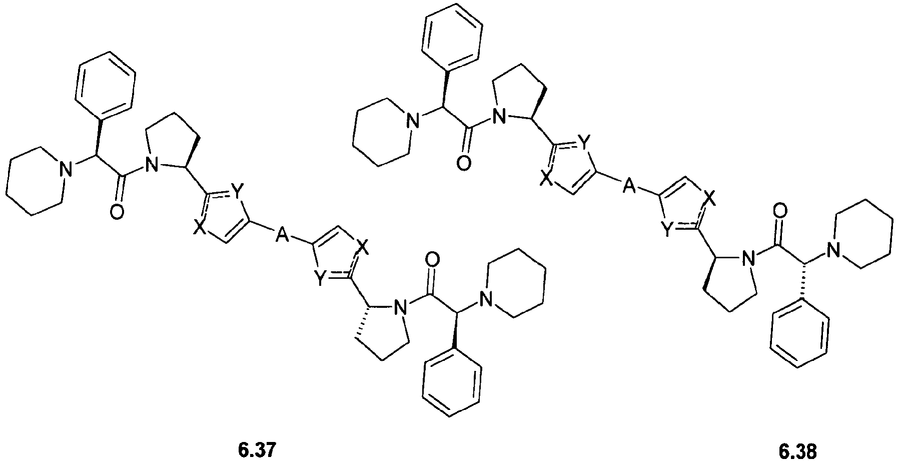

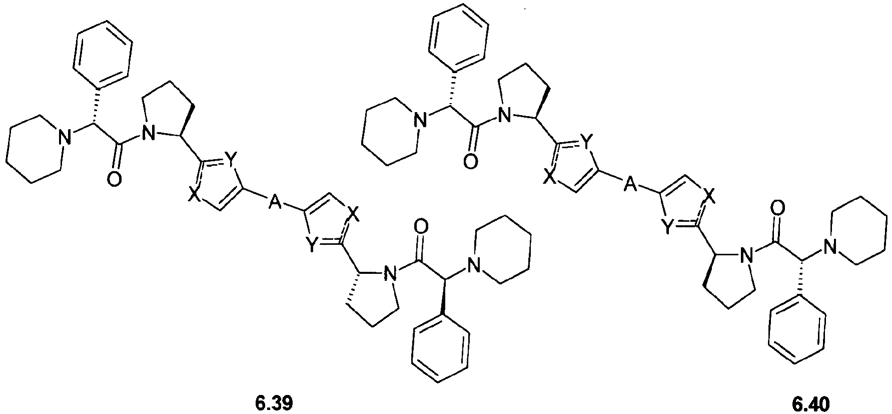

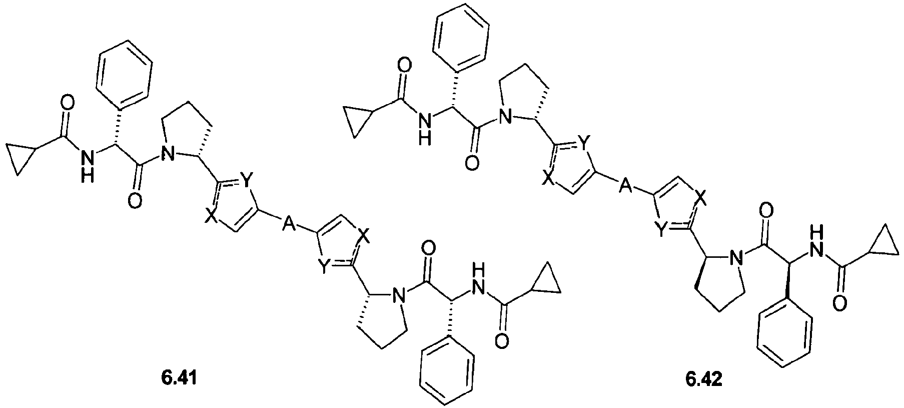

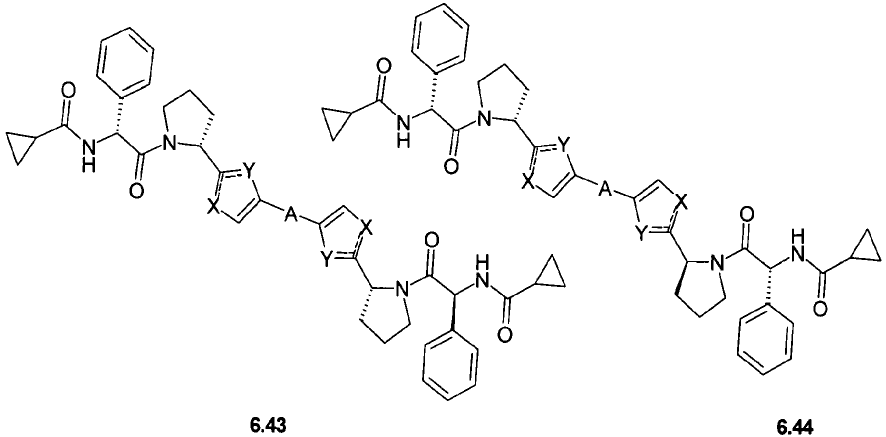

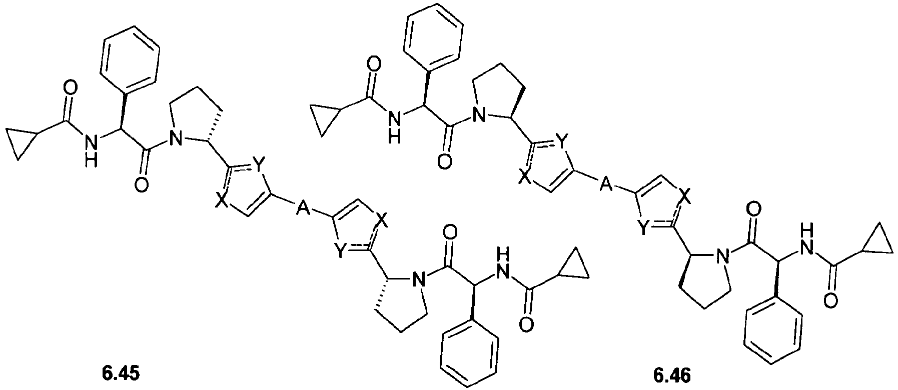

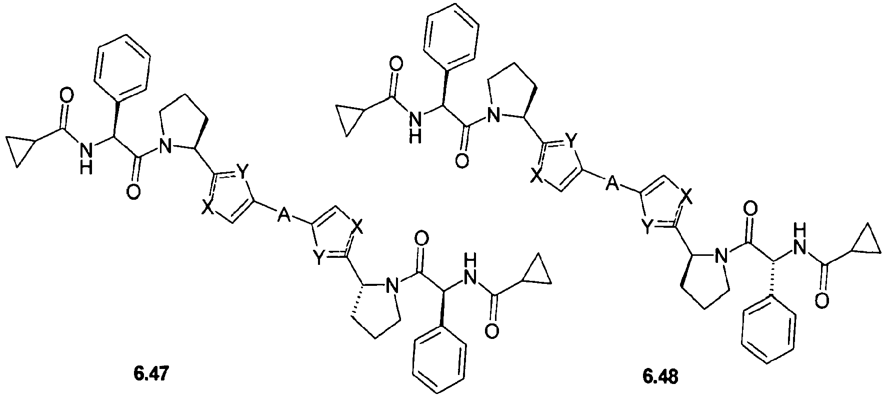

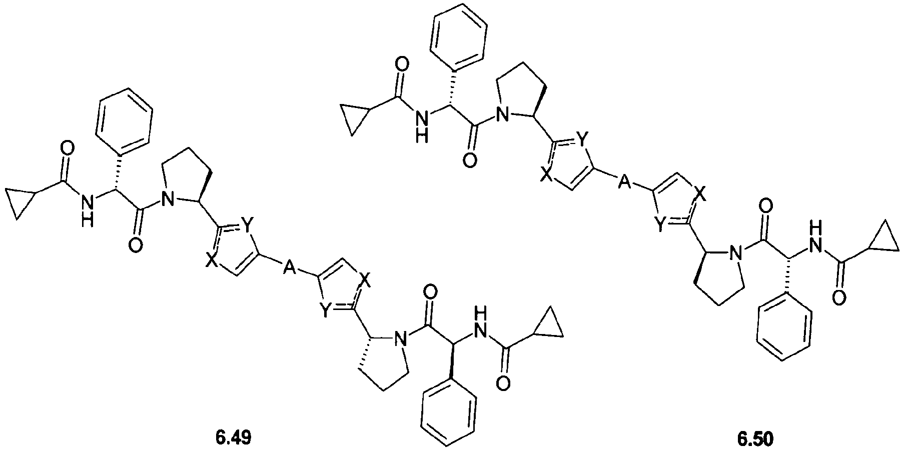

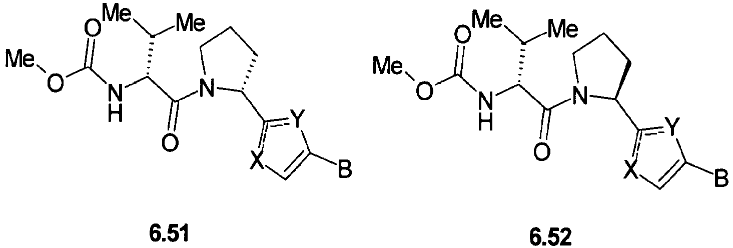

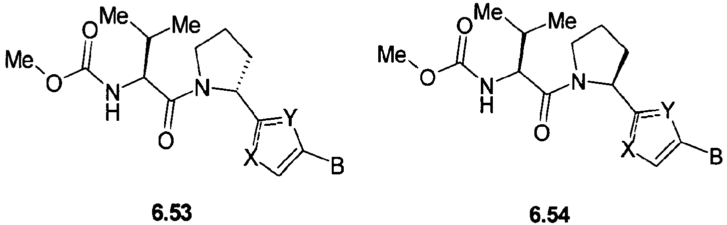

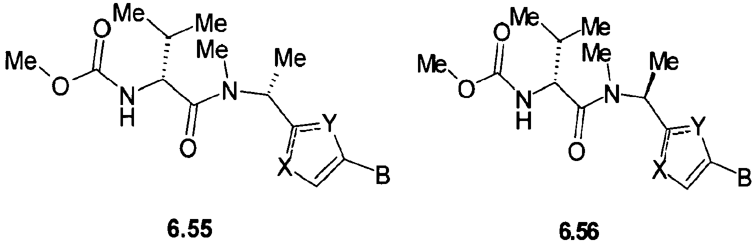

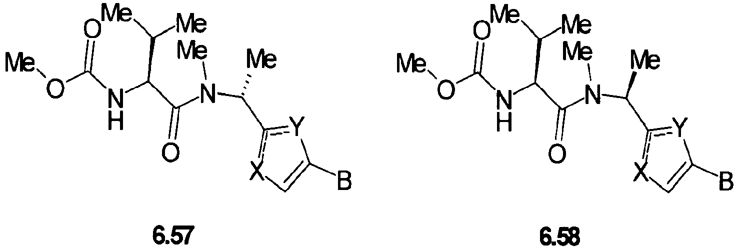

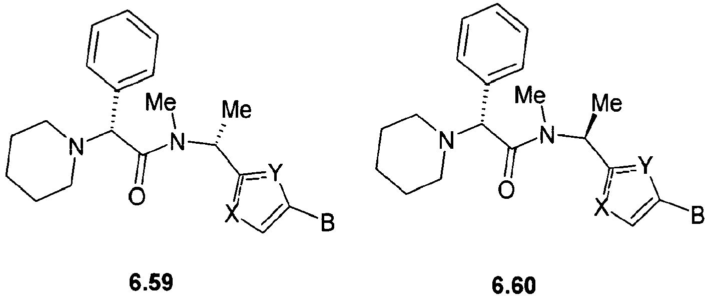

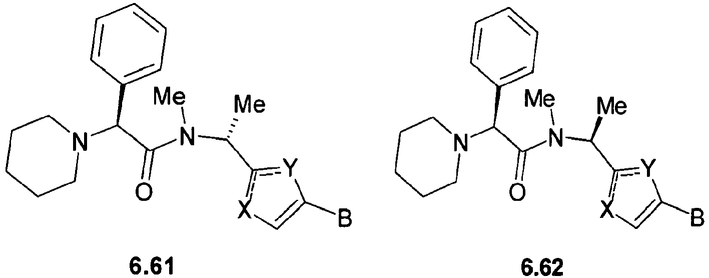

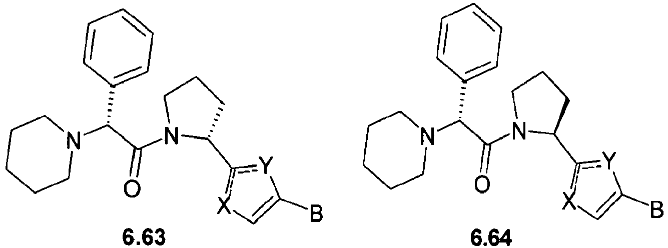

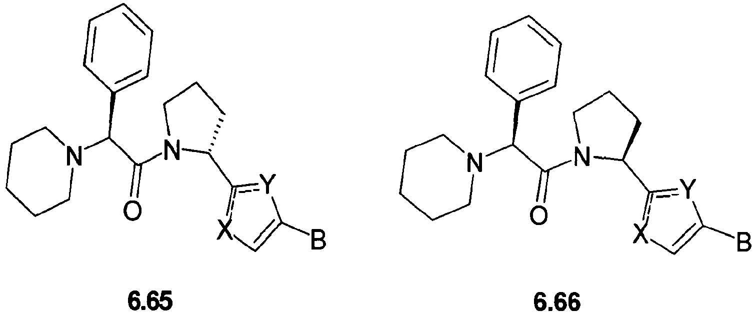

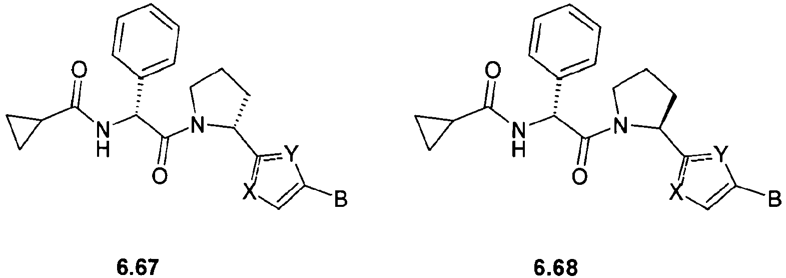

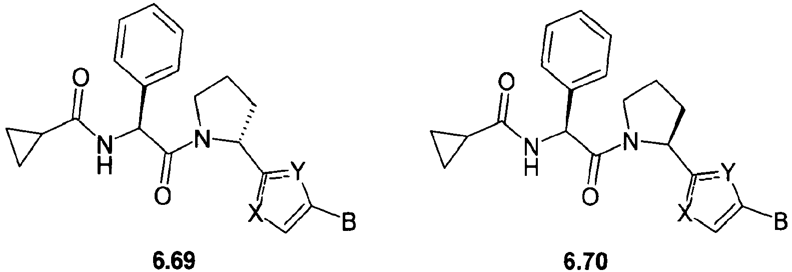

The more preferable substituted azoles are the compounds of formulas **6** - **62.**

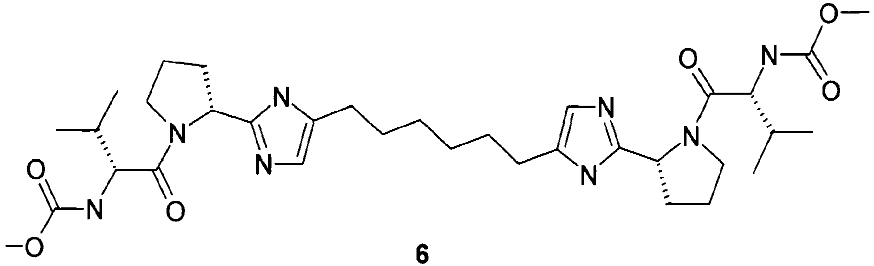

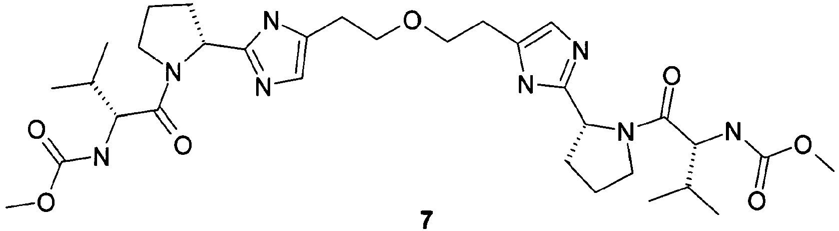

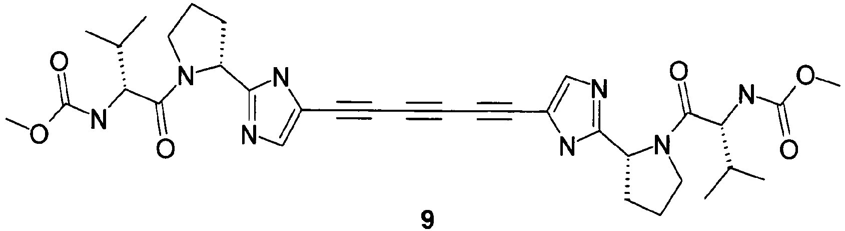

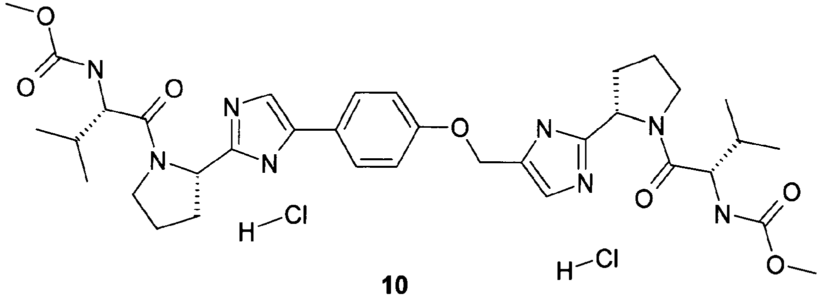

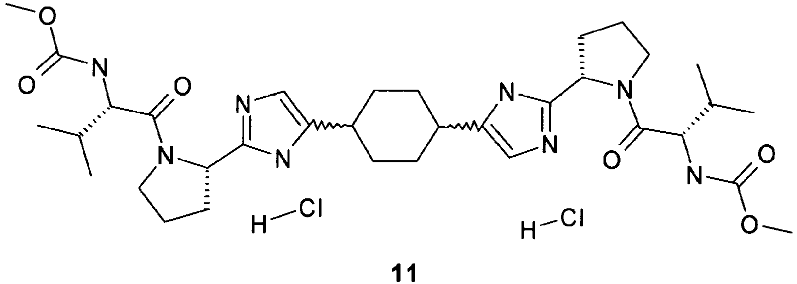

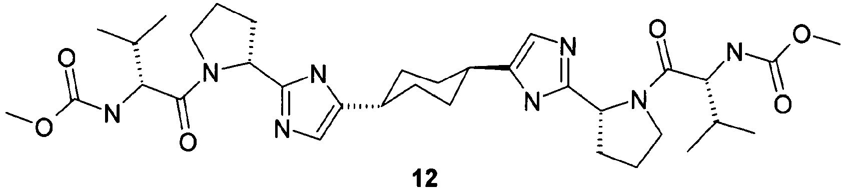

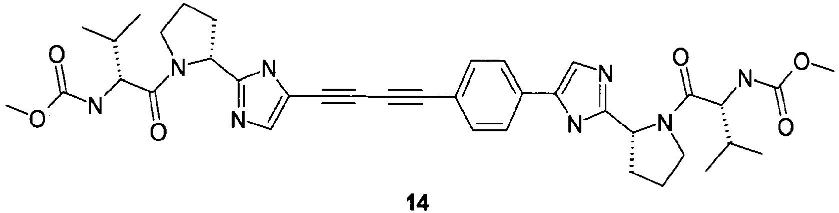

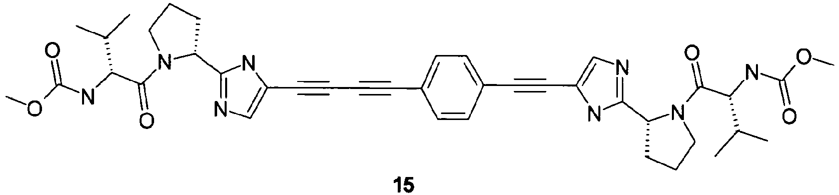

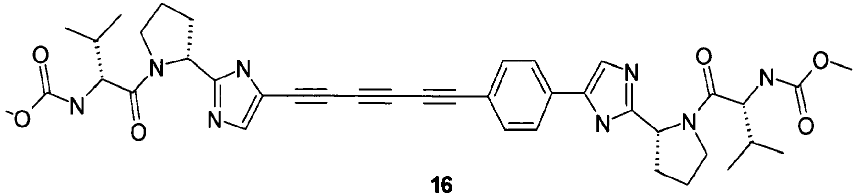

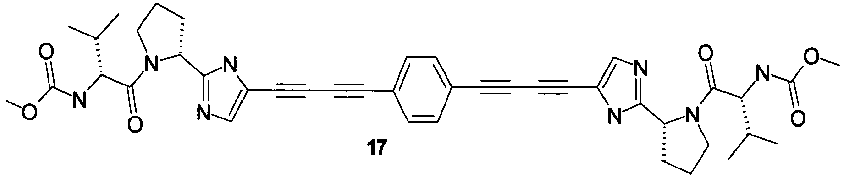

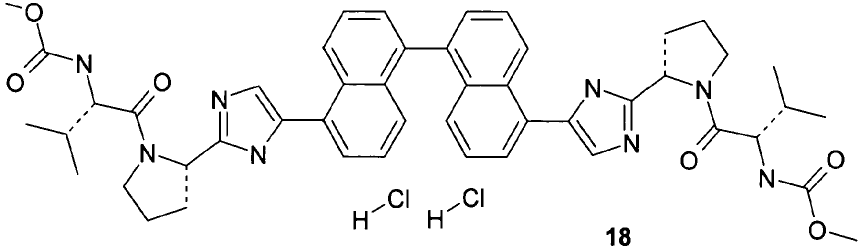

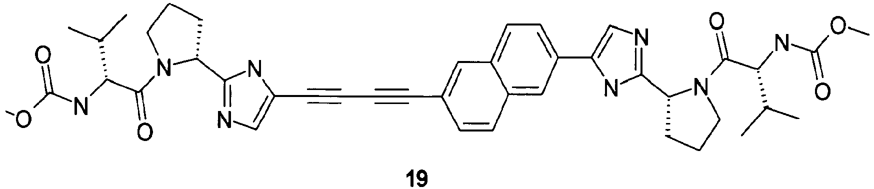

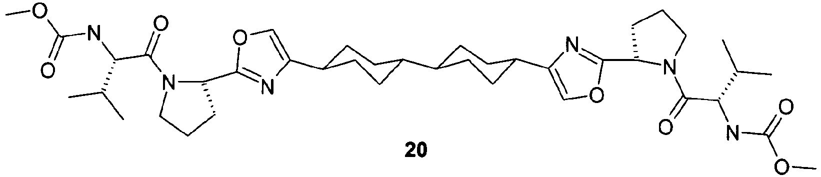

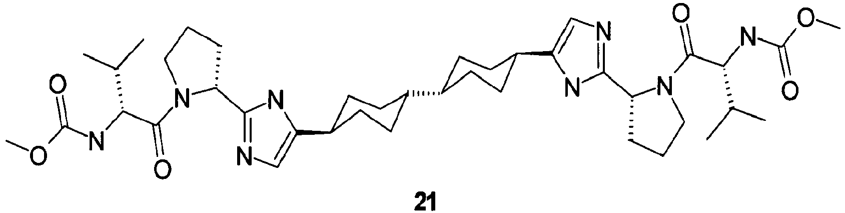

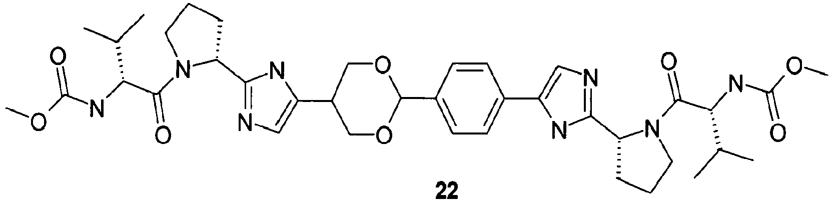

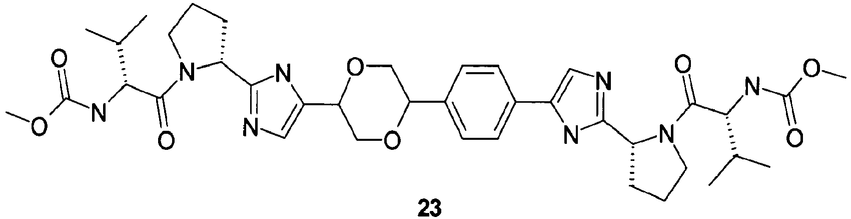

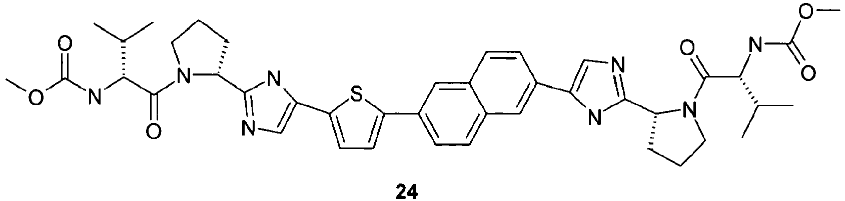

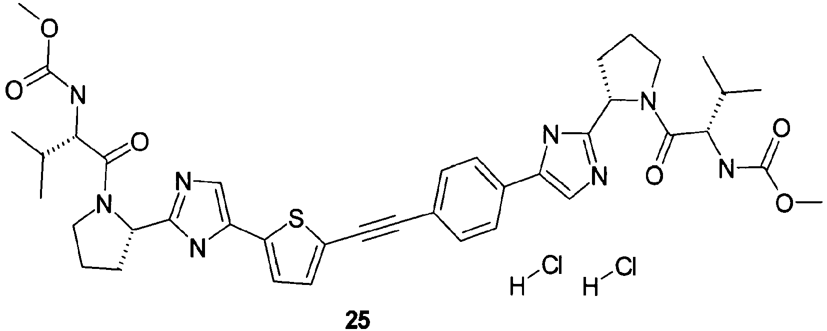

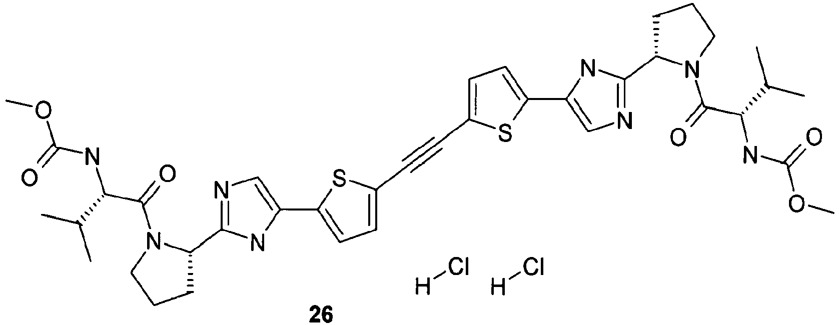

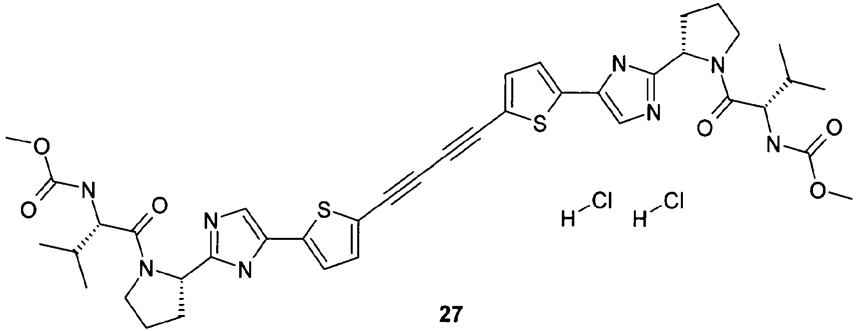

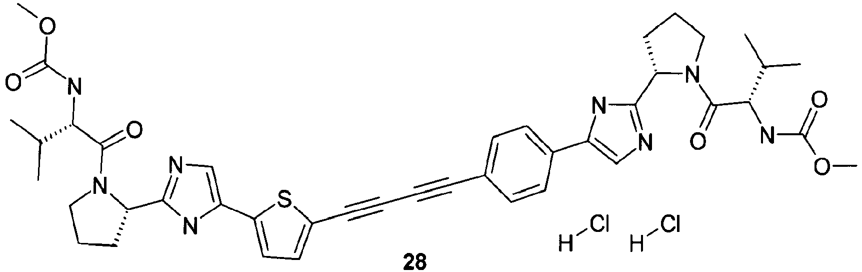

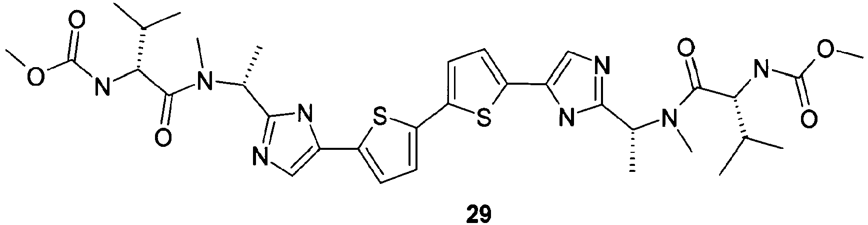

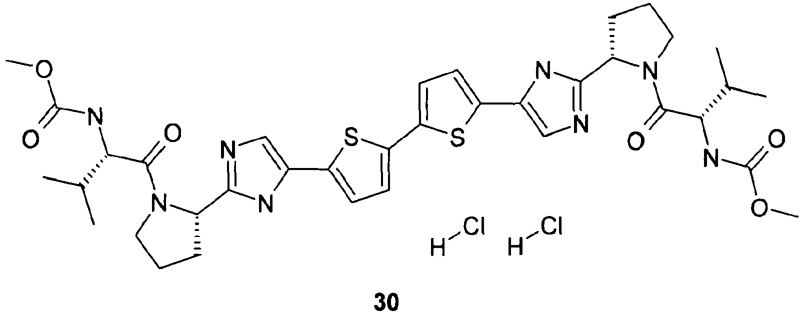

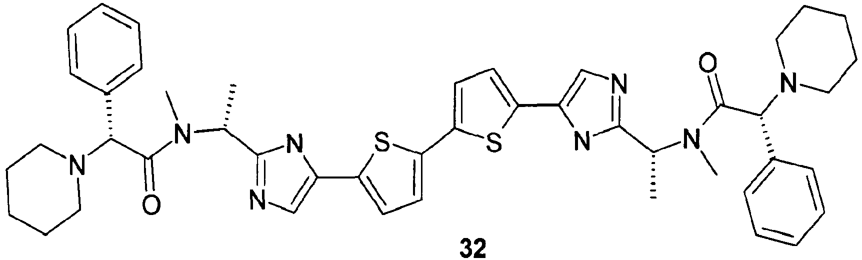

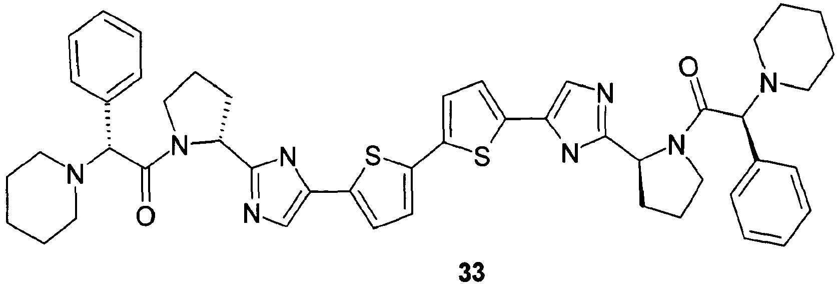

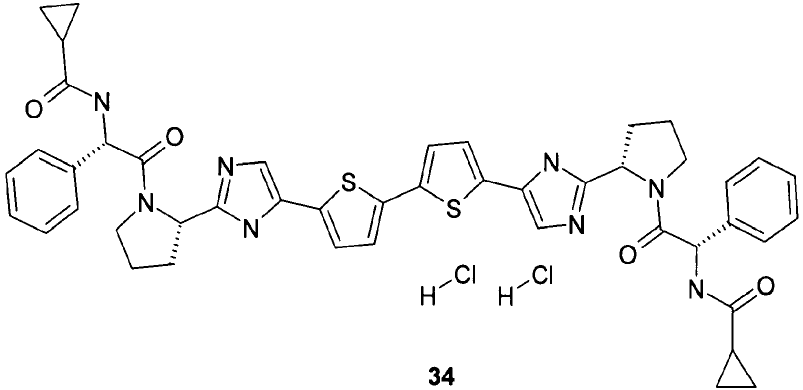

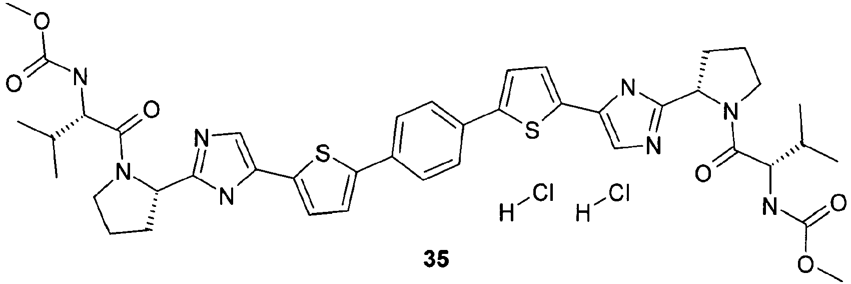

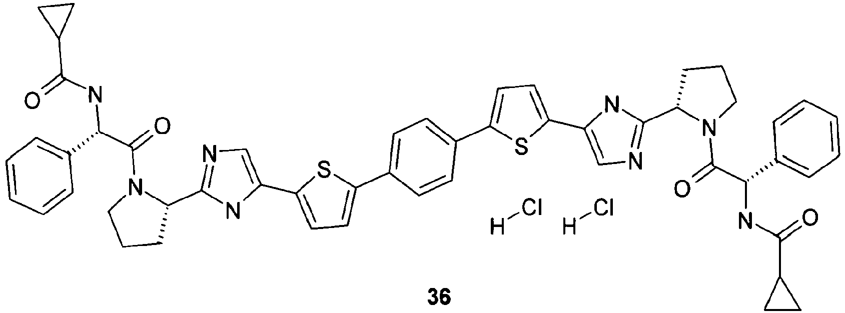

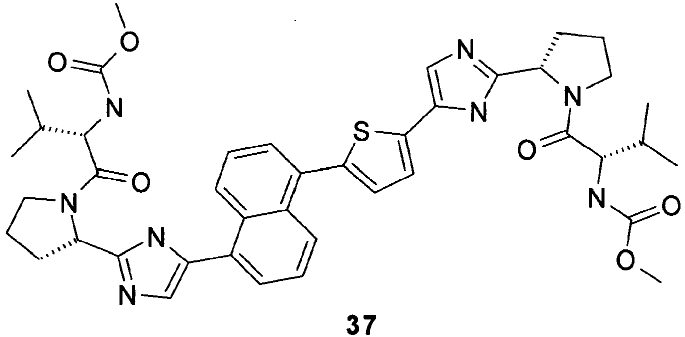

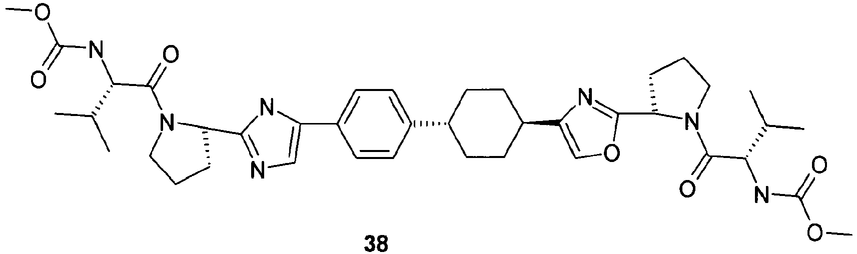

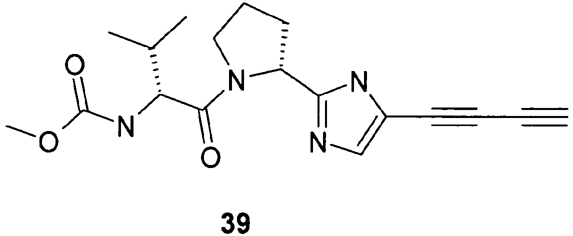

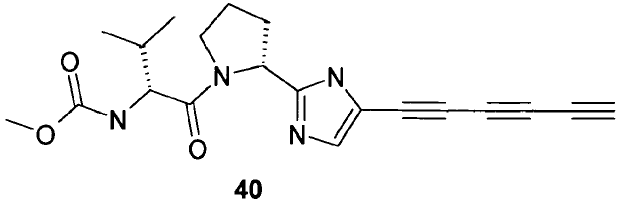

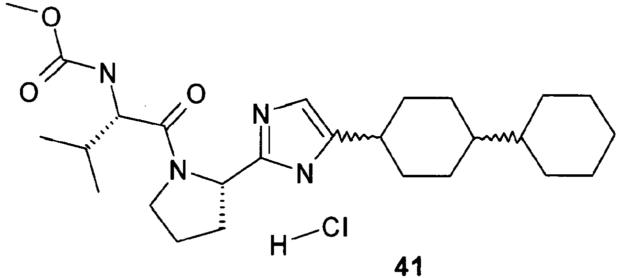

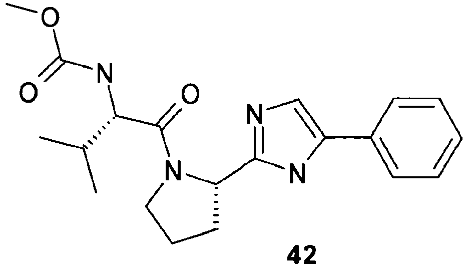

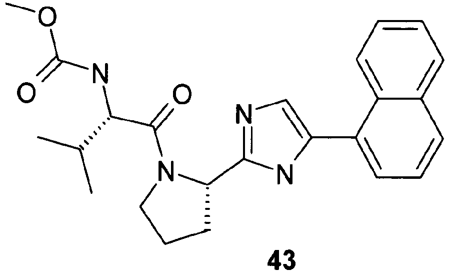

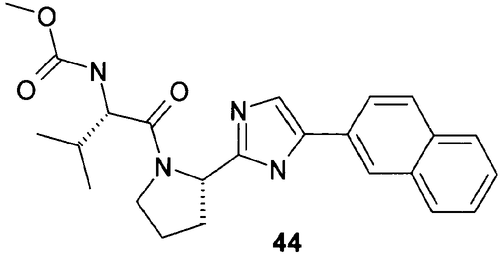

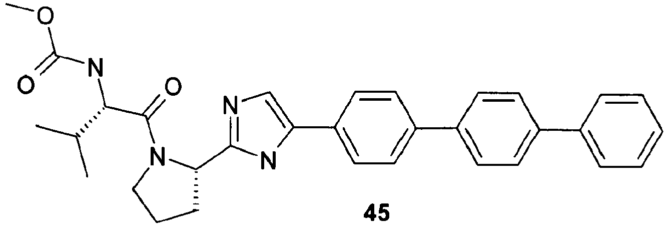

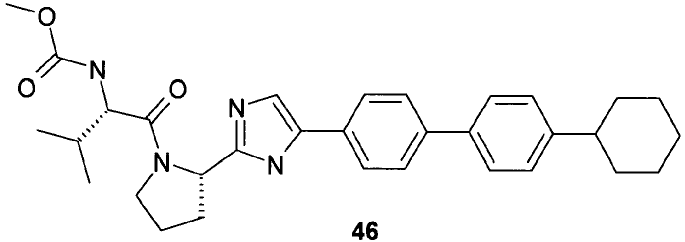

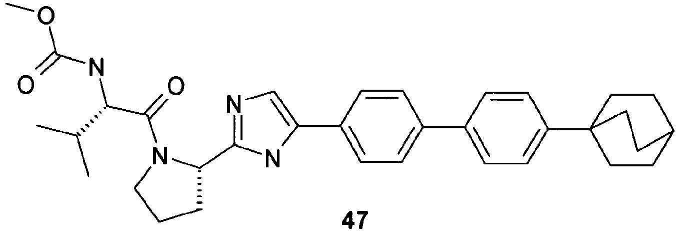

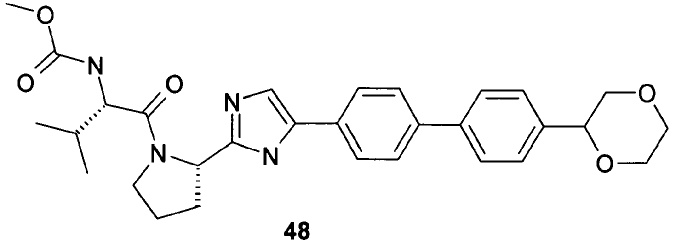

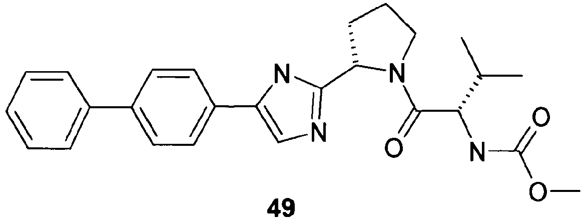

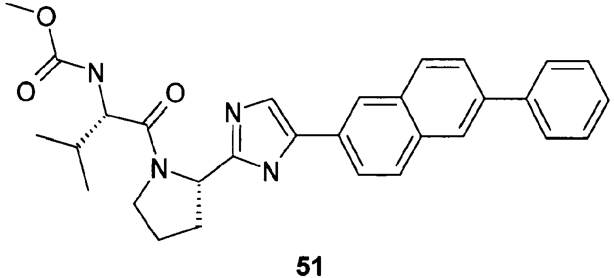

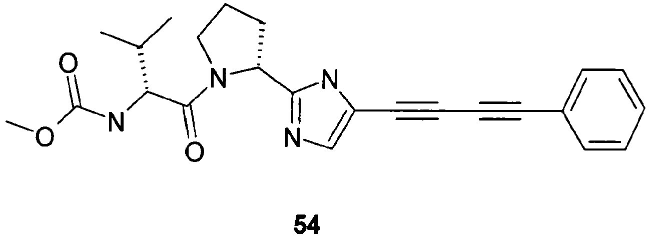

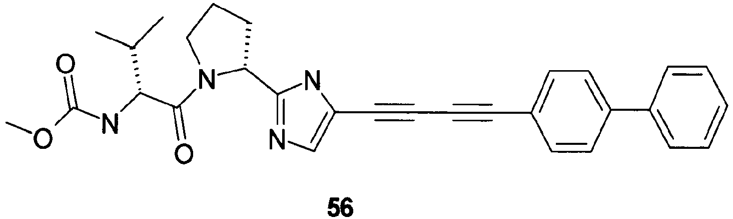

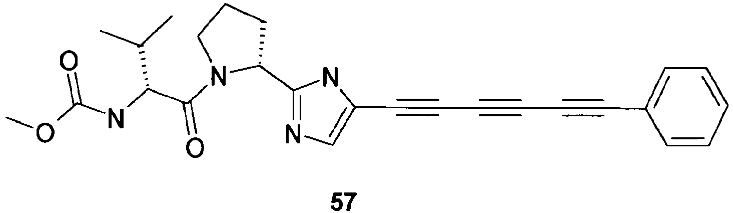

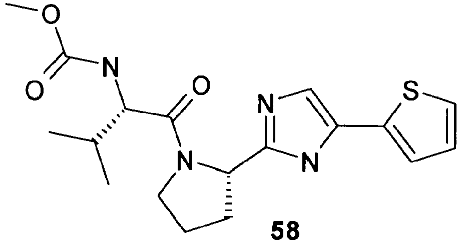

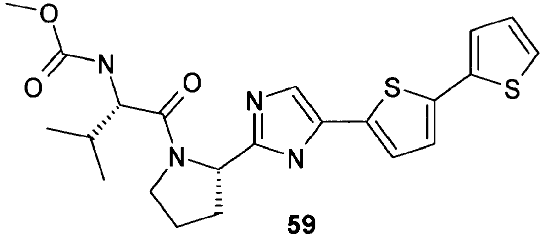

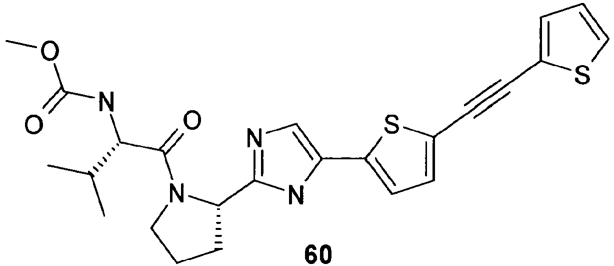

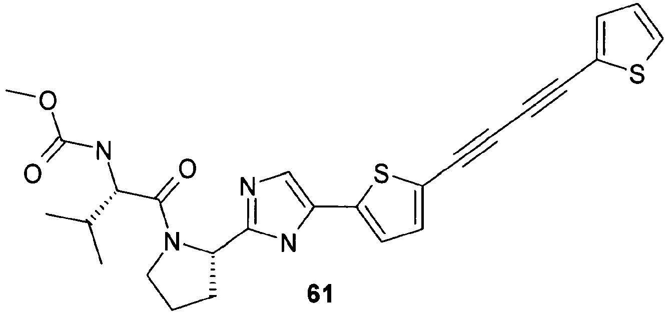

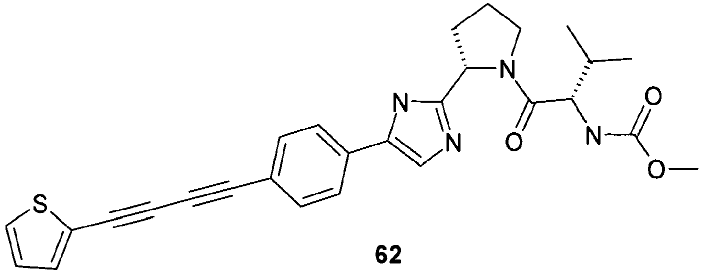

According to the invention substituted azoles of the general formulas **1A** and **1B** were prepared using known chemical reactions and commercial reagents. Structure of the compounds prepared was confirmed by LCMS and NMR data. The compounds were named using Chem Draw (Chembridge Soft Inc.) programme.

The folowing abbreviations were used in the scemes: DIPEA-diisopropylethylamine, EDAC - *N*-(3-dimethylaminopropyl)-*N*'-ethylcarbodiimide hydrochloride, HOBt - 1-hydroxybenzotriazole, N-Boc-L-Pro-OH - *N-*(*tert-*butoxycarbonyl)-L-proline, N-Moc-L-Val-OH = *N*-(methoxycarbonyl)-L-valine, RP HPLC - reverse-phase high performance liquid chromatography, HPLC - high-performance liquid chromatography, DCM - dichloromethane, DMF - N,N-dimethylformamide, PdCl2dppf - [1,1'-bis(diphenylphosphino)ferrocene]dichloropalladium(II), TBTU - O-(benzotriazol-1-yl)-*N,N,N',N*'-tetramethyluronium tetrafluoroborate, DBU - 1,8-diazabicyclo[5.4.0]undec-7-ene, HCV - hepatitis C virus, DMEM - Dulbecco's Modified Eagle Medium - culture medium.

Thus, [(1*S*)-1-[[(2*S*)-2-[5-[4-[4-[2-[(2*S*)-1-[(2*S*)-2-[(methoxycarbonyl)amino]-3-methyl-1-oxobutyl]-2-pyrrolidinyl]-1*H*-imidazol-5-yl]-1,3-butadiynyl]phenyl]-1H-imidazol-2-yl]-1-pyrrolidinyl]carbonyl]-2-methylpropyl]-carbamic acid methyl ester **14** was prepared according to the following scheme:

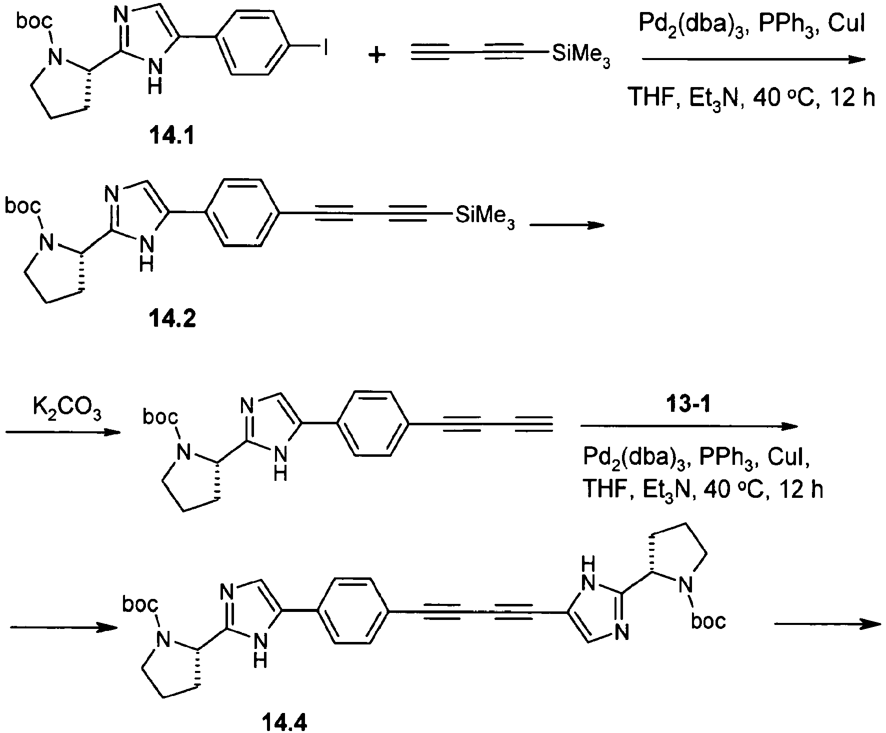

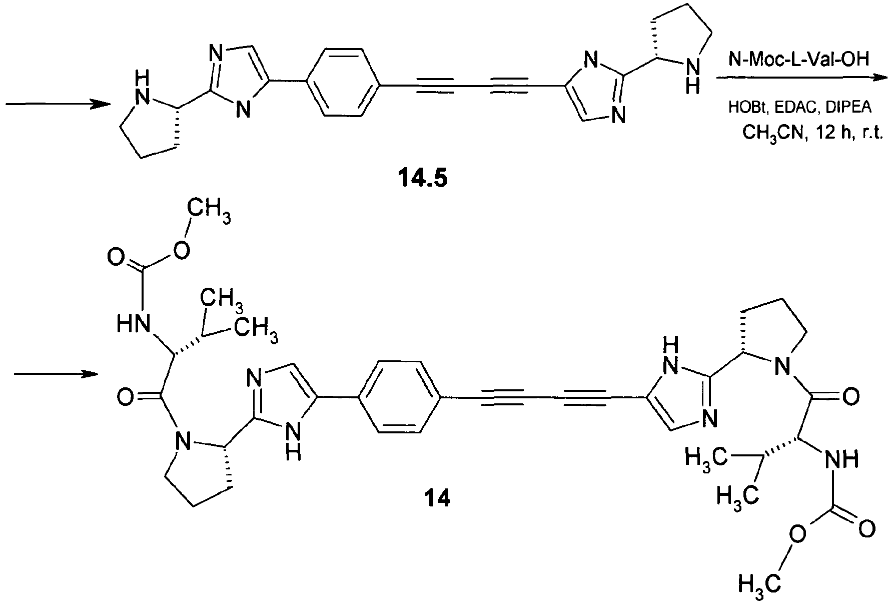

[[1,1'-*trans*,*trans*-Bicyclohexyl]-4,4'-diylbis[4,2-oxazolediyl(2*S*)-2,1-pyrrolidinediyl[(1*S*)-1-(1-methylethyl)-2-oxo-2,1-ethanediyl]]]bis-carbamic acid dimethyl ester **20** was prepared starting from biphenyl-4,4'-dicarboxylic acid dimethyl ester **20.1** according to the scheme given below.

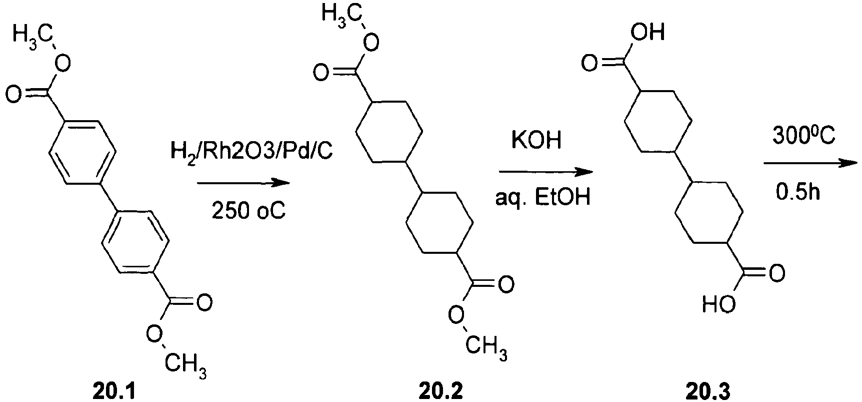

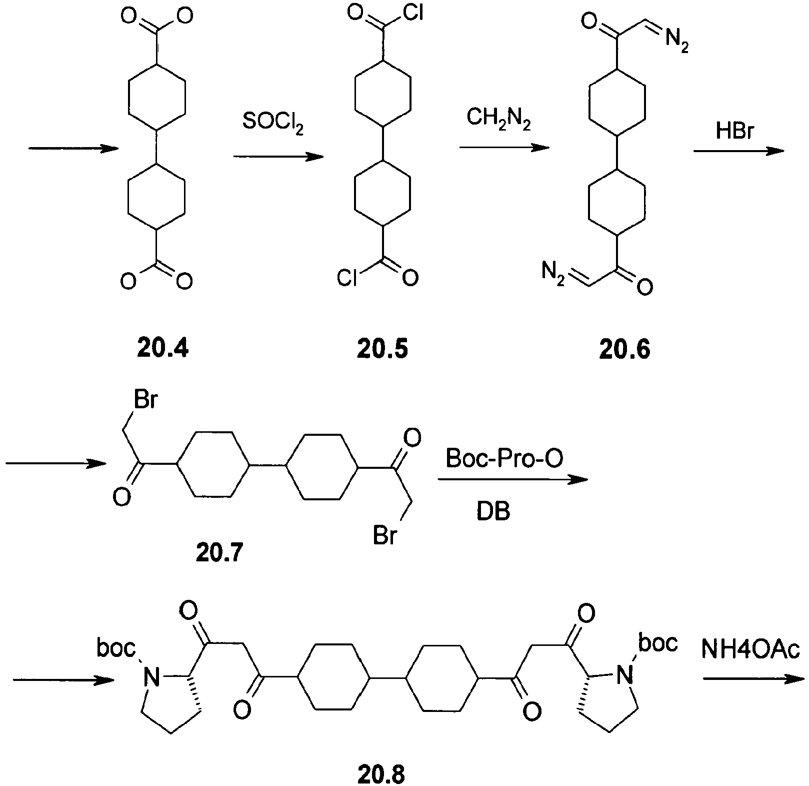

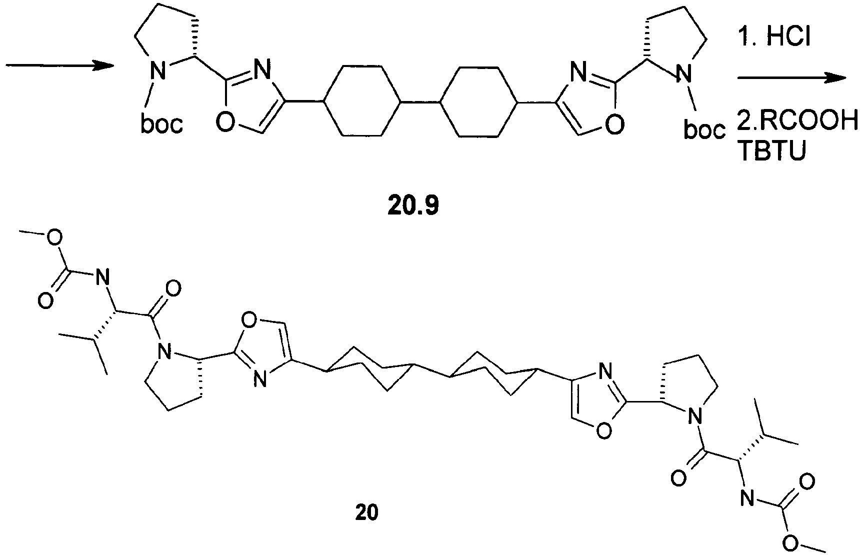

[(S)-1-((S)-2-{5-[5-(6-{2-[(S)-1-((S)-2-Methoxycarbonylamino-3-methyl-butyryl)-pyrrolidin-2-yl]-3H-imidazol-4-yl}-naphthalen-2-yl)-thiophen-2-yl]-1H-imidazol-2-yl}-pyrrolidine-1-carbonyl)-2-methyl-propyl]-carbamic acid methyl ester **24** was prepared according to the scheme given below.

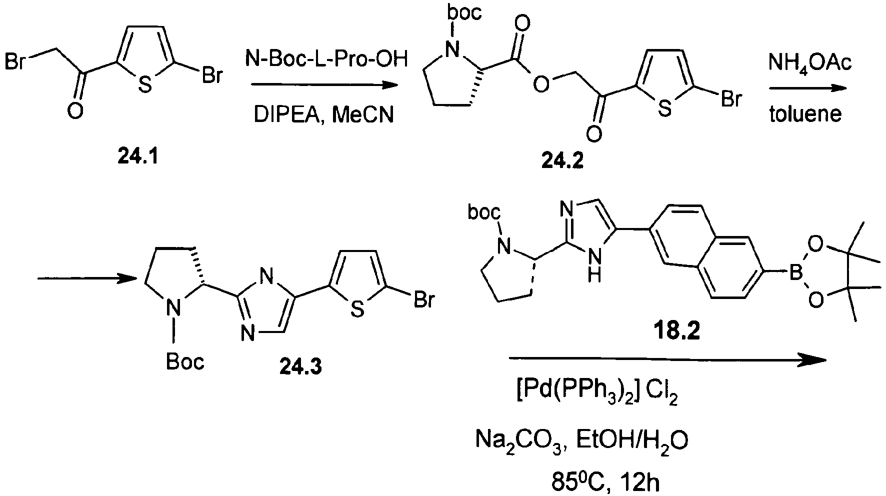

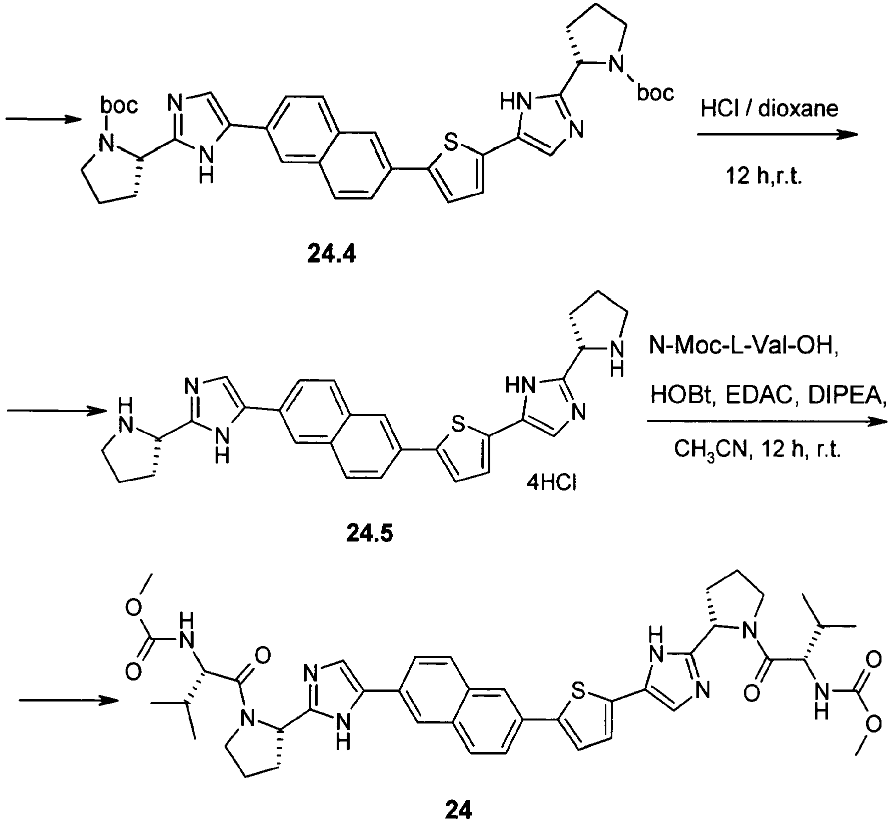

[(S)-1-((S)-2-{5-[5-(4-{2-[(S)-1-((S)-2-Methoxycarbonylamino-3-methyl-butyryl)-pyrrolidin-2-yl]-3H-imidazol-4-yl}-phenylethynyl)-thiophen-2-yl]-1H-imidazol-2-yl}-pyrrolidine-1-carbonyl)-2-methyl-propyl]-carbamic acid methyl ester **25** was prepared according to the scheme given below.

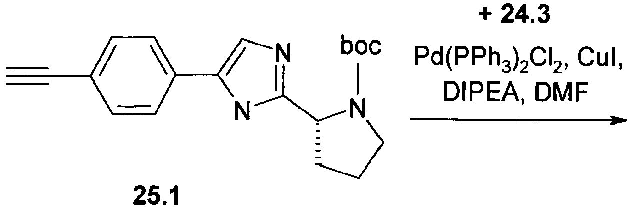

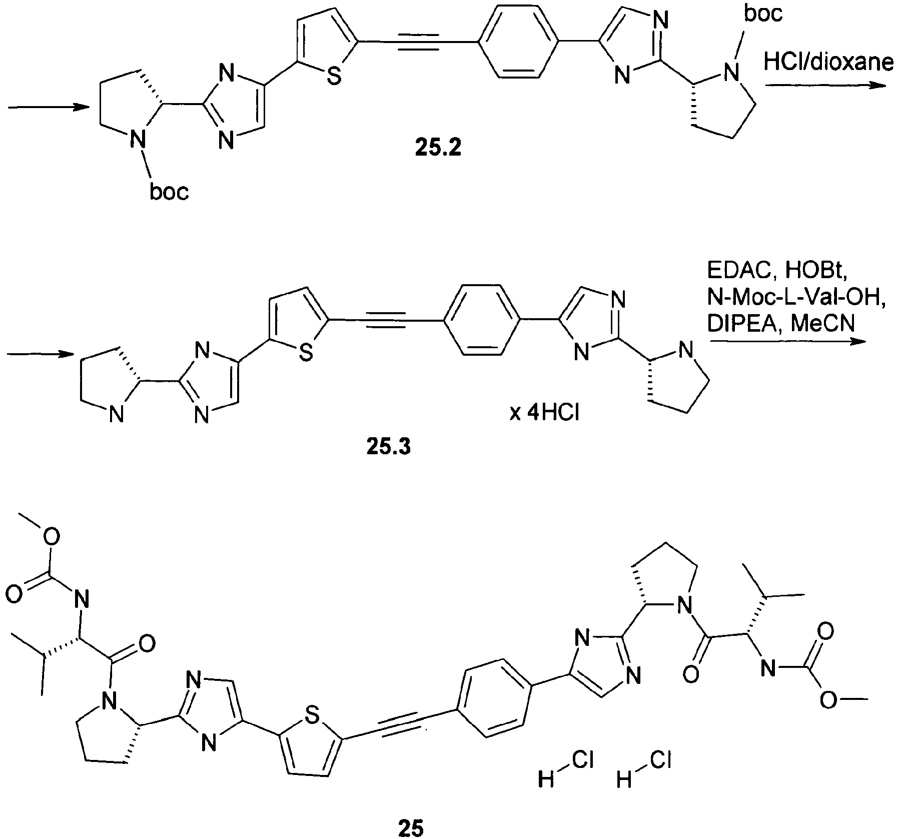

((S)-1-{(S)-2-[5-(5'-{2-[(S)-1-((S)-2-Methoxycarbonylamino-3-methyl-butyryl)-pyrrolidin-2-yl]-3H-imidazol-4-yl}-[2,2']bithiophenyl-5-yl)-1H-imidazol-2-yl]-pyrrolidine-1-carbonyl}-2-methyl-propyl)-carbamic acid methyl ester **30** was prepared according to the scheme given below.

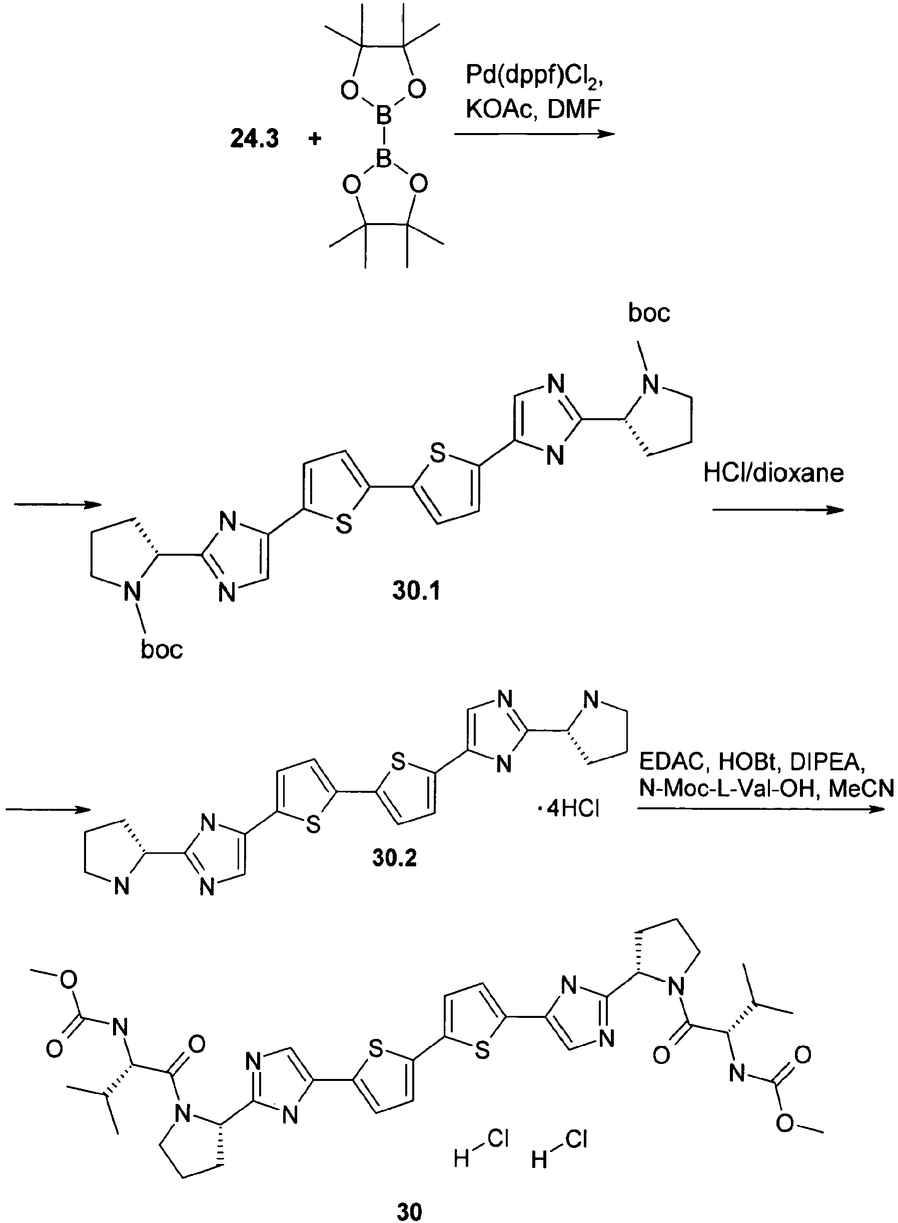

{(S)-1-[(S)-2-(5-{5-[4-(5-{2-[(S)-1-((S)-2-Methoxycarbonylamino-3-methyl-butyryl)-pyrrolidin-2-yl]-3H-imidazol-4-yl}-thiophen-2-yl)-phenyl]-thiophen-2-yl}-1H-imidazol-2-yl)-pyrrolidine-1-carbonyl]-2-methyl-propyl}-carbamic acid methyl ester 35 was prepared according to the scheme given below.

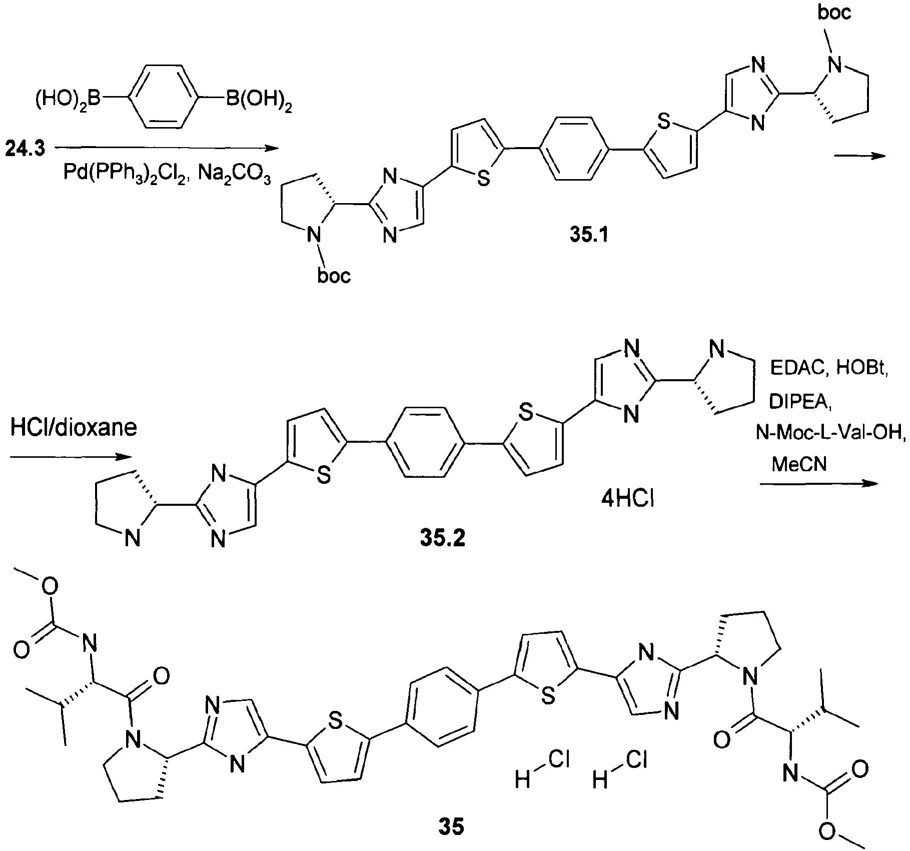

*N*-[(1S)-2-[(2*S*)-2-[5-[5-[4-[5-[2-[(2*S*)-1-[(2*S*)-2-[(1-Cyclopropylethenyl)amino]-2-phenylacetyl]-2-pyrrolidinyl]-1*H*-imidazol-5-yl]-2-thienyl]phenyl]-2-thienyl]-1H-imidazol-2-yl]-1-pyrrolidinyl]-2-oxo-1-phenylethyl]-cyclopropanecarboxamide 36 was prepared according to the scheme given below.

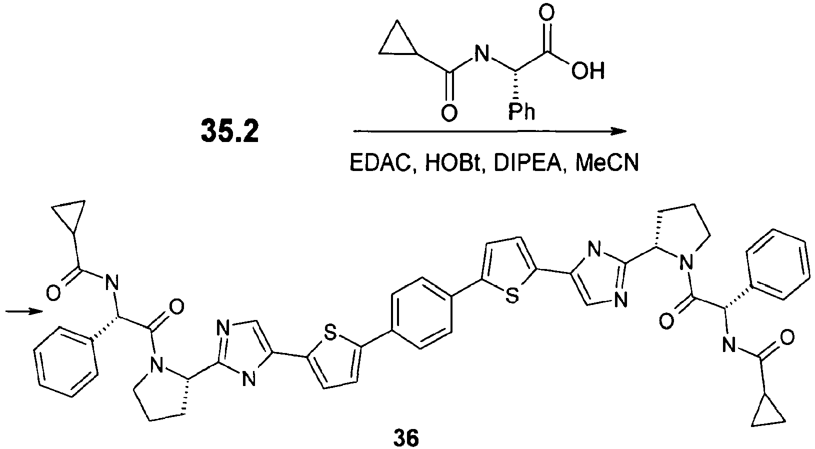

[(S)-1-((S)-2-{5-[5-(5-{2-[(S)-1-((S)-2-Methoxycarbonylamino-3-methyl-butyryl)-pyrrolidin-2-yl]-3H-imidazol-4-yl}-naphthalen-1-yl)-thiophen-2-yl]-1H-imidazol-2-yl}-pyrrolidine-1-carbonyl)-2-methyl-propyl]-carbamic acid methyl ester **37** was prepared according to the scheme given below.

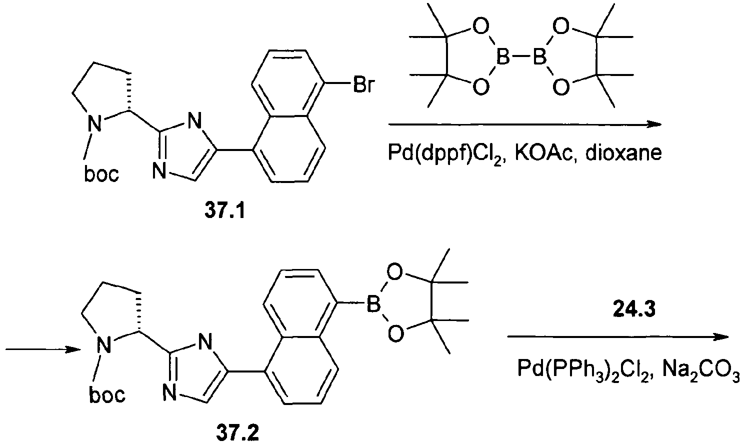

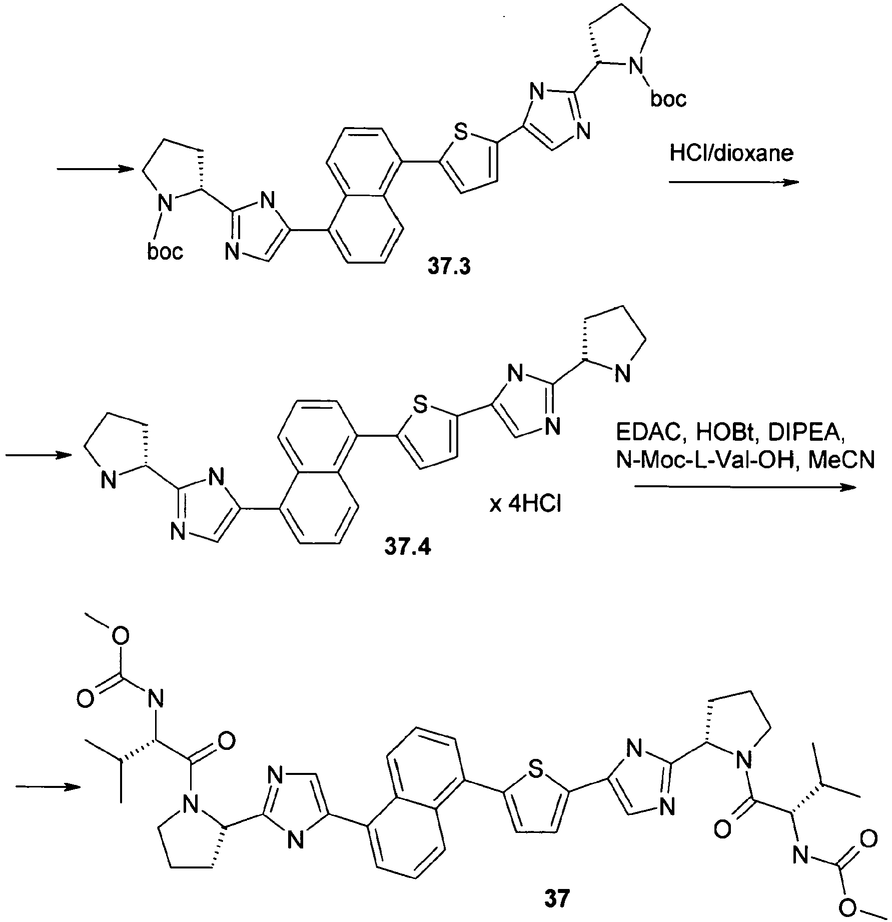

[(1S)-1-[[(2S)-2-[4-[4-[2-[(2S)-1-[(2S)-2-[(Methoxycarbonyl)amino]-3-methyl-1-oxobutyl]-2-pyrrolidinyl]-1*H*-imidazol-5-yl]phenyl]cyclohexyl]-2-oxazolyl]-1-pyrrolidinyl]carbonyl]-2-methylpropyl]-carbamic acid methyl ester **38** was prepared starting from biphenyl-4,4'-dicarboxylic acid dimethyl ester **20.1,** according to the scheme given below.

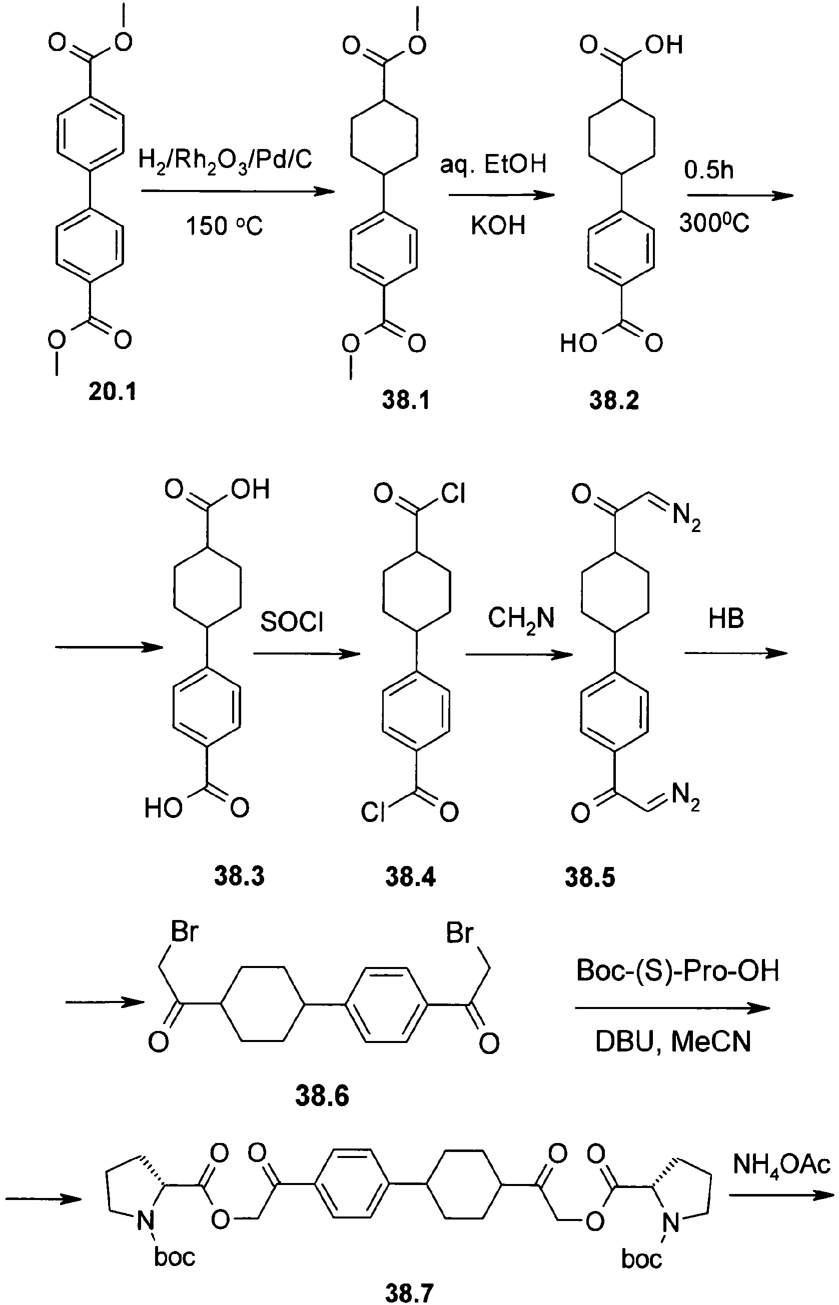

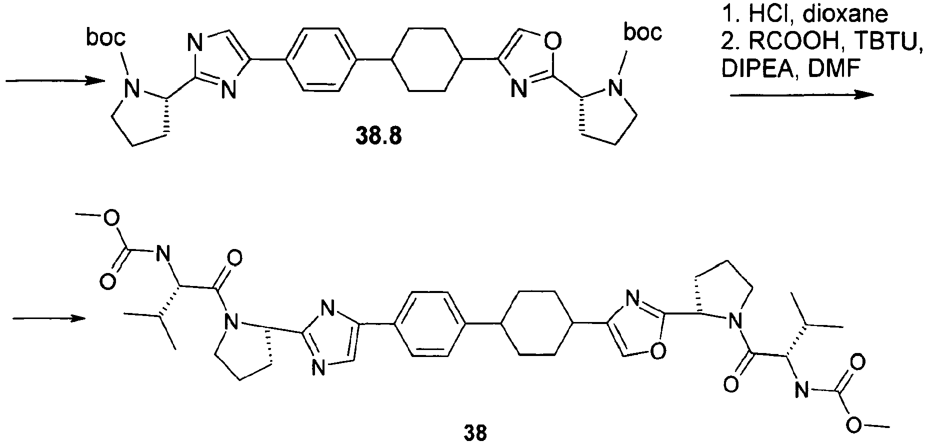

{(S)-2-Methyl-1-[(S)-2-(5-phenyl-1H-imidazol-2-yl)-pyrrolidine-1-carboline]-propyl}-carbamic acid methyl ester **42** was prepared according to the scheme given below.

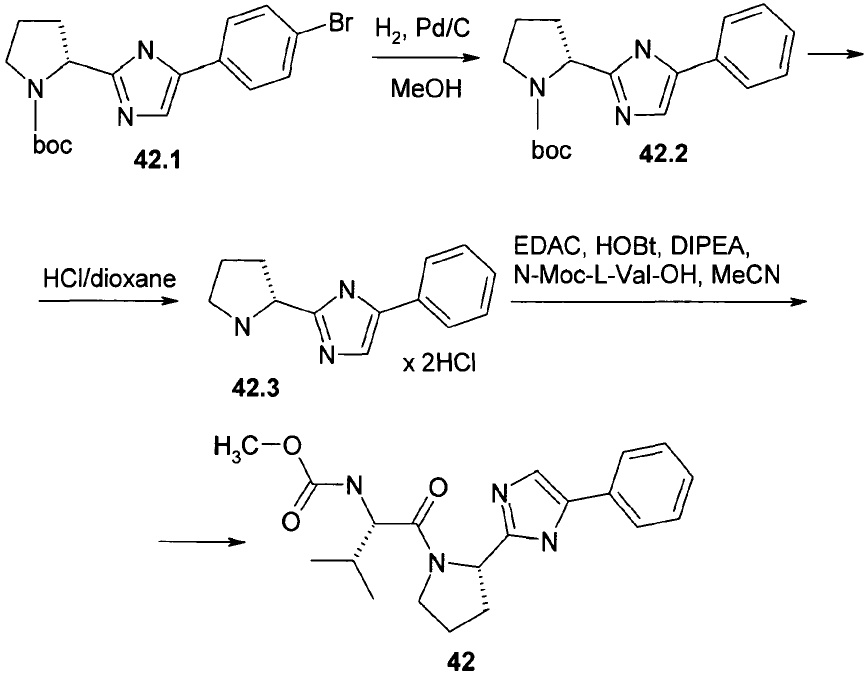

{(S)-2-Methyl-1-[(S)-2-(5-naphthalen-1-yl)-1H-imidazol-2-yl)-pyrrolidine-1-carboline]-propyl}-carbamic acid methyl ester 43 was prepared according to the scheme given below.

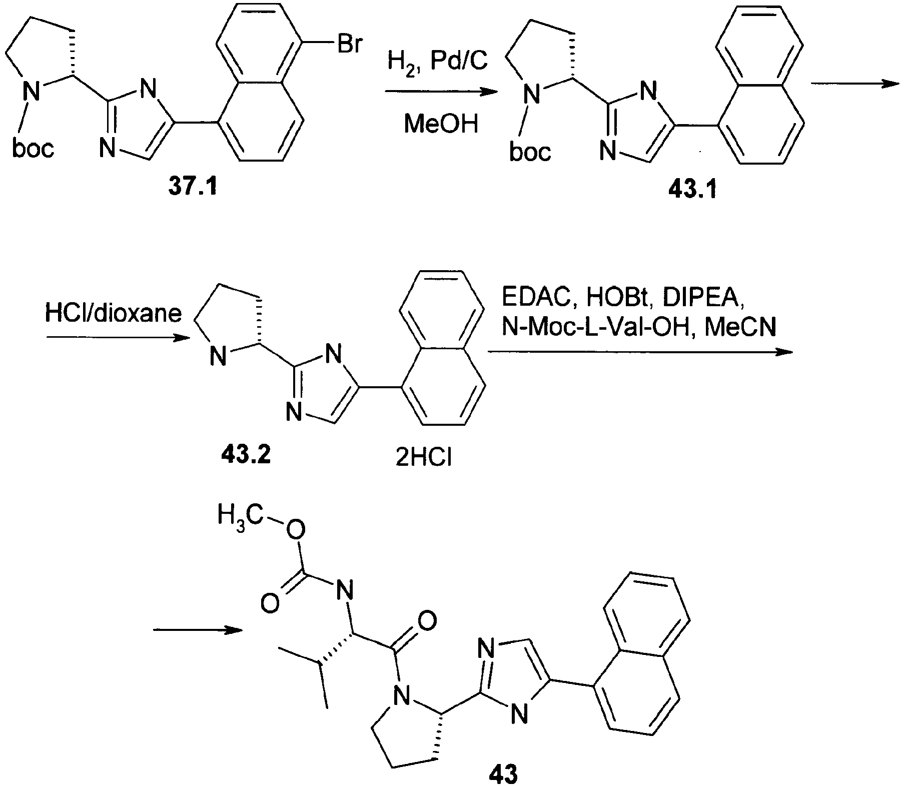

{(S)-2-Methyl-1-[(S)-2-(5-naphthalen-2-yl-1H-imidazol-2-yl)-pyrrolidine-1-carboline]-propyl}-carbamic acid methyl ester **44** was prepared according to the scheme given below.

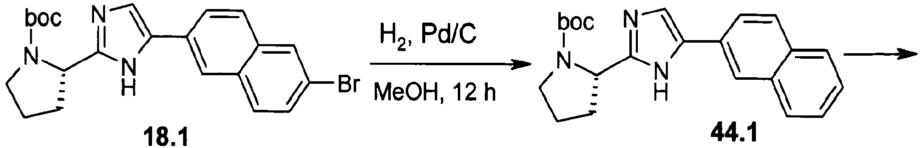

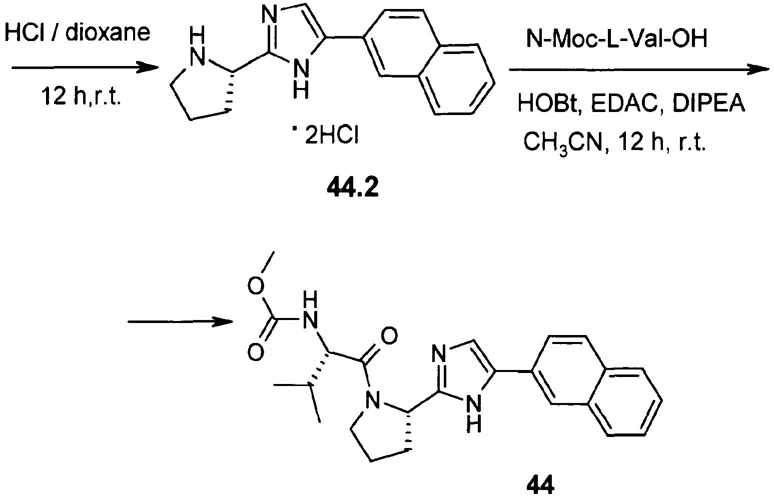

{(S)-2-Methyl-1-[(S)-2-(5-thiophen-2-yl-1H-imidazol-2-yl)-pyrrolidine-1-carboline]-propyl}-carbamic acid methyl ester **58** and {(S)-2-methyl-1-[(S)-2-[5-(2,2'-dithiophen-5-yl)-1H-imidazol-2-yl]-pyrrolidine-1-carboline]-propyl}-carbamic acid methyl ester **59** were prepared according to the scheme given below.

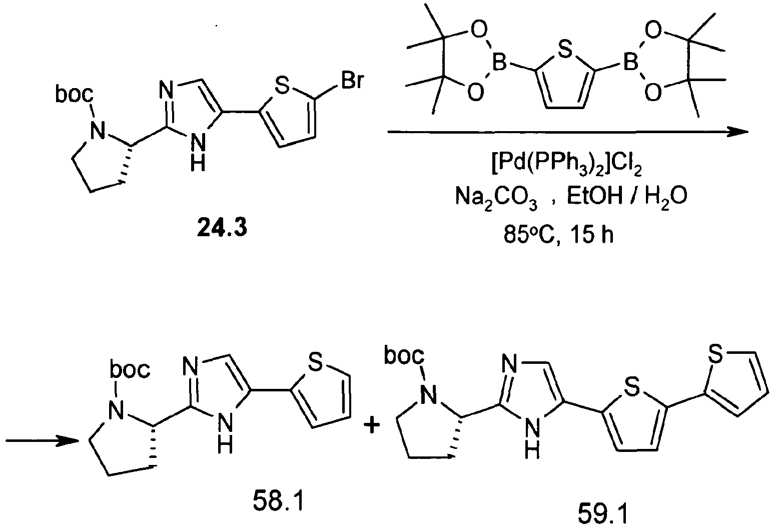

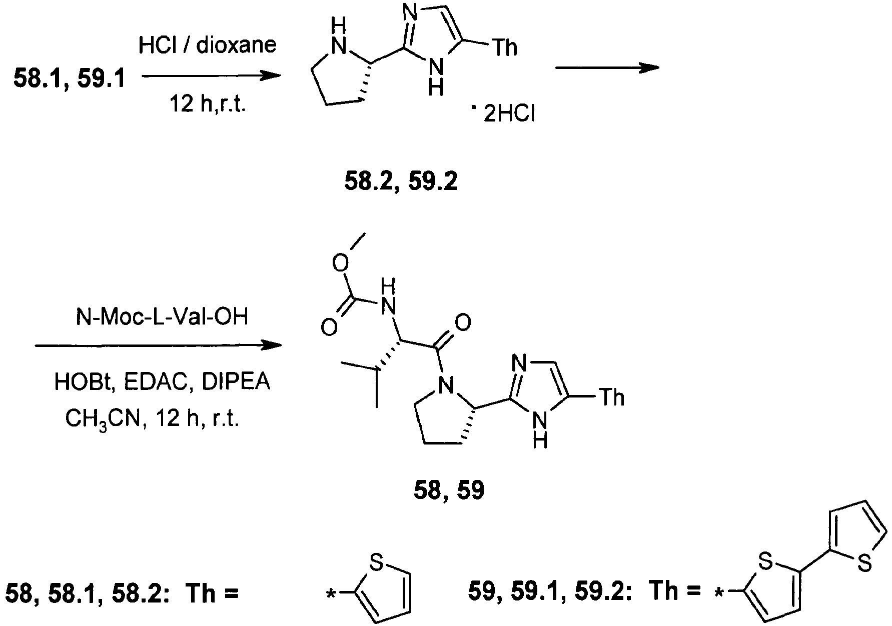

### Biological activity of azoles of the general formulas 1A and 1B.

Antiviral activity of substituted azoles of the general formulas **1A** and **1B** was determined in the human hepatoma cell line Huh7, comprising subgenomic RNA-replicon HCV (genotype 1b, clon Con1). A version of immumoenzymatic assay (IEA) on viral protein NS5A in 96-well plate was used as an experimental method. Cytotoxicity of the compounds was estimated in parallel regime.

Cells Huh7 were seeded in 96-well plate (7.5x10³ cells to each well in 100 µl of culture medium). Solutions of the tested compounds in DMEM medium (DMEM) 1X; Source: Cellgro; Catalogue: 10-013-CV} were prepared immediately before use. Eleven serial three fold dilutions with variation of concentrations from 20 nM to 0.2 pM were prepared. In 4 hours after seeding, serial dilutions of the compounds were added to the cells (100 µl to each well). Final concentration of tested compounds was varied from 10 nM to 0.1 pM, and DMSO - 0.5%. If it was necessary, higher concentrations of the disclosed azoles were investigated.

Each dilution of the compound was tested on two identical wells. Then the cells were incubated for three days at 37°C/5% CO₂ and fixed by addition of acetone/methanol (1:1) mixture in amount of 250 µl/well. In 1 min the cells were washed 3 times with PBS (Phosphate Buffered Saline) solution. Then the cells were blocked by addition of 10% fetal calf serum in PBS solution in amount of 150 µl/well for 1 h at room temperature. Then, the cells were incubated with mouse monoclonal antibodies to cor-antigen HCV, clon C7-50 (Source: Affinity BioReagents; Catalogue: MA1-080) (100 µl/well, working dilution - 1:500 in 10% fetal calf serum in PBS solution) for 2 h at 37°C. The cells were washed 6 times with PBS/0.05% Tween 20 solution, then, they were incubated for 1 h with goat anti-mouse immunoglobulin antibodies (conjugated with horseradish peroxidase, 100 µl/well, working dilution - 1:2500 in 10% fetal calf serum in PBS solution). The cells were washed 6 times with PBS/0.05% Tween 20 solution, once with PBS solution, after that substrate (1 tablet of o-phenylenediamine (oPD) + 12 ml citrate/phosphate buffer + 5 µl 30% H₂O₂) in amount of 100 µl/well was added. The plates were kept for 30 min in the dark at room temperature. The reaction was arrested by the addition of 2N H₂SO₄ in amount of 100 µl/well, and optical density (wavelength 490 nm) was measured by means of multiscan plate reader Victor3 V 1420 (Perkin Elmer). IC₅₀ values (azole concentration, lowering the level of virus RNA-replicon on 50%) for every tested azole were calculated with the help of XLfit 4 program.

Cytotoxicity of the disclosed azoles was tested in experiments in the human hepatoma cell line Huh7. The amount of living cells was determined with the help of ATPLite kit (Perkin Elmer, Boston, USA) in accordance with manufacturer instructions. Cytotoxic action was estimated by seeding the cells into black microplate with transparent bottom (96 wells, 10⁴ cells to each well). Three independent repeatings were used for each bis-azole. The tested bis-azoles were added in 18 h, after that the cells were incubated together with the compounds for 96 h. Each well was washed two times with phosphate buffered saline (0.2 ml/well) and then the cells were lysed by addition of cell buffer (50 µl/well) (all mentioned reagents are included in ATPLite kit). The microplate was incubated for 5 min on a rotating platform at 600 r/min, after that 50 µl of substrate solution (a part of ATPLite kit) was added into each well. The microplate was incubated for additional 5 min on a rotating platform at 600 r/min, kept for 10 min in the dark, after that luminescence was measured using TopCount NXT instrument (Packard, Perkin Elmer).

CC₅₀ Value corresponding to bis-azole concentration at which 50% of cells were ruined was used as quantitative characteristic for cytotoxicity estimation.

Calculation of CC₅₀ value: for calculation of inhibition effectiveness (% Inh) the following equation was used: % Inh = [(L^{pos}- L^{ex})/ L^{pos} - L^{neg})] * 100%, where L^{pos} - positive control, luminescence in the wells with cells without compounds; L^{neg} - negative control, luminescence in the wells with medium without cells; L^{ex}-luminescence in wells with a compound of definite concentration. Then, CC₅₀ values were calculated with the help of XLfit 4 program. Test results for novel azoles of the general formulas **1A** and **1B** testify their high (nanomolar) or very high (picomolar) activity. Inhibition activity towards genotype gT1b, gT1a and gT2a HCV of novel azoles of the general formulas **1A** and **1B** are shown in the Table given below and denoted as: * > 1000 nM, ** from 999 nM till 10 nM, *** from 9.9 nM till 1 nM and **** < 1 nM.

| **Nº comp.** | **gT1b** | **gT2a** | **pT1a** |
|---|---|---|---|
| **14·2HCl** | **** | **** | **** |
| **18·2HCl** | **** | **** | **** |
| **30** | **** | **** | *** |
| **25** | **** | *** | *** |
| **35** | **** | ** | **** |
| **42** | ** | ** | ** |
| **36** | **** | *** | *** |
| **38·2HCl** | ** | ** | * |
| **20·2HCl** | ** | * | ** |
| **43** | ** | * | * |
| **59** | ** | * | ** |
| **44** | ** | * | * |
| **37·2HCl** | **** | *** | **** |
| **58** | *** | ** | ** |
| **37·2HCl** | **** | **** | *** |
| **17·2HCl** | * | * | ** |

The subject of the present invention is novel ligans, the range of biological activity of which includes viral protein NS5A, which are substituted azoles of the general formulas **1A** and **1B** and pharmaceutically acceptable salts thereof.

The subject of the present invention is an active component for pharmaceutical compositions and medicaments intended for treatment and prophylaxis of flavivirus (genus Flaviviridae) diseases caused by hepatisis C virus, hepatisis GBV-C virus, yellow fever virus, West Nile virus, Dengue virus, representing substituted azoles of the general formulas **1A** and **1B** and pharmaceutically accaptable salts thereof.

The subject of the present invention is pharmaceutical composition comprising as an active component pharmaceutically effective amount of substituted azole of the general formulas **1A** and **1B** and pharmaceutically acceptable salts thereof.

Pharmaceutical compositions may include pharmaceutically acceptable excipients. Pharmaceutically acceptable excipients mean diluents, auxiliary agents and/or carriers applied in the sphere of pharmaceutics. According to the invention pharmaceutical composition in addition to the active component of the general formulas **1A** and **1B** may include other active ingredients provided that they do not give rise to undesirable effects, for example, allergic reactions.

If needed, according to the present invention pharmaceutical compositions can be used in clinical practice in various forms prepared by mixing the said compositions with traditional pharmaceutical carries; for example, peroral forms (such as, tablets, gelatinous capsules, pills, solutions or suspensions); forms for injections (such as, solutions or suspensions for injections, or a dry powder for injections which requires only addition of water for injections before utilization); local forms (such as, ointments or solutions).

According to the present invention the carriers used in pharmaceutical compositions represent carriers which are used in the sphere of pharmaceutics for preparation of commonly applied forms including: binding agents, greasing agents, disintegrators, solvents, diluents, stabilizers, suspending agents, colorless agents, taste flavors are used for peroral forms; antiseptic agents, solubilizers, stabilizers are used in the forms for injections; base materials, diluents, greasing agents, antiseptic agents are used in local forms.

The subject of the present invention is also method for the preparation of pharmaceutical compositions, which consists in mixing of at least one active component of the general formulas **1A** and **1B** or its pharmaceutically acceptable salt with inert exicipient and/or solvent.

The subject of the present invention is also a pharmaceutical composition in the form of tablets, capsules, or injections, placed in pharmaceutically acceptable packing.

The subject of the present invention is also a method for treatment of flaviviruses diseases caused by hepatitis C virus, yellow fever virus, West Nile virus, Dengue virus by introduction of pharmacologically effective amount of substituted azole of the general formulas **1A** and **1B** or its pharmaceutically acceptable salt or novel pharmaceutical composition.

Clinical doses of pharmaceutical composition comprising as active component azole of the general formulas **1A** and **1B** may be corrected depending on: therapeutic efficiency and bio-accessibility of the active ingredients in patients' organism, rate of their exchange and removal from organism, and age, gender, and severity of patient's symptoms. Thus, the daily intake for adults normally being

10∼500 mg. Accordingly, the above effective doses are to be taken into consideration while preparing medicament from the pharmaceutical composition of the present invention, each dose unit of the medicament contains 10 ∼ 500 mg of azole of the general formula **1A** and **1B.** Following the instructions of physician or pharmacist, the medicaments may be taken several times over specified periods of time (preferably, from one to six times).

The subject of the present invention is also a therapeutic kit for prophylaxis and treatment of flavivirus diseases among them diseases caused by hepatisis C viruses, yellow fever viruses, West Nile virus, Dengue virus, hepatitis GBV-C virus including as one of the component substituted azole of the general formulas **1A** and **1B** or its pharmaceutically acceptable salt or pharmaceutical composition comprising an azole mentioned above.

The therapeutic kits for prophylaxis and treatment of flavivirus diseases mentioned above, among them hepatisis C, along with the drug substances disclosed in the invention, may include: inhibitors inosine-5-monophosphate dehydrogenase, for example, Ribavirin (allowed) and Ribamidine; inhibitors of NS3 hepatisis C protease, for example, Telaprevir and Boceprevir; inhibitors of RNK-polimeraze NS5B, for example, VX222, R7128, PF-868554, ANA598; alpha-glucosidase inhibitors, for example, aminocarbohydrate Selgozivir; and also TLR-receptor agonists, hepatoprotectors, cyclosporines, various proteins (for example, interferons), antibodies, vaccines etc.

For combination therapies any classes of agents that may be useful when combined with substituted azoles of the present invention include, for example, nucleoside and non-nucleoside inhibitors of the HCV polymerase, protease inhibitoprs, helicase inhibitors, NS4B inhibitors and medicinal agents that functionally inhibit the internal ribosomal entry site (IRES) and other medicaments that inhibit HCV cell attachment or virus entry, HCV RNA translation, replication or HCV maturation or virus release. Specific compounds in these classes and useful

in this invention include, but are not limited to, macrocyclic, heterocyclic and linear HCV protease inhibitors such as telaprevir (VX-950), boceprevir (SCH-503034), narlaprevir (SCH-900518), ITMN-191 (R-7227), TMC-435350 (a.k.a. TMC-435), MK-7009, BI-201335, BI-2061 (ciluprevir), BMS-650032, ACH-1625, ACH-1095 (HCV NS4A protease co-factor inhibitor) VX-500, VX-813, PHX-1766, PHX2054, IDX-136, IDX-316, ABT-450 EP-013420 (and congeners) and VBY-376; the Nucleosidic HCV polymerase (replicase) inhibitors useful in the invention include, but are not limited to, R7128, PSI-7851, IDX-184, IDX-102, R1479, UNX-08189, PSI-6130, PSI-938 and PSI-879 and various other nucleoside and nucleotide analogs and HCV inhibitors including (but not limited to) those derived as 2'-C-methyl modified nucleoside and nucleotide; and 7'-deaza modified nucleoside and nucleotide. Non-nuclosidic HCV polymerase (replicase) inhibitors useful in the invention, include, but are not limited to, HCV-796, HCV-371, VCH-759, VCH-916, VCH-222, ANA-598, MK-3281, ABT-333, ABT-072, PF-00868554, BI-207127, GS-9190, A-837093, JKT-109, GL-59728 and GL-60667.

In addition, NS5A inhibitors of the present invention may be used in combination with cyclophyllin and immunophyllin antagonists (for example, without limitation, DEBIO compounds, NM-811, as well as cyclosporine and its derivatives), kinase inhibitors, inhibitors of heat shock proteins (for example, HSP90, HSP70), other immunomodulatory agents that may include, without limitation, interferons (alpha-, beta-, omega-, gamma-, lambda or synthetic), such as Intron A™, Roferon- A™, Canferon-A300™, Advaferon™, Infergen™, Humoferon™, Sumiferon MP™, Alfaferon™, IFN-β™, Feron ™, and the like, polyethylene glycol derivatized (pegylated) interferon compounds, such as: PEG interferon-α-2a (Pegasys™), PEG interferon-α-2b (PEGIntron™), pegylated IFN-α-con 1 and the like; long acting formulations and derivatives of interferon compounds, such as albumin-fused interferon, Albuferon™, Locteron™, and the like; interferons with various types of controlled delivery systems (e.g. ITCA-638, omega-interferon delivered by the DUROS subcutaneous delivery system); compounds that stimulate the synthesis of interferon in cells, such as resiquimod and the like; interleukins;
compounds that enhance the development of type 1 helper T cell response, such as SCV-07 and the like; TOLL-like receptor agonists, such as: CpG-10101 (action), isotorabine, ANA773 and the like; thymosin α-1, ANA-245 and ANA-246, histamine dihydrochloride, propagermanium; tetrachlorodecaoxide; ampligen; IMP-321; KRN-7000; antibodies, such as: civacir, XTL-6865 and the like and prophylactic and therapeutic vaccines, such as: Inno Vac, HCV E1E2/MF59 and the like. In addition, any of the above-described methods involving adminestering an NS5A inhibitor, a Type 1 interferon receptor agonist (e.g., an IFN-α) and a Type 2 interferon receptor agonist (e.g., IFN-γ) can be augmented by administration of an effective amount of TNF-α antagonist. Exemplary, non-limiting TNF-α antagonists that are suitable for use in such combination therapies include ENBREL™ and HUMIRA™.

In addition, NS5A inhibitors of the present invention may be used in combination with antiprotozoans and other antivirals thought to be effective in the treatment of HCV infection, such as, the prodrug nitazoxanide. Nitazoxanide can be used as an agent in combination with the compounds disclosed in this invention as well as in combination with other agents useful in treating HCV infection such as peginterferon alfa-2a and ribavarin (see, for example, Rossignol, JF and Keeffe, EB, Future Microbiol. 3:539-545, 2008).

NS5A inhibitors of the present invention may also be used in combination with alternative forms of interferons and pegylated interferons, ribavirin or its analogs (e.g., Tarabavarin, levovirion), microRNA, small interfering RNA compounds (e.g., SIRPLEX-140-N) and the like, nucleotide or nucleoside analogs, immonoglobulins, hepatoprotectants, anti-inflammatory agents and other inhibitiors of NS5A.

Inhibitors of other targets in the HCV life cycle include NS3 helicase inhibitors; NS4A co-factor inhibitors, antisense oligonucleotide inhibitors, such as ISIS-14803, AVI-4065 and the like; vector-encoded short hairpin RNA (shRNA); HCV specific ribozymes such as heptazyme, RPI, 139199 and the like; entry inhibitors such as HepeX-C, HuMax-HepC and the like; alpha glucosidase inhibitors such as celgosivir, UT-231B and the like; KPE-02003002 and BIVN 401 and IMPDH inhibitors. Other illustrative compounds HCV inhibitor compounds include those disclosed in the known scientific and patent publications.

Additionally, combinations of, for example, ribavirin and interferon may be administered as multiple combination therapy with at least one azole of the present invention. The present invention is not limited to the aforementioned classes or compounds and contemplates known and new compounds and combinations of biologically active agents. It is intended that combination therapies of the present invention include any chemically compatible combination of an bis-azole of this inventive group with other compounds of the inventive group or other compounds outside of the inventive group, as long as the combination does not eliminate the anti-viral activity of the compound of this inventive group or the anti-viral activity of the pharmaceutical composition itself.

Combination therapy can be sequential, that is treatment with one agent first and then a second agent (for example, where each treatment comprises a different compound of the present invention or where one treatment comprises a compound of the present invention and the other comprises one or more biologically active agents) or it can be treatment with both agents at the same time. Sequential therapy can include a reasonable time after the completion of the first therapy before beginning the second therapy. Treatment with both agents at the same time can be in the same daily dose or in separate doses. Combination therapy need not be limited to two agents and may include three or more agents. The dosages for both concurrent and sequential combination therapy will depend on absorption, distribution, metabolism and excretion rates of the components of the combination therapy as well as other factors known to one of skill in the art. Dosage values will also vary with the severity of the condition to be alleviated. For any particular subject specific dosage regimens and schedules may be adjusted over time according to the individual's need and the professional judgement of the person administering or superivising the administration of the combination therapy.

Although the foregoing invention has been described in some detail by way of illustration and example for purposes of clarity of understanding, it will be readily apparent to one of ordinary skill in the art in light of the teaching of this invention that certain changes and modifications may be made thereto without departing from the spirit or scope of the invention as defined in the appended claims.

### Best Embodiment of the invention

Below the invention is described by means of specific examples, which illustrate but not limit the scope of the invention.

### Example 1.

[(1*S*)-1-[[(2*S*)-2-[5-[4-[4-[2-[(2*S*)-1-[(2*S*)-2-[(Methoxycarbonyl)amino]-3-methyl-1-oxobutyl]-2-pyrrolidinyl]-1*H*-imidazol-5-yl]-1,3-butadiynyl]phenyl]-1*H*-imidazol-2-yl]-1-pyrrolidinyl]carbonyl]-2-methylpropyl]-carbamic acid methyl ester **14.**

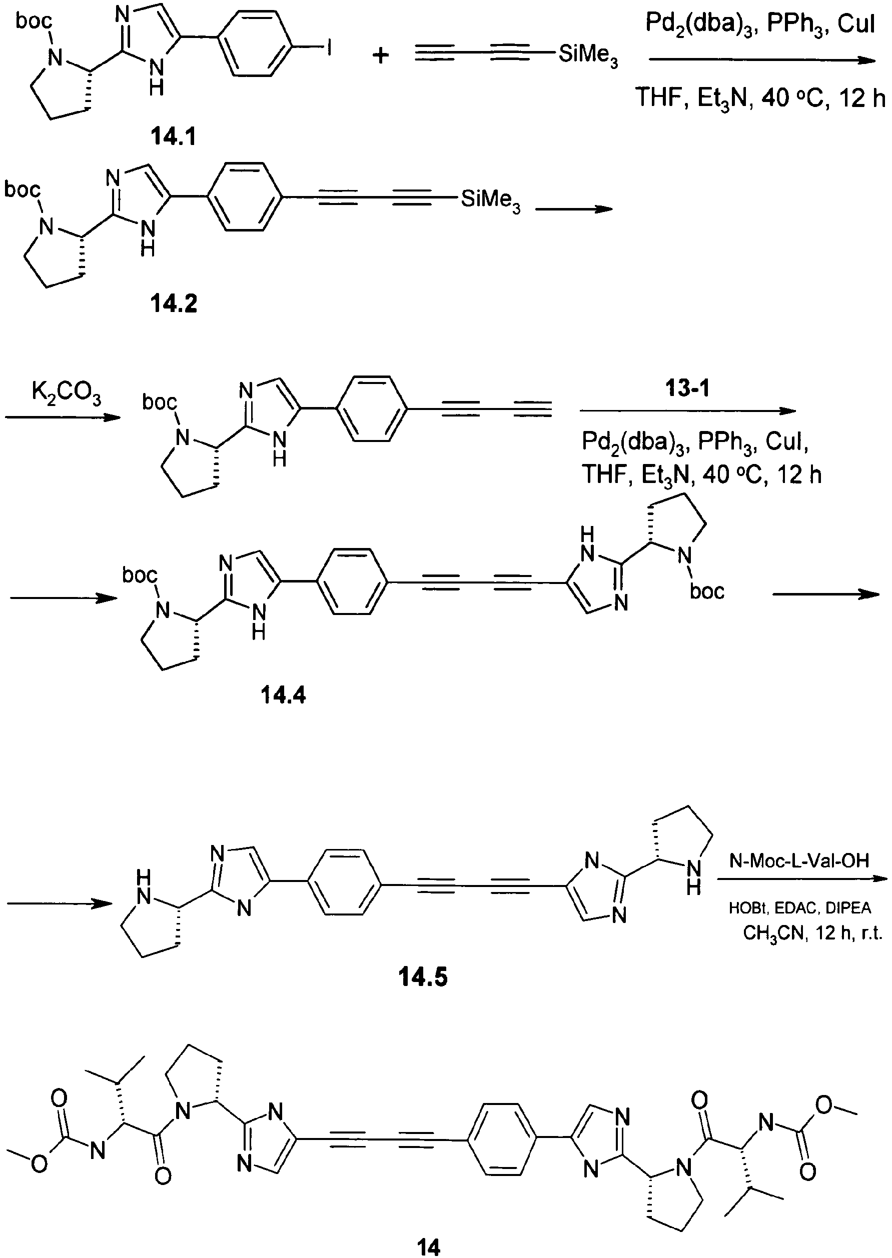

1,5 M MeLi × LiBr solution in ether (31 ml, 46.5 mmol) was added to solution of 1,4-bis-(trimethylsilyl)-1,3-butadiyne (8.15 g, 42 mmol) in dry ether (50 ml) under argon. The mixture was stirred at room temperature for 6 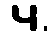 . Then, saturated NH₄Cl solution (50 ml) was slowly added to the mixture, extracted with pentane, dried over Na₂SO₄ and the solvents were evaporated in soft *vacuo.* Liquid residue was dissolved in THF (70 ml), then triethylamine (20 ml), compound **14.1** (8.8 g, 20 mmol), Pd₂(dba)₃ (458 mg, 0.5 mmol), triphenyl phosphine (524 mg, 2 mmol), CuI 190 mg (1 mmol) were added one after another and the resultant mixture was stirred for 12 h at 40°C under argon. The mixture was filtered through celit, applied to silica gel and compound **2** was isolated by flash-chromatography (eluent CHCl₃ : EtOAc = 10:1). Yield is 7.56 g (87 %). LCMS (M+H)⁺ 434.

K₂CO₃ (7.04 g, 51 mmol) was added to solution of compound **14.2** (7.36 g, 17 mmol) in THF (120 ml) and methnol (120 ml) and the resultant mixture was stirred for 2 h. The solvents were evaporated *in vacuo,* the residue was treated with THF (150 ml) and filtered. Compound **13-1** (5-iodo-2-[(2*S*)-1-*boc*-pyrrolidin-2-yl]-1H-imidazol) (5.56 g, 15.3 mmol), Pd₂(dba)₃ (366 mg, 0.4 mmol), triphenyl phosphine (630 mg, 2.4 mmol), and CuI (152 mg, 0.8 mmol) were subsequently added to the obtained solution of compound **14.3,** and the resultant mixture was stirred for 12 h at 40 °C under argon. Then, the reaction mixture was filtered through celit, applied to silica gel and compound **14.4** was separated from the main part of admixtures by flash-chromatography (eluent CHCl₃ : MeOH = 80:1). After evaporation of the solvent the residue was treated with acetonitrile (60 ml), kept in ultrasound-bath till the beginning of crystallisation and left for 3 h. The precipitated solid was filtered off, washed with acetonitrile, ether and dried *in vacuo.* Yield is 5.47 r (54 %). LCMS (M+H)⁺ 597. 3M HCl solution in dioxane (15 ml) was added to compound **14.4** (0.695 g) and stirred for 12 h. After that the mixture was evaporated *in vacuo,* it gave compound **14.5,** yield is 55%. LCMS (ESI): LCMS (M+H)⁺ 397. ¹H NMR (DMSO-d₆, 400 MHz) δ 2.02 (br.s., 8H), 2.99 (m, 4H), 3.40 (br.s., 2H), 6.22 (m, 4H), 6.75 (s, 1 H), 7.22 (s, 1 H), 7.77 (m, 2H), 7.83 (d, 2H). Mixture of N-methoxycarbonyl-L-valine (50 mg, 0.283 mmol, 2.4 eq.), 1-hydroxybenzotriazole (40 mg, 0.295 mmol, 2.5 eq.) and N-(3-dimethylaminopropyl)-N'-ethylcarbodiimide hydrochloride (53 mg, 0.277 mmol, 2.35 eq.) in acetonitrile (1 ml) was stirred for 1 h, then compound **14.5** (64 mg, 0.118 mmol, 1eq.) and 82 mkl (61 mg, 0.472 mmol, 4 eq.) diisopropylamine were added. The reaction mixture was stirred for 12 h at room temperature. Completeness of the reaction was controlled by LCMS method. After the reaction was completed the solvent was evaporated to dryness on rotary evaporator, the residue was dissolved in dichloromethane. Extract was washed with 10% Na₂CO₃ solution, dried over Na₂SO₄ and evaporated on rotary evaporator. Further purification was carried out by HPLC method. Dihydrochloride

**14·2HCl** was prepared by addition of excess of 3M HCl solution in dioxane to a solution of **14** base in CH₂Cl₂ and precipitation with ether. It gave 56 mg (67 %) of compound **14·2HCl.** LCMS (M+1)⁺ 711. ¹H NMR (DMSO-*d*₆, 400 MHz) δ 0.87 (t, J₁ = 6.50, J₂ = 0.93, 6H), 0.97 (t, J₁ = 6.50, J₂ = 0.93, 6H), 1.64 (m, 2H), 1.84 (m, 2H), 1.99 (m, 4H), 2.06 (m, 2H), 3.56 (m, 2H), 3.63 (m, 8H), 4.02 (d, J = 0.42, 2H), 4.73 (m, 2H), 6.86 (s, 1 H), 7.33 (s, 1 H), 7.76 (d, J = 8.26, 2H), 7.90 (m, 2H), 8.80 (m, 4H).

### Example 2.

[[1,1'-*trans*,*trans*-Bicyclohexyl]-4,4'-diylbis[4,2-oxazoldiyl(2*S*)-2,1-pyrrolidinyl[(1*S*)-1-(1-methylethyl)-2-oxo-2,1-ethanediyl]]]bis-carbamic acid dimethyl ester **20.**

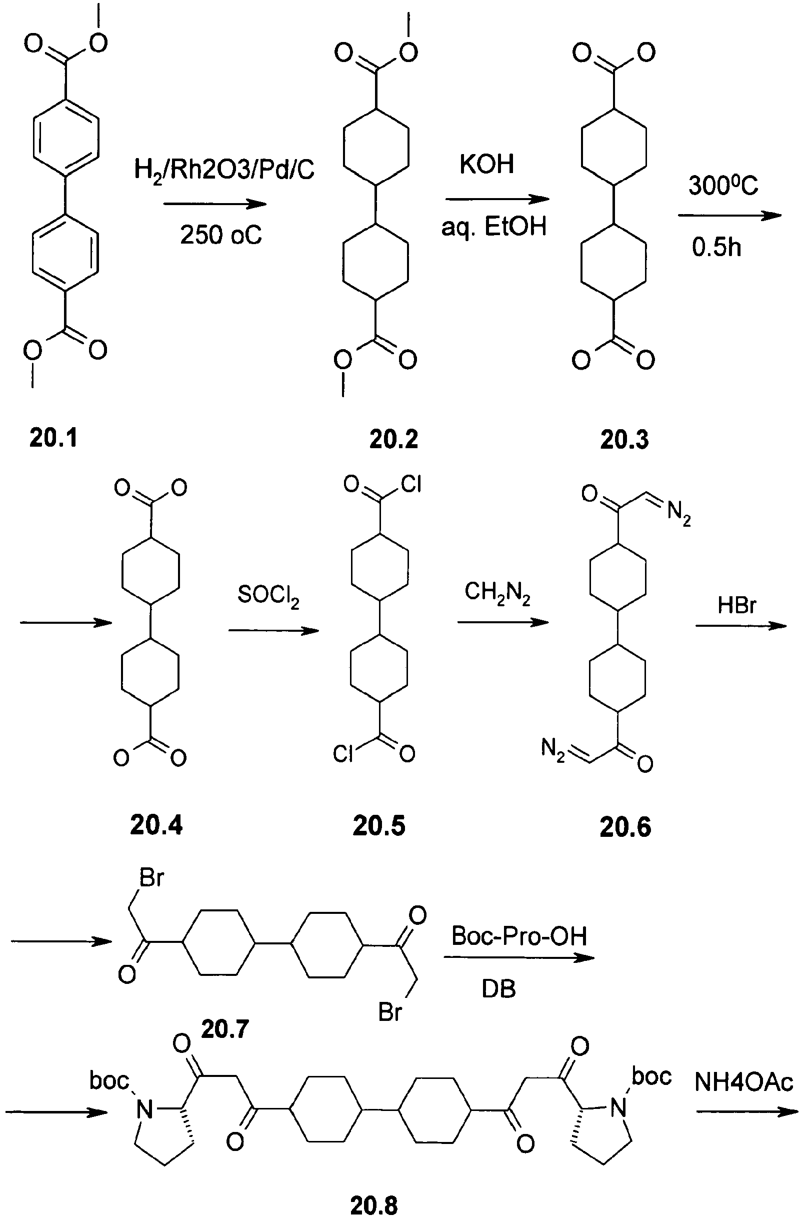

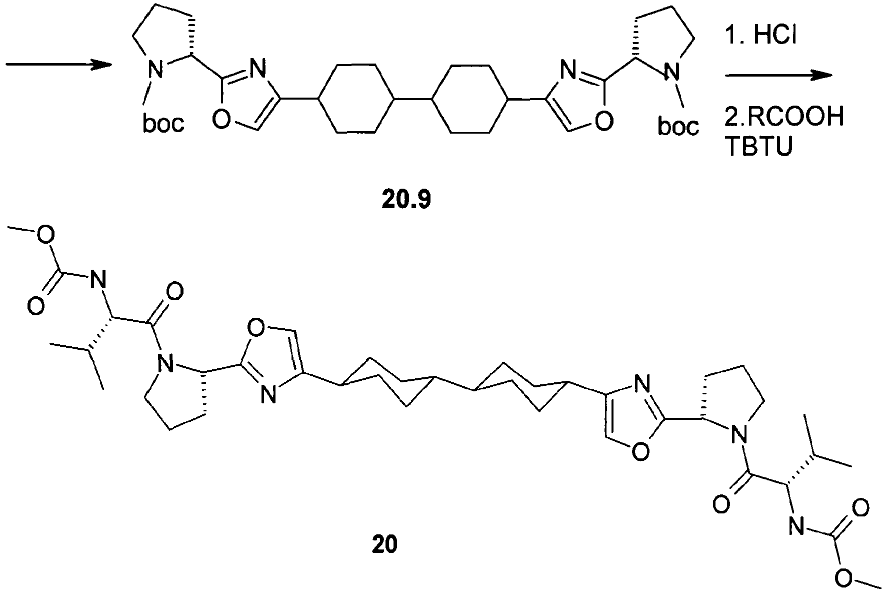

4,4'-Dimethyl biphenyl-4,4'-dicarboxylate **20.1** (9 g, 33 mmol) was hydrogenated in AcOH (100 ml) in the presence of 10 % Pd/C (3 g) and Rh₂O₃ (0.15 g) at 250 °C for 16 h and pressure of 60-20 atm H₂. After that the mixture was filtered off, evaporated at reduced pressure, excess of 10 % K₂CO₃ solution in water was added and the mixture was extracted with CHCl₃. Organic layer was evaporated, it gave 9 g (96%) of compound **20.2.** ¹H NMR (CDCl₃): 3.67(s); 3.65 (s) (total 6H, 2Me) 2.56 (1H, br.s) 2.20 (br.t., 1H) 2.0 (m, 4H) 1.80 (br.t, 2H) 1.5-1.0 (m, 12H). Compound **20.2** (9 g, 31 mmol) was boiled in EtOH (100 ml) and H₂O (20 ml) in the presence of 50 % KOH (9 g, 80 mmol) water solution for 30 min. The solution was evaporated, the residue was dissolved in water (0.5 l) and excess of HCl was added. The precipitated solid was filtered off, it gave di-acid **20.3** 7.2 g (91 %). ¹H NMR (DMSO- *d*₆): 2.44 (1H, br.s) 2.05 (br.t., 1 H) 1.88 (m, 4H) 1.68 (m, 2H) 1.43 (m, 4H) 1.18 (m, 4H) 0.98 (m, 4H). The acid **20.3** (7.2 g, 28 mmol) was heated at stirring at 300 °C for 0.5 h under Ar. Sublimate and the residue were dissolved in hot 20 % KOH solution (50 ml) and stirred with 1 g of activated carbon. Solution was filtered, diluted with water (0.5 l) and treated with excess of HCl.

The precipitate was filtered of, it gave *trans*,*trans*-dicarboxylic acid **20.4** 5 g (69 %) . ¹H NMR 12.0 (br.s, 2OH) 2.50 (t, 2H, ³*J* 1.8 *Hz)* 2.07 (t.t., 2H, ³*J 12 Hz, ³J 1.8 Hz*) 1.95-1.85 (m, **4H**) 1.75-1.65 (m, **4H)** 1.30-1.20 (m, 4H) 1.00-0.90 (m, **4H**). *Trans,trans*-dicarboxylic acid **20.4** (4.3 g, 17 mmol) was heated with SOCl₂ (50 ml) in the presence of DMF (0.5 ml) for 2 h. Solution was evaporated, it gave crude solid diacyl chloride **20.5** (NMR LDA-2450), which was used in the next stage without additional purification. The solution of the prepared compound **20.5** in ether was slowly added to the solution of diazomethane (136 mmol) in Et₂O at 0-10 °C. The mixture was stirred for 10 h, precipitated solid was filtered off and dried in the air. NMR LDA-2260, yield is 5.4 g (42 %) of pure *trans*,*trans*-compound **20.6.** ¹H NMR LDA-2260 (CDCl₃) 5.24 (br.s, 2H) 2.16 (br.m. 2H), 1.92-1.80 (m, 8H) 1.40 (m, 4H) 1.05 (m, 6H). Bis-diazoketone **20.6** (1.3 g, 4.3 mmol) was dissolved in DCM (50 ml) and 40% HBr solution in acetic acid (1 ml) was added. After the effervescence was completed the solution was additionally stirred for 30 min, then powdered K₂CO₃ (10 g) was added and mixture was stirred for additional 30 min; K₂CO₃ excess was filtered of, filtrate was evaporated, the residue was recrystallized from heptane. Yield of compound **20.7** is 1.5 g (85%). ¹H NMR (CDCl₃) LDA-2459. 3.97 (s, 4H, 2CH2Br), 2.65 (t.t., 2H, *³J* 12 *Hz ³J* 3 *Hz)* 2.0-1.8 (m, 8H) 1.3-1.4 (m, 4H) 1.1-1.0 (m, 6H). DIPEA (460 mg, 3.6 mmol) was added at srirring to solution of N-boc-(S)-proline-OH (765 mg, 3.6 mmol) in MeCN (30 ml), and in 5 min compound **20.7** (1.8 mmol) was added. The solution was refluxed by night, cooled, solvent was evaporated, the residue was dissolved in DCM (50 ml) and washed successively with 0.1 N HCl solution (2x20 ml) and saturated NaHCO₃/NaCl solution (20 ml). Yield of compound **20.8** is 1.4 g (95%). NH₄OAc (10 g) was added to solution of compound **20.8** (1.7 mmol) in toluene (50 ml). The mixture was refluxed at stirring for 24 h, then organic layer was evaporated, it gave 1.08 g (95 %) of compound **20.9.** LCMS (M+1) 639. Compound **20.9** (1.6 mmol) was dissolved in 5 M HCl solution in dioxane (50 ml) and stirred at 40°C for 1h. Then the solution was evaporated to dryness, the residue was extracted with CHCl₃ (50 ml), extracts were successively washed with 10 % K₂CO₃ (50 ml) solution, dried over K₂CO₃ and evaporated again to dryness. DIPEA (1.02 g, 7.9 mmol), N-methoxycarbonyl-(5)-valine-OH (1.04 g, 5.9 mmol) and TBTU (1.92 g, 5.9 mmol) were successively added to the residue dissolved in DCM (50 ml). The mixture was stirred for 16 h, then washed with 0.1 N HCl solution (2 x 50 ml) and 10 % K₂CO₃ (2 x 50 ml) solution, the organic layer was evaporated and subjected to RP HPLC. It gave compound **20.** Dihydrochloride **20·2HCl** was prepared by the addition of excess of 3M HCl solution in dioxane to solution of base **20** in CH₂Cl₂ and precipitation with ether. LCMS (M+1) 753. ¹H NMR (CDCl₃) 0.73 (d, 3H, Me), 0.87 (d, 6H, 2Me), 1.06 (d, 3H, Me), 1.4-2.7 (m, 32H), 3.64 (m, 1H), 3.69 (s, 3H, OMe), 3.75 (s, 3H, OMe), 3.80 (m, 3H), 4.34 (m, 1H), 5.24 (m, 1 H), 5.42 (m, 1H), 7.12 (s, 1H), 7.30 (s, 1H).

### Example 3.

[(S)-1-((S)-2-{5-[5-(6-{2-[(S)-1-((S)-2-Methoxycarbonylamino-3-methyl-butyryl)-pyrrolidin-2-yl]-3H-imidazol-4-yl}-naphthalen-2-yl)-thiophen-2-yl]-1H-imidazol-2-yl}-pyrrolidine-1-carbonyl)-2-methylpropyl]-carbamic acid methyl ester **24.**

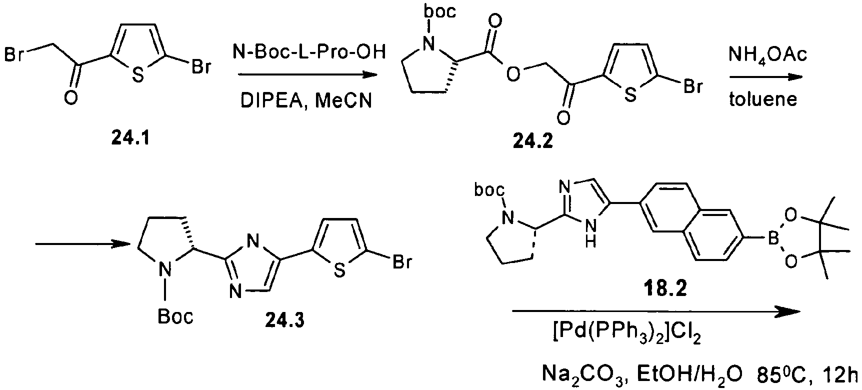

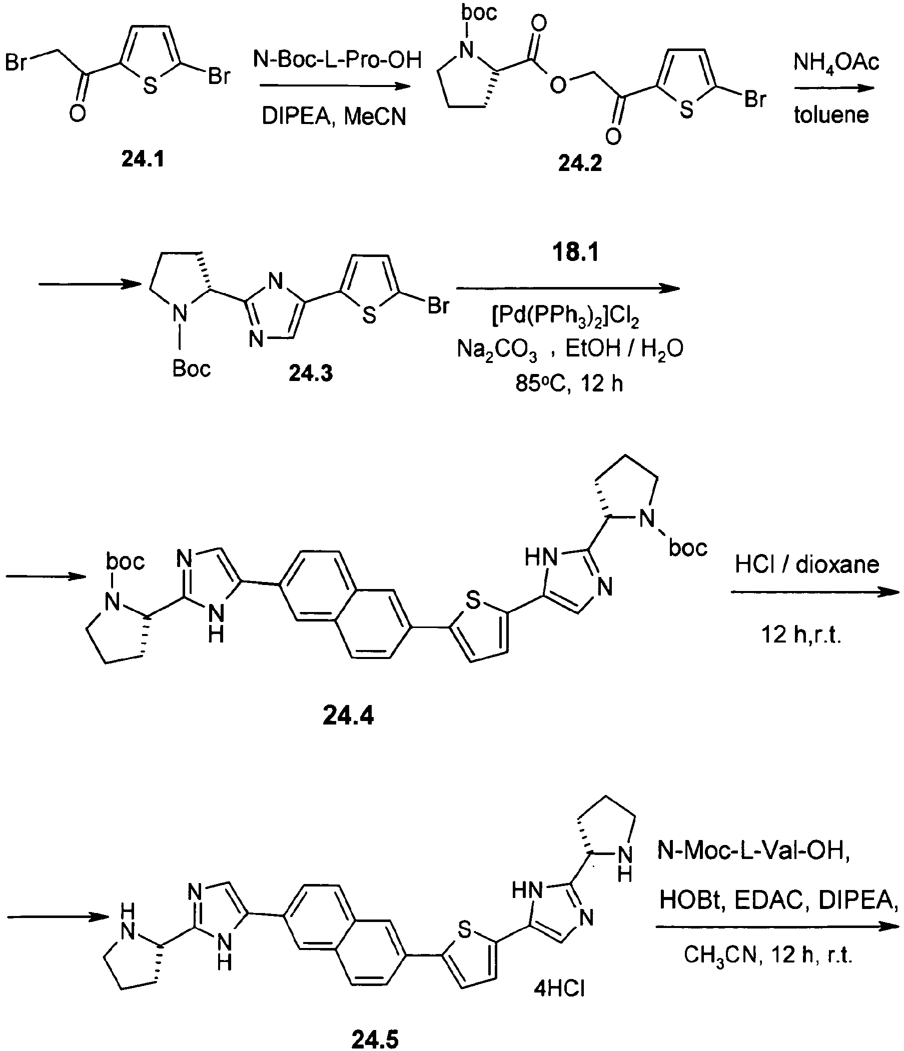

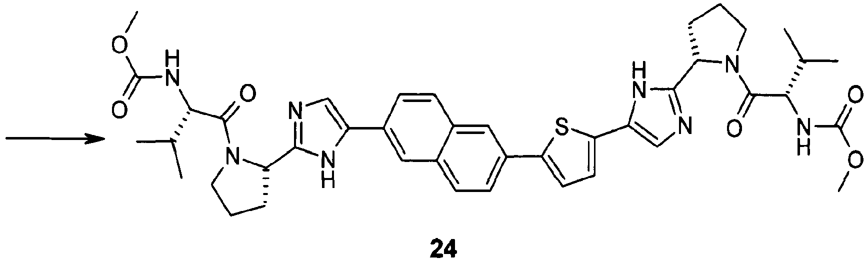

Diisopropylethylamine (4.3 ml, 24.8 mmol) was added to mixture of compound **24.1** (6.87 g, 24.2 mmol) and N-boc-L-proline (5.47g, 25.4 mmol) in acetonitrile (40 ml). The mixture was stirred for 3 h at room temperature, acetonitrile was evaporated *in vacuo,* the residue was diluted with toluene (100 ml), washed with saturated saline solution, 5 % NaHCO₃ solution, dried over Na₂SO₄ and evaporated *in vacuo.* AcONH₄ (8.5 g, 0.24 mol) was added to the solution of the prepared ester **24.2** dissolved in toluene (75 ml) and stirred at 100 °C for 18 h. Then the mixture was washed with water, dried over Na₂SO₄, applied to SiO₂ and compound **24.3** was isolated by colomn chromatography (eluent hexane-ethyl acetate = 4:1). Yield of compound **24.3** is 4.76 g (49 %). LCMS (ESI): m/z 398.1, 400.0 (M+H)⁺. ¹H NMR (CDCl₃, 400 MHz) δ 10.80 (br. s, 0.15H), 10.52 (br. s, 0.75H), 7.09 (d, *J* = 1.2 *Hz*, 1 H), 6.96 (m, 2H), 4.93 (m, 1 H), 3.40 (br. m, 2H), 3.00 (br. m, 1 H), 2.13 (m, 2H), 1.96 (m, 1H), 1.50 (s, 9H). Mixture of boronic ester **18.2** (0.613 mmol, 1.1 eq.) and Na₂CO₃ (1.40 mmol, 2.5 eq.) in ethanol (6 ml) and water (1.4 ml) was blown through with argon, then compound **24.3** (203 mg, 0.56 mmol, 1 eq.) and Pd(PPh₃)₂Cl₂ (20 mg, 0.028 mmol, 0.05 eq.) were added, and the resultant mixture was stirred for 12 h at 85°C under argon. Completeness of the reaction was controlled by LCMS method. After the reaction was over, the reaction mixture was filtered through celit and evaporated on rotary evaporator. The residue was dissolved in CH₂Cl₂, washed with water, dried over Na₂SO₄ and evaporated on rotary evaporator. Compound **24.4** was isolated by colomn chromatography (eluent - toluene:ethyl acetate =2:1), purity 85%. Further purufucation was carried out by HPLC method. It gave compound **24.4** (174 mg, 41 %) LCMS (ESI): *m*/*z* 681.4 (M+H)⁺, which was boc-deprotected with 3M HCl solution in dioxane. It gave compound **24.5** as tetrahydrochloride, yield is 68%. LCMS (ESI): *m*/*z* 481.4 (M+H)⁺. ¹H NMR (DMSO-d₆, 400 MHz) δ 10.40 (br. s, 1 H), 10.30 (br. s, 1 H), 9.96 (br. s, 0.8 H), 9.49 (br.s, 0.8H), 8.53 (s, 1 H), 8.23 (d, *J* = 13.6 Hz, 2H), 8.07 (m, 2H), 7.95 (m, 2H), 7.83 (s, 1H), 7.69(s, 1H), 7.56 (s, 1H), 5.07 (br. m, 1H), 4.86 (br. m, 1 H), 3.76 (br. m, 1 H), 3.48 (br. m, 1 H), 3.38 (br. m, 2H), 2.41 (br.m, 2H), 2.34 (br. m, 2H), 2.21 (br. m, 2H), 2.02 (br. m, 2H). The prepared compound **24.5** was converted to compound **24** with the yield of 53 %, by analogy with the synthesis of compound **14** from compound **14.5.** Dihydrochloride **24**·**2HCl** was prepared by addition of excess of 3M HCl solution in dioxane to the solution of **24** base in CH₂Cl₂ and precipitation with ether. LCMS (ESI): *m*/*z* 795.8 (M+H)⁺. ¹H NMR (DMSO-*d*₆, 400 MHz) δ 15.29 (br. s, 1 H), 14.76 (br. s, 1.5H), 8.49 (s, 1H), 8.21 (d, *J* = 16.0 *Hz,* 2H), 8.10 (d, *J* = 8.8 *Hz,* 1 H), 7.99 (m, 2H), 7.93 (d, *J* = 8.8 Hz, 1 H), 7.88 (br. s, 1 H), 7.74 (s, 1 H), 7.71 (br. s, 1H), 5.22 (t, *J =* 6.4 *Hz,* 1 H), 5.13 (t, *J =* 6.4 *Hz,* 1 H), 4.13 (m, 2H), 3.97 (m, 2H), 3.84 (br. m, 2H), 3.54 (s, 6H), 2.36 (m, 2H), 2.19 (m, 4H), 2.06 (m, 4H), 0.92 (m, 1.6H), 0.86 (d, *J* = 6.8 *Hz,* 5.2H), 0.79 d, *J* = 6.8 *Hz,* 5.2H).

### Example 4.

[(S)-1-((S)-2-{5-[5-(4-{2-[(S)-1-((S)-2-Methoxycarbonylamino-3-methyl-butyryl)-pyrrolidin-2-yl]-3H-imidazol-4-yl}-phenylethynyl)-thiophen-2-yl]-1H-imidazol-2-yl}-pyrrolidine-1-carbonyl)-2-methylpropyl]-carbamic acid methyl ester **25.**

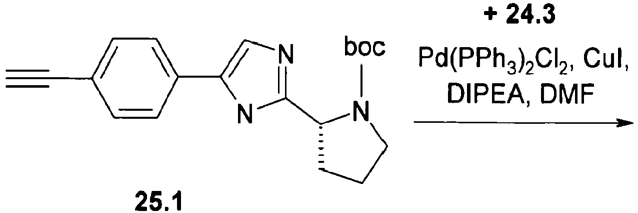

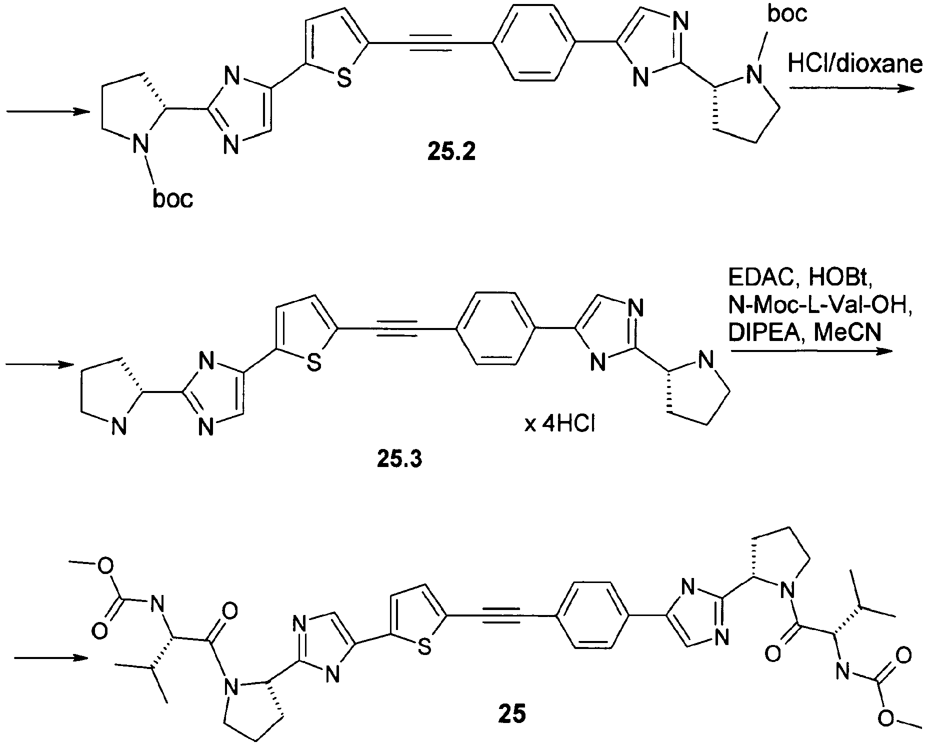

Diisopropylethylamine (0.15 ml, 0.87 mmol) and Pd(PPh₃)Cl₂ (31 mg, 0.09 mmol) were added to mixture of compound **24.3** (348 mg, 1 mmol), compound **25.1** (295 mg, 0.87 mmol) and CuI (17 mg, 0.09 mmol) in DMF (3 ml) under argon, and the resultant mixture was stirred at 80 °C for 12 h. After the reaction was completed the mixture was diluted with CHCl₃, washed with water, dried over Na₂SO₄ and evaporated *in vacuo.* Compound **25.2** was isolated by HPLC method. Yield is 173 mg (30 %). LCMS (ESI): *m*/*z* 655.3 (M+H)⁺. Compound **25.2** was boc-deprotected with 3M HCl solution in dioxane with quantitative yield, it gave compound **25.3** (LC-MS (ESI): *m*/*z* 455.6 (M+H)⁺), which was converted into compound **25** with 41 % yield by analogy with the conversion of compound **14.5** into compound **14.** LCMS (ESI): *m*/*z* 769.7 (M+H)⁺. Dihydrochloride of **25** was prepared by addition of excess of 3M HCl solution in dioxane to solution of **25** base in CH₂Cl₂ and precipitation with ether. ¹H NMR (DMSO-d₆, 400 MHz) δ 15.11 (br. s, 0.95H), 14.69 (br. s, 1.25H), 8.15 (s, 1 H), 7.93 (d, *J =* 8.0 Hz, 2H), 7.87 (br. s, 1 H), 7.71 (d, *J* = 8.0 *Hz,* 2H), 7.58 (br. s, 1 H), 7.45 (d, *J* = 2.8 *Hz,* 1 H), 7.27 (m, 2H), 5.16 (t, *J* = 6.8 Hz, 1 H), 5.10 (t, *J =* 6.6 Hz, 1H), 4.11 (m, 2H), 3.92 (m, 2H), 3.83 (m, 2H), 3.56 (s, 0.1 H), 3.54 (s, 5.9H), 2.34 (m, 2H), 2.16 (m, 4H), 2.03 (m, 4H), 0.90 (m, 1.2H), 0.84 (t, *J* = 7.2 *Hz,* 5.4H), 0.77 (t, *J* = 7.2 *Hz,* 5.4H).

### Example 5.

((S)-1-{(S)-2-[5-(5'-{2-[(S)-1-((S)-2-Methoxycarbonylamino-3-methyl-butyryl)-pyrrolidin-2-yl]-3H-imidazol-4-yl}-[2,2']bithiophen-5-yl)-1H-imidazol-2-yl]-pyrrolidine-1-carbonyl}-2-methylpropyl)-carbamic acid methyl ester **30.**

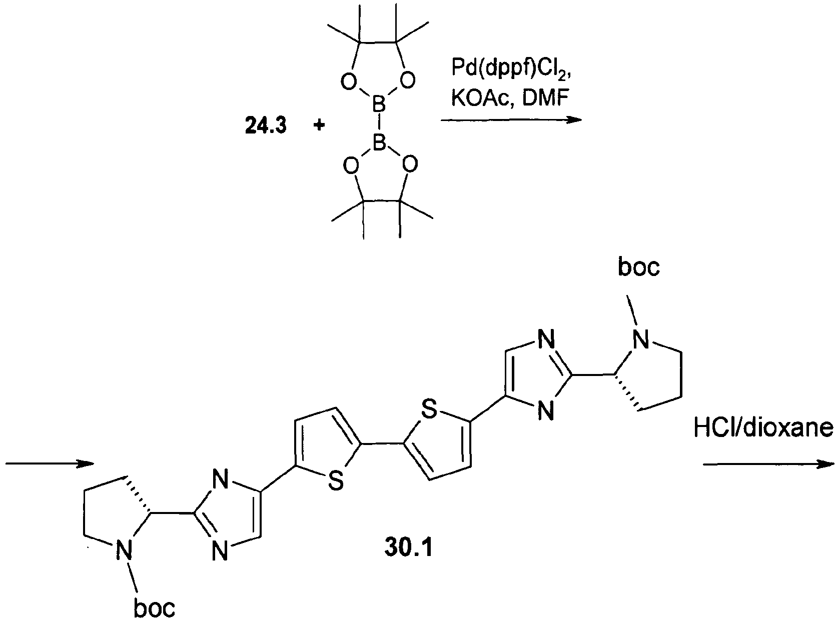

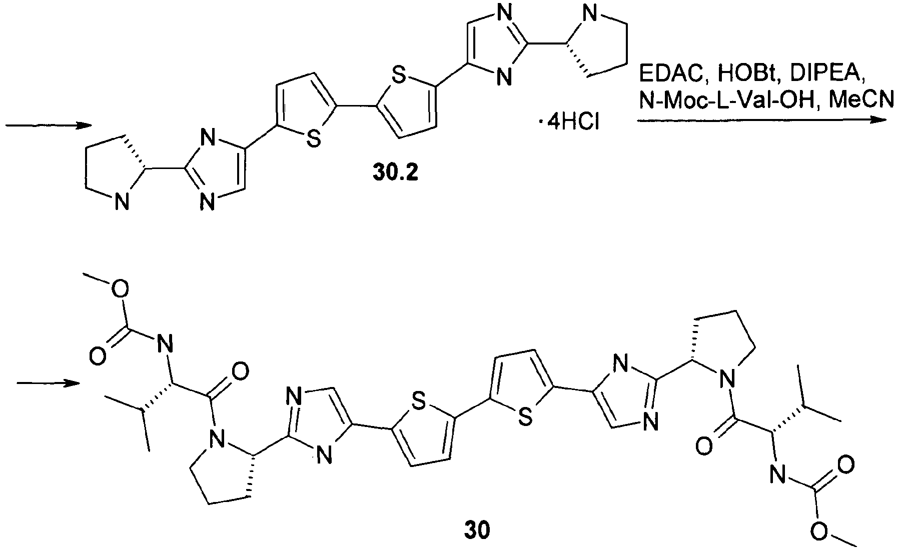

Pd(dppf)Cl₂·CH₂Cl₂ (75 mg, 0.09 mmol) was added to mixture of compound **24.3** (1.194 g, 3 mmol), bis(pinacolato)diborone (840 mg, 3.3 mmol) and AcOK (882 mg, 9 mmol) in DMF (9 ml) under argon and the resultant mixture was stirred at 90 °C for 12 h. After the reaction was completed the mixture was diluted with CHCl₃, washed with water, dried over Na₂SO₄, applied to SiO₂, and compound **29.1** was isolated by colomn chromatography (eluent hexane-ethyl acetate = 1 : 2). Yield is 324 mg (34 %). LCMS (ESI): *m*/*z* 637.5 (M+H)⁺. The prepared compound **30.1** was boc-deprotected with 3M HCl solution in dioxane with quantitative yield, giving compound **30.2.** LCMS (ESI): *m*/*z* 437.3 (M+H)⁺. ¹H NMR (DMSO-*d*₆, 400 MHz) δ 10.31 (br. s, 2H), 9.50 (br. s, 2H), 7.81 (s, 2H), 7.47 (d, *J* = 3.2 *Hz,* 2H), 7.31 (d. d, *J*₁ = 4.0 *Hz, J*₂ = 0.8 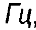 , 2H), 4.85 (br. m, 2H), 3.35 (m, 4H), 2.41 (m, 2H), 2.33 (m, 2H), 2.15 (m, 2H), 2.00 (m, 2H). Compound **30.2** was converted to compound **30** by analogy with the conversion of compound **14.5** to compound **14.** Compound **30** was prepared with yield 77 %. LCMS (ESI): *m*/*z* 751.8 (M+H)⁺. Dihydrochloride **30** was prepared by the addition of excess of 3M HCl solution in dioxane to the solution of base **30** in CH₂Cl₂ and precipitation with ether. ¹H NMR (DMSO-*d*₆, 400 MHz) δ 14.42 (br. s, 0.8H), 7.91 (s, 2H), 7.91 (s, 2H), 7.63 (s, 2H), 7.41 (d, *J* = 3.6 *Hz,* 2H), 7.27 (d, *J* = 8.4 *Hz,* 2H), 5.10 (t, *J* = 7.0 *Hz,* 2H), 4.10 (t, *J* = 7.8 *Hz,* 2H), 3.92 (m, 2H), 3.83 (m, 2H), 3.53 (s, 6H), 2.34 (m, 2H), 2.15 (m, 4H), 2.02 (m, 4H), 0.89 (m, 1.2H), 0.83 (d, *J* = 6.8 Hz, 5.4H), 0.77 (d, *J* = 6.8 Hz, 5.4H).

### Example 6.

{(S)-1-[(S)-2-(5-{5-[4-(5-{2-[(S)-1-((S)-2-Methoxycarbonylamino-3-methyl-butyryl)-pyrrolidin-2-yl]-3H-imidazol-4-yl}-thiophen-2-yl)-phenyl]-thiophen-2-yl}-1H-imidazol-2-yl)-pyrrolidine-1-carbonyl]-2-methyl-propyl}-carbamic acid methyl ester **35.**

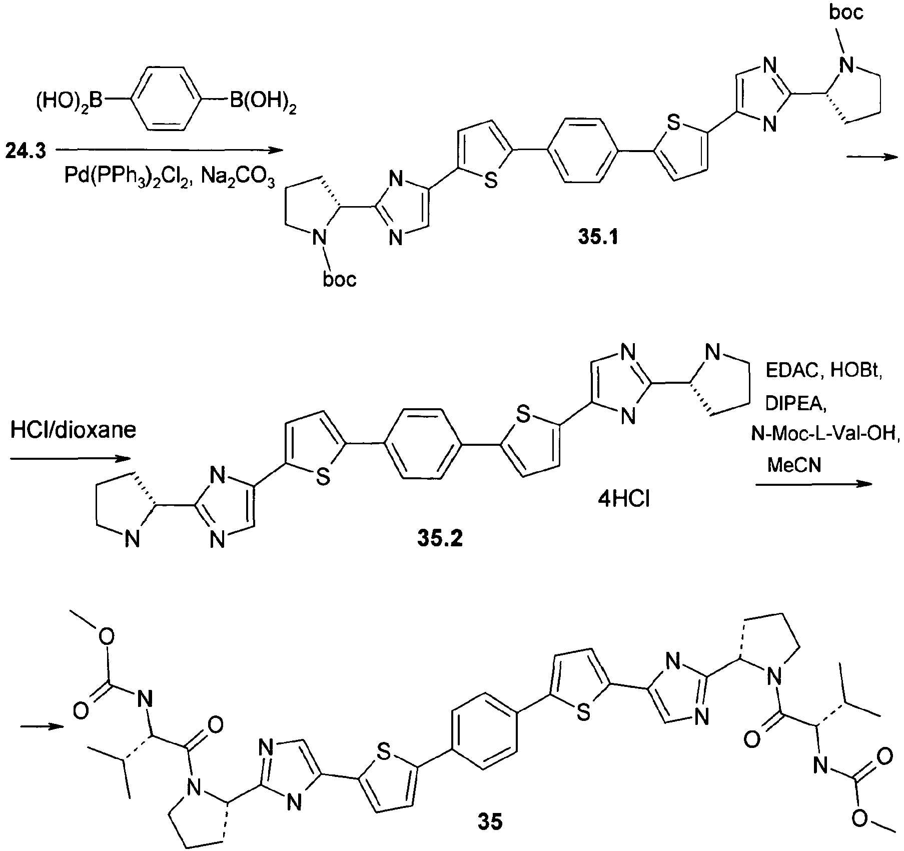

Mixture of compound **24.3** (598 mg, 1.5 mmol), benzene-1,4-diboronic (124 mg, 0.75 mmol) and Na₂CO₃ (320 mg, 3 mmol) in alcohol (4 ml) and water (0.35 ml) was blown through with argon. Pd(PPh₃)₂Cl₂ (105 mg, 0.15 mmol) was added and the resultant mixture was stirred in a closed vial at 80 °C for 12 h under argon. After the reaction was completed the mixture was diluted with CHCl₃, washed with water, dried over Na₂SO₄, the solvent was evaporated and the residue was applied to SiO₂, compound **35.1** was isolated by colomn chromatography (eluent hexane-ethyl acetate = 1 : 2). Yield is 318 mg (59 %). LC-MS (ESI): *m*/*z* 713.7 (M+H)⁺. 3M HCl solution in dioxane (3 ml) was added to compound **35.1** (310 mg) and stirred for 12 h. Mixture was evaporated *in vacuo,* it gave compound **35.2.** with quantitative yield. LCMS (ESI): *m*/*z* 513.5 (M+H)⁺. ¹H NMR (DMSO-*d*₆, 400 MHz) δ 10.32 (br. s, 2H), 9.56 (br. s, 2H), 7.83 (s, 2H), 7.72 (s, 4H), 7.58 (br. m, 2H), 7.55 (br. m, 2H), 4.88 (br. m, 2H), 3.37 (m, 4H), 2.40 (m, 4H), 2.17 (m, 2H), 2.01 (m, 2H). Mixture of *N*-(methoxycarbonyl)-L-valine (46 mg, 0.26 mmol), 1-hydroxybenzotriazole (37 mg, 0.27 mmol) and *N*-(3-dimethylaminopropyl)-*N*'-ethylcarbodiimide hydrochloride (49 mg, 0.26 mmol) in acetonitrile (1 ml) was stirred for 1 h, then compound **35.2** (70 mg, 0.11mmol) and diisopropylethylamine (76 mkl, 0.44 mmol) were added. Mixture was stirred for 12 h at room temperature, evaporated to dryness *in vacuo,* compound **35** was isolated by HPLC method. Yield is 59 mg (67 %). LCMS (ESI): *m*/*z* 827.8 (M+H)⁺. Dihydrochloride **35** was prepared by addition of excess of 3M HCl solution in dioxane to solution of base **6** in CH₂Cl₂ and precipitation with ether. ¹H NMR (DMSO-*d*₆, 400 MHz) δ 14.40 (br. s, 0.6H), 7.75 (s, 4H), 7.65 (br. m, 4H), 7.28 (d, *J* = *8.0 Hz,* 2H), 5.11 (t, *J* = *6.8 Hz,* 2H), 4.11 (t, *J = 7.8 Hz,* 2H), 3.92 (m, 2H), 3.83 (br. m, 2H), 3.54 (s, 6H), 2.34 (m, 2H), 2.15 (m, 4H), 2.03 (m, 4H), 0.90 (m, 1.1 H), 0.84 (d, *J = 6.4 Hz,* 5.45H), 0.78 (d, *J* = *6.4 Hz,* 5.45H).

### Example 7.

1,4-Bis-{5-[5-{(S)-1-[(S)-2-(cyclopropanecarbonyl-amino)-2-phenyl-acetyl]-pyrrolidin-2-yl}-3H-imidazol-4-yl]-thiophen-2-yl}-benzene **36.**

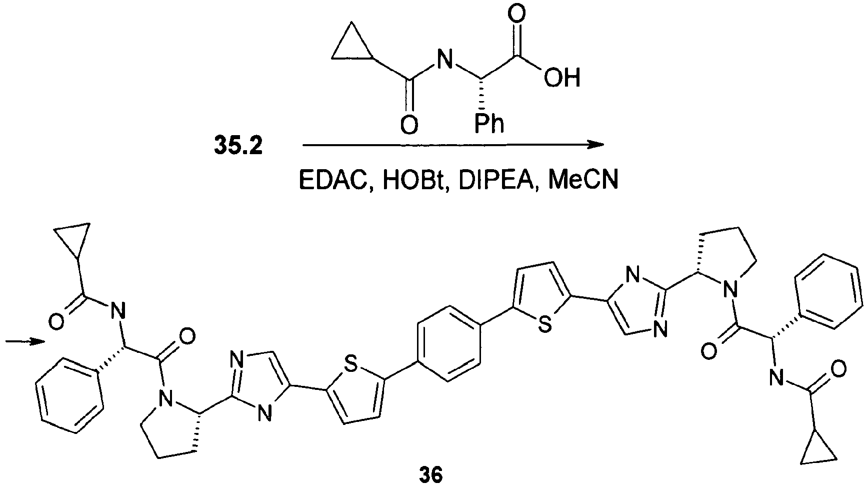

Compound **36** was prepared by analogy with the conversion of compound **14.5** to compound **14,** but *N*-cyclopropionyl-L-phenylglycine was used as an acid. LCMS (ESI): *m*/*z* 915.7 (M+H)⁺. Dihydrochloride **36** was prepared by addition of excess of 3M HCl solution in dioxane to solution of base **36** in CH₂Cl₂ and precipitation with ether.

**Example 8.** [(S)-1-((S)-2-{5-[5-(5-{2-[(S)-1-((S)-2-Methoxycarbonylamino-3-methyl-butyryl)-pyrrolidin-2-yl]-3H-imidazol-4-yl}-naphthalen-1-yl)-thiophen-2-yl]-1H-imidazol-2-yl}-pyrrolidine-1-carbonyl)-2-methyl-propyl]-carbamic acid methyl ester **37.**

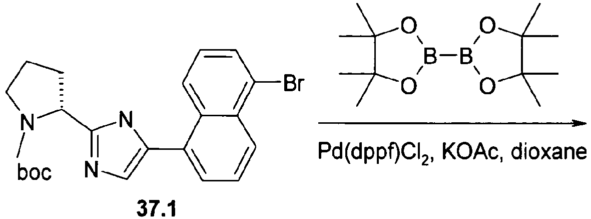

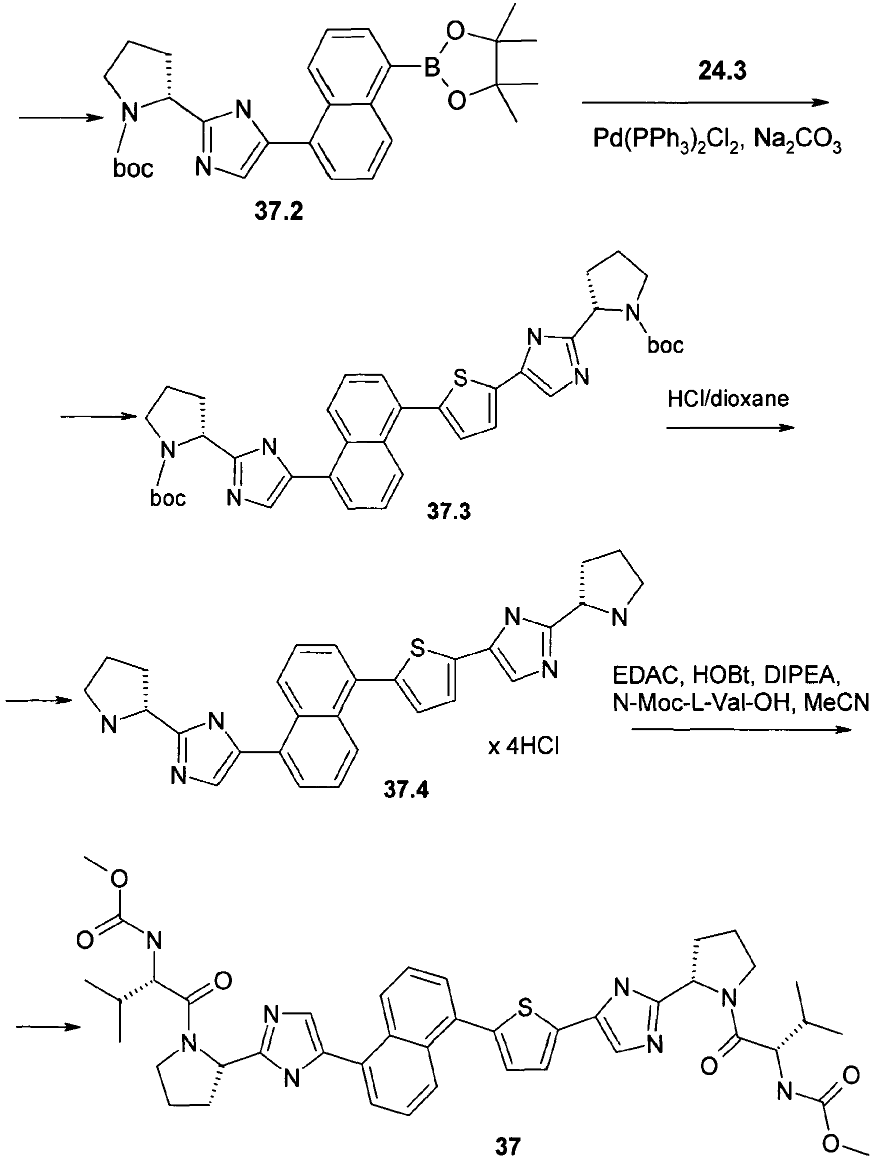

Mixture of compound **37.1** (442 mg, 1 mmol), bis(pinacolato)diborone (279 mg, 1.1 mmol) and AcOK (294 mg, 3 mmol) in dioxane (4 ml) was blown through with argon, then Pd(dppf)Cl₂·CH₂Cl₂ (24 mg, 0.03 mmol) was added and the resultant mixture was stirred at 85 °C for 24 h under argon. After the reaction was completed the mixture was diluted with CHCl₃, washed with water, dried over Na₂SO₄ and evaporated *in vacuo.* Compound **37.2** was isolated by HPLC method. Yield is 250 mg (51 %). LCMS (ESI): *m*/*z* 490.8 (M+H)⁺. Mixture of compound **37.2** (225 mg, 0.46 mmol), compound **24.3** (182 mg, 0.46 mmol) and Na₂CO₃ (98 mg, 0.92 mmol) in alcohol (4 ml) and water (0.35 ml) was blown through with argon. Pd(PPh₃)₂Cl₂ (32 mg, 0.05 mmol) was added and the resultant mixture was stirred in a closed vial at 80 °C for 12 h under argon. After the reaction was completed the mixture was diluted with CHCl₃, washed with water, dried over Na₂SO₄, applied to SiO₂ and compound **37.3** was isolated by colomn chromatography (eluent hexane-ethyl acetate-Et₃N = 1 : 1 : 0.05). Yield of compound **37.3** is 257 mg (82 %, LCMS (ESI): *m*/*z* 681.5 (M+H)⁺), which was boc-deprotected with 3M HCl solution in dioxane, compound **37.4** was prepared with quantitative yield (LCMS (ESI): *m*/*z* 481.3 (M+H)⁺), it was converted to compound **37** (LCMS (ESI): *m*/*z* 795.7 (M+H)⁺) by analogy with the conversion of compound **14.5** to compound **14.** Dihydrochloride **37** was prepared by the addition of an excess of 3M HCl solution in dioxane to solution of base **37** in CH₂Cl₂ and precipitation with ether.

### Example 9

Preparation of [(1S)-1-[[(2S)-2-[4-[4-[2-[(2S)-1-[(2S)-2-[(methoxycarbonyl)amino]-3-methyl-1-oxobutyl]-2-pyrrolidinyl]-1*H*-imidazol-5-yl]phenyl]cyclohexyl]-2-oxazolyl]-1-pyrrolidinyl]carbonyl]-2-methylpropyl]-carbamic acid methyl ester **38.**

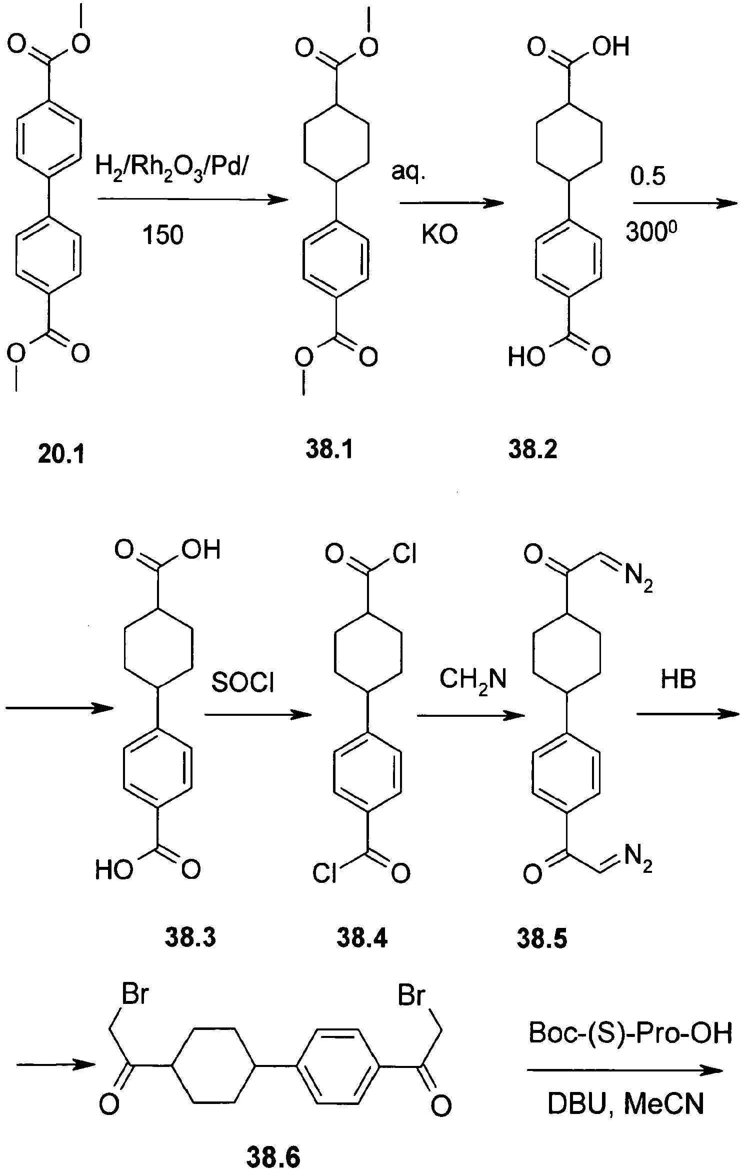

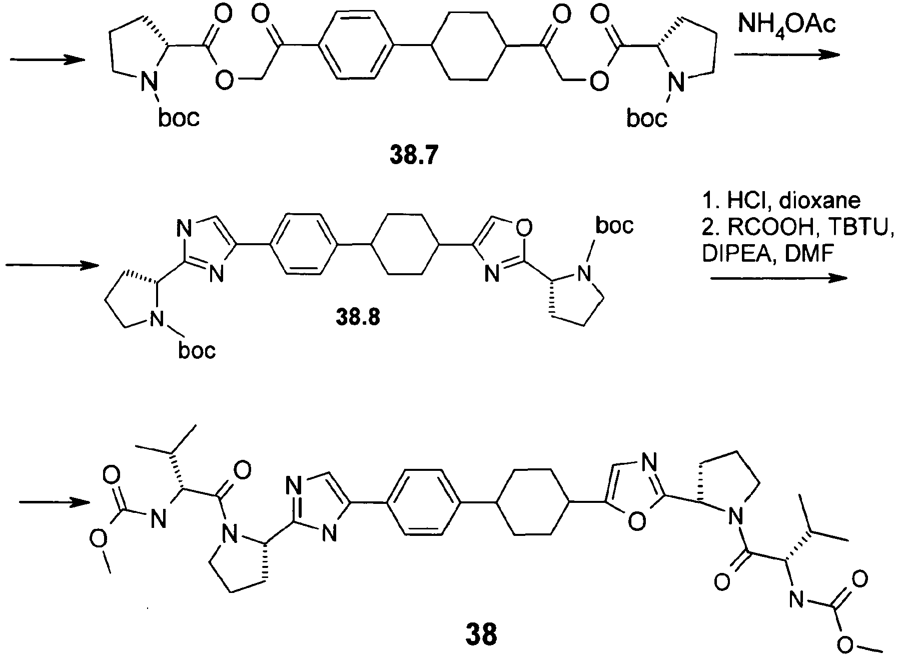

4,4'- Dimethyl biphenyl-4,4'-dicarboxylate **20.1** (9 g, 33 mmol) was hydrogenated in AcOH (70 ml) in the presence of 10 % Pd/C (3 g) and Rh₂O₃ (0.05 g) at 150°C for 16 h at pressure 60-20 atm H₂. The mixture was filtered, evaporated at reduced pressure, an excess of 10 % K₂CO₃ water solution was added to the residue, mixture was extracted with CHCl₃. Organic extracts were evaporated and subjected to colomn chromatography, eluent 10 % EtOAc-hexane. Yield of compound **38.1** is 6 g (65 %). Compound **27** (6 g, 22 mmol) was refluxed in EtOH (100 ml) and H₂O (20 ml) in the presence of 50% KOH (9 g, 80 mmol) solution for 30 min. The solution was evaporated, the residue was dissolved in water (0.5 l) and excess of HCl was added. Solid was filtered off, it gave 5 g (92 %) of *cis*+*trans*-dicarboxylic acids **38.2** mixture. The mixture of dicarboxylic acids **38.2** (5 g, 20 _{MMO} 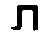 ) was heated at 300 °C at stirring in argon current for 30 min (at this temperature the compound melted). Sublimate and the residue were dissolved in hot 20 % KOH (50 ml) solution and stirred with activated carbon (1 g). The solution was filtered, diluted with water (0,5 l) and treated with an excess of HCl.

Precipitated solid was filtered off, it gave dicarboxylic acid **38.3** 3 g (60 %) (*trans*isomer 90%, *cis*-isomer 10%). ¹H NMR (DMSO-*d*₆). 12.5 (br.s, 2H) 7.88 (d, 2H, *J 8 Hz)* 7.34 (d, 2H, *J 8 Hz)* 2.56 (br.m, 1 H) 2.26 (br.m, 1 H) 2.0 (m, 2H) 1.82 (m, 2H) 1.50-1.40 (m, 4H). Compound **38.5** was prepared by analogy with preparation of compound **20.6,** yield is 50 %, ¹H NMR LDA-2488 (CDCl₃ ). 7.70 (d, 2H) 7.28 (d, 2H) 5.88 (s, 1 H) 5.31 (br.s,1H) 2.60 (t.t., 1H, ³*J 12 Hz, ³J 2 Hz)* 2.33 (br.m, 1 H) 2.05-2.00 (m, 4H) 1.65-1.45 (m,4H). Compound **38.6** was prepared by analogy with preparation of compound **20.7,** dibromide was purified by colomn chromatography, yield is 42 %, ¹H NMR (CDCl₃): 7.94 (d, 2H) 7.33 (d, 2H) 4.44 (s, 2H, ArCOC*H*2Br) 4.00 (s, 2H, CyCOC*H*2Br) 2.84 (m, 1 H) 2.63 (m, 1 H) 2.15-2.05 (m, 4H) 1.60 (m, 4H). Compound **38.7** was prepared by analogy with the preparation of compound **20.8,** yield is quantitative. Compound **38.8** was prepared by analogy with the preparation of compound **20.9,** yield is quantitative, LCMS 632 (M+1). Compound **38** (free base, LCMS 746 (M+1) was prepared by analogy with the preparation of compound **20.** Dihydrochloride **38·2HCl** was prepared by addition of an excess of 3M HCl in dioxane to solution of base **38** in CH₂Cl₂ and precipitation with ether. ¹H NMR (CDCl₃) 0.87 (d, 6H, 2Me), 0.93 (d, 3H, Me), 1.03 (d, 3H, Me), 1.5-2.5 (m, 22H), 3.59 (m,1H), 3.68 (s, 3H, OMe), 3.71 (s, 3H, OMe), 3.80 (m,3H), 4.34 (m, 1 H), 5.24 (m, 1 H), 5.42 (m, 1 H), 7.16 (s, 1 H), 7.24 (s, 1H), 7.22 (br.d, 2H), 7.68 (br.m.,2H).

In analogous manner, using the corresponding starting materials and suitable chiral building blocks, the following compounds have been prepared:
[(1*S*)-1-[[(2*S*)-2-[5-[2-[2-[2-[(2*S*)-2-[(Methoxycarbonyl)amino]-3-methyl-1-oxobutyl]-1-pyrrolidinyl]-1*H*-imidazol-5-yl]ethyl]-1*H*-imidazol-2-yl]-1-pyrrolidinyl]carbonyl]-2-methylpropyl]-carbamic acid methyl ester, LCMS (M+1) 615 ¹H NMR (DMSO-D6, 400 MHz) δ 0.88 (t, J₁ = 6.50, J₂ = 0.93, 6H), 0.96 (t, J₁ = 6.50, J₂ = 0.93, 6H), 1.67 (m, 2H), 1.84 (m, 2H), 1.95 (m, 2H), 1.99 (m, 2H), 2.10 (m, 2H), 2.85 (s, 4H), 3.57 (m, 2H), 3.61 (d, J = 9.40, 6H), 4.02 (m, 2H), 4.75 (m, 2H), 6.91 (d, J = 0.93, 2H), 9.06 (m, 4H).
Bis[1,3-Propanediylbis[1*H*-imidazole-5,2-diyl(2*S*)-2,1- pyrrolidinediyl[(1*S*)-1-(1-methylethyl)-2-oxo-2,1-ethanediyl]]]-carbamic acid dimethyl ester, LCMS (M+1) 629 ¹H NMR (DMSO-D6, 400 MHz) δ 0.88 (t, J₁ = 6.50, J₂ = 0.93, 6H), 0.96 (t, J₁ = 6.50, J₂ = 0.93, 6H), 1.64 (m, 2H), 1.84 (m, 2H), 1.97 (m, 4H), 2.10 (m, 2H), 2.55 (d, J = 4.76, 4H), 3.56 (m, 2H), 3.61 (d, J = 9.40, 6H), 3.63 (m, 2H), 4.02 (m, 2H), 4.73 (m, 2H), 6.73 (d, J = 0.93, 2H), 9.06 (m, 4H).
[1,4-Butanediylbis[1*H*-imidazole-5,2-diyl(2*S*)-2,1-pyrrolidinediyl[(1*S*)-1-(1-methylethyl)-2-oxo-2,1-ethanediyl]]]bis-carbamic acid dimethyl ester, LCMS (M+1) 643 ¹H NMR (DMSO-D6, 400 MHz) δ 0.88 (t, J₁ = 6.50, J₂ = 0.93, 6H), 0.96 (t, J₁ = 6.50, J₂ = 0.93, 6H), 1.60 (t, J₁ = 7.17, J₂ = 0.25, 4H), 1.64 (m, 2H), 1.84 (m, 2H), 1.99 (m, 2H), 2.10 (m, 4H), 2.49 (d, J = 14.80, 4H), 3.55 (m, 4H), 3.61 (d, J = 9.40, 6H), 3.63 (m, 2H), 4.01 (t, J₁ = 8.50, J₂ = 0.42, 2H), 4.75 (m, 2H), 6.84 (d, J = 0.93, 2H), 9.06 (m, 4H).
[1,5-Pentanediylbis[1*H*-imidazole-5,2-diyl(2*S*)-2,1-pyrrolidinediyl[(1*R*)-1-(1-methylethyl)-2-oxo-2,1-ethanediyl]]]bis-carbamic acid dimethyl ester, LCMS (M+1) 657 ¹H NMR (DMSO-D6, 400 MHz) δ 0.88 (t, J₁ = 6.75, J₂ = 0.93, 6H), 0.95 (t, J₁ = 6.75, J₂ = 0.93, 6H), 1.38 (t, J₁ = 7.60, J₂ = 0.25, 2H), 1.62 (m, 6H), 1.84 (m, 2H), 1.99 (m, 4H), 2.10 (m, 2H), 2.48 (t, J₁ = 14.80, J₂ = 0.25, 4H), 3.56 (m, 2H), 3.61 (d, J = 9.40, 6H), 3.63 (m, 2H), 3.96 (d, J = 0.42, 2H), 4.75 (d, J = 0.42, 2H), 6.79 (d, J = 0.93, 2H), 8.71 (m, 4H).
[1,6-Hexanediylbis[1*H*-imidazole-5,2-diyl(2*S*)-2,1-pyrrolidinediyl[(1S)-1-(1-methylethyl)-2-oxo-2,1-ethanediyl]]]bis-carbamic acid dimethyl ester, compound 6, LCMS (M+1) 671 ¹H NMR (DMSO-D6, 400 MHz) δ 0.88 (t, J₁ = 6.75, J₂ = 0.93, 6H), 0.96 (t, J₁ = 6.75, J₂ = 0.93, 6H), 1.36 (d, J = 6.00, 4H), 1.58 (t, J₁ = 7.60, J₂ = 0.25, 4H), 1.64 (m, 2H), 1.84 (m, 2H), 1.99 (m, 4H), 2.10 (m, 2H), 2.48 (t, J₁ = 14.80, J₂ = 0.25, 4H), 3.56 (m, 2H), 3.61 (d, J = 9.40, 6H), 3.67 (m, 2H), 3.96 (d, J = 0.42, 2H), 4.75 (m, 2H), 6.79 (d, J = 0.93, 2H), 8.71 (m, 4H).
[(*E*)-1,2-Ethenediylbis[1*H*-imidazole-5,2-diyl(2*R*)-2,1-pyrrolidinediyl[(1*S*)-1-(1-methylethyl)-2-oxo-2,1-ethanediyl]]]bis-carbamic acid dimethyl ester, LCMS (M+1) 613 ¹H NMR (DMSO-D6, 400 MHz) δ 0.88 (t, J₁ = 6.50, J₂ = 0.93, 6H), 0.96 (t, J₁ = 6.50, J₂ = 0.93, 6H), 1.64 (m, 2H), 1.84 (m, 2H), 1.99 (m, 4H), 2.10 (m, 2H), 3.56 (m, 2H), 3.61 (d, J = 9.40, 6H), 3.64 (m, 2H), 4.02 (d, J = 0.42, 2H), 4.56 (m, 2H), 7.35 (s, 2H), 7.43 (d, J = 16.47, 2H), 7.70 (m, 4H).
[(1*E*)-1-Propene-1,3-diylbis[1*H*-imidazole-5,2-diyl(2*R*)-2,1-pyrrolidinediyl[(1*R*)-1-(1-methylethyl)-2-oxo-2,1-ethanediyl]]]bis-carbamic acid dimethyl ester, LCMS (M+1) 628 ¹H NMR (DMSO-D6, 400 MHz) δ 0.88 (t, J₁ = 6.75, J₂ = 0.93, 6H), 0.94 (t, J₁ = 6.75, J₂ = 0.93, 6H), 1.64 (m, 2H), 1.83 (m, 4H), 1.99 (d, J = 7.70, 2H), 2.10 (m, 2H), 3.31 (d, J = 14.34, 2H), 3.56 (m, 2H), 3.61 (d, J = 9.40, 6H), 3.63 (m, 2H), 3.96 (d, J = 7.70, 2H), 4.55 (m, 1 H), 4.69 (m, 1 H), 6.71 (d, J = 1.17, 1 H), 6.83 (t, J₁ = 16.77, J₂ = 2.10, 1 H), 6.98 (s, 1 H), 7.17 (d, J = 2.10, 1 H), 8.03 (m, 4H).
[(1*E*)-1-Butene-1,4-diylbis[1*H*-imidazole-5,2-diyl(2*S*)-2,1-pyrrolidinediyl[(1*S*)-1-(1-methylethyl)-2-oxo-2,1-ethanediyl]]]bis-carbamic acid dimethyl ester, LCMS (M+1) 641 ¹H NMR (DMSO-D6, 400 MHz) δ 0.88 (t, J₁ = 6.50, J₂ = 0.93, 6H), 0.94 (t, J₁ = 6.50, J₂ = 0.93, 6H), 1.64 (m, 2H), 1.84 (m, 2H), 1.99 (m, 2H), 2.10 (m, 2H), 2.38 (d, J = 14.20, 2H), 2.58 (t, J₁ = 14.02, J₂ = 7.67, 2H), 3.54 (m, 2H), 3.61 (d, J = 9.40, 6H), 3.63 (m, 2H), 4.02 (d, J = 0.42, 2H), 4.61 (m, 2H), 4.75 (m, 2H), 6.53 (t, J₁ = 6.90, J₂ = 1.50, 2H), 6.99 (d, J = 0.93, 1 H), 7.28 (m, 1 H), 8.38 (m, 4H).
2,2'-[(2*E*)-2-Butene-1,4-diylbis(1*H*-imidazole-5,2-diyl)]bis[1-[(2*R*)-2-phenyl-2-(1-piperidinyl)acetyl]-(2*S*,2'*S*)-pyrrolidine, LCMS (M+1) 729 ¹H NMR (DMSO-D6, 400 MHz) δ 1.54 (m, 2H), 1.62 (m, 2H), 1.67 (m, 6H), 1.75 (m, 4H), 1.84 (m, 2H), 1.99 (m, 2H), 2.10 (m, 2H), 2.66 (m, 4H), 2.73 (m, 4H), 3.27 (d, J = 14.34, 4H), 3.45 (m, 2H), 3.53 (m, 2H), 3.95 (d, J = 0.42, 2H), 4.63 (m, 2H), 6.15 (t, J₁ = 7.10, J₂ = 1.17, 2H), 7.10 (s, 2H), 7.22 (t, J₁ = 7.32, J₂ = 1.25, 4H), 7.38 (t, J₁ = 7.03, J₂ = 0.71, 4H), 7.43 (m, 2H), 11.73 (m, 2H).
2,2'-[(1*E*)-1-Pentene-1,5-diylbis(1*H*-imidazole-5,2-diyl)bis[1-[(2*R*)-2-phenyl-2-(1-piperidinyl)acetyl]-, (2R,2'R)-pyrrolidine, LCMS (M+1) 743 ¹H NMR (DMSO-D6, 400 MHz) δ 1.54 (m, 2H), 1.62 (m, 2H), 1.67 (m, 8H), 1.75 (m, 4H), 1.83 (m, 2H), 1.99 (m, 2H), 2.10 (m, 2H), 2.31 (d, J = 12.85, 2H), 2.49 (t, J₁ = 14.80, J₂ = 1.70, 2H), 2.65 (m, 4H), 2.73 (m, 4H), 3.46 (m, 2H), 3.53 (m, 2H), 3.94 (d, J = 0.42, 2H), 4.45 (m, 1 H), 4.59 (m, 1 H), 6.54 (t, J₁ = 16.77, J₂ = 1.50, 2H), 6.84 (d, J = 0.93, 1 H), 7.20 (t, J₁ = 7.32, J₂ = 1.25, 5H), 7.38 (t, J₁ = 7.03, J₂ = 0.71, 4H), 7.43 (m, 2H), 10.37 (m, 2H).
2,2'-[(2*E*)-2-Pentene-1,5-diylbis(1*H*-imidazole-5,2-diyl)]bis[1-[(2*S*)-2-phenyl-2-(1-piperidinyl)acetyl]-, (2*S*,2'*S*)-pyrrolidine, LCMS (M+1) 743 ¹H NMR (DMSO-D6, 400 MHz) δ 1.54 (m, 2H), 1.62 (m, 2H), 1.67 (m, 6H), 1.75 (m, 4H), 1.84 (m, 2H), 1.99 (m, 2H), 2.10 (m, 2H), 2.33 (t, J₁ = 14.20, J₂ = 1.20, 2H), 2.55 (t, J₁ = 14.02, J₂ = 1.00, 2H), 2.66 (m, 4H), 2.73 (m, 4H), 3.21 (d, J = 14.34, 2H), 3.44 (m, 2H), 3.53 (m, 2H), 3.92 (s, 2H), 4.64 (m, 2H), 5.43 (t, J₁ = 15.17, J₂ = 1.17, 1 H), 5.99 (t, J₁ = 7.10, J₂ = 1.22, 1 H), 6.88 (d, J = 0.93, 2H), 7.05 (s, 2H), 7.22 (t, J₁ = 7.80, J₂ = 0.71, 4H), 7.38 (t, J₁ = 7.80, J₂ = 0.71, 4H), 7.43 (m, 2H), 11.73 (m, 2H).
2,2'-[(1*E*,4*E*)-1,4-Pentadiene-1,5-diylbis(1*H*-imidazole-5,2-diyl)]bis[1-[(2*S*)-2-phenyl-2-(1-piperidinyl)acetyl]-, (2*R*,2'*R*)-pyrrolidine, LCMS (M+1) 741 ¹H NMR (DMSO-D6, 400 MHz) δ 1.54 (m, 2H), 1.62 (m, 2H), 1.67 (m, 6H), 1.75 (m, 4H), 1.84 (m, 2H), 1.99 (m, 2H), 2.10 (m, 2H), 2.63 (m, 4H), 2.73 (m, 4H), 2.83 (d, J = 6.00, 2H), 3.46 (m, 2H), 3.53 (m, 2H), 3.92 (d, J = 0.42, 2H), 4.45 (m, 2H), 6.17 (t, J₁ = 6.47, J₂ = 2.10, 2H), 6.69 (d, J = 1.65, 2H), 7.22 (d, J = 7.80, 4H), 7.28 (d, J = 2.10, 2H), 7.38 (t, J₁ = 2.28, J₂ = 0.71, 4H), 7.43 (t, J₁ = 7.32, J₂ = 2.10, 2H), 9.01 (m, 2H).
*N*,*N*'-[2,3- Pentadiene-1,5-diylbis[1*H*-imidazole-5,2-diyl(2*S*)-2,1-pyrrolidinediyl[(1*R*)-2-oxo-1-phenyl-2,1-ethanediyl]]]bis-cyclopropanecarboxamide, LCMS (M+1) 741 ¹H NMR (DMSO-D6, 400 MHz) δ 0.83 (m, 4H), 0.91 (m, 4H), 1.64 (m, 2H), 1.73 (m, 2H), 1.76 (m, 2H), 1.83 (m, 2H), 1.99 (m, 2H), 2.10 (m, 2H), 3.43 (m, 2H), 3.50 (m, 6H), 4.62 (m, 2H), 5.00 (t, J₁ = 12.88, J₂ = 6.67, 2H), 5.17 (d, J = 0.42, 2H), 7.04 (d, J = 0.93, 2H), 7.18 (d, J = 7.17, 4H), 7.28 (m, 6H), 9.82 (m, 4H).
*N*,*N*'-[(1*E*)-1-Hexene-1,6-diylbis[1*H*-imidazole-5,2-diyl(2*R*)-2,1-pyrrolidinediyl[(1R)-2-oxo-1-phenyl-2,1-ethanediyl]]]bis-cyclopropanecarboxamide, LCMS (M+1) 757 ¹H NMR (DMSO-D6, 400 MHz) δ 0.83 (m, 4H), 0.91 (m, 4H), 1.39 (t, J₁ = 7.19, J₂ = 0.25, 2H), 1.60 (d, J = 7.17, 2H), 1.64 (m, 4H), 1.76 (m, 2H), 1.83 (m, 2H), 1.99 (m, 2H), 2.10 (m, 2H), 2.50 (t, J₁ = 12.85, J₂ = 1.70, 2H), 3.43 (m, 2H), 3.50 (m, 2H), 4.43 (m, 1H), 4.56 (m, 1H), 5.17 (d, J = 0.42, 2H), 6.53 (t, J₁ = 16.77, J₂ = 1.22, 2H), 6.84 (s, 2H), 7.18 (t, J₁ = 7.03, J₂ = 1.46, 4H), 7.27 (m, 6H), 9.14 (m, 4H).
*N*,*N*'-[(2*E*)-2-Hexene-1,6-diylbis[1*H*-imidazole-5,2-diyl(2*S*)-2,1-pyrrolidinediyl[(1*S*)-2-oxo-1-phenyl-2,1-ethanediyl]]]bis-cyclopropanecarboxamide,
   LCMS (M+1) 757 ¹H NMR (DMSO-D6, 400 MHz) δ 0.84 (m, 4H), 0.92 (m, 4H), 1.64 (m, 4H), 1.76 (d, J = 6.60, 2H), 1.84 (m, 2H), 1.99 (m, 2H), 2.10 (m, 4H), 2.49 (d, J = 14.80, 2H), 3.26 (d, J = 14.34, 2H), 3.43 (m, 2H), 3.50 (m, 2H), 4.62 (m, 2H), 5.17 (d, J = 0.42, 2H), 5.46 (t, J₁ = 6.64, J₂ = 1.17, 1 H), 5.97 (t, J₁ = 15.17, J₂ = 1.20, 1 H), 6.84 (s, 1 H), 7.05 (s, 1 H), 7.17 (t, J₁ = 7.60, J₂ = 0.71, 4H), 7.27 (m, 6H), 9.99 (m, 4H).
*N*,*N*'-[(1*E*,3*E*)-1,3-Hexadiene-1,6-diylbis[1*H*-imidazole-5,2-diyl(2*R*)-2,1-pyrrolidinediyl[(1*S*)-2-oxo-1-phenyl-2,1-ethanediyl]]]bis-cyclopropanecarboxamide,
   LCMS (M+1) 755 ¹H NMR (DMSO-D6, 400 MHz) δ 0.84 (m, 4H), 0.90 (m, 4H), 1.64 (m, 2H), 1.76 (d, J = 6.60, 2H), 1.83 (m, 2H), 1.99 (m, 2H), 2.10 (m, 2H), 2.34 (t, J₁ = 14.20, J₂ = 7.67, 2H), 2.61 (d, J = 14.02, 2H), 3.43 (m, 2H), 3.50 (m, 2H), 4.43 (m, 1 H), 4.56 (m, 1 H), 5.17 (d, J = 0.42, 2H), 5.80 (t, J₁ = 7.30, J₂ = 0.95, 1H), 5.98 (t, J₁ = 14.46, J₂ = 1.22, 1 H), 6.85 (m, 1 H), 6.94 (s, 1 H), 7.17 (t, J₁ = 7.60, J₂ = 0.71, 4H), 7.27 (m, 6H), 7.36 (m, 2H), 9.32 (m, 4H).
[(2*E*,4*E*)-2,4-Hexadiene-1,6-diylbis[1*H*-imidazole-5,2-diyl(2*S*)-2,1-pyrrolidinediyl[(1*S*)-1-(1-methylethyl)-2-oxo-2,1-ethanediyl]]]bis-carbamic acid methyl ester, LCMS (M+1) 667 ¹H NMR (DMSO-D6, 400 MHz) δ 0.87 (t, J₁ = 6.50, J₂ = 0.93, 6H), 0.97 (t, J₁ = 6.50, J₂ = 0.93, 6H), 1.65 (m, 2H), 1.84 (m, 2H), 1.97 (m, 4H), 2.10 (m, 2H), 3.28 (d, J = 14.34, 4H), 3.54 (m, 2H), 3.61 (d, J = 9.40, 8H), 4.02 (d, J = 8.50, 2H), 4.75 (m, 2H), 6.16 (t, J₁ = 15.00, J₂ = 7.10, 4H), 7.10 (s, 2H), 9.06 (m, 4H).
(1*S*)-1-[[(2*S*)-2-[5-[4-[[2-[(2*S*)-1-[(2*S*)-2-[(Methoxycarbonyl)amino]-3-methyl-1-oxobutyl]-2-pyrrolidinediyl]-1*H*-imidazol-5-yl]methoxy]phenyl]-1*H*-imidazol-2-yl]-1-pyrrolidinyl]carbonyl]-2-methylpropyl]-carbamic acid methyl ester, LCMS (M+1) 693.
[2,3-Hexadiene-1,6-diylbis[1*H*-imidazole-5,2-diyl(2*S*)-2,1-pyrrolidinediyl[(1*S*)-1-(1-methylethyl)-2-oxo-2,1-ethanediyl]]]bis-carbamic acid dimethyl ester, LCMS (M+1) 667 ¹H NMR (DMSO-D6, 400 MHz) δ 0.87 (t, J₁ = 6.50, J₂ = 0.93, 6H), 0.97 (t, J₁ = 6.50, J₂ = 0.93, 6H), 1.64 (m, 2H), 1.84 (m, 2H), 1.97 (m, 4H), 2.10 (m, 2H), 2.21 (t, J₁ = 15.40, J₂ = 3.00, 2H), 2.67 (d, J = 14.02, 2H), 3.54 (m, 4H), 3.61 (d, J = 9.40, 6H), 3.66 (m, 2H), 4.02 (d, J = 0.42, 2H), 4.75 (m, 2H), 4.88 (t, J₁ = 7.00, J₂ = 1.63, 1 H), 5.05 (t, J₁ = 6.87, J₂ = 3.00, 1 H), 6.68 (d, J = 0.93, 1 H), 7.04 (d, J = 0.93, 1 H), 9.06 (m, 4H).
[1,2-Ethynediylbis[1*H*-imidazole-5,2-diyl(2*S*)-2,1-pyrrolidinediyl[(1*S*)-1-(1-methylethyl)-2-oxo-2,1-ethanediyl]]]bis-carbamic acid dimethyl ester, LCMS (M+1) 611 ¹H 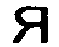 MP (DMSO-D6, 400 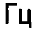 ) δ 0.87 (τ, J₁ = 6.50, J₂ = 0.93, 6H), 0.97 ( 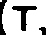 , J₁ = 6.50, J₂ = 0.93, 6H), 1.64 (M, 2H), 1.84 (M, 2H), 1.97 (M, 4H), 2.10 (M, 2H), 3.56 (M, 2H), 3.61 ( 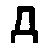 , J = 9.40, 6H), 3.63 (M, 2H), 4.02 (M, 2H), 4.75 ( 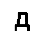 J = 0.42, 2H), 6.90 (c, 2H), 8.80 (M, 4H).
[1-Propyne-1,3-diylbis[1*H*-imidazole-5,2-diyl(2*S*)-2,1-pyrrolidinediyl[(1*S*)-1-(1-methylethyl)-2-oxo-2,1-ethanediyl]]]bis-carbamic acid dimethyl ester, LCMS (M+1) 625 ¹H NMR (DMSO-D6, 400 MHz) δ 0.87 (t, J₁ = 6.50, J₂ = 0.93, 6H), 0.97 (t, J₁ = 6.50, J₂ = 0.93, 6H), 1.65 (m, 2H), 1.84 (m, 2H), 1.97 (m, 4H), 2.10 (m, 2H), 3.47 (d, J = 14.02, 2H), 3.56 (m, 2H), 3.63 (m, 8H), 4.02 (m, 2H), 4.75 (m, 2H), 6.71 (s, 1 H), 6.85 (d, J = 0.93, 1 H), 8.93 (m, 4H).
[1-Butyne-1,4-diylbis[1*H*-imidazole-5,2-diyl(2*S*)-2,1-pyrrolidinediyl[(1*S*)-1-(1-methylethyl)-2-oxo-2,1-ethanediyl]]]bis-carbamic acid dimethyl ester, LCMS (M+1) 625 ¹H NMR (DMSO-D6, 400 MHz) δ 0.87 (t, J₁ = 6.50, J₂ = 0.93, 6H), 0.97 (t, J₁ = 6.50, J₂ = 0.93, 6H), 1.64 (m, 2H), 1.84 (m, 2H), 1.97 (m, 4H), 2.10 (m, 2H), 2.63 (d, J = 12.50, 2H), 2.75 (t, J₁ = 14.80, J₂ = 6.89, 2H), 3.53 (m, 2H), 3.61 (m, 8H), 4.02 (m, 2H), 4.75 (m, 2H), 6.83 (s, 1 H), 7.35 (d, J = 0.93, 1 H), 8.93 (m, 4H).
[2-Butyne-1,4-diylbis[1*H*-imidazole-5,2-diyl(2*S*)-2,1-pyrrolidinediyl[(1*S*)-1-(1-methylethyl)-2-oxo-2,1-ethanediyl]]]bis-carbamic acid dimethyl ester, LCMS (M+1) 639 ¹H NMR (DMSO-D6, 400 MHz) δ 0.88 (t, J₁ = 6.50, J₂ = 0.93, 6H), 0.96 (t, J₁ = 6.50, J₂ = 0.93, 6H), 1.64 (m, 2H), 1.84 (m, 2H), 1.97 (m, 4H), 2.10 (m, 2H), 3.41 (t, J₁ = 14.76, J₂ = 2.51, 4H), 3.56 (m, 2H), 3.63 (m, 8H), 4.02 (d, J = 0.42, 2H), 4.75 (m, 2H), 6.96 (d, J = 14.02, 2H), 9.06 (m, 4H).
[1-Pentyne-1,5-diylbis[1*H*-imidazole-5,2-diyl(2*S*)-2,1-pyrrolidinediyl[(1*S*)-1-(1-methylethyl)-2-oxo-2,1-ethanediyl]]]bis-carbamic acid dimethyl ester, LCMS (M+1) 653 ¹H NMR (DMSO-D6, 400 MHz) δ 0.88 (t, J₁ = 6.50, J₂ = 0.93, 6H), 0.96 (t, J₁ = 6.50, J₂ = 0.93, 6H), 1.64 (m, 2H), 1.84 (m, 4H), 1.97 (m, 4H), 2.10 (m, 2H), 2.49 (d, J = 12.50, 2H), 3.56 (m, 4H), 3.63 (m, 8H), 4.02 (d, J = 0.42, 2H), 4.75 (d, J = 0.42, 2H), 6.66 (d, J = 0.93, 1 H), 6.77 (s, 1H), 8.93 (m, 4H).
[2-Pentyne-1,5-diylbis[1*H*-imidazole-5,2-diyl(2*S*)-2,1-pyrrolidinediyl[(1*S*)-1-(1-methylethyl)-2-oxo-2,1-ethanediyl]]]bis-carbamic acid dimethyl ester, LCMS (M+1) 653 ¹H NMR (DMSO-D6, 400 MHz) δ 0.88 (t, J₁ = 6.50, J₂ = 0.93, 6H), 0.97 (t, J₁ = 6.50, J₂ = 0.93, 6H), 1.64 (m, 2H), 1.84 (m, 2H), 1.97 (m, 4H), 2.10 (m, 2H), 2.58 (t, J₁ = 12.50, J₂ = 2.45, 2H), 2.74 (t, J₁ = 14.80, J₂ = 6.89, 2H), 3.40 (t, J₁ = 14.76, J₂ = 0.93, 2H), 3.56 (m, 2H), 3.63 (m, 8H), 4.02 (m, 2H), 4.75 (m, 2H), 6.91 (d, J = 0.93, 1 H), 7.30 (s, 1 H), 9.06 (m, 4H).
[1,3-Pentadiynyl-1,5-diylbis[1*H*-imidazole-5,2-diyl(2*S*)-2,1-pyrrolidinediyl[(1*S*)-1-(1-methylethyl)-2-oxo-2,1-ethanediyl]]]bis-carbamic acid dimethyl ester, LCMS (M+1) 649 ¹H NMR (DMSO-D6, 400 MHz) δ 0.88 (t, J₁ = 6.50, J₂ = 0.93, 6H), 0.97 (t, J₁ = 6.50, J₂ = 0.93, 6H), 1.64 (m, 2H), 1.84 (m, 2H), 1.97 (m, 4H), 2.10 (m, 2H), 3.45 (d, J = 14.76, 2H), 3.56 (m, 2H), 3.63 (m, 8H), 4.01 (d, J = 0.42, 2H), 4.75 (m, 2H), 6.79 (d, J = 0.93, 1 H), 7.28 (s, 1 H), 8.93 (m, 4H).
[1,4-Pentadiynyl-1,5-diylbis[1*H*-imidazole-5,2-diyl(2*S*)-2,1-pyrrolidinediyl[(1*R*)-1-(1-methylethyl)-2-oxo-2,1-ethanediyl]]]bis-carbamic acid dimethyl ester, LCMS (M+1) 649 ¹H NMR (DMSO-D6, 400 MHz) δ 0.88 (t, J₁ = 6.75, J₂ = 0.93, 6H), 0.97 (t, J₁ = 6.75, J₂ = 0.93, 6H), 1.64 m, 2H), 1.84 (m, 2H), 1.99 (m, 4H), 2.10 (m, 2H), 3.48 (d, J = 12.50, 2H), 3.56 (m, 2H), 3.63 (m, 8H), 3.96 (d, J = 0.42, 2H), 4.75 (m, 2H), 6.65 (s, 2H), 8.44 (m, 4H).
[1-Hexyne-1,6-diylbis[1*H*-imidazole-5,2-diyl(2*S*)-2,1-pyrrolidinediyl[(1*R*)-1-(1-methylethyl)-2-oxo-2,1-ethanediyl]]]bis-carbamic acid dimethyl ester, LCMS (M+1) 667 ¹H NMR (DMSO-D6, 400 MHz) δ 0.88 (t, J₁ = 6.75, J₂ = 0.93, 6H), 0.95 (t, J₁ = 6.75, J₂ = 0.93, 6H), 1.50 (d, J = 6.79, 2H), 1.64 (m, 2H), 1.70 (t, J₁ = 7.24, J₂ = 0.25, 2H), 1.84 (m, 2H), 1.99 (m, 2H), 2.10 (m, 2H), 2.55 (d, J = 12.50, 2H), 2.64 (t, J₁ = 14.80, J₂ = 0.25, 2H), 3.48 (d, J = 12.50, 2H), 3.56 (m, 2H), 3.63 (m, 8H), 3.96 (d, J = 0.42, 2H), 4.75 (m, 2H), 6.77 (d, J = 0.93, 2H), 8.58 (m, 4H).
[2-Hexyne-1,6-diylbis[1*H*-imidazole-5,2-diyl(2*S*)-2,1-pyrrolidinediyl[(1*R*)-1-(1-methylethyl)-2-oxo-2,1-ethanediyl]]]bis-carbamic acid dimethyl ester, LCMS (M+1) 667 ¹H NMR (DMSO-D6, 400 MHz) δ 0.88 (t, J₁ = 6.75, J₂ = 0.93, 6H), 0.95 (t, J₁ = 6.75, J₂ = 0.93, 6H), 1.64 (m, 2H), 1.75 (d, J = 7.00, 2H), 1.84 (m, 2H), 1.99 (m, 4H), 2.10 (m, 2H), 2.25 (t, J₁ = 12.50, J₂ = 2.45, 2H), 2.53 (t, J₁ = 14.80, J₂ = 0.25, 2H), 3.40 (t, J₁ = 14.76, J₂ = 0.93, 2H), 3.56 (m, 2H), 3.63 (m, 8H), 3.96 (d, J = 0.42, 2H), 4.75 (m, 2H), 6.67 (d, J = 0.93, 1 H), 6.91 (d, J = 0.93, 1 H), 8.71 (m, 4H).
[3-Hexyne-1,6-diylbis[1*H*-imidazole-5,2-diyl(2*R*)-2,1-pyrrolidinediyl[(1*R*)-1-(1-methylethyl)-2-oxo-2,1-ethanediyl]]]bis-carbamic acid dimethyl ester, LCMS (M+1) 667 ¹H NMR (DMSO-D6, 400 MHz) δ 0.88 (t, J₁ = 6.75, J₂ = 0.93, 6H), 0.95 (t, J₁ = 6.75, J₂ = 0.93, 6H), 1.64 (m, 2H), 1.84 (m, 2H), 1.99 (m, 4H), 2.10 (m, 2H), 2.57 (t, J₁ = 12.50, J₂ = 6.89, 4H), 2.74 (t, J₁ = 14.80, J₂ = 6.89, 4H), 3.56 (m, 2H), 3.63 (m, 8H), 3.96 (d, J = 0.42, 2H), 4.69 (m, 2H), 7.30 (d, J = 0.93, 2H), 8.71 (m, 4H).
[1,3-Hexadiynyl-1,6-diylbis[1*H*-imidazole-5,2-diyl(2*R*)-2,1-pyrrolidinediyl[(1*R*)-1-(1-methylethyl)-2-oxo-2,1-ethanediyl]]]bis-carbamic acid dimethyl ester, LCMS (M+1) 663 ¹H NMR (DMSO-D6, 400 MHz) δ 0.88 (t, J₁ = 6.75, J₂ = 0.93, 6H), 0.95 (t, J₁ = 6.75, J₂ = 0.93, 6H), 1.64 (m, 2H), 1.83 (m, 2H), 1.99 (m, 4H), 2.10 (m, 2H), 2.61 (d, J = 12.50, 2H), 2.89 (t, J₁ = 14.80, J₂ = 6.89, 2H), 3.56 (m, 2H), 3.63 (m, 8H), 3.96 (d, J = 0.42, 2H), 4.69 (m, 2H), 7.30 (d, J = 0.93, 2H), 8.58 (m, 4H).
[1,5-Hexadiynyl-1,6-diylbis[1*H*-imidazole-5,2-diyl(2*R*)-2,1-pyrrolidinediyl[(1*R*)-1-(1-methylethyl)-2-oxo-2,1-ethanediyl]]]bis-carbamic acid dimethyl ester, LCMS (M+1) 663 ¹H NMR (DMSO-D6, 400 MHz) δ 0.88 (t, J₁ = 6.75, J₂ = 0.93, 6H), 0.95 (t, J₁ = 6.75, J₂ = 0.93, 6H), 1.64 (m, 2H), 1.83 (m, 2H), 1.99 (m, 4H), 2.10 (m, 2H), 2.40 (t, J₁ = 12.50, J₂ = 6.89, 4H), 3.56 (m, 2H), 3.63 (m, 8H), 3.96 (d, J = 0.42, 2H), 4.69 (m, 2H), 6.77 (s, 2H), 8.44 (m, 4H).
[(4*E*)-4-Hexen-1-yl-1,6-diylbis[1*H*-imidazole-5,2-diyl(2*R*)-2,1-pyrrolidinediyl[(1*S*)-1-(1-methylethyl)-2-oxo-2,1-ethanediyl]]]bis-carbamic acid dimethyl ester, LCMS (M+1) 665 ¹H NMR (DMSO-D6, 400 MHz) δ 0.88 (t, J₁ = 6.50, J₂ = 0.93, 6H), 0.95 (t, J₁ = 6.50, J₂ = 0.93, 6H), 1.64 (m, 2H), 1.83 (m, 2H), 1.99 (m, 4H), 2.10 (m, 2H), 2.92 (m, 2H), 3.41 (t, J₁ = 14.34, J₂ = 1.63, 2H), 3.56 (m, 2H), 3.63 (m, 8H), 4.01 (d, J = 0.42, 2H), 4.69 (m, 2H), 5.61 (t, J₁ = 5.50, J₂ = 1.17, 1H), 6.24 (t, J₁ = 15.10, J₂ = 1.50, 1 H), 6.83 (s, 1H), 7.05 (s, 1 H), 8.93 (m, 4H).
[(1*E*)-1-Hexen-5-yl-1,6-diylbis[1*H*-imidazole-5,2-diyl(2*R*)-2,1-pyrrolidinediyl[(1*S*)-1-(1-methylethyl)-2-oxo-2,1-ethanediyl]]]bis-carbamic acid dimethyl ester, LCMS (M+1) 665 ¹H NMR (DMSO-D6, 400 MHz) δ 0.88 (t, J₁ = 6.50, J₂ = 0.93, 6H), 0.95 (t, J₁ = 6.50, J₂ = 0.93, 6H), 1.64 (m, 2H), 1.83 (m, 2H), 1.99 (m, 4H), 2.10 (m, 2H), 2.22 (d, J = 13.52, 2H), 2.41 (d, J = 12.50, 2H), 3.56 (m, 2H), 3.63 (m, 8H), 4.01 (d, J = 0.42, 2H), 4.55 (m, 1 H), 4.69 (m, 1H), 6.54 (t, J₁ = 6.90, J₂ = 1.50, 1 H), 6.78 (t, J₁ = 16.77, J₂ = 1.22, 1H), 6.83 (s, 1H), 7.23(t, J₁ = 2.10, J₂ = 0.93, 1H), 8.25 (m, 4H).
[1,3,5-Hexatriynyl-1,6-diylbis[1*H*-imidazole-5,2-diyl(2*S*)-2,1-pyrrolidinediyl[(1*S*)-1-(1-methylethyl)-2-oxo-2,1-ethanediyl]]]bis-carbamic acid dimethyl ester, (compound 9), LCMS (M+1) 659 ¹H NMR (DMSO-D6, 400 MHz) δ 0.88 (t, J₁ = 6.50, J₂ = 0.93, 6H), 0.95 (t, J₁ = 6.50, J₂ = 0.93, 6H), 1.64 (m, 2H), 1.83 (m, 2H), 1.99 (m, 4H), 2.10 (m, 2H), 3.56 (m, 2H), 3.63 (m, 8H), 4.01 (d, J = 0.42, 2H), 4.75 (m, 2H), 7.28 (s, 2H), 8.80 (m, 4H).
[1,3-Dioxane-2,5-diylbis[1*H*-imidazole-5,2-diyl(1*S*)ethylidene(methylimino)[(1*S*)-1-(1-methylethyl)-2-oxo-2,1-ethanediyl]]]bis-carbamic acid dimethyl ester, LCMS (M+1) 649 ¹H NMR (DMSO-D6, 400 MHz) δ 0.88 (t, J₁ = 6.75, J₂ = 0.93, 6H), 0.95 (t, J₁ = 6.75, J₂ = 0.93, 6H), 1.40 (d, J = 7.12, 6H), 1.99 (d, J = 7.70, 2H), 3.02 (t, J₁ = 14.23, J₂ = 1.50, 6H), 3.22 (m, 1 H), 3.61 (d, J = 9.40, 6H), 3.87 (m, 2H), 3.97 (m, 2H), 4.08 (m, 2H), 4.59 (t, J₁ = 6.97, J₂ = 1.50, 2H), 5.53 (m, 1H), 6.69 (d, J = 0.97, 1 H), 6.83 (s, 1 H), 8.64 (m, 4H).
[1,4-Dioxane-2,5-diylbis[1*H*-imidazole-5,2-diyl(1*S*)ethylidene(methylimino)[(1*S*)-1-(1-methylethyl)-2-oxo-2,1-ethanediyl]]]bis-carbamic acid dimethyl ester, LCMS (M+1) 649.
[(1*S*)-1-[[[(1*S*)-1-[5-[2-[4-[2-[(1*S*)-1-[[(2*S*)-2-[(Methoxycarbonyl)amino]-3-methyl-1-oxobutyl]methylamino]ethyl]-1*H*-imidazol-5-yl]cyclohexyl]-1,3-dioxan-5-yl]-1*H-*imidazol-2-yl]ethyl]methylamino]carbonyl]-2-methylpropyl]-carbamic acid methyl ester, LCMS (M+1) 729.
[(1*S*)-1-[[[(1*S*)-1-[5-[5-[4-[2-[(1*S*)-1-[[(2*S*)-2-[(Methoxycarbonyl)amino]-3-methyl-1-oxobutyl]methylamino]ethyl]-1*H*-imidazol-5-yl]cyclohexyl]-1,3-dioxan-2-yl]-1*H-*imidazol-2-yl]ethyl]methylamino]carbonyl]-2-methylpropyl]-carbamic acid methyl ester, LCMS (M+1) 729.
[(1*S*)-1-[[[(1*R*)-1-[5-[5-[4-[2-[(1*R*)-1-[[(2*S*)-2-[(Methoxycarbonyl)amino]-3-methyl-1-oxobutyl]methylamino]ethyl]-1*H*-imidazol-5-yl]cyclohexyl]-1,4-dioxan-2-yl]-1*H-*imidazol-2-yl]ethyl]methylamino]carbonyl]-2-methylpropyl]-carbamic acid methyl ester, LCMS (M+1) 729.
[(1*S*)-1-[[[(1*R*)-1-[5-[5-[4-[2-[(1*R*)-1-[[(2*S*)-2-[(Methoxycarbonyl)amino]-3-methyl-1-oxobutyl]methylamino]ethyl]-1*H*-imidazol-5-yl]cyclohexyl]-1,4-dioxan-2-yl]-1*H-*imidazol-2-yl]ethyl]methylamino]carbonyl]-2-methylpropyl]-carbamic acid methyl ester, LCMS (M+1) 731.
[1,4-Cyclohexanediylbis[1*H*-imidazole-5,2-diyl(2*S*)-2,1-pyrrolidinediyl[(1*S*)-1-(1-methylethyl)-2-oxo-2,1-ethanediyl]]]bis-carbamic acid dimethyl ester, dihydrochloride, compound 11, LCMS (M+1) 669, ¹H 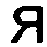 MP (DMSO-D6, 400 M 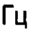 ) δ 0.87 (τ, J₁ = 6.50, J₂ = 0.93, 6H), 0.97 (τ, J₁ = 6.50, J₂ = 0.93, 6H), 1.64 (M, 6H), 1.86 (M, 6H), 1.97 (M, 4H), 2.10 (M, 2H), 2.51 (M, 2H), 3.56 (M, 2H), 3.61 ( 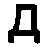 (, J = 9.40, 6H), 3.63 (M, 2H), 4.02 (M, 2H), 4.75 ( 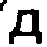 , J = 0.42, 2H), 6.42 (c, 2H),9.06 (M, 4H) 52.
[[1,1'-*trans*-Bicyclohexyl]-4,4'-diylbis[1*H*-imidazole-5,2-diyl(2*S*)-2,1-pyrrolidinediyl[(1*S*)-1-(1-methylethyl)-2-oxo-2,1-ethanediyl]]]bis-carbamic acid dimethyl ester, compound 21, LCMS (M+1) 751 ¹H NMR (DMSO-D6, 400 MHz) δ 0.88 (t, J₁ = 6.50, J₂ = 0.93, 6H), 0.97 (t, J₁ = 6.50, J₂ = 0.93, 6H), 1.46 (m, 8H), 1.56 (m, 8H), 1.64 (m, 2H), 1.84 (m, 2H), 1.97 (m, 2H), 2.10 (m, 2H), 2.15 (m, 2H), 2.42 (m, 2H), 3.45 (d, J = 14.76, 2H), 3.56 (m, 2H), 3.63 (m, 8H), 4.01 (d, J = 0.42, 2H), 4.75 (m, 2H), 6.37 (s, 2H), 9.06 (m, 4H).
[Bicyclo[2.2.2]octane-1,4-diylbis[1*H*-imidazole-5,2-diyl(2*S*)-2,1-pyrrolidinediyl[(1*S*)-1-(1-methylethyl)-2-oxo-2,1-ethanediyl]]]bis-carbamic acid dimethyl ester, LCMS (M+1) 695.
[(1*S*)-1-[[(2*S*)-2-[5-[4-[4-[2-[(2*S*)-1-[(2*S*)-2-[(Methoxycarbonyl)amino]-3-methyl-1-oxobutyl]-2-pyrrolidinyl]-1*H*-imidazol-5-yl]bicyclo[2.2.2]oct-1-yl]-*trans*-cyclohexyl]-1*H*-imidazol-2-yl]-1-pyrrolidinyl]carbonyl]-2-methylpropyl]-carbamic acid methyl ester, LCMS (M+1) 778.
[(1*R*)-1-[[(2*R*)-2-[5-[2-[2-[5-[(1*R*)-2-[(2*R*)-2-[(Methoxycarbonyl)amino]-3-methyl-1-oxobutyl]cyclopentyl]-1*H*-imidazol-2-yl]ethoxy]ethyl]-1*H*-imidazol-2-yl]-1-pyrrolidinyl]carbonyl]-2-methylpropyl]-carbamic acid methyl ester, compound 7. LCMS (M+1) 659. ¹H NMR (DMSO-D6, 400 MHz) δ 0.88 (t, J₁ = 6.45, J₂ = 0.93, 6H), 0.97 (t, J₁ = 6.45, J₂ = 0.93, 6H), 1.64 (m, 2H), 1.84 (m, 2H), 1.97 (m, 4H), 2.10 (m, 2H), 2.46 (t, J₁ = 14.76, J₂ = 8.00, 4H), 3.31 (t, J₁ = 9.55, J₂ = 0.81, 4H), 3.56 (m, 2H), 3.61 (m, 8H), 3.96 (d, J = 0.42, 2H), 4.76 (m, 2H), 6.95 (s, 2H), 8.91 (m, 4H).
[Thiobis[2,1-ethanediyl-1*H*-imidazole-5,2-diyl(1*S*)ethylidene(methylimino)[(1*R*)-1-(1-methylethyl)-2-oxo-2,1-ethanediyl]]]bis-carbamic acid dimethyl ester, LCMS (M+1) 651.
[(1*R*)-1-[[[(1*S*)-1-[5-[2-[[(*E*)-2-[2-[(1*S*)-1-[[(2*R*)-2-[(methoxycarbonyl)amino]-3-methyl-1-oxobutyl]methylamino]ethyl]-1*H*-imidazol-5-yl]-ethenyl]oxy]ethyl]-1*H-*imidazol-2-yl]ethyl]methylamino]carbonyl]-2-methylpropyl]-carbamic acid methyl ester, LCMS (M+1) 633.
[1,2-ethanediylbis[oxy-2,1-ethanediyl-1*H*-imidazole-5,2-diyl(1*S*)ethylidene(methylimino)[(1*R*)-1-(1-methylethyl)-2-oxo-2,1-ethanediyl]]]bis-carbamic acid dimethyl ester dihydrochloride, LCMS (M+1) 679
[oxybis[(1*E*)-1-propyne-3,1-diyl-1*H*-imidazole-5,2-diyl(1*S*)ethylidene(methylimino)[(1*R*)-1-(1-methylethyl)-2-oxo-2,1-ethanediyl]]]bis-carbamic acid dimethyl ester, LCMS (M+1) 659
[(1*R*)-1-[[[(1*R*)-1-[5-[3-[2-[2-[(1*R*)-1-[[(2*R*)-2-[(methoxycarbonyl)amino]-3-methyl-1-oxobutyl]methylamino]ethyl]-1*H*-imidazol-5-yl]ethoxy]-1-propynyl]-1H-imidazol-2-yl]ethyl]methylamino]carbonyl]-2-methylpropyl]-carbamic acid methyl ester, LCMS (M+1) 645.
[Oxybis[1-propyne-3,1-diyl-1*H*-imidazole-5,2-diyl(1*R*)ethylidene(methylimino)[(1*R*)-1-(1-methylethyl)-2-oxo-2,1-ethanediyl]]]bis-carbamic acid dimethyl ester, LCMS (M+1) 645.
[(1*R*)-1-[[[(1*R*)-1-[5-[3-[[(2*E*)-3-[2-[(1*R*)-1-[[(2*R*)-2-[(Methoxycarbonyl)amino]-3-methyl-1-oxobutyl]methylamino]ethyl]-1*H*-imidazol-5-yl]-2-propenyl]oxy]-1-propynyl]-1*H*-imidazol-2-yl]ethyl]methylamino]carbonyl]-2-methylpropyl]-carbamic acid methyl ester, LCMS (M+1) 657.
[(1*R*)-1-[[[(1*R*)-1-[5-[3-[2-[2-[(1*R*)-1-[[(2*R*)-2-[(Methoxycarbonyl)amino]-3-methyl-1-oxobutyl]methylamino]ethyl]-1*H*-imidazol-5-yl]ethoxy]-1-propynyl]-1*H*-imidazol-2-yl]ethyl]methylamino]carbonyl]-2-methylpropyl]-carbamic acid methyl ester, LCMS (M+1) 645.
2,2'-[1,4-Naphthalenediylbis(1*H*-imidazole-5,2-diyl)]bis[1-[(2*S*)-2-phenyl-2-(1-piperidinyl)acetyl], - (2S,2'S)pyrrolidine LCMS (M+1) 802.
2,2'-[1,5-Naphthalenediylbis(1*H*-imidazole-5,2-diyl)]bis[1-[(2*S*)-2-phenyl-2-(1-piperidinyl)acetyl], - (2S,2'S)pyrrolidine, LCMS (M+1) 802
2,2'-[2,5-Thienenediylbis(1*H*-imidazole-5,2-diyl)]bis[1-[(2*S*)-2-phenyl-2-(1-piperidinyl)acetyl], -(2*S*,2'*S*)pyrrolidine LCMS (M+1) 758.
[[2,2'-Bithiophene]-5,5'-diylbis[1*H*-imidazole-5,2-diyl(1*S*)ethelydene(methylimino)[(1*R*)-1-(1-methylethyl)-2-oxo-2,1-ethanediyl]]]bis-carbamic acid dimethyl ester, compound 29, LCMS (M+1) 727, ¹H NMR (DMSO-D6, 400 MHz) δ 0.87 (t, J₁ = 6.50, J₂ = 0.93, 6H), 0.97 (t, J₁ = 6.50, J₂ = 0.93, 6H), 1.40 (t, J₁ = 7.12, J₂ = 6.78, 6H), 1.95 (d, J = 8.50, 2H), 3.02 (t, J₁ = 14.23, J₂ = 1.50, 6H), 3.61 (d, J = 9.40, 6H), (m, 2H), 4.03 (d, J = 8.50, 2H), 4.89 (m, 2H), 6.51 (d, J = 4.00, 2H), 6.86 (d, J = 0.25, 2H), 7.24 (d, J = 0.25, 2H), 8.79 (m, 4H).
*N*,*N*'-[[2,2'-bithiophene]-5,5'-diylbis[1*H*-imidazole-5,2-diyl(1*S*)ethelydene]]bis[*N*'-methyl-□-phenyl-, (□*R*)- 1-piperidineacetamide, compound 32, LCMS (M+1) 816, ¹H NMR (DMSO-D6, 400 MHz) δ 1.39 (t, J₁ = 7.12, J₂ = 6.97, 6H), 1.54 (m, 2H), 1.67 (m, 6H), 1.75 (m, 4H), 2.63 (m, 4H), 2.71 (m, 4H), 2.97 (t, J₁ = 14.23, J₂ = 1.50, 6H), 3.94 (m, 2H), 5.37 (d, J = 0.30, 2H), 6.52 (d, J = 4.00, 2H), 6.87 (d, J = 0.25, 2H), 7.22 (m, 6H), 7.39 (m, 4H), 7.46 (t, J₁ = 7.32, J₂ = 2.10, 2H), 11.18 (m, 2H).
1-[(2*R*)-2-Phenyl-2-(1-piperidinyl)acetyl]-2-[5-[5'-[2-[(2*S*)-1-[(2*S*)-2-phenyl-2-(1-piperidinyl)acetyl]-2-pyrrolidinyl]-1*H*-imidazol-5-yl][2,2'-bithiophen]-5-yl]-1H-imidazol-2-yl]-,(2*R*)-pyrrolidine, compound 33, LCMS (M+1) 840, ¹H NMR (DMSO-D6, 400 MHz) δ 1.51 (m, 2H), 1.70 (m, 10H), 1.84 (m, 2H), 1.99 (m, 2H), 2.10 (m, 2H), 2.65 (m, 4H), 2.72 (m, 4H), 3.45 (m, 2H), 3.53 (m, 2H), 3.96 (d, J = 0.42, 2H), 4.58 (m, 2H), 4.64 (m, 2H), 6.51 (d, J = 4.00, 2H), 6.92 (d, J = 0.25, 2H), 7.25 (br.d, 6H), 7.38 (m, 4H), 7.44 (t, J₁ = 7.32, J₂ = 2.10, 2H), 11.18 (m, 2H).
*N*,*N*'-[[2,2'-bithiophene]-5,5'-diylbis[1*H*-imidazole-5,2-diyl(2*S*)-2,1-pyrrolidinediyl[(1*S*)-2-oxo-1-phenyl-2,1-ethanediyl]]]bis-cyclopropanecarboxamide dihydrochloride, compound 34, LCMS (M+1) 840, ¹H NMR (DMSO-D6, 400 MHz) δ 0.83 (m, 4H), 0.91(m, 4H), 1.64 (m, 2H), 1.76 (m, 2H), 1.84 (m, 2H), 1.99 (m, 2H), 2.10 (m, 2H), 3.43 (d, J = 4.78, 2H), 3.50 (m, 2H), 4.60 (m, 2H), 5.17 (d, J = 0.42, 2H), 6.51 (d, J = 4.00, 2H), 6.92 (d, J = 0.25, 2H), 7.19 (m, 4H), 7.27 (m, 8H), 9.72 (m, 4H).
1-[(2*S*)-2-Phenyl-2-(1-piperidinyl)acetyl]-2-[5-[5-[4'-[2-[(2*S*)-1-[(2*S*)-2-phenyl-2-(1-piperidinyl)acetyl]-2-pyrrolidinyl]-1*H*-imidazol-5-yl][1,1'-biphenyl]-4-yl]-2-thienyl]-1*H-*imidazol-2-yl]-, (2*S*)-pyrrolidine, LCMS (M+1) 910.
2,2'-[2,5-Thienediylbis(4,1-phenylene-1*H*-imidazole-5,2-diyl)]bis[1-[(2*S*)-2-phenyl-2-(1-piperidinyl)acetyl]-, (2*S*,2'*S*)-pyrrolidine, LCMS (M+1) 910.
1-[(2*S*)-2-Phenyl-2-(1-piperidinyl)acetyl]-2-[5-[4-[5'-[2-[(2*R*)-1-[(2*S*)-2-phenyl-2-(1-piperidinyl)acetyl]-2-pyrrolidinyl]-1*H*-imidazol-5-yl][2,2'-bithiophen]-5-yl]phenyl]-1*H-*imidazol-2-yl]-, (2*R*)-pyrrolidine, LCMS (M+1) 916.
2,2'-[2,6-Naphthalenediylbis(5,2-thienediyl-1*H*-imidazol-5,2-diyl)]bis[1-[(2*S*)-2-phenyl-2-(1-piperidinyl)acetyl]-, (2*R*,2'*R*)-pyrrolidine, LCMS (M+1) 966.
1-[(2*S*)-2-Phenyl-2-(1-piperidinyl)acetyl]-2-[5-[4-[5-[6-[2-[(2*R*)-1-[(2*S*)-2-phenyl-2-(1-piperidinyl)acetyl]-2-pyrrolidinyl]-1*H*-imidazol-5-yl]-2-naphthalenyl]-2-thienyl]phenyl]-1*H*-imidazol-2-yl]-, (2*R*)-pyrrolidine, LCMS (M+1) 966.
1-[(2*S*)-2-Phenyl-2-(1-piperidinyl)acetyl]-2-[5-[[4-[4-[2-[(2*R*)-1-[(2*S*)-2-phenyl-2-(1-piperidinyl)acetyl]-2-pyrrolidinyl]-1*H*-imidazol-5-yl]-1,3-butadiynyl]-*trans-*cyclohexyl]ethynyl]-1*H*-imidazol-2-yl]-, (2*R*)-pyrrolidine, LCMS (M+1) 830.
1-[(2*R*)-2-Phenyl-2-(1-piperidinyl)acetyl]-2-[5-[4-[4-[2-[(2*S*)-1-[(2*R*)-2-phenyl-2-(1-piperidinyl)acetyl]-2-pyrrolidinyl]-1*H*-imidazol-5-yl]-1,3-butadiynyl]-trans-cyclohexyl]-1*H*-imidazol-2-yl]-, (2*S*)-pyrrolidine, LCMS (M+1) 806.
1-[(2*R*)-2-Phenyl-2-(1-piperidinyl)acetyl]-2-[5-[4-[4-[2-[(2*S*)-1-[(2*R*)-2-phenyl-2-(1-piperidinyl)acetyl]-2-pyrrolidinyl]-1*H*-imidazol-5-yl]-1,3-butadiynyl]cyclphexyl]-1*H-*imidazol-2-yl]-, (2*S*)-pyrrolidine, dihydrochloride, LCMS (M+1) 806.
1-[(2*R*)-2-Phenyl-2-(1-piperidinyl)acetyl]-2-[5-[6-[4-[2-[(2*S*)-1-[(2*R*)-2-phenyl-2-(1-piperidinyl)acetyl]-2-pyrrolidinyl]-1*H*-imidazol-5-yl]cyclohexyl]-1,3,5-hexatriynyl]-1*H*-imidazol-2-yl]-, (2*S*)-pyrrolidine, LCMS (M+1) 830.
1-[(2*R*)-2-Phenyl-2-(1-piperidinyl)acetyl]-2-[5-[[4-[4-[2-[(2*S*)-1-[(2*R*)-2-phenyl-2-(1-piperidinyl)acetyl]-2-pyrrolidinyl]-1*H*-imidazol-5-yl]-1 ,3-butadiynyl]cyclohexyl]ethynyl]-1*H*-imidazol-2-yl]-,(2*S*)-pyrrolidine,LCMS (M+1) 830.
2,2'-[1,4-Cyclohexanediylbis(1,3-butadiynyl-4,1-diyl-1*H*-imidazole-5,2-diyl)]bis[1-[(2*R*)-2-phenyl-2-(1-piperidinyl)acetyl]-, (2*R*,2'*R*)-pyrrolidine, LCMS (M+1) 854.
1-[(2*R*)-2-Phenyl-2-(1-piperidinyl)acetyl]-2-[5-[4-[4-[2-[(2*R*)-1-[(2*R*)-2-phenyl-2-(1-piperidinyl)acetyl]-2-pyrrolidinyl]-1*H*-imidazol-5-yl]bicyclo[2.2.2]oct-1-yl]-1,3-butadiynyl]-1*H*-imidazol-2-yl]-, (2*R*)-pyrrolidine, LCMS (M+1) 832.
1-[(2*R*)-2-Phenyl-2-(1-piperidinyl)acetyl]-2-[5-[6-[4-[2-[(2*R*)-1-[(2*R*)-2-phenyl-2-(1-piperidinyl)acetyl]-2-pyrrolidinyl]-1*H*-imidazol-5-yl]bicyclo[2.2.2]oct-1-yl]-1,3,5-hexatriynyl]-1*H*-imidazol-2-yl]-, (2*R*)-pyrrolidine, LCMS (M+1) 856.
1-[(2*R*)-2-Phenyl-2-(1-piperidinyl)acetyl]-2-[5-[[4-(4-[2-[(2*R*)-1-[(2*R*)-2-phenyl-2-(1-piperidinyl)acetyl]-2-pyrrolidinyl]-1*H*-imidazol-5-yl]-1,3-butadiynyl]bicyclo[2.2.2]oct-1-yl]ethynyl]-1*H*-imidazol-2-yl]-, (2*R*)-pyrrolidine, LCMS (M+1) 856.
*N*-[(1*S*)-2-[(2*S*)-2-[5-[[5-[2-[(2*S*)-1-[(2*S*)-2-[(Cyclopropylcarbonyl)amino]-2-phenylacetyl]-2-pyrrolidinyl]-1*H*-imidazol-5-yl]-1,3-dioxan-2-yl]ethynyl]-1*H*-imidazol-2-yl]-1-pyrrolidinyl]-2-oxo-1-phenylethyl]-cyclopropanecarboxamide, LCMS (M+1) 785.
*N*-[(1*S*)-2-[(2*S*)-2-[5-[[2-[2-[(2*S*)-1-[(2*S*)-2-[(Cyclopropylcarbonyl)amino]-2-phenylacetyl]-2-pyrrolidinyl]-1*H*-imidazol-5-yl]-1,3-dioxan-5-yl]ethynyl]-1*H*-imidazol-2-yl]-1-pyrrolidinyl]-2-oxo-1-phenylethyl]-cyclopropanecarboxamide, LCMS (M+1) 785.
*N*-[(1*S*)-2-[(2*S*)-2-[5-[2-[4-[2-[(2*S*)-1-[(2*S*)-2-[(Cyclopropylcarbonyl)amino]-2-phenylacetyl]-2-pyrrolidinyl]-1*H*-imidazol-5-yl]-1,3-butadiynyl]-1,3-dioxan-5-yl]-1*H-*imidazol-2-yl]-1-pyrrolidinyl]-2-oxo-1-phenylethyl]-cyclopropanecarboxamide, LCMS (M+1) 809.
*N*-[(1*S*)-2-[(2*S*)-2-[5-[5-[4-[2-[(2*S*)-1-[(2*S*)-2-[(Cyclopropylcarbonyl)amino]-2-phenylacetyl]-2-pyrrolidinyl]-1*H*-imidazol-5-yl]-1,3-butadiynyl]-1,3-dioxan-2-yl]-1*H-*imidazol-2-yl]-1-pyrrolidinyl]-2-oxo-1-phenylethyl]-cyclopropanecarboxamide, LCMS (M+1) 809.
*N*-[(1*S*)-2-[(2*S*)-2-[5-[6-[5-[2-[(2*S*)-1-[(2*S*)-2-[(Cyclopropylcarbonyl)amino]-2-phenylacetyl]-2-pyrrolidinyl]-1*H*-imidazol-5-yl]-1,3-dioxan-2-yl]-1,3,5-hexatriynyl]-1*H*-imidazol-2-yl]-1-pyrrolidinyl]-2-oxo-1-phenylethyl]-cyclopropanecarboxamide, LCMS (M+1) 833.
*N*-[(1*S*)-2-[(2*S*)-2-[5-[[5-[4-[2-[(2*S*)-1-[(2*S*)-2-[(Cyclopropylcarbonyl)amino]-2-phenylacetyl]-2-pyrrolidinyl]-1*H*-imidazol-5-yl]-1,3-butadiynyl]-1,3-dioxan-2-yl]ethynyl]-1*H*-imidazol-2-yl]-1-pyrrolidinyl]-2-oxo-1-phenylethyl]-cyclopropanecarboxamide, LCMS (M+1) 833.
*N*-[(1*S*)-2-[(2*S*)-2-[5-[[2-[4-[2-[(2*S*)-1-[(2*S*)-2-[(Cyclopropylcarbonyl)amino]-2-phenylacetyl]-2-pyrrolidinyl]-1*H*-imidazol-5-yl]-1,3-butadiynyl]-1,3-dioxan-5-yl]ethynyl]-1*H*-imidazol-2-yl]-1-pyrrolidinyl]-2-oxo-1-phenylethyl]-cyclopropanecarboxamide, LCMS (M+1) 833.
*N*-[(1*S*)-2-[(2*R*)-2-[5-[[5-[4-[2-[(2*R*)-1-[(2*S*)-2-[(Cyclopropylcarbonyl)amino]-2-phenylacetyl]-2-pyrrolidinyl]-1*H*-imidazol-5-yl]-1,3-butadiynyl]-1,3-dioxan-2-yl]ethynyl]-1*H*-imidazol-2-yl]-1-pyrrolidinyl]-2-oxo-1-phenylethyl]-cyclopropanecarboxamide, LCMS (M+1) 833.
*N*-[(1*S*)-2-[(2*R*)-2-[5-[[5-[2-[(2*R*)-1-[(2*S*)-2-[(Cyclopropylcarbonyl)amino]-2-phenylacetyl]-2-pyrrolidinyl]-1*H*-imidazol-5-yl]-1,4-dioxan-2-yl]ethynyl]-1H-imidazol-2-yl]-1-pyrrolidinyl]-2-oxo-1-phenylethyl]-cyclopropanecarboxamide, LCMS (M+1) 785.
*N*-[(1*S*)-2-[(2*R*)-2-[5-[5-[4-[2-[(2*R*)-1-[(2*S*)-2-[(Cyclopropylcarbonyl)amino]-2-phenylacetyl]-2-pyrrolidinyl]-1*H*-imidazol-5-yl]-1,3-butadiynyl]-1,4-dioxan-2-yl]-1*H-*imidazol-2-yl]-1-pyrrolidinyl]-2-oxo-1-phenylethyl]-cyclopropanecarboxamide, LCMS (M+1) 809.
*N,N*'-[1,4-Dioxane-2,5-diylbis[2,1-ethynediyl-1*H*-imidazole-5,2-diyl(2*R*)-2,1-pyrrolidinediyl[(1*S*)-2-oxo-1-phenyl-2,1-ethanediyl]]]bis-, cyclopropanecarboxamide, LCMS (M+1) 809.
*N*-[(1*R*)-2-[(2*S*)-2-[5-[[5-[4-[2-[(2*S*)-1-[(2*R*)-2-[(Cyclopropylcarbonyl)amino]-2-phenylacetyl]-2-pyrrolidinyl]-1*H*-imidazol-5-yl]-1,3-butadiynyl]-1,4-dioxan-2-yl]ethynyl]-1*H*-imidazol-2-yl]-1-pyrrolidinyl]-2-oxo-1-phenylethyl]-cyclopropanecarboxamide, LCMS (M+1) 833.
*N*-[(1*R*)-2-[(2*S*)-2-[5-[6-[5-[2-[(2*S*)-1-[(2*R*)-2-[(Cyclopropylcarbonyl)amino]-2-phenylacetyl]-2-pyrrolidinyl]-1*H*-imidazol-5-yl]-1,4-dioxan-2-yl]-1,3,5-hexatriynyl]-1*H*-imidazol-2-yl]-1-pyrrolidinyl]-2-oxo-1-phenylethyl]-cyclopropanecarboxamide, LCMS (M+1) 833.
*N*-[(1*R*)-2-[(2*S*)-2-[5-[6-[5-[[2-[(2*S*)-1-[(2*R*)-2-[(Cyclopropylcarbonyl)amino]-2-phenylacetyl]-2-pyrrolidinyl]-1*H*-imidazol-5-yl]ethynyl]-1,4-dioxan-2-yl]-1,3,5-hexatriynyl]-1*H*-imidazol-2-yl]-1-pyrrolidinyl]-2-oxo-1-phenylethyl]-cyclopropanecarboxamide, LCMS (M+1) 857.
*N,N*'-[1,4-Dioxane-2,5-diylbis[1,3-butadiyne-4,1-diyl-1*H*-imidazole-5,2-diyl(2*S*)-2,1-pyrrolidinediyl[(1*R*)-2-oxo-1-phenyl-2,1-ethanediyl]]]bis-, cyclopropanecarboxamide, LCMS (M+1) 857.
[(1*S*)-1-[[(2*S*)-2-[5-[4-[6-[2-[(2*S*)-1-[(2*S*)-2-[(Methoxycarbonyl)amino]-3-methyl-1-oxobutyl]-2-pyrrolidinyl]-1*H*-imidazol-5-yl]-1,3,5-hexatriynyl]phenyl]-1*H*-imidazol-2-yl]-1-pyrrolidinyl]carbonyl]-2-methylpropyl]-carbamic acid methyl ester, compound 16, LCMS (M+1) 735 40 ¹H NMR (DMSO-D6, 400 MHz) δ 0.88 (t, J₁ = 6.50, J₂ = 0.93, 6H), 0.97 (t, J₁ = 6.50, J₂ = 0.93, 6H), 1.64 (m, 2H), 1.84 (m, 2H), 1.97 (m, 2H), 2.10 (m, 2H), 3.45 (d, J = 14.76, 2H), 3.56 (m, 2H), 3.63 (m, 8H), 4.01 (d, J = 0.42, 2H), 4.75 (m, 2H), 6.86 (s, 1 H), 7.28 (s, 1 H), 7.72 (d, J = 8.26, 2H), 7.90 (d, J = 1.95, 2H), 8.80 (m, 4H).
[(1*S*)-1-[[(2*S*)-2-[5-[[4-[4-[2-[(2*S*)-1-[(2*S*)-2-[(Methoxycarbonyl)amino]-3-methyl-1-oxobutyl]-2-pyrrolidinyl]-1*H*-imidazol-5-yl]-1,3-butadiynyl]phenyl]ethynyl]-1*H-*imidazol-2-yl]-1-pyrrolidinyl]carbonyl]-2-methylpropyl]-carbamic acid methyl ester, compound 15, LCMS (M+1) 735 40 ¹H NMR (DMSO-D6, 400 MHz) δ 0.88 (t, J₁ = 6.50, J₂ = 0.93, 6H), 0.97 (t, J₁ = 6.50, J₂ = 0.93, 6H), 1.64 (m, 2H), 1.84 (m, 2H), 1.97 (m, 2H), 2.10 (m, 2H), 3.45 (d, J = 14.76, 2H), 3.56 (m, 2H), 3.63 (m, 8H), 4.01 (d, J = 0.42, 2H), 4.75 (m, 2H), 6.82 (d, J = 0.93, 1 H), 7.33 (s, 1 H), 7.88 (t, J₁ = 2.02, J₂ = 0.71, 2H), 7.93 (m, 2H), 8.80 (m, 4H).
[1,4-Phenylenebis[1,3-butadiyne-4,1-diyl-1*H*-imidazole-5,2-diyl(2*S*)-2,1-pyrrolidinediyl[(1*S*)-1-(1-methylethyl)-2-oxo-2,1-ethanediyl]]]bis-carbamic acid dimethyl ester, compound 17, LCMS (M+1) 759 40 ¹H NMR (DMSO-D6, 400 MHz) δ 0.88 (t, J₁ = 6.50, J₂ = 0.93, 6H), 0.97 (t, J₁ = 6.50, J₂ = 0.93, 6H), 1.64 (m, 2H), 1.84 (m, 2H), 1.97 (m, 2H), 2.10 (m, 2H), 3.45 (d, J = 14.76, 2H), 3.56 (m, 2H), 3.63 (m, 8H), 4.01 (d, J = 0.42, 2H), 4.75 (m, 2H), 7.33 (s, 2H), 7.81 (s, 4H), 8.80 (m, 4H).
[(1*S*)-1-[[(2*S*)-2-[5-[6-[4-[2-[(2*S*)-1-[(2*S*)-2-[(Methoxycarbonyl)amino]-3-methyl-1-oxobutyl]-2-pyrrolidinyl]-1*H*-imidazol-5-yl]-1,3-butadiynyl]-2-naphthalenyl]-1*H-*imidazol-2-yl]-1-pyrrolidinyl]carbonyl]-2-methylpropyl]-carbamic acid methyl ester, compound 19, LCMS (M+1) 761 40 ¹H NMR (DMSO-D6, 400 MHz) δ 0.88 (t, J₁ = 6.50, J₂ = 0.93, 6H), 0.97 (t, J₁ = 6.50, J₂ = 0.93, 6H), 1.64 (m, 2H), 1.84 (m, 2H), 1.97 (m, 2H), 2.10 (m, 2H), 3.45 (d, J = 14.76, 2H), 3.56 (m, 2H), 3.63 (m, 8H), 4.01 (d, J = 0.42, 2H), 4.75 (m, 2H), 6.94 (s, 1 H), 7.33 (s, 1 H), 7.65 (d, J = 8.56, 1 H), 7.85 (m, 1 H), 7.91 (m, 1H), 8.05 (br. d, 2H), 8.61 (t, J₁ = 1.65, J₂ = 0.33, 1 H), 8.79 (m, 4H).
*N*-[(1*R*)-2-[(2*R*)-2-[5-[5-[6-[2-[(2*R*)-1-[(2*R*)-2-[(Cyclopropylcarbonyl)amino]-2-phenylacetyl]-2-pyrrolidinyl]-1*H*-imidazol-5-yl]-1,3,5-hexatriynyl]-1-naphthalenyl]-1 *H*-imidazol-2-yl]-1-pyrrolidinyl]-2-oxo-1-phenylethyl]-cyclopropanecarboxamide, LCMS (M+1) 874.
*N*-[(1*R*)-2-[(2*R*)-2-[5-[[4-[4-[2-[(2*R*)-1-[(2*R*)-2-[(Cyclopropylcarbonyl)amino]-2-phenylacetyl]-2-pyrrolidinyl-1*H*-imidazol-5-yl]-1,3-butadiynyl]-1-naphthalenyl]ethynyl]-1*H*-imidazol-2-yl]-1-pyrrolidinyl]-2-oxo-1-phenylethyl]-cyclopropanecarboxamide, LCMS (M+1) 874.
*N*-[(1*R*)-2-[(2*R*)-2-[5-[[5-[4-[2-[(2*R*)-1-[(2*R*)-2-[(Cyclopropylcarbonyl)amino]-2-phenylacetyl]-2-pyrrolidinyl]-1*H*-imidazol-5-yl]-1,3-butadiynyl]-1-naphthalenyl]ethynyl]-1*H*-imidazol-2-yl]-1-pyrrolidinyl]-2-oxo-1-phenylethyl]-cyclopropanecarboxamide, LCMS (M+1) 874.
*N*-[(1*R*)-2-[(2*R*)-2-[5-[5-[2-[2-[(2*R*)-1-[(2*R*)-2-[(Cyclopropylcarbonyl)amino]-2-phenylacetyl]-2-pyrrolidinyl]-1*H*-imidazol-5-yl]ethyl]-2-thienyl]-1*H*-imidazol-2-yl]-1-pyrrolidinyl]-2-oxo-1-phenylethyl]-cyclopropanecarboxamide, LCMS (M+1) 785.
*N,N*'-[2,5-Thienediylbis[2,1-ethenediyl-1*H*-imidazole-5,2-diyl(1*S*)ethylidene]]bis[*N*'-methyl-□-phenyl-, (□*S*)-1-piperidineacetamide, LCMS (M+1) 790.
*N*-Methyl-*N*-[(1*S*)-1-[5-[(*E*)-2-[5-[2-[(1*S*)-1-[methyl[(2*S*)-2-phenyl-2-(1-piperidinyl)acetyl]amino]ethyl]-1*H*-imidazol-5-yl]-2-thienyl]ethenyl]-1*H*-imidazol-2-yl]ethyl]-□-phenyl-, (□*S*)-1-piperidineacetamide, LCMS (M+1) 760.
*N*-Methyl-*N*-[(1*S*)-1-[5-[(*E*)-2-[5-[2-[2-[(1*S*)-1-[methyl[(2*S*)-2-phenyl-2-(1-piperidinyl)acetyl]amino]ethyl]-1*H*-imidazol-5-yl]ethyl]-2-thienyl]ethenyl]-1*H-*imidazol-2-yl]ethyl]-□-phenyl-, (□*S*)-1-piperidineacetamide, LCMS (M+1) 788.
*N,N*'-[2,5-Thienylbis[(*E*)-2,1-ethenediyl-1*H*-imidazole-5,2-diyl(1*S*)ethylydene]]bis[*N*'-methyl-□-phenyl-, (□*S*)-1-piperidineacetamide, LCMS (M+1) 786.
*N*-Methyl-N-[(1*R*)-1-[5-[[5-[2-[2-[(1*R*)-1-[methyl[(2*S*)-2-phenyl-2-(1-piperidinyl)acetyl]amino]ethyl]-1*H*-imidazol-5-yl]ethyl]-2-thienyl]ethynyl]-1*H-*imidazol-2-yl]ethyl]-□-phenyl-, (□*S*)-1-piperidineacetamide, LCMS (M+1) 786.
*N*-Methyl-*N*-[(1*R*)-1-[5-[(*E*)-2-[5-[[2-[(1*R*)-1-[methyl[(2*S*)-2-phenyl-2-(1-piperidinyl)acetyl]amino]ethyl]-1*H*-imidazol-5-yl]ethynyl]-2-thienyl]ethenyl]-1*H-*imidazol-2-yl]ethyl]-□-phenyl-, (□*S*)-1-piperidineacetamide, LCMS (M+1) 784. *N*-Methyl-N-[(1*R*)-1-[5-[[5-[2-[(1*R*)-1-[methyl[(2*S*)-2-phenyl-2-(1-piperidinyl)acetyl]amino]ethyl]-1*H*-imidazol-5-yl]-2-thienyl]ethynyl]-1*H*-imidazol-2-yl]ethyl]-□-phenyl-, (□*S*)-1-piperidineacetamide, LCMS (M+1) 758.
*N*-Methyl-*N*-[(1*R*)-1-[5-[4-[5-[2-[(1*R*)-1-[methyl[(2*S*)-2-phenyl-2-(1-piperidinyl)acetyl]amino]ethyl]-1*H*-imidazol-5-yl]-2-thienyl]-1,3-butadiynyl]-1*H-*imidazol-2-yl]ethyl]-□-phenyl-, (□*S*)-1-piperidineacetamide, LCMS (M+1) 782.
*N*-Methyl-*N*-[(1*S*)-1-[5-[5-[6-[2-[(1*S*)-1-[methyl[(2*R*)-2-phenyl-2-(1-piperidinyl)acetyl]amino]ethyl]-1*H*-imidazol-5-yl]-1,3,5-hexatriynyl]-2-thienyl]-1*H-*imidazol-2-yl]ethyl]-□-phenyl-, (□*R*)-1-piperidineacetamide, LCMS (M+1) 806.
*N*-Methyl-N-[(1*S*)-1-[5-[[5-[4-[2-[(1*S*)-1-[methyl[(2*R*)-2-phenyl-2-(1-piperidinyl)acetyl]amino]ethyl]-1*H*-imidazol-5-yl]-1,3-butadiynyl]-2-thienyl]ethynyl]-1*H*-imidazol-2-yl]ethyl]-□-phenyl-, (□*R*)-1-piperidineacetamide, LCMS (M+1) 806.
*N,N*'-[2,5-Thienediylbis[1,3-butadiynyl-4,1-diyl-1*H*-imidazole-5,2-diyl(1 *S*)ethylidene]]bis[*N*'-methyl-□-phenyl-, (□*R*)-1-piperidineacetamide, LCMS (M+1) 830.
*N*-Methyl-*N*-[(1*S*)-1-[5-[[5-[4-[2-[(1*S*)-1-[methyl[(2*R*)-2-phenyl-2-(1-piperidinyl)acetyl]amino]ethyl]-1*H*-imidazol-5-yl]-1,3-butadiynyl]-2-thienyl]methyl]-1*H*-imidazol-2-yl]ethyl]-□-phenyl-, (□*R*)-1-piperidineacetamide, LCMS (M+1) 796.
*N*-Methyl-N-[(1*R*)-1-[5-[2-[5-[4-[2-[(1*R*)-1-[methyl[(2*R*)-2-phenyl-2-(1-piperidinyl)acetyl]amino]ethyl]-1*H*-imidazol-5-yl]-1,3-butadiynyl]-2-thienyl]ethyl]-1*H-*imidazol-2-yl]ethyl]-□-phenyl-, (□*R*)-1-piperidineacetamide, LCMS (M+1) 810.
*N*-Methyl-N-[(1*R*)-1-[5-[(*E*)-2-[5-[4-[2-[(1*R*)-1-[methyl[(2*R*)-2-phenyl-2-(1-piperidinyl)acetyl]amino]ethyl]-1*H*-imidazol-5-yl]-1,3-butadiynyl]-2-thienyl]ethenyl]-1*H*-imidazol-2-yl]ethyl]-□-phenyl-, (□*R*)- 1-piperidineacetamide, LCMS (M+1) 808. [(1*S*)-1-[[(2*S*)-2-[5-[5-[[4-[2-[(2*S*)-1-[(2*S*)-2-[(Methoxycarbonyl)amino]-3-methyl-1-oxobutyl]-2-pyrrolidinyl]-1*H*-imidazol-5-yl]phenyl]ethynyl]-2-thienyl]-1*H*-imidazol-2-yl]-1-pyrrolidinyl]carbonyl]-2-methylpropyl]-carbamic acid methyl ester, LCMS (M+1) 769.
[1,2-Ethynediyl[5,2-thienediyl-1*H*-imidazole-5,2-diyl(2*S*)-2,1-pyrrolidinediyl[(1*S*)-1-(1-methylethyl)-2-oxo-2,1-ethanediyl]]]bis-carbamic acid dimethyl ester, dihydrochloride, compound 26, LCMS (M+1) 775,¹H NMR (DMSO-D6, 400 MHz) δ 0.88 (t, J₁ = 6.50, J₂ = 0.93, 6H), 0.97 (t, J₁ = 6.50, J₂ = 0.93, 6H), 1.64 (m, 2H), 1.84 (m, 2H), 1.97 (m, 4H), 2.10 (m, 2H), 3.56 (m, 2H), 3.63 (m, 8H), 4.03 (m, 2H), 4.74 (m, 2H), 6.61 (d, J = 3.50, 2H), 7.00 (d, J = 0.25, 2H), 7.17 (d, J = 3.50, 2H), 8.79 (m, 4H).
[1,3-Butadiyne-1,4-diylbis[5,2-thienediyl-1*H*-imidazole-5,2-diyl(2*S*)-2,1-pyrrolidinediyl[(1*S*)-1-(1-methylethyl)-2-oxo-2,1-ethanediyl]]]bis-carbamic acid dimethyl ester, dihydrochloride, compound 27, LCMS (M+1) 799, ¹H NMR (DMSO-D6, 400 MHz) δ 0.88 (t, J₁ = 6.50, J₂ = 0.93, 6H), 0.97 (t, J₁ = 6.50, J₂ = 0.93, 6H), 1.64 (m, 2H), 1.84 (m, 2H), 1.97 (m, 4H), 2.10 (m, 2H), 3.56 (m, 2H), 3.63 (m, 8H), 4.03 (m, 2H), 4.74 (m, 2H), 6.54 (d, J = 3.50, 2H), 7.00 (d, J = 0.25, 2H), 7.67 (d, J = 3.50, 2H), 8.79 (m, 4H).
[(1*S*)-1-[[(2*S*)-2-[5-[5-[4-[4-[2-[(2*S*)-1-[(2*S*)-2-[(Methoxycarbonyl)amino]-3-methyl-1-oxobutyl]-2-pyrrolidinyl]-1*H*-imidazol-5-yl]phenyl]-1,3-butadiynyl]-2-thienyl]-1*H-*imidazol-2-yl]-1-pyrrolidinyl]carbonyl]-2-methylpropyl]-carbamic acid methyl ester, dihydrochloride, compound 28, LCMS (M+1) 793, ¹H NMR (DMSO-D6, 400 MHz) δ 0.88 (t, J₁ = 6.50, J₂ = 0.93, 6H), 0.97 (t, J₁ = 6.50, J₂ = 0.93, 6H), 1.64 (m, 2H), 1.84 (m, 2H), 1.97 (m, 4H), 2.10 (m, 2H), 3.56 (m, 2H), 3.63 (m, 8H), 4.03 (m, 2H), 4.74 (m, 2H), 6.54 (d, J = 3.50, 1 H), 6.86 (s, 1H), 7.00 (d, J = 0.25, 1H), 7.67 (s, 1H), 7.76 (m, 2H), 7.91 (d, J = 8.26, 2H), 8.79 (m, 4H).
[(1*S*)-1-[[(2*S*)-2-[5-[[5'-[2-[(2*S*)-1-[(2*S*)-2-[(Methoxycarbonyl)amino]-3-methyl-1-oxobutyl]-2-pyrrolidinyl]-1*H*-imidazol-5-yl][2,2'-bithiophen]-5yl]ethynyl]-1*H*-imidazol-2-yl]-1-pyrrolidinyl]carbonyl]-2-methylpropyl]-carbamic acid methyl ester, LCMS (M+1) 775.
[(1*S*)-1-[[(2*S*)-2-[5-[4-[5'-[2-[(2*S*)-1-[(2*S*)-2-[(Methoxycarbonyl)amino]-3-methyl-1-oxobutyl]-2-pyrrolidinyl]-1*H*-imidazol-5-yl][2,2'-bithiophen]-5-yl]-1,3-butadiynyl]-1*H-*imidazol-2-yl]-1-pyrrolidinyl]carbonyl]-2-methylpropyl]-carbamic acid methyl ester, LCMS (M+1) 799.
[(1*S*)-1-[[(2*S*)-2-[5-[5-[4-[[2-[(2*S*)-1-[(2*S*)-2-[(Methoxycarbonyl)amino]-3-methyl-1-oxobutyl]-2-pyrrolidinyl]-1*H*-imidazol-5-yl]ethynyl]phenyl]-2-thienyl]-1*H*-imidazol-2-yl]-1-pyrrolidinyl]carbonyl]-2-methylpropyl]-carbamic acid methyl ester, LCMS (M+1) 769.
[(1*S*)-1-[[(2*S*)-2-[5-[5-[4-[4-[2-[(2*S*)-1-[(2*S*)-2-[(Methoxycarbonyl)amino]-3-methyl-1-oxobutyl]-2-pyrrolidinyl]-1*H*-imidazol-5-yl]-1,3-butadiynyl]phenyl]-2-thienyl]-1*H-*imidazol-2-yl]-1-pyrrolidinyl]carbonyl]-2-methylpropyl]-carbamic acid methyl ester, LCMS (M+1) 793.
[(1*S*)-1-[[(2*S*)-2-[5-[4-[5-[[2-[(2*S*)-1-[(2*S*)-2-[(Methoxycarbonyl)amino]-3-methyl-1-oxobutyl]-2-pyrrolidinyl]-1*H*-imidazol-5-yl]ethynyl]-2-thienyl]phenyl]-1*H*-imidazol-2-yl]-1-pyrrolidinyl]carbonyl]-2-methylpropyl]-carbamic acid methyl ester, LCMS (M+1) 769.
[(1*S*)-1-[[(2*S*)-2-[5-[4-[5-[4-[2-[(2*S*)-1-[(2*S*)-2-[(Methoxycarbonyl)amino]-3-methyl-1-oxobutyl]-2-pyrrolidinyl]-1*H*-imidazol-5-yl]-1,3-butadiynyl]-2-thienyl]phenyl]-1*H-*imidazol-2-yl]-1-pyrrolidinyl]carbonyl]-2-methylpropyl]-carbamic acid methyl ester, LCMS (M+1) 793.
[(*S*)-1-[[(2*S*)-2-[5-[4-[[2-[(2*S*)-1-[(2*S*)-2-[(Methoxycarbonyl)amino]-3-methyl-1-oxobutyl]-2-pyrrolidinyl]-1*H*-imidazol-5-yl]methoxy]phenyl]-1*H*-imidazol-2-yl]-1-pyrrolidinyl]carbonyl]-2-methylpropyl]-carbamic acid methyl ester, hydrochloride, compound 10, LCMS (M+1) 693; ¹H NMR (DMSO-D6, 400 MHz) δ 0.88 (t, J₁ = 6.50, J₂ = 0.93, 6H), 0.97 (t, J₁ = 6.50, J₂ = 0.93, 6H), 1.64 (m, 2H), 1.84 (m, 2H), 1.97 (m, 4H), 2.10 (m, 2H), 3.56 (m, 2H), 3.63 (m, 8H), 4.01 (d, J = 0.42, 2H), 4.75 (m, 2H), 5.12 (s, 2H), 6.71 (d, J = 0.93, 1 H), 6.87 (s, 1 H), 7.07 (d, J = 0.7.89, 2H), 7.89 (d, J = 0.7.89, 2H), 8.98 (m, 4H).
*N*-Methyl-*N*-[(1*R*)-1-[5-[2-[4-[2-[(1*R*)-1-[methyl[(2*R*)-2-phenyl-2-(1-piperidinyl)acetyl]amino]ethyl]-1*H*-imidazol-5-yl]phenoxy]ethyl]-1*H*-imidazol-2-yl]ethyl]-□-phenyl-, (□*R*)-1-piperidineacetamide, LCMS (M+1) 772.
*N*-Methyl-*N*-[(1*R*)-1-[5-[3-[4-[2-[(1*R*)-1-[methyl[(2*R*)-2-phenyl-2-(1-piperidinyl)acetyl]amino]ethyl]-1*H*-imidazol-5-yl]phenoxy]propyl]-1*H*-imidazol-2-yl]ethyl]-□-phenyl-, (□*R*)-1-piperidineacetamide, LCMS (M+1) 786.
[(1*S*)-1-[[[(1*S*)-1-[5-[(1*E*)-3-[4-[2-[(1*S*)-1-[[(2*S*)-2-[(Methoxycarbonyl)amino]-3-methyl-1-oxobutyl]methylaminoethyl]-1*H*-imidazol-5-yl]phenoxy]-1-propenyl]-1*H-*imidazol-2-yl]ethyl]methylamino]carbonyl]-2-methylpropyl]-carbamic acid methyl ester, LCMS (M+1) 695.
[(1*S*)-1-[[[(1*S*)-1-[5-[3-[4-[2-[(1*S*)-1-[[(2*S*)-2-[(Methoxycarbonyl)amino]-3-methyl-1-oxobutyl]methylamino]ethyl]-1*H*-imidazol-5-yl]phenoxy]-1-propynyl]-1*H*-imidazol-2-yl]ethyl]methylamino]carbonyl]-2-methylpropyl]-carbamic acid methyl ester, LCMS (M+1) 693.
[1,4-Phenylenbis[oxy-2,1-ethanediyl-1*H*-imidazole-5,2-diyl(1*S*)ethylyden(methylimino)[(1*S*)-1-(1-methylethyl)-2-oxo-2,1-ethanediyl]]]bis-carbamic acid dimethyl ester, LCMS (M+1) 727.
[1,4-Phenylenbis[oxy-1-propyne-3,1-diyl-1*H*-imidazole-5,2-diyl(1*S*)ethylyden(methylimino)[(1*S*)-1-(1-methylethyl)-2-oxo-2,1-ethanediyl]]]bis-carbamic acid dimethyl ester, LCMS (M+1) 747.
[(1*S*)-1-[[[(1*R*)-1-[5-[5-[4-[2-[(1*R*)-1-[[(2*S*)-2-[(Methoxycarbonyl)amino]-3-methyl-1-oxobutyl]methylamino]ethyl]-1*H*-imidazol-5-yl]cyclohexyl]-2-thienyl]-1*H*-imidazol-2-yl]ethyl]methylamino]carbonyl]-2-methylpropyl]-carbamic acid methyl ester, LCMS (M+1) 747.
[(1*S*)-1-[[[(1*R*)-1-[5-[5-[4-[2-[(1*R*)-1-[[(2*S*)-2-[(Methoxycarbonyl)amino]-3-methyl-1-oxobutyl]-2-pyrrolidinyl]-1*H*-imidazol-5-yl]-trans-cyclohexyl]-2-thienyl]-1*H*-imidazol-2-yl]ethyl]methylamino]carbonyl]-2-methylpropyl]-carbamic acid methyl ester, LCMS (M+1) 747.
[(1*S*)-1-[[[(1*R*)-1-[5-[5-[4-[2-[(1*R*)-1-[[(2*S*)-2-[(Methoxycarbonyl)amino]-3-methyl-1-oxobutyl]-2-pyrrolidinyl]-1*H*-imidazol-5-yl]bicyclo[2.2.2]oct-1-yl]-2-thienyl]-1*H-*imidazol-2-yl]ethyl]methylamino]carbonyl]-2-methylpropyl]-carbamic acid methyl ester, LCMS (M+1) 753.
[(1*S*)-1-[[(2*S*)-2-[5-[4-[5-[2-[(2*S*)-1-[(2*S*)-2-[(Methoxycarbonyl)amino]-3-methyl-1-oxobutyl]-2-pyrrolidinyl]-1*H*-imidazol-5-yl]-1,3-dioxan-2-yl]phenyl]-1*H*-imidazol-2-yl]-1-pyrrolidinyl]carbonyl]-2-methylpropyl]-carbamic acid methyl ester, compound 22, LCMS (M+1) 749 ¹H NMR (DMSO-D6, 400 MHz) δ 0.88 (t, J₁ = 6.50, J₂ = 0.93, 6H), 0.97 (t, J₁ = 6.50, J₂ = 0.93, 6H), 1.64 (m, 2H), 1.84 (m, 2H), 1.97 (m, 2H), 2.10 (m, 2H), 3.22 (m, 1 H), 3.45 (d, J = 14.76, 2H), 3.56 (m, 2H), 3.63 (m, 8H), 3.84 (m, 2H), 4.01 (d, J = 0.42, 2H), 4.08 (m, 2H), 4.75 (m, 2H), 5.48 (m, 1 H), 6.75 (s, 1 H), 6.86 (s, 1 H), 7.68 (d, J = 8.06, 2H), 7.92 (d, J = 1.99, 2H), 8.92 (m, 4H).
[(1*S*)-1-[[[(1*R*)-1-[5-[4-[2-[2-[(1*R*)-1-[[(2*S*)-2-[(Methoxycarbonyl)amino]-3-methyl-1-oxobutyl]methylamino]ethyl]-1*H*-imidazol-5-yl]-1,3-dioxan-5-yl]phenyl]-1*H*-imidazol-2-yl]ethyl]methylamino]carbonyl]-2-methylpropyl]-carbamic acid methyl ester, LCMS (M+1) 725.
[(1*S*)-1-[((2*S*)-2-[5-[4-[5-[2-[(2*S*)-1-[(2*S*)-2-[(Methoxycarbonyl)amino]-3-methyl-1-oxobutyl]-2-pyrrolidinyl]-1*H*-imidazol-5-yl]-1,4-dioxan-2-yl]phenyl]-1*H*-imidazol-2-yl]-1-pyrrolidinyl]carbonyl]-2-methylpropyl]-carbamic acid methyl ester, compound 23, LCMS (M+1) 749 ¹H NMR (DMSO-D6, 400 MHz) δ 0.88 (t, J₁ = 6.50, J₂ = 0.93, 6H), 0.97 (t, J₁ = 6.50, J₂ = 0.93, 6H), 1.64 (m, 2H), 1.84 (m, 2H), 1.97 (m, 2H), 2.10 (m, 2H), 3.45 (d, J = 14.76, 2H), 3.56 (m, 2H), 3.63 (m, 8H), 3.73 (m, 2H), 3.90 (m, 2H), 4.03 (m, 2H), 4.47 (m, 1 H), 4.64 (m, 1 H), 4.75 (m, 2H), 6.82 (s, 1 H), 6.86 (s, 1 H), 7.68 (d, J = 8.06, 2H), 7.92 (m, 2H), 8.92 (m, 4H).
[(1*R*)-1-[[[(1*S*)-1-[5-[6-[5-[2-[(1*S*)-1-[[(2*R*)-2-[(Methoxycarbonyl)amino]-3-methyl-1-oxobutyl]methylamino]ethyl]-1*H*-imidazol-5-yl]-1,3-dioxan-2-yl]-2-naphthalenyl]-1*H-*imidazol-2-yl]ethyl]methylamino]carbonyl]-2-methylpropyl]-carbamic acid methyl ester, LCMS (M+1) 775.
[(1*R*)-1-[[[(1*S*)-1-[5-[6-[2-[2-[(1*S*)-1-[[(2*R*)-2-[(Methoxycarbonyl)amino]-3-methyl-1-oxobutyl]methylamino]ethyl]-1*H*-imidazol-5-yl]-1,3-dioxan-5-yl]-2-naphthalenyl]-1*H-*imidazol-2-yl]ethyl]methylamino]carbonyl]-2-methylpropyl]-carbamic acid methyl ester, LCMS (M+1) 775.
[(1*R*)-1-[[[(1*S*)-1-[5-[6-[5-[2-[(1*S*)-1-[[(2*R*)-2-[(Methoxycarbonyl)amino]-3-methyl-1-oxobutyl]methylamino]ethyl]-1*H*-imidazol-5-yl]-1,4-dioxan-2-yl]-2-naphthalenyl]-1*H-*imidazol-2-yl]ethyl]methylamino]carbonyl]-2-methylpropyl]-carbamic acid methyl ester, LCMS (M+1) 775.
[(1*R*)-1-[[[(1*S*)-1-[5-[2-[4'-[2-[(1*S*)-1-[[(2*R*)-2-[(Methoxycarbonyl)amino]-3-methyl-1-oxobutyl]methylamino]ethyl]-1*H*-imidazol-5-yl][1,1'-biphenyl]-4-yl]-1,3-dioxan-5-yl]-1*H*-imidazol-2-yl]ethyl]methylamino]carbonyl]-2-methylpropyl]-carbamic acid methyl ester, LCMS (M+1) 801.
[(1 *R*)-1-[[[(1*R*)-1-[5-[5-[4'-[2-[(1*R*)-1-[[(2*R*)-2-[(Methoxycarbonyl)amino]-3-methyl-1-oxobutyl]methylamino]ethyl]-1*H*-imidazol-5-yl][1,1'-biphenyl]-4-yl]-1,3-dioxan-2-yl]-1 H-imidazol-2-yl]ethyl]methylamino]carbonyl]-2-methylpropyl]-carbamic acid methyl ester, LCMS (M+1) 801.
[(1*R*)-1-[[[(1*R*)-1-[5-[5-[4'-[2-[(1*R*)-1-[[(2*R*)-2-[(Methoxycarbonyl)amino]-3-methyl-1-oxobutyl]methylamino]ethyl]-1*H*-imidazol-5-yl][1,1'-biphenyl]-4-yl]-1,4-dioxan-2-yl]-1 H-imidazol-2-yl]ethyl]methylamino]carbonyl]-2-methylpropyl]-carbamic acid methyl ester, LCMS (M+1) 801.
[(1*R*)-1-[[[(1*R*)-1-[5-[5-[5-[2-[(1*R*)-1-[[(2*R*)-2-[(Methoxycarbonyl)amino]-3-methyl-1-oxobutyl]methylamino]ethyl]-1*H*-imidazol-5-yl]-1,3-dioxan-2-yl]-2-thienyl]-1*H-*imidazol-2-yl]ethyl]methylamino]carbonyl]-2-methylpropyl]-carbamic acid methyl ester, LCMS (M+1) 731.
[(1*R*)-1-[[[(1*R*)-1-[5-[5-[2-[2-[(1*R*)-1-[[(2*R*)-2-[(Methoxycarbonyl)amino]-3-methyl-1-oxobutyl]methylamino]ethyl]-1*H*-imidazol-5-yl]-1,3-dioxan-5-yl]-2-thienyl]-1*H-*imidazol-2-yl]ethyl]methylamino]carbonyl]-2-methylpropyl]-carbamic acid methyl ester, LCMS (M+1) 731.
[(1*R*)-1-[[[(1*R*)-1-[5-[5-[5-[2-[(1*R*)-1-[[(2*R*)-2-[(Methoxycarbonyl)amino]-3-methyl-1-oxobutyl]methylamino]ethyl]-1*H*-imidazol-5-yl]-1,4-dioxan-2-yl]-2-thienyl]-1*H-*imidazol-2-yl]ethyl]methylamino]carbonyl]-2-methylpropyl]-carbamic acid methyl ester, LCMS (M+1) 731.
[(1*S*)-1-[[(2*S*)-2-[5-[2-[5'-[2-[(2*S*)-1-[(2*S*)-2-[(Methoxycarbonyl)amino]-3-methyl-1-oxobutyl]-2-pyrrolidinyl]-1*H*-imidazol-5-yl][2,2'-bithiophen]-5-yl]-1,3-dioxan-5-yl]-1*H-*imidazol-2-yl]-1-pyrrolidinyl]carbonyl]-2-methylpropyl]-carbamic acid methyl ester, LCMS (M+1) 838.
[(1 *S*)-1-[[(2*S*)-2-[5-[5-[5'-[2-[(2*S*)-1-[(2*S*)-2-[(Methoxycarbonyl)amino]-3-methyl-1-oxobutyl]-2-pyrrolidinyl]-1*H*-imidazol-5-yl][2,2'-bithiophen]-5-yl]-1,3-dioxan-2-yl]-1*H-*imidazol-2-yl]-1-pyrrolidinyl]carbonyl]-2-methylpropyl]-carbamic acid methyl ester, LCMS (M+1) 838.
[(1*S*)-1-[[(2*S*)-2-[5-[5-[5'-[2-[(2*S*)-1-[(2*S*)-2-[(Methoxycarbonyl)amino]-3-methyl-1-oxobutyl]-2-pyrrolidinyl]-1*H*-imidazol-5-yl][2,2'-bithiophen]-5-yl]-1,4-dioxan-2-yl]-1H-imidazol-2-yl]-1-pyrrolidinyl]carbonyl]-methylpropyl]-carbamic acid methyl ester, LCMS (M+1) 838.

### Example 10.

{(S)-2-Methyl-1-[(S)-2-(5-phenyl-1H-imidazol-2-yl)-pyrrolidine-1-carboline]-propyl}-carbamic acid methyl ester **42.**

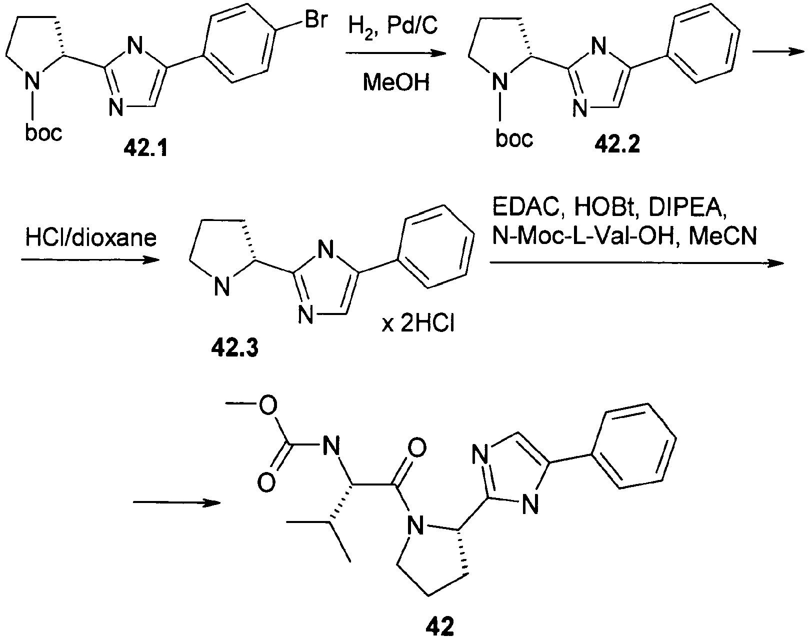

10 % Pd/C (10 mg) was added to solution of compound **42.1** (196 mg, 0.5 mmol) in methanol (10 ml) and mixture was stirred in the current of H₂ for 12 h. The mixture was filtered through celit and evaporated *in vacuo.* Yield of compound **42.2** is 53 mg (98 %). LCMS (ESI): *m*/*z* 314 (M+H)⁺. Compound **42.2** was boc-deprotected with 3M HCl solution in dioxane, yield of compound **42.3** is quantitative (LCMS (ESI): *m*/*z* 214 (M+H)⁺); it was converted into compound **42** (LCMS (ESI): *m*/*z* 371 (M+H)⁺) by analogy with the conversion of compound **14.5** into compound **14.** Dihydrochloride of **42** was prepared by addition of excess of 3M HCl solution in dioxane to solution of base **42** in CH₂Cl₂ and precipitation with ether. ¹H NMR (DMSO-*d*₆, 400 MHz) δ 14.85 (br. m, 2H), 8.05 (s, 1H), 7.86 (m, 2H), 7.50 (m, 2H), 7.41 (m, 1 H), 7.27 (d, *J* = 8.8 *Hz,* 1 H), 5.17 (t, *J* = 6.8 *Hz,* 1 H), 4.11 (t, *J* = 7.8 *Hz,* 1H), 4.00 (m, 1H), 3.83 (br. m, 1 H), 3.56 (s, 0.25H), 3.53 (s, 2.75H), 2.36 (m, 1 H), 2.16 (m, 2H), 2.06 (m, 1 H), 2.00 (m, 1H), 0.90 (m, 0.5H), 0.84 (d, J = 6.4 *Hz,* 2.75H), 0.77 (d, *J* = 6.4 Hz, 2.75H).

### Example 11.

### {(S)-2-Methyl-1-[(S)-2-(5-naphthalen-1-yl-1H-imidazol-2-yl)-pyrrolidine-1-carboline]-propyl}-carbamic acid methyl ester 43.

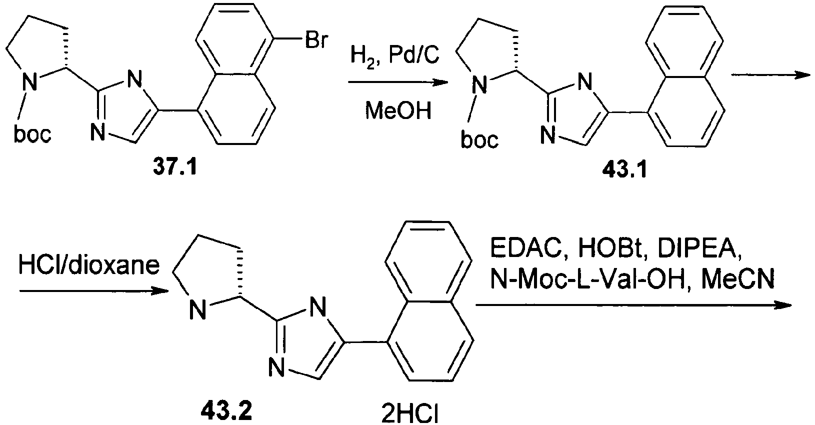

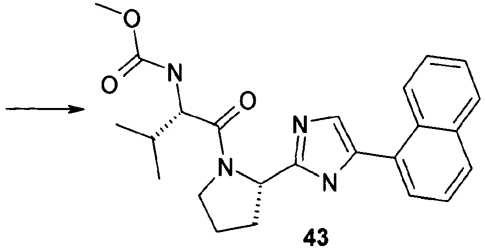

Compound **43.1** was prepared according to the method given for the preparation of compound **42.2.** Yield is 92 %. LCMS (ESI): *m*/*z* 364 (M+H)⁺. Compound **43.1** was boc-deprotected with 3M HCl solution in dioxane with quantitative yield, it gave compound **43.2** (LCMS (ESI): *m*/*z* 264 (M+H)⁺), which was converted into compound **43** (LCMS (ESI): *m*/*z* 421 (M+H)⁺)·by analogy with the conversion of compound **14.5** into compound **14.** Dihydrochloride **43** was prepared by addition of excess of 3M HCl solution in dioxane to solution of base **42** in CH₂Cl₂ and precipitation with ether.

### Example 12.

{(S)-2-Methyl-1-[(S)-2-(5-naphthalen-2-yl-1H-imidazol-2-yl)-pyrrolidine-1-carboline]-propyl}-carbamic acid methyl ester **44.**

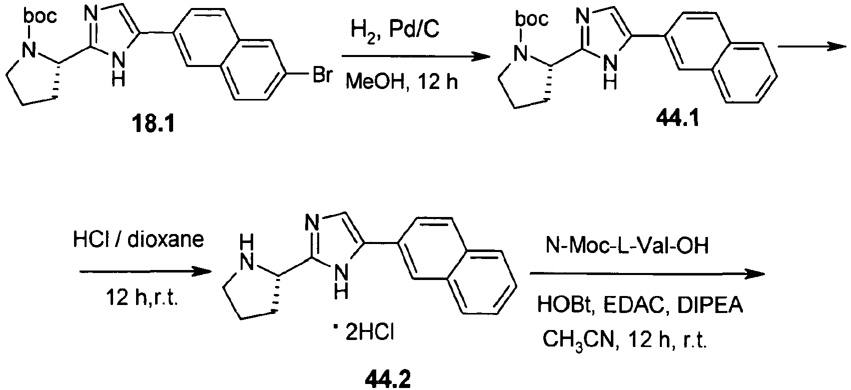

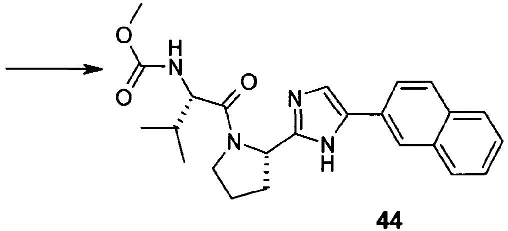

Synthesis of compound **44** was carried out by analogy with the synthesis of compound **43.** Compound **44.1** was prepared with yield of 97%. LCMS (ESI): *m*/*z* 364,4 (M+H)⁺. NMR (DMSO-*d*₆, 400 MHz) δ 14.6 (br. s, 1,5H), 8.36 (s, 1H), 8.22 (d, *J*=*21.6 Hz,* 1 H), 8.09 (d, *J*=*18.8 Hz,* 1 H), 7.97 (m, 2H), 7.91 (d, *J*=8.8 *Hz,* 1 H), 7.60 (m, 2H), 5.07 (m, 1H), 7.20 (m, 1 H), 3.44 (m, 1 H), 2.43 (br. m, 1H), 2.08 (m, 1 H), 1.98 (m, 2H), 1.41 (s, 3.5H), 1.18 (s, 5.5H). Yield of compound **44.2** is 94%. LCMS (ESI): *m*/*z* 264,3 (M+H)⁺. NMR (DMSO-*d*₆, 400 MHz) δ 10.09 (br. s, 1 H), 9.58 (br. s, 0.6H), 8.43 (s, 1 H), 8.11 (s, 1 H), 8.00 (br. s, 2H), 7.93 (br. m, 2H), 7.54 (m, 2H), 4.97 (br. m, 1H), 3.40 (br. m, 1 H), 2.42 (m, 1 H), 2.20 (br. m, 1 H), 2.03 (m, 2H). Yield of compound **44** is 33%. Dihydrochloride **44·2HCl** was prepared by addition of excess of 3M HCl solution in dioxane to solution of base **44** in CH₂Cl₂ and precipitation with ether. LCMS (ESI): *m*/*z* 421,2 (M+H)⁺. NMR (DMSO-*d*₆, 400 MHz) δ 15.02 (br. s, 1 H), 14.67 (br. s, 1 H), 8.43 (s, 1H), 8.18 (s, 1 H), 8.06 (d, *J =* 8.8 *Hz,* 1 H), 7.94 (m, 3H), 7.59 (m, 2H), 7.29 (d, *J =* 8.4 *Hz,* 1 H), 5.19 (t, *J* = 6.6 *Hz,* 1 H), 4.13 (t, *J* = 7.6 *Hz,* 1 H), 3.96 (br. m, 1 H), 3.86 (br. m, 1 H), 3.56 (s, 0.8H), 3.54 (s, 2.2H), 2.40 (m, 1 H), 2.18 (br. m, 2H), 2.05 (m, 2H), 0.91 (m, 0.6H), 0.81 (d. d, *J*₁ = 27.2 *Hz, J*₂ = 6.4 *Hz,* 5.4H).

### Example 13.

{(S)-2-Methyl-1-[(S)-2-(5-thiophen-2-yl)-1H-(imidazol-2-yl)-pyrrolidine-1-carboline]-propyl}-carbamic acid methyl ester **58** and {(S)-2-methyl-1-[(S)-2-[5-(2,2'-bithiophen-5-yl)-1H-imidazol-2-yl]-pyrrolidine-1-carboline]-propyl}-carbamic acid methyl ester **59.**

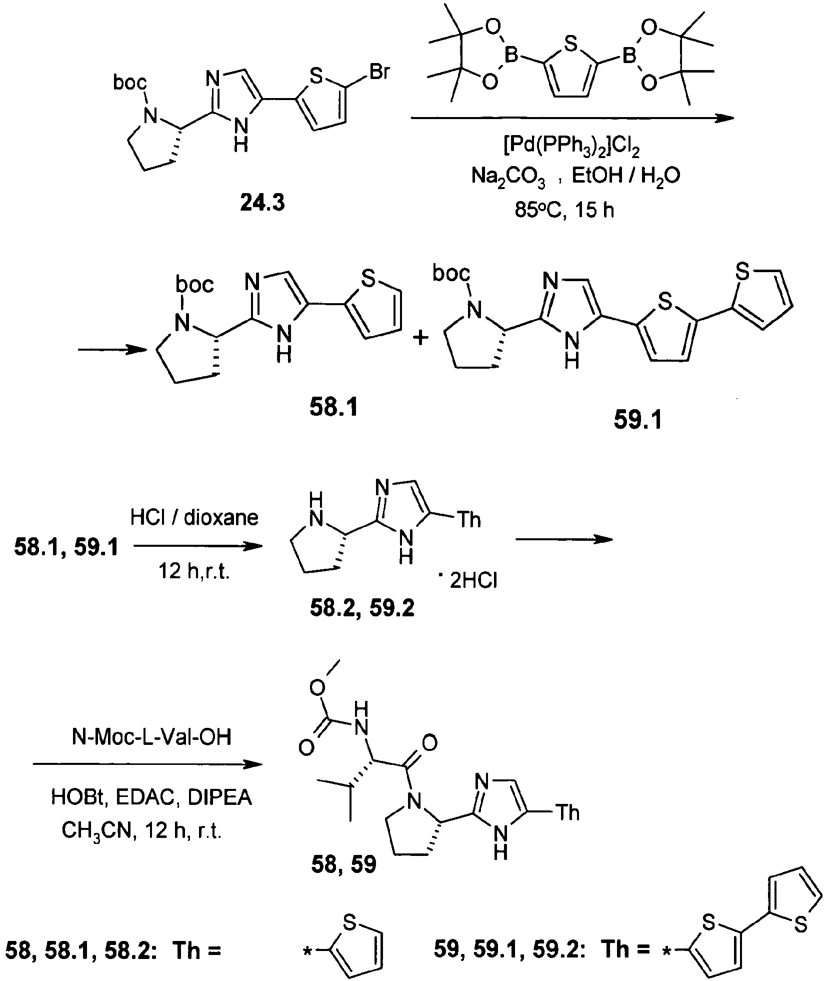

Boronic ester (256 mg, 0.762 mmol, 1 eq.) and Na₂CO₃ (404 mg, 3.81 mmol, 5 eq.) were suspended in mixture of ethanol (7.5 ml) and water (1.8 ml). Argon was passed through the suspension for 40 min at 90°C. Then the mixture was cooled to 80°C, compound **24.3** (667 mg, 1.68 mmol, 2.2 eq.) was added, and argon was passed for additional 10 min. Then [Pd(PPh₃)₂]Cl₂ (53 mg, 0.0762 mmol, 0.1 eq.) was added, and passing of the gas was prolonged for 5 min. At the end of this period NaBH₄ (2.9 mg, 0.0762 mmol, 0.1 eq.) was added to the reaction mixture.

It was stirred for 15 h at 85°C under argon. The completeness of the rection was controlled by LCMS method. After that the reaction mixture was filtered through the layer of Celite, filtrate was evaporated on rotary evaporator. The residue was dissolved in CH₂Cl₂, extract was washed with water, dried over Na₂SO₄ and evaporated on rotary evaporator. Compound **7** was purified by colomn chromatography (eluent - gradient hexane:THF: triethylamine = from 12:1:0.1 till 8:1:0.1). Compound **6** was purified by HPLC method. It gave compound **58.1** - 71 mg, yield 29%, compound **59.1** -120 mg, yield 39%.

Compound **58.1 -** LCMS (ESI): *m*/*z* 320,0 (M+H)⁺. NMR (CDCl₃-*d*₃, 400 MHz) δ 10.5 (br. s, 1.4H), 7.21 (br. m, 1H), 7.17 (d, *J* = 4.0 *Hz*, 1 H), 7.12 (s, 1 H), 7.02 (t, J = 4.0 *Hz,* 1 H), 4.96 (m, 1 H), 3.50 (br. m, 1 H), 3.41 (br. m, 1 H), 3.00 (br. m, 1 H), 2.15 (br. m, 2H), 1.96 (m, 1 H), 1.50 (s, 9H). Compound **59.1 -** LCMS (ESI): *m*/*z* 402,1 (M+H)⁺. Synthesis of compounds **58** and **59** was carried out by analogy with the synthesis of compound **14** from compound **14.5.** Dihydrochlorides **58** and **59** were prepared by addition of excess of 3M HCl solution in dioxane to solutions of bases **58** and **59** in CH₂Cl₂ respectively and precipitation with ether. Compound **58.2:** LCMS (ESI): *m*/*z* 220,1 (M+H)⁺. Compound 58: LCMS (ESI): *m*/*z* 377,2 (M+H)⁺. Compound **59.2:** LCMS (ESI): *m*/*z* 302,3 (M+H)⁺. NMR (DMSO-*d*₆, 400 MHz) δ 10.3 (br. s, 1 H), 9.48 (br. s, 1 H), 7.80 (s, 1 H), 7.52 (d, *J* = 4.8 *Hz,* 1 H), 7.44 (d, *J* = 3.2 *Hz,* 1H), 7,32 (d, *J* = 4.8 *Hz,* 1H), 7.29 (t, *J* = 3.6 *Hz,* 1H), 7.10 (d.d., *J*₁ = 4.8 *Hz, J*₂ = 4.0 *Hz,* 1 H), 4,84 (br. m, 1H), 3.34 (br. m, 2H), 2.41 (m, 1H), 2.33 (m, 1 H), 2.15 (m, 1 H), 1.99 (m, 1 H). Compound 59: LCMS (ESI): *m*/*z* 459,5 (M+H)⁺. NMR (DMSO-*d*₆, 400 MHz) δ 14.45 (br. s, 1 H), 7.90 (s, 1 H), 7.58 (m, 2H), 7.37 (s, 2H), 7.28 (d, *J =* 8.4 *Hz,* 1 H), 7.13 (m, 1 H), 5.09 (t, *J =* 6.4 *Hz*, 1 H), 4.10 (t, *J* = 7.2 *Hz,* 1 H), 3.90 (br. m, 1 H), 3.84 (br. m, 1H), 3.56 (s, 0.5H), 3.54 (s, 2.5H), 2.35 (m, 1 H), 2.15 (br. m, 2H), 2.02 (m, 2H), 0.88 (m, 0.7H), 0.80 (d. d, *J*₁ = 23.0 *Hz, J*₂ = 6.6 *Hz,* 5.3H).

[(1*S*)-1-[[(2*R*)-2-(5-Ethynyl-1*H*-imidazol-2-yl)-1-pyrrolidinyl]carbonyl]-2-methylpropyl]-carbamic acid methyl ester, LCMS (M+1) 319.

[(1*S*)-2-Methyl-1-[[(2*R*)-2-[5-(2-propynyl)-1*H*-imidazol-2-yl]-1-pyrrolidinyl]carbonyl]propyl]-carbamic acid methyl ester, LCMS (M+1) 333.

[(1*S*)-1-[[(2*R*)-2-[5-(3-Butynyl)-1*H*-imidazol-2-yl]-1-pyrrolidinyl]carbonyl]-2-methylpropyl]-carbamic acid methyl ester, LCMS (M+1) 347.

[(1*S*)-1-[[(2*R*)-2-[5-(2-Butynyl)-1*H*-imidazol-2-yl]-1-pyrrolidinyl]carbonyl]-2-methylpropyl]-carbamic acid methyl ester, LCMS (M+1) 347.

[(1*S*)-1-[[(2*S*)-2-[5-(1,3-Butadiynyl)-1*H*-imidazol-2-yl]-1-pyrrolidinyl]carbonyl]-2-methylpropyl]-carbamic acid methyl ester, compound 39, LCMS (M+1) 343. ¹H NMR (DMSO-D6, 400 MHz) δ 0.88 (t, J₁ = 6.50, J₂ = 0.93, 3H), 0.95 (t, J₁ = 6.75, J₂ = 0.93, 3H), 1.64 (m, 1H), 1.85 (m, 1H), 1.92 (s, 1 H), 1.99 (m, 2H), 2.10 (m, 1 H), 3.56 (m, 1 H), 3.61 (d, J = 9.40, 3H), 3.68 (m, 1 H), 4.03 (d, J = 0.42, 1 H), 4.73 (m, 1 H), 7.25 (s, 1 H), 8.80 (m, 2H).

[(1*S*)-2-Methyl-1-[[(2*S*)-2-[5-(2,4-pentadiynyl)-1*H*-imidazol-2-yl]-1-pyrrolidinyl]carbonyl]propyl]-carbamic acid methyl ester, LCMS (M+1) 357.

[(1*S*)-2-Methyl-1-[[(2*S*)-2-[5-(1,3-pentadiynyl)-1*H*-imidazol-2-yl]-1-pyrrolidinyl]carbonyl]propyl]-carbamic acid methyl ester, LCMS (M+1) 357.

[(1*R*)-2-Methyl-1-[[(2*R*)-2-[5-(1,4-pentadiynyl)-1*H*-imidazol-2-yl]-1-pyrrolidinyl]carbonyl]propyl]-carbamic acid methyl ester, LCMS (M+1) 357.

[(1*R*)-1-[[(2*R*)-2-[5-(3,5-Hexadiynyl)-1*H*-imidazol-2-yl]-1-pyrrolidinyl]carbonyl]-2-methylpropyl]-carbamic acid methyl ester, LCMS (M+1) 371.

[(1*S*)-1-[[(2*S*)-2-[5-(1,3-Hexadiynyl)-1*H*-imidazol-2-yl]-1-pyrrolidinyl]carbonyl]-2-methylpropyl]-carbamic acid methyl ester, LCMS (M+1) 371.

[(1 *S*)-1-[[(2*S*)-2-[5-(1,5-Hexadiynyl)-1*H*-imidazol-2-yl]-1-pyrrolidinyl]carbonyl]-2-methylpropyl]-carbamic acid methyl ester, LCMS (M+1) 371.

[(1*S*)-1-[[(2*S*)-2-[5-(1,3,5-Hexatriynyl)-1*H*-imidazol-2-yl]-1-pyrrolidinyl]carbonyl]-2-methylpropyl]-carbamic acid methyl ester, compound 40, LCMS (M+1) 367. ¹H NMR (DMSO-D6, 400 MHz) δ 0.88 (t, J₁ = 6.50, J₂ = 0.93, 3H), 0.95 (t, J₁ = 6.75, J₂ = 0.93, 3H), 1.64 (m, 1 H), 1.85 (m, 1 H), 1.92 (s, 1 H), 1.99 (m, 2H), 2.10 (m, 1 H), 3.56 (m, 1 H), 3.61 (d, J = 9.40, 3H), 3.68 (m, 1 H), 4.03 (d, J = 0.42, 1 H), 4.73 (m, 1 H), 7.28 (s, 1 H), 8.80 (m, 2H).

[(1*S*)-2-Methyl-1-[[(2*S*)-2-[5-(2-naphthalenyl)-1*H*-imidazol-2-yl]-1-pyrrolidinyl]carbonyl]propyl]-carbamic acid methyl ester, compound 44, LCMS (M+1) 421. ¹H NMR (DMSO-D6, 400 MHz) δ 0.88 (t, J₁ = 6.50, J₂ = 0.93, 3H), 0.95 (t, J₁ = 6.75, J₂ = 0.93, 3H), 1.64 (m, 1 H), 1.85 (m, 1 H), 1.96 (m, 1 H), 2.10 (m, 1 H), 3.56 (m, 1 H), 3.61 (d, J = 9.40, 3H), 3.63 (m, 2H), 4.03 (m, 1 H), 4.73 (m, 1 H), 6.94 (s, 1 H), 7.57 (t, J₁ = 7.00, J₂ = 0.28, 2H), 7.88 (t, J₁ = 8.13, J₂ = 1.48, 2H), 7.99 (t, J₁ = 8.13, J₂ = 0.63, 1H), 8.18 (m, 1 H), 8.58 (m, 1 H), 8.79 (m, 2H).

[(1*S*)-2-Methyl-1-[[(2*S*)-2-(5-[1,1':4',1 "-terphenyl]-4-yl-1*H*-imidazol-2-yl)-1-pyrrolidinyl]carbonyl]propyl]-carbamic acid methyl ester, compound 45, LCMS (M+1) 523. ¹H NMR (DMSO-D6, 400 MHz) δ 0.88 (t, J₁ = 6.50, J₂ = 0.93, 3H), 0.95 (t, J₁ = 6.75, J₂ = 0.93, 3H), 1.64 (m, 1H), 1.85 (m, 1 H), 1.96 (m, 2H), 2.10 (m, 1 H), 3.56 (m, 1 H), 3.61 (d, J = 9.40, 3H), 3.66 (m, 1 H), 4.03 (m, 1 H), 4.73 (m, 1 H), 6.79 (s, 1H), 7.02 (d, J = 8.60, 2H), 7.36 (t, J₁ = 7.13, J₂ = 1.44, 3H), 7.53 (m, 4H), 7.62 (d, J = 8.60, 2H), 7.67 (m, 2H), 8.79 (m, 2H).

[(1*S*)-2-Methyl-1-[[(2*S*)-2-[5-(1-naphthalenyl)-1*H*-imidazol-2-yl]-1-pyrrolidinyl]carbonyl]propyl]-carbamic acid methyl ester, compound 43, LCMS (M+1) 421. ¹H NMR (DMSO-D6, 400 MHz) δ 0.88 (t, J₁ = 6.50, J₂ = 0.93, 3H), 0.95 (t, J₁ = 6.75, J₂ = 0.93, 3H), 1.64 (m, 1H), 1.85 (m, 1H), 1.92 (s, 1 H), 1.99 (m, 1H), 2.10 (m, 1 H), 3.56 (m, 1 H), 3.61 (d, J = 9.40, 3H), 3.68 (m, 1 H), 4.03 (d, J = 0.42, 1 H), 4.73 (m, 1H), 7.04 (s, 1H), 7.46 (d, J = 0.28, 2H), 7.59 (d, J = 8.32, 1 H), 7.64 (d, J = 6.95, 1H), 7.77 (d, J = 7.72, 1H), 8.04 (t, J₁ = 8.13, J₂ = 1.46, 1H), 8.10 (m, 1H), 8.79 (m, 2H).

[(1 *S*)-2-Methyl-1-[[(2*S*)-2-[5-(1,1'-biphenyl)-1*H*-imidazol-2-yl]-1-pyrrolidinyl]carbonyl]propyl]-carbamic acid methyl ester, compound 49, LCMS (M+1) 447. ¹H NMR (DMSO-D6, 400 MHz) δ 0.88 (t, J₁ = 6.50, J₂ = 0.93, 3H), 0.95 (t, J₁ = 6.75, J₂ = 0.93, 3H), 1.64 (m, 1 H), 1.85 (m, 1 H), 1.96 (m, 2H), 2.10 (m, 1 H), 3.56 (m, 1 H), 3.61 (d, J = 9.40, 3H), 3.66 (m, 1 H), 4.03 (m, 1 H), 4.73 (m, 1 H), 6.79 (s, 1 H), 7.06 (d, J = 8.60, 2H), 7.36 (t, J₁ = 7.13, J₂ = 1.44, 1 H), 7.40 (m, 2H), 7.55 (m, 4H), 8.79 (m, 2H).

[(1*S*)-2-Methyl-1-[[(2*S*)-2-[5-(6-phenyl-2-naphthalenyl)-1*H*-imidazol-2-yl]-1-pyrrolidinyl]carbonyl]propyl]-carbamic acid methyl ester, LCMS (M+1) 497, compound 51 ¹H NMR (DMSO-D6, 400 MHz) δ 0.88 (t, J₁ = 6.50, J₂ = 0.93, 3H), 0.95 (t, J₁ = 6.75, J₂ = 0.93, 3H), 1.64 (m, 1 H), 1.85 (m, 1 H), 1.96 (m, 2H), 2.10 (m, 1H), 3.56 (m, 1H), 3.61 (d, J = 9.40, 3H), 3.66 (m, 1 H), 4.03 (m, 1 H), 4.73 (m, 1H), 6.94 (s, 1 H), 7.42 (t, J₁ = 7.13, J₂ = 1.44, 1 H), 7.65 (d, J = 7.58, 2H), 7.92 (d, J = 0.70, 1 H), 8.06 (t, J₁ = 2.03, J₂ = 0.62, 2H), 8.27 (m, 4H), 8.64 (s, 1 H), 8.79 (m, 2H).

[(1*S*)-2-Methyl-1-[[(2*S*)-2-[5-(5-phenyl-2-thienyl)-1H-imidazol-2-yl]-1-pyrrolidinyl]carbonyl]propyl]-carbamic acid methyl ester, LCMS (M+1) 453.

[(1R)-2-Methyl-1-[[(2R)-2-[5-[4-(2-thienyl)phenyl]-1H-imidazol-2-yl]-1-pyrrolidinyl]carbonyl]propyl]-carbamic acid methyl ester, LCMS (M+1) 453 ¹H NMR (DMSO-D6, 400 MHz) δ 0.88 (t, J₁ = 6.50, J₂ = 0.93, 3H), 0.95(t, J₁ = 6.75, J₂ = 0.93, 3H), 1.64 (m, 1H), 1.83 (t, J₁ = 9.72, J₂ = 1.00, 1 H), 1.98 (m, 2H), 2.13 (m, 2H), 3.56 (m, 1 H), 3.61 (d, J = 9.40, 3H), 3.68 (m, 1 H), 3.97 (d, J = 7.70, 1 H), 4.68 (m, 1H), 6.79 (s, 1H), 7.01 (t, J₁= 4.00, J₂=3.29, 1H), 7.32 (m, 2H), 7.41 (m, 2H), 7.60 (t, J₁=1.95, J₂=0.71, 2H), 8.43 (m, 1H);

[(1 *S*)-1-[[(2*R*)-2-[5-(5-[1,1'-Biphenyl]-4-yl-2-thienyl)-1*H*-imidazol-2-yl]-1-pyrrolidinyl]carbonyl]-2-methylpropyl]-carbamic acid methyl ester, LCMS (M+1) 529 ¹H NMR (DMSO-D6, 400 MHz) δ 0.87 (t, J₁ = 6.50, J₂ = 0.93, 3H), 0.97(t, J₁ = 6.50, J₂ = 0.93, 3H), 1.64 (m, 1 H), 1.83 (m, 1 H), 1.98 (m, 4H), 2.12 (m, 1 H), 3.56 (t, J₁ = 6.89, J₂ = 2.18, 1 H), 3.63 (s, 1 H), 3.67 (m, 2H), 4.03 (d, J = 8.50, 1 H), 4.68 (m, 2H), 6.62 (t, J₁ = 8.60, J₂ = 2.47, 2H), 6.92 (s, 1 H), 7.02 (s, 1 H), 7.11 (s, 1 H), 7.20 (t, J₁ = 2.42, J₂ = 0.71, 2H), 7.40 (t, J₁ = 7.58, J₂ = 1.39, 2H), 7.50 (t, J₁ = 2.03, J₂ = 0.62, 2H), 8.79 (m, 2H);

[(1*R*)-2-Methyl-1-[[(2*S*)-2-[5-[4'-(2-thienyl)[1,1'-biphenyl]-4-yl]-1*H*-imidazol-2-yl]-1-pyrrolidinyl]carbonyl]propyl]-carbamic acid methyl ester, LCMS (M+1) 543 ¹H NMR (DMSO-D6, 400 MHz) δ 0.88 (t, J₁ = 6.75, J₂ = 0.93, 3H), 0.95 (t, J₁ = 6.75, J₂ = 0.93, 3H), 1.64 (m, 1 H), 1.84 (m, 1 H), 1.99 (m, 2H), 2.10 (m, 1 H), 3.56 (m, 1 H), 3.61 (d, J = 9.40, 3H), 3.63 (m, 1 H), 3.96 (d, J =7.70, 1 H), 4.73 (m, 1 H), 6.62 (t, J₁ = 8.60, J₂ = 0.71, 2H), 6.79 (s, 1H), 7.02 (t, J₁ = 8.60, J₂ = 0.71, 3H), 7.12 (t, J₁ = 8.60, J₂ = 0.71, 3H), 7.32 (d, J = 1.36, 2H), 7.55 (t, J₁ = 8.60, J₂ = 1.95, 2H), 8.43 (m, 2H);

[(1*S*)-2-Methyl-1-[[(2*S*)-2-[5-[6-(2-thienyl)-2-naphthalenyl]-1*H*-imidazol-2-yl]-1-pyrrolidinyl]carbonyl]propyl]-carbamic acid methyl ester, LCMS (M+1) 517 ¹H NMR (DMSO-D6, 400 MHz) δ 0.88 (t, J₁ = 6.75, J₂ = 0.93, 3H), 0.96 (t, J₁ = 6.75, J₂ = 0.93, 3H), 1.64 (m, 1H), 1.84 (m, 1 H), 1.96 (m, 2H), 2.10 (m, 1 H), 3.56 (m, 1H), 3.61 (d, J = 9.40, 3H), 3.63 (m, 1 H), 4.02 (m, 1 H), 4.74 (m, 1H), 6.94 (s, 1 H), 7.06 (d, J = 3.29, 1 H), 7.31 (t, J₁ = 8.50, J₂ = 0.33, 2H), 7.49 (t, J₁ = 3.29, J₂ = 1.36, 1 H), 7.82 (m, 1 H), 7.95 (m, 2H), 8.27 (t, J₁ = 8.50, J₂ = 0.56, 1H), 8.64 (m, 1H), 8.79 (m, 2H).

[(1*S*)-2-Methyl-1-[[(2*S*)-2-[5-[5-(2-naphthalenyl)-2-thienyl]-1*H*-imidazol-2-yl]-1-pyrrolidinyl]carbonyl]propyl]-carbamic acid methyl ester, LCMS (M+1) 517 ¹H NMR (DMSO-D6, 400 MHz) δ 0.88 (t, J₁ = 6.50, J₂ = 0.93, 3H), 0.96 (t, J₁ = 6.50, J₂ = 0.93, 3H), 1.64 (m, 1 H), 1.84 (m, 1 H), 1.99 (m, 2H), 2.10 (m, 1 H), 3.56 (m, 1H), 3.60 (d, J = 9.40, 3H), 3.66 (m, 1 H), 4.02 (m, 1 H), 4.74 (m, 1 H), 6.92 (s, 1 H), 7.08 (d, J = 3.29, 1 H), 7.17 (d, J = 4.76, 1 H), 7.32 (m, 1 H), 7.56 (t, J₁ = 8.13, J₂ = 0.21, 2H), 7.73 (d, J = 8.50, 1 H), 7.82 (t, J₁ = 1.65, J₂ = 0.56, 1H), 7.82 (m, 1H), 7.91 (m, 2H), 8.79 (m, 2H).

[(1*R*)-2-Methyl-1-[[(2*R*)-2-[5-[5-[6-(2-thienyl)-2-naphthalenyl]-2-thienyl]-1H-imidazol-2-yl]-1-pyrrolidinyl]carbonyl]propyl]-carbamic acid methyl ester, LCMS (M+1) 599 ¹H NMR (DMSO-D6, 400 MHz) δ 0.88 (t, J₁ = 6.50, J₂ = 0.93, 3H), 0.9 (t, J₁ = 6.50, J₂ = 0.93, 3H), 1.64 (m, 1 H), 1.83 (m, 1 H), 1.99 (m, 2H), 2.10 (m, 1 H), 3.56 (m, 1 H), 3.61 (d, J = 9.40, 3H), 3.63 (m, 1 H), 3.97 (d, J = 7.70, 1 H), 4.68 (m, 1 H), 6.92 (s, 1 H), 7.07 (d, J = 4.76, 2H), 7.17 (d, J = 4.76, 1 H), 7.32 (t, J₁ = 8.50, J₂ = 1.56, 2H), 7.36 (m, 1 H), 7.49 (t, J₁ = 3.29, J₂ = 1.36, 1 H), 7.82 (d, J = 0.42, 2H), 7.90 (d, J = 0.43, 2H), 8.43 (m, 2H).

1-[(2*S*)-2-Phenyl-2-(1-piperidinyl)acetyl]-2-[5-[4-(5-phenyl-2-thienyl)phenyl]-1*H-*imidazol-2-yl]-(2*S*)-pyrrolidine, LCMS (M+1) 573 ¹H NMR (DMSO-D6, 400 MHz) δ 1.67 (m, 7H), 1.84 (m, 2H), 2.06 (m, 3H), 2.65 (m, 1 H), 2.73 (m, 1 H), 3.45 (m, 1 H), 3.53 (m, 1 H), 3.92 (s, 1 H), 4.64 (s, 1H), 6.79 (s, 1H), 6.93 (d, J = 4.00, 1 H), 7.01 (d, J = 4.00, 1 H), 7.22 (m, 5H), 7.46 (m, 3H), 7.53 (m, 4H), 7.63 (m, 2H), 11.18 (m, 1H);

2-[5-(4-[2,2'-Bithiophen]-5-ylphenyl)-1*H*-imidazol-2-yl]-1-[(2R)-2-phenyl-2-(1-piperidinyl)acetyl]-(2R)-pyrrolidine, LCMS (M+1) 579 ¹H NMR (DMSO-D6, 400 MHz) δ 1.54 (m, 2H), 1.67 (m, 2H), 1.83 (m, 2H), 1.98 (m, 1 H), 2.10 (m, 1H), 2.62 (m, 2H), 2.72 (m, 2H), 3.48 (m, 2H), 3.53 (m, 2H), 3.93 (s, 1H), 4.64 (m, 1H), 6.79 (s, 1 H), 7.00 (s, 1 H), 7.09 (d, J = 4.00, 1 H), 7.22 (m, 4H), 7.38 (d, J = 0.71, 2H), 7.43 (m, 1H), 7.50 (d, J = 8.06, 2H), 7.56 (m, 2H), 7.68 (d, J = 4.00, 1 H), 11.18 (m, 1H);

*N*-[(1*S*)-2-Oxo-1-phenyl-2-[(2*S*)-2-[5-(5'-phenyl[2,2'-bithiophen]-5-yl)-1*H*-imidazol-2-yl]-1-pyrrolidinyl]ethyl]-cyclopropanecarboxamide, LCMS (M+1) 579 ¹H NMR (DMSO-D6, 400 MHz) δ 0.83 (m, 2H), 0.93 (m, 2H), 1.64 (m, 1 H), 1.76 (s, 1 H), 1.84 (m, 1H), 1.99 (m, 1H), 2.10 (m, 1H), 3.44 (d, J = 4.78, 1H), 3.50 (t, J₁ = 10.60, J₂ = 4.78, 1 H), 4.62 (m, 1 H), 5.17 (s, 1 H), 6.51 (d, J = 4.00, 1 H), 6.92 (d, J = 0.25, 1 H), 7.17 (m, 4H), 7.27 (m, 6H), 7.56 (d, J = 7.78, 2H), 7.69 (d, J = 4.00, 1 H), 9.72 (m, 2H);

*N*-[(1*R*)-2-Oxo-1-phenyl-2-[(2*R*)-2-[5-[5-[4-(2-thienyl)phenyl]-2-thienyl]-1H-imidazol-2-yl]-1-pyrrolidinyl]ethyl]-cyclopropanecarboxamide, LCMS (M+1) 579 ¹H NMR (DMSO-D6, 400 MHz) δ 0.84 (m, 2H), 0.91 (m, 2H), 1.64 (m, 1 H), 1.76 (s, 1 H), 1.83 (t, J₁ = 6.89, J₂ = 1.00, 1 H), 1.99 (m, 1 H), 2.10 (t, J₁ = 12.93, J₂ = 3.40, 1 H), 3.43 (d, J = 4.78, 1 H), 3.51 (t, J₁ = 10.35, J₂ = 4.78, 1H), 4.55 (m, 1 H), 5.17 (d, J = 0.42, 1 H), 6.95 (d, J = 8.25, 1 H), 7.04 (m, 5H), 6.98 (t, J₁ = 8.25, J₂ = 0.71, 2H), 7.30 (m, 2H), 9.55 (m, 2H).

2-[5-[4'-(2-Naphthalenyl)[1,1'-biphenyl]-4-yl]-1*H*-imidazol-2-yl]-1-[(2*R*)-2-phenyl-2-(1-piperidinyl)acetyl]-(2S)-pyrrolidine, LCMS (M+1) 617 ¹H NMR (DMSO-D6, 400 MHz) δ 1.57 (m, 1H), 1.65 (m, 4H), 1.75 (m, 2H), 1.84 (m, 1 H), 1.99 (m, 1 H), 2.10 (m, 1H), 2.65 (m, 2H), 2.73 (m, 2H), 3.45 (m, 1H), 3.53 (m, 1H), 3.96 (d, J = 0.42, 1 H), 4.63 (s, 1 H), 6.79 (s, 1 H), 7.02 (t, J₁ = 8.60, J₂ = 0.71, 2H), 7.22 (m, 2H), 7.38 (t, J₁ = 7.03, J₂ = 1.46, 2H), 7.43 (t, J₁ = 7.32, J₂ = 1.46, 1 H), 7.56 (m, 3H), 7.61 (m, 2H), 7.64 (t, J₁ = 8.60, J₂ = 0.71, 2H), 7.89 (d, J = 0.70, 2H), 7.98 (d, J = 8.50, 1 H), 8.16 (d, J = 1.65, 1 H), 8.20 (m, 1 H), 11.18 (m, 1H).

[(1*S*)-2-Methyl-1-[[(2*S*)-2-[5-(4-phenyl-1,3-butadiynyl)-1*H*-imidazol-2-yl]-1-pyrrolidinyl]carbonyl]propyl]-carbamic acid methyl ester, compound 54, LCMS (M+1) 419, ¹H NMR (DMSO-D6, 400 MHz) δ 0.87 (t, J₁ = 6.50, J₂ = 0.93, 3H), 0.97 (t, J₁ = 6.50, J₂ = 0.93, 3H), 1.61 (m, 1 H), 1.84 (m, 1H), 1.96 (m, 2H), 2.10 (m, 1 H), 3.56 (m, 1H), 3.64 (br.d, 4H), 4.02 (m, 1 H), 4.74 (m, 1 H), 7.24 (d, J = 7.73, 3H), 7.33 (s, 1 H), 7.47 (br. t, 2H), 8.79 (m, 2H).

2-[5-(4-Phenyl-1,3-butadiynyl)-1*H*-imidazol-2-yl]-1-[(2*R*)-2-phenyl-2-(1-piperidinyl)acetyl]-(2R)-pyrrolidine, LCMS (M+1) 463 ¹H NMR (DMSO-D6, 400 MHz) δ 1.52 (m, 1 H), 1.67 (m, 4H), 1.75 (m, 2H), 1.84 (m, 1 H), 1.99 (m, 1 H), 2.10 (m, 1 H), 2.65 (m, 2H), 2.73 (m, 2H), 3.20 (m, 1 H), 3.31 (m, 1 H), 3.92 (s, 1 H), 4.64 (m, 1 H), 7.24 (m, 5H), 7.33 (s, 1 H), 7.39 (m, 2H), 7.43 (d, J = 7.32, 1 H), 7.47 (t, J₁ = 7.73, J₂ = 1.76, 2H), 11.20 (m, 1 H).

[(*S*)-2-Methyl-1-[[(2*S*)-2-[5-(6-phenyl-1,3,5-hexatriynyl)-1*H*-imidazol-2-yl]-1-pyrrolidinyl]carbonyl]propyl]-carbamic acid methyl ester, compound 57, LCMS (M+1) 443, ¹H NMR (DMSO-D6, 400 MHz) δ 0.87 (t, J₁ = 6.50, J₂ = 0.93, 3H), 0.97 (t, J₁ = 6.50, J₂ = 0.93, 3H), 1.61 (m, 1 H), 1.84 (m, 1H), 1.96 (m, 2H), 2.10 (m, 1 H), 3.56 (m, 1 H), 3.64 (br.d, 4H), 4.02 (m, 1 H), 4.74 (m, 1 H), 7.26 (m, 4H), 7.43 (d, J = 1.36, 2H), 8.80 (m, 2H).

*N*-[(1*S*)-2-Oxo-1-phenyl-2-[(2*R*)-2-[5-(6-phenyl-1,3,5-hexatriynyl)-1*H*-imidazol-2-yl]-1-pyrrolidinyl]ethyl]-cyclopropanecarboxamide, LCMS (M+1) 487 ¹H NMR (DMSO-D6, 400 MHz) δ 0.84 (m, 2H), 0.92 (m, 2H), 1.64 (m, 1 H), 1.76 (m, 1 H), 1.83 (m, 1H), 1.96 (m, 1H), 2.10 (m, 1 H), 3.43 (m, 1H), 3.50 (m, 1H), 4.56 (m, 1 H), 5.17 (s, 1 H), 7.17 (t, J₁ = 2.28, J₂ = 0.71, 2H), 7.26 (m, 7H), 7.42 (t, J₁ = 7.73, J₂ = 1.36, 2H), 9.73 (m, 2H).

*N*-[(1*R*)-2-[(2*S*)-2-[5-(4-[1,1'-Biphenyl]-4-yl-1,3-butadiynyl)-1*H*-imidazol-2-yl]-1-pyrrolidinyl]-2-oxo-1-phenylethyl]-cyclopropanecarboxamide, LCMS (M+1) 539 ¹H NMR (DMSO-D6, 400 MHz) δ 0.82 (m, 2H), 0.92 (m, 2H), 1.64 (m, 1 H), 1.76 (s, 1H), 1.84 (m, 1H), 1.99 (m, 1H), 2.10 (m, 1H), 3.43 (m, 1H), 3.50 (m, 1H), 4.62 (m, 1H), 5.17 (s, 1 H), 7.17 (t, J₁ = 2.28, J₂ = 0.71, 2H), 7.26 (m, 6H), 7.36 (t, J₁ = 7.13, J₂ = 1.44, 1 H), 7.40 (t, J₁ = 7.58, J₂ = 1.39, 2H), 7.53 (m, 2H), 7.78 (m, 2H), 9.56 (m, 2H).

*N*-Methyl-*N*-[(1*S*)-1-[5-[4-(2-naphthalenyl)-1,3-butadiynyl]-1*H*-imidazol-2-yl]ethyl]-□-phenyl-(□*R*)-1-piperidineacetamide, LCMS (M+1) 501 ¹H NMR (DMSO-D6, 400 MHz) δ 1.40 (d, J = 7.12, 3H), 1.55 (m, 1 H), 1.62 (m, 3H), 1.75 (m, 2H), 2.61 (m, 2H), 2.73 (m, 2H), 2.97 (t, J₁ = 14.23, J₂ = 1.50, 3H), 3.94 (s, 1 H), 5.09 (d, J = 0.30, 1 H), 7.22 (t, J₁ = 7.80, J₂ = 0.71, 2H), 7.28 (s, 1H), 7.37 (m, 2H), 7.42 (t, J₁ = 7.32, J₂ = 2.10, 1H), 7.53 (m, 2H), 7.62 (t, J₁ = 8.13, J₂ = 0.28, 1H), 7.72 (d, J = 8.56, 1 H), 7.88 (m, 2H), 8.02 (d, J = 1.66, 1 H), 11.20 (m, 1H).

*N*-Methyl-□-phenyl-*N*-[(1*R*)-1-[5-(2-thienylethynyl)-1*H*-imidazol-2-yl]ethyl]-, (□*S*)-1-piperidineacetamide, LCMS (M+1) 433 ¹H NMR (DMSO-D6, 400 MHz) δ 1.40 (d, J = 7.12, 3H), 1.54 (m, 1 H), 1.62 (m, 1 H), 1.67 (m, 2H), 1.75 (m, 2H), 2.63 (m, 2H), 2.73 (m, 2H), 2.97 (t, J₁ = 14.23, J₂ = 1.50, 3H), 3.97 (m, 1 H), 5.39 (m, 2H), 6.77 (s, 1H), 6.89 (d, J = 3.79, 1H), 7.13 (d, J = 4.76, 1 H), 7.22 (d, J = 7.03, 2H), 7.37 (m, 3H), 7.43 (t, J₁ = 7.32, J₂ = 1.46, 1 H), 11.20 (m, 1 H).

*N*-Methyl-□-phenyl-*N*-[(1*S*)-1-[5-[4-(2-thienyl)-1,3-butadiynyl]-1*H*-imidazol-2-yl]ethyl]-, (□*S*)- 1-piperidineacetamide, LCMS (M+1) 457 ¹H NMR (DMSO-D6, 400 MHz) δ 1.41 (t, J₁ = 7.12, J₂ = 6.97, 3H), 1.54 (m, 1 H), 1.62 (m, 3H), 1.75 (m, 2H), 2.65 (m, 2H), 2.73 (m, 2H), 2.97 (t, J₁ = 14.23, J₂ = 1.50, 3H), 3.97 (m, 1 H), 5.07 (d, J = 0.30, 1 H), 6.82 (t, J₁ = 4.76, J₂ = 3.79, 1 H), 7.06 (d, J = 1.10, 1 H), 7.22 (d, J = 7.03, 2H), 7.28 (s, 1H),7.38 (t, J₁ = 7.32, J₂ = 0.71, 2H), 7.44 (t, J₁ = 7.32, J₂ = 1.46, 1 H), 7.79 (d, J = 3.79, 1 H), 11.20 (m, 1H).

*N*-Methyl-□-phenyl-*N*-[(1*R*)-1-[5-[5-(phenylethynyl)-2-thienyl]-1*H*-imidazol-2-yl]ethyl]-, (□*R*)- 1-piperidineacetamide, LCMS (M+1) 509 ¹H NMR (DMSO-D6, 400 MHz) δ 1.39 (t, J₁ = 7.12, J₂ = 6.81, 3H), 1.52 (m, 1H), 1.67 (m, 3H), 1.75 (m, 2H), 2.66 (m, 2H), 2.73 (m, 2H), 2.97 (t, J₁ = 14.23, J₂ = 1.50, 3H), 3.94 (m, 1 H), 5.69 (d, J = 0.30, 1 H), 6.60 (d, J = 0.25, 1 H), 6.95 (s, 1 H), 7.17 (d, J = 3.50, 1H), 7.22 (d, J = 7.80, 2H), 7.33 (m, 5H), 7.42 (t, J₁ = 7.32, J₂ = 2.10, 1 H), 7.50 (t, J₁ = 1.76, J₂ = 0.63, 2H), 11.18 (m, 1 H).

*N*-Methyl-□-phenyl-*N*-[(1*R*)-1-[5-[4-(2-thienylethynyl)phenyl]-1H-imidazol-2-yl]ethyl]-, (□*R*)-1-piperidineacetamide, LCMS (M+1) 509 ¹H NMR (DMSO-D6, 400 MHz) δ 1.39 (t, J₁ = 7.12, J₂ = 6.81, 3H), 1.52 (m, 1H), 1.67 (m, 3H), 1.75 (m, 2H), 2.66 (m, 2H), 2.73 (m, 2H), 2.97 (t, J₁ = 14.23, J₂ = 1.50, 3H), 3.94 (m, 1H), 5.69 (d, J = 0.30, 1 H), 6.81 (s, 1 H), 6.90 (d, J = 3.79, 1 H), 7.13 (d, J = 1.10, 1 H), 7.22 (t, J₁ = 7.80, J₂ = 0.71, 2H), 7.28 (d, J = 3.79, 1H), 7.37 (t, J₁ = 7.80, J₂ = 0.71, 2H), 7.43 (t, J₁ = 7.32, J₂ = 2.10, 1H), 7.79 (t, J₁ = 8.26, J₂ = 0.71, 2H), 7.98 (t, J₁ = 1.95, J₂ = 0.71, 2H), 11.18 (m, 1H).

[(1*S*)-2-Methyl-1-[[(2*S*)-2-[5-[6-(2-thienyl)-1,3,5-hexatriynyl]-1*H*-imidazol-2-yl]-1-pyrrolidinyl]carbonyl]propyl]-carbamic acid methyl ester, LCMS (M+1) 449 ¹H NMR (DMSO-D6, 400 MHz) δ 0.87 (t, J₁ = 6.50, J₂ = 0.93, 3H), 0.97 (t, J₁ = 6.50, J₂ = 0.93, 3H), 1.64 (m, 1 H), 1.84 (m, 1 H), 1.95 (d, J = 8.50, 1 H), 1.99 (m, 1 H), 2.10 (m, 1 H), 3.56 (m, 1 H), 3.61 (d, J = 9.40, 3H), 3.63 (m, 1 H), 4.02 (d, J = 0.42, 1 H), 4.76 (m, 1H), 6.84 (d, J = 3.79, 1 H), 7.07 (d, J = 1.10, 1 H), 7.28 (s, 1 H), 7.74 (d, J = 3.79, 1H), 8.80 (m, 2H).

[(1*S*)-2-Methyl-1-[[(2*S*)-2-[5-[4-(phenylethynyl)phenyl]-1*H*-imidazol-2-yl]-1-pyrrolidinyl]carbonyl]propyl]-carbamic acid methyl ester, LCMS (M+1) 471 ¹H NMR (DMSO-D6, 400 MHz) δ 0.88 (t, J₁ = 6.50, J₂ = 0.93, 3H), 0.98 (t, J₁ = 6.50, J₂ = 0.93, 3H), 1.66 (m, 1 H), 1.86 (m, 1 H), 1.96 (d, J = 8.50, 1 H), 2.00 (m, 1 H), 2.12 (m, 1 H), 3.56 (m, 1H), 3.61 (d, J = 9.40, 3H), 3.63 (m, 1 H), 4.02 (d, J = 0.42, 1H), 4.76 (m, 1 H), 6.86 (s, 1 H), 7.22 (d, J = 7.73, 3H), 7.48 (t, J₁ = 1.76, J₂ = 0.63, 2H), 7.79 (t, J₁ = 8.26, J₂ = 0.71, 2H), 7.98 (t, J₁ = 1.95, J₂ = 0.71, 2H), 8.79 (m, 2H).

[(1*S*)-1-[[(2*S*)-2-[5-[4-([1,1'-Biphenyl]-4-ylethynyl)phenyl]-1*H*-imidazol-2-yl]-1-pyrrolidinyl]carbonyl]-2-methylpropyl]-carbamic acid methyl ester, LCMS (M+1) 547 ¹H NMR (DMSO-D6, 400 MHz) δ 0.88 (t, J₁ = 6.50, J₂ = 0.93, 3H), 0.98 (t, J₁ = 6.50, J₂ = 0.93, 3H), 1.66 (m, 1H), 1.86 (m, 1 H), 1.96 (d, J = 8.50, 1 H), 2.00 (m, 1 H), 2.12 (m, 1H), 3.56 (m, 1 H), 3.61 (d, J = 9.40, 3H), 3.63 (m, 1 H), 4.02 (d, J = 0.42, 1H), 4.76 (m, 1 H), 6.86 (s, 1 H), 7.40 (m, 5H), 7.53 (t, J₁ = 2.03, J₂ = 0.63, 2H), 7.80 (m, 4H), 7.98 (m, 2H), 8.79 (m, 2H).

[(1*S*)-1-[[(2*S*)-2-[5-(4-[1,1'-Biphenyl]-4-yl-1,3-butadiynyl)-1*H*-imidazol-2-yl]-1-pyrrolidinyl]carbonyl]-2-methylpropyl]-carbamic acid methyl ester, compound 56, LCMS (M+1) 495, ¹H NMR (DMSO-D6, 400 MHz) δ 0.87 (t, J₁ = 6.50, J₂ = 0.93, 3H), 0.97 (t, J₁ = 6.50, J₂ = 0.93, 3H), 1.64 (m, 1 H), 1.84 (m, 1 H), 1.96 (m, 2H), 2.10 (m, 1 H), 3.56 (m, 1 H), 3.64 (br.d, 4H), 4.02 (m, 1 H), 4.74 (m, 1 H), 7.28 (t, J₁ = 8.26, J₂ = 0.71, 2H), 7.38 (m, 4H), 7.54 (t, J₁ = 2.03, J₂ = 0.62, 2H), 7.78 (t, J₁ = 2.02, J₂ = 0.71, 2H), 8.80 (m, 2H).

[(1*S*)-2-Methyl-1-[[(2*S*)-2-[5-[5-(2-thienylethynyl)-2-thienyl]-1*H*-imidazol-2-yl]-1-pyrrolidinyl]carbonyl]propyl]-carbamic acid methyl ester, compound 60, LCMS (M+1) 483. ¹H NMR (DMSO-D6, 400 MHz) δ 0.88 (t, J₁ = 6.50, J₂ = 0.93, 3H), 0.95 (t, J₁ = 6.75, J₂ = 0.93, 3H), 1.64 (m, 1 H), 1.85 (m, 1 H), 1.96 (m, 2H), 2.10 (m, 1H), 3.56 (m, 1H), 3.61 (d, J = 9.40, 3H), 3.66 (m, 1 H), 4.03 (m, 1 H), 4.73 (m, 1 H), 6.60 (t, J₁ = 4.78, J₂ = 0.25, 1 H), 6.90 (d, J = 4.76, 1 H), 7.00 (s, 1H), 7.13 (d, J = 1.10, 1 H), 7.19 (d, J = 4.78, 1 H), 7.29 (t, J₁ = 3.79, J₂ = 1.10, 1 H), 8.79 (m, 2H).

[(1*S*)-2-Methyl-1-[[(2*S*)-2-[5-[5-[4-(2-thienyl)-1,3-butadiynyl]-2-thienyl]-1*H*-imidazol-2-yl]-1-pyrrolidinyl]carbonyl]propyl]-carbamic acid methyl ester, compound 61, LCMS (M+1) 507, ¹H NMR (DMSO-D6, 400 MHz) δ 0.87 (t, J₁ = 6.50, J₂ = 0.93, 3H), 0.97 (t, J₁ = 6.50, J₂ = 0.93, 3H), 1.61 (m, 1 H), 1.84 (m, 1 H), 1.96 (m, 2H), 2.10 (m, 1H), 3.56 (m, 1 H), 3.64 (br.d, 4H), 4.02 (m, 1 H), 4.74 (m, 1 H), 6.54 (t, J₁ = 4.78, J₂ = 0.25, 1 H), 6.82 (t, J₁ = 4.76, J₂ = 3.79, 1H), 7.00 (s, 1 H), 7.06 (d, J = 1.10, 1 H), 7.67 (d, J = 4.78, 1 H), 7.79 (t, J₁ = 3.79, J₂ = 1.10, 1 H), 8.80 (m, 2H).

[(1*S*)-2-Methyl-1-[[(2*S*)-2-[5-[4-[4-(2-thienyl)-1,3-butadiynyl]phenyl]-1*H*-imidazol-2-yl]-1-pyrrolidinyl]carbonyl]propyl]-carbamic acid methyl ester, compound 62, LCMS (M+1) 501. ¹H NMR (DMSO-D6, 400 MHz) δ 0.88 (t, J₁ = 6.50, J₂ = 0.93, 3H), 0.95 (t, J₁ = 6.75, J₂ = 0.93, 3H), 1.64 (m, 1 H), 1.85 (m, 1 H), 1.96 (m, 2H), 2.10 (m, 1H), 3.56 (m, 1H), 3.61 (d, J = 9.40, 3H), 3.66 (m, 1H), 4.03 (m, 1 H), 4.73 (m, 1H), 6.83 (d, J = 3.89, 1 H), 6.86 (s, 1H), 7.07 (d, J = 1.10, 1 H), 7.78 (m, 3H), 7.89 (m, 2H), 8.79 (m, 2H).

[(1*R*)-2-Methyl-1-[[(2*R*)-2-[5-(4-phenylcyclohexyl)-1*H*-imidazol-2-yl]-1-pyrrolidinyl]carbonyl]propyl]-carbamic acid methyl ester, LCMS (M+1) 453 ¹H NMR (DMSO-D6, 400 MHz) δ 0.87 (t, J₁ = 6.75, J₂ = 0.93, 3H), 0.95 (t, J₁ = 6.75, J₂ = 0.93, 3H), 1.39 (m, 2H), 1.61 (m, 3H), 1.70 (m, 2H), 1.79 (m, 1 H), 1.86 (m, 2H), 1.99 (m, 2H), 2.10(m, 1 H), 2.39 (m, 1 H), 2.51 (m, 1 H), 3.56 (m, 1 H), 3.63 (m, 4H), 3.97 (m, 1 H), 4.68 (d, J = 4.69, 1 H), 6.42 (s, 1H), 7.16 (d, J = 1.20, 2H), 7.22 (t, J₁ = 7.13, J₂ = 1.25, 1 H), 7.35 (d, J = 7.26, 2H), 8.71 (m, 2H).

[(1*R*)-1-[[(2*S*)-2-[5-(4-[1,1'-Biphenyl]-4-ylcyclohexyl)-1*H*-imidazol-2-yl]-1-pyrrolidinyl]carbonyl]-2-methylpropyl]-carbamic acid methyl ester, LCMS (M+1) 529 ¹H NMR (DMSO-D6, 400 MHz) δ 0.87 (t, J₁ = 6.75, J₂ = 0.93, 3H), 0.96 (t, J₁ = 6.75, J₂ = 0.93, 3H), 1.37 (m, 2H), 1.60 (m, 4H), 1.70 (m, 2H), 1.84 (m, 3H), 1.99 (m, 2H), 2.39 (m, 1 H), 2.51 (m, 1 H), 3.56 (m, 1H), 3.63 (m, 4H), 3.97 (d, J = 7.70, 1 H), 4.72 (m, 1 H), 6.42 (s, 1 H), 7.16 (t, J₁ = 2.06, J₂ = 0.71, 2H), 7.24 (t, J₁ = 7.58, J₂ = 0.62, 2H), 7.36 (t, J₁ = 7.13, J₂ = 1.44, 1H), 7.41 (t, J₁ = 8.20, J₂ = 0.71, 2H), 7.53 (m, 2H), 8.71 (m, 2H).

[(1*S*)-1-[[(2*R*)-2-[5-(4-Cyclohexylphenyl)-1*H*-imidazol-2-yl]-1-pyrrolidinyl]carbonyl]-2-methylpropyl]-carbamic acid methyl ester, LCMS (M+1) 453¹H NMR (DMSO-D6, 400 MHz) δ 0.87 (t, J₁ = 6.50, J₂ = 0.93, 3H), 0.96 (t, J₁ = 6.50, J₂ = 0.93, 3H), 1.53 (m, 5H), 1.63 (m, 6H), 1.83 (m, 1 H), 1.95 (d, J = 8.50, 2H), 2.10 (m, 1 H), 2.33 (m, 1H), 3.56 (m, 1 H), 3.61 (s, 1 H), 3.63 (m, 2H), 4.02 (m, 1 H), 4.69 (m, 1 H), 6.86 (s, 1H), 7.37 (t, J₁ = 8.06, J₂ = 0.71, 2H), 7.74 (t, J₁ = 8.06, J₂ = 0.71, 2H), 8.79 (m, 2H).

[(1*S*)-1-[[(2*S*)-2-[5-(4'-Cyclohexyl[1,1'-biphenyl]-4-yl)-1*H*-imidazol-2-yl]-1-pyrrolidinyl]carbonyl]-2-methylpropyl]-carbamic acid methyl ester, compound 46, LCMS (M+1) 529, ¹H NMR (DMSO-D6, 400 MHz) δ 0.87 (t, J₁ = 6.50, J₂ = 0.93, 3H), 0.97 (t, J₁ = 6.50, J₂ = 0.93, 3H), 1.52 (m, 4H), 1.60 (m, 2H), 1.65 (m, 2H), 1.83 (m, 1 H), 1.96 (m, 2H), 2.10 (m, 2H), 2.32 (m, 1 H), 3.57 (m, 2H), 3.61 (d, J = 9.40, 3H), 3.65 (m, 2H), 4.03 (m, 1 H), 4.72 (m, 1 H), 6.79 (s, 1 H), 7.03 (t, J₁ = 8.60, J₂ = 0.71, 2H), 7.13 (d, J = 8.20, 2H), 7.36 (t, J₁ = 2.47, J₂ = 0.71, 2H), 7.55 (m, 2H), 8.79 (m, 2H).

[(1*S*)-1-[[(2*R*)-2-[5-(4'-Cyclohexyl[1,1'-biphenyl]-4-yl)-1*H*-imidazol-2-yl]-1-pyrrolidinyl]carbonyl]-2-methylpropyl]-carbamic acid methyl ester, LCMS (M+1) 529 ¹H NMR (DMSO-D6, 400 MHz) δ 0.87 (t, J₁ = 6.50, J₂ = 0.93, 3H), 0.96 (t, J₁ = 6.50, J₂ = 0.93, 3H), 1.52 (m, 3H), 1.55 (m, 2H), 1.60 (m, 6H), 1.83 (m, 1 H), 1.95 (d, J = 6.50, 2H), 2.10 (m, 1 H), 2.32 (m, 1 H), 3.56 (m, 1 H), 3.62 (m, 4H), 4.02 (d, J = 0.42, 1 H), 4.67 (m, 1 H), 6.79 (s, 1 H), 7.04 (t, J₁ = 8.60, J₂ = 0.71, 2H), 7.15 (t, J₁ = 8.20, J₂ = 0.71, 2H), 7.36 (t, J₁ = 8.20, J₂ = 0.71, 2H), 7.55 (t, J₁ = 8.60, J₂ = 1.95, 2H), 8.79 (m, 2H).

[(1*S*)-2-Methyl-1-[[(2*S*)-2-[5-(4-phenylbicyclo[2.2.2]oct-1-yl)-1*H*-imidazol-2-yl]-1-pyrrolidinyl]carbonyl]propyl]-carbamic acid methyl ester, LCMS (M+1) 479¹H NMR (DMSO-D6, 400 MHz) δ 0.87 (t, J₁ = 6.50, J₂ = 0.93, 3H), 0.96 (t, J₁ = 6.50, J₂ = 0.93, 3H), 1.56 (m, 3H), 1.63 (m, 4H), 1.83 (m, 1 H), 1.95 (d, J = 8.50, 1H), 1.99 (m, 1 H), 2.10 (m, 1 H), 2.20 (m, 3H), 2.31 (m, 4H), 3.56 (m, 1 H), 3.62 (m, 4H), 4.02 (d, J = 0.42, 1 H), 4.75 (m, 1 H), 6.40 (s, 1 H), 7.16 (t, J₁ = 7.13, J₂ = 1.22, 1 H), 7.26 (m, 4H), 9.06 (m, 2H).

[(1*S*)-1-[[[(1*S*)-1-[5-(4-[1,1'-Biphenyl]-4-ylbicyclo[2.2.2]oct-1-yl)-1*H*-imidazol-2-yl]ethyl]methylamino]cabonyl]-2-methylpropyl]-carbamic acid methyl ester, LCMS (M+1) 543 ¹H NMR (DMSO-D6, 400 MHz) δ 0.88 (t, J₁ = 6.50, J₂ = 0.93, 3H), 0.94 (t, J₁ = 6.50, J₂ = 0.93, 3H), 1.40 (d, J = 6.97, 3H), 1.56 (m, 3H), 1.63 (m, 3H), 2.00 (d, J = 7.70, 1 H), 2.21 (m, 3H), 2.31 (m, 3H), 3.02 (t, J₁ = 14.23, J₂ = 1.50, 3H), 3.61 (d, J = 9.40, 3H), 3.97 (m, 1 H), 4.58 (d, J = 0.30, 1 H), 6.35 (s, 1 H), 7.11 (m, 2H), 7.24 (t, J₁ = 7.58, J₂ = 0.62, 4H), 7.36 (t, J₁ = 7.13, J₂ = 1.44, 1H), 7.53 (t, J₁ = 7.58, J₂ = 2.03, 2H), 8.71 (m, 2H).

[(1 *S*)-1-[[[(1*R*)-1-[5-(4-Bicyclo[2.2.2]oct-1-y)phenyl)-1H-imidazol-2-yl]ethyl]methylamino]carbonyl]-2-methylpropyl]-carbamic acid methyl ester, LCMS (M+1) 467 ¹H NMR (DMSO-D6, 400 MHz) δ 0.87 (t, J₁ = 6.75, J₂ = 0.93, 3H), 0.95 (t, J₁ = 6.75, J₂ = 0.93, 3H), 1.24 (m, 3H), 1.40 (d, J = 7.12, 3H), 1.47 (m, 6H), 1.63 (d, J = 3.05, 1 H), 1.99 (m, 4H), 3.08 (t, J₁ = 14.23, J₂ = 1.50, 3H), 3.61 (d, J = 9.40, 3H), 3.98 (m, 1 H), 5.22 (d, J = 0.30, 1 H), 6.81 (s, 1 H), 7.34 (d, J = 8.06, 2H), 7.71 (t, J₁ = 1.95, J₂ = 0.71, 2H), 8.43 (m, 2H).

[(1*S*)-1-[[(2*S*)-2-[5-(4'-Bicyclo[2.2.2]oct-1-yl[1,1'-biphenyl]-4-yl)-1*H*-imidazol-2-yl]-1-pyrrolidinyl]carbonyl]-2-methylpropyl]-carbamic acid methyl ester, compound 47, LCMS (M+1) 555, ¹H NMR (DMSO-D6, 400 MHz) δ 0.87 (t, J₁ = 6.50, J₂ = 0.93, 3H), 0.97 (t, J₁ = 6.50, J₂ = 0.93, 3H), 1.25 (m, 2H), 1.46 (m, 4H), 1.61 (m, 2H), 1.84 (m, 2H), 1.99 (m, 5H), 2.10 (m, 1 H), 3.57 (m, 1 H), 3.61 (d, J = 9.40, 4H), 3.65 (m, 2H), 4.03 (m, 1H), 4.72 (m, 1H), 6.79 (s, 1H), 7.04 (d, J = 8.60, 2H), 7.13 (d, J = 8.20, 2H), 7.20 (t, J₁ = 2.47, J₂ = 0.71, 2H), 7.55 (m, 2H), 8.79 (m, 2H).

[(1*R*)-1-[[[(1*S*)-1-[5-(4'-Bicyclo[2.2.2]oct-1-yl[1,1'-biphenyl]-4-yl)-1*H*-imidazol-2-yl]ethyl]methylamino]carbonyl]-2-methylpropyl]-carbamic acid methyl ester LCMS (M+1) 543 ¹H NMR (DMSO-D6, 400 MHz) δ 0.87 (t, J₁ = 6.50, J₂ = 0.93, 3H), 0.96 (t, J₁ = 6.50, J₂ = 0.93, 3H), 1.24 (m, 3H), 1.40 (d, J = 7.12, 3H), 1.46 (m, 5H), 1.63 (d, J = 3.05, 1 H), 1.95 (m, 5H), 3.02 (t, J₁ = 14.23, J₂ = 1.50, 3H), 3.61 (d, J = 9.40, 3H), 4.03 (m, 1H), 4.86 (m, 1H), 6.74 (s, 1H), 7.03 (t, J₁ = 8.60, J₂ = 2.87, 2H), 7.11 (t, J₁ = 8.20, J₂ = 0.71, 2H), 7.20 (t, J₁ = 2.47, J₂ = 0.71, 2H), 7.55 (m, 2H), 8.79 (m, 2H).

[(1*R*)-1-[[[(1*R*)-1-[5-[4-(1,3-Dioxan-2-yl)phenyl]-1*H*-imidazol-2-yl]ethyl]methylamino]carbonyl]-2-methylpropyl]-carbamic acid methyl ester, LCMS (M+1) 445 ¹H NMR (DMSO-D6, 400 MHz) δ 0.87 (t, J₁ = 6.50, J₂ = 0.93, 3H), 0.96 (t, J₁ = 6.50, J₂ = 0.93, 3H), 1.40 (d, J = 7.12, 3H), 1.52 (m, 1 H), 1.95 (d, J = 8.50, 1 H), 2.09 (m, 1 H), 3.02 (t, J₁ = 14.23, J₂ = 1.50, 3H), 3.61 (d, J = 9.40, 3H), 3.95 (m, 2H), 4.03 (d, J = 8.50, 1 H), 4.13 (m, 2H), 5.21 (d, J = 0.30, 1H), 5.27 (m, 1H), 6.81 (s, 1H), 7.66 (t, J₁ = 8.06, J₂ = 0.71, 2H), 7.91 (t, J₁ = 1.95, J₂ = 0.71, 2H), 8.79 (m, 2H).

*N*-[(1*S*)-1-[5-[4'-(1,3-dioxan-2-yl)[1,1'-biphenyl]-4-yl]-1*H*-imidazol-2-yl]ethyl]-*N-*methyl-□-phenyl-, (□*S*)-1-piperidineacetamide, LCMS (M+1) 565 ¹H NMR (DMSO-D6, 400 MHz) δ 1.39 (d, J₁ = 7.12, 3H), 1.52 (m, 2H), 1.67 (m, 3H), 1.72 (m, 2H), 2.07 (m, 1 H), 2.63 (m, 2H), 2.73 (m, 2H), 2.97 (t, J₁ = 14.23, J₂ = 1.50, 3H), 3.94 (m, 3H), 4.15 (m, 2H), 5.26 (m, 1 H), 5.37 (d, J₁ = 0.30, 1 H), 6.74 (d, J₁ = 0.30, 1 H), 7.12 (t, J₁ = 8.60, J₂ = 0.71, 2H), 7.22 (d, J = 7.03, 2H), 7.26 (d, J = 2.47, 2H), 7.37 (t, J₁ = 2.28, J₂ = 0.71, 4H), 7.43 (t, J₁ = 7.32, J₂ = 1.46, 1H), 7.55 (d, J = 1.95, 2H), 11.18 (m, 1 H).

*N*-[(1*S*)-1-[5-[4-(1,3-Dioxan-5-yl)phenyl]-1*H*-imidazol-2-yl]ethyl]-*N*-methyl-□-phenyl-, (□*S*)-1-piperidineacetamide, LCMS (M+1) 489 ¹H NMR (DMSO-D6, 400 MHz) δ 1.39 (d, J₁ = 7.12, 3H), 1.54 (m, 1 H), 1.62 (m, 3H), 1.72 (m, 2H), 2.65 (m, 2H), 2.73 (m, 2H), 2.97 (t, J₁ = 14.23, J₂ = 1.50, 3H), 3.34 (m, 1 H), 3.67 (m, 2H), 3.90 (m, 2H), 3.97 (m, 1 H), 4.64 (m, 1 H), 4.85 (m, 1 H), 5.37 (d, J = 0.30, 1H), 6.81 (d, J₁ = 0.30, 1 H), 7.22 (d, J₁ = 7.03, 2H), 7.37 (t, J₁ = 2.18, J₂ = 1.46, 2H), 7.45 (t, J₁ = 7.32, J₂ = 1.46, 1 H), 7.61 (t, J₁ = 8.06, J₂ = 0.71, 2H), 7.93 (d, J = 1.95, 2H), 11.18 (m, 1H).

*N*-[(1*R*)-1-[5-[4'-(1,3-Dioxan-5-yl)[1,1'-biphenyl]-4-yl]-1*H*-imidazol-2-yl]ethyl]-*N-*methyl-□-phenyl-, (□*S*)-1-piperidineacetamide, LCMS (M+1) 565 ¹H NMR (DMSO-D6, 400 MHz) δ 1.39 (t, J₁ = 7.12, J₂ = 1.50, 3H), 1.53 (m, 1 H), 1.67 (m, 3H), 1.75 (m, 2H), 2.66 (m, 2H), 2.73 (m, 2H), 2.97 (t, J₁ = 14.23, J₂ = 1.50, 3H), 3.36 (m, 1 H), 3.67 (m, 2H), 3.89 (m, 2H), 3.97 (m, 1 H), 4.64 (m, 1 H), 4.86 (m, 1 H), 5.69 (d, J = 0.30, 1 H), 6.74 (d, J₁ = 0.30, 1 H), 7.04 (t, J₁ = 8.60, J₂ = 0.71, 2H), 7.22 (t, J₁ = 7.32, J₂ = 0.71, 2H), 7.33 (t, J₁ = 8.20, J₂ = 0.71, 4H), 7.44 (t, J₁ = 7.32, J₂ = 1.46, 1 H), 7.55 (m, 4H), 11.18 (m, 1 H).

*N*-[(1*S*)-2-[(2*R*)-2-[5-[4-(1,4-Dioxan-2-yl)phenyl]-1*H*-imidazol-2-yl]-1-pyrrolidine]-2-oxo-1-phenylethyl]-cyclopropanecarboxamide, LCMS (M+1) 507 ¹H NMR (DMSO-D6, 400 MHz) δ 0.84 (m, 2H), 0.91 (m, 2H), 1.64 (m, 1H), 1.76 (s, 1 H), 1.83 (m, 1 H), 1.97 (m, 1 H), 2.10 (m, 1H), 3.16 (m, 1H), 3.24 (m, 1H), 3.42 (m, 1H), 3.50 (m, 1 H), 3.86 (m, 1 H), 3.97 (m, 1 H), 4.10 (m, 1 H), 4.18 (m, 1 H), 4.59 (m, 2H), 5.17 (d, J₁ = 0.42, 1 H), 6.86 (s, 1 H), 7.17 (t, J₁ = 7.60, J₂ = 0.71, 2H), 7.27 (m, 3H), 7.67 (t, J₁ = 8.06, J₂ = 0.60, 2H), 7.93 (d, J₁ = 1.95, 2H), 9.72 (m, 2H).

[(1*S*)-1-[[(2*S*)-2-[5-[4'-(1,4-Dioxan-2-yl)[1,1'-biphenyl]-4-yl]-1*H*-imidazol-2-yl]-1-pyrrolidinyl]carbonyl]-2-methylpropyl]-carbamic acid methyl ester, compound 48, LCMS (M+1) 533, ¹H NMR (DMSO-D6, 400 MHz) δ 0.87 (t, J₁ = 6.50, J₂ = 0.93, 3H), 0.97 (t, J₁ = 6.50, J₂ = 0.93, 3H), 1.65 (m, 1H), 1.84 (m, 1H), 1.99 (m, 2H), 2.10 (m, 1 H), 3.15 (m, 1 H), 3.25 (m, 1 H), 3.56 (m, 1 H), 3.64 (br.d, 4H), 3.89 (m, 1 H), 3.97 (m, 1 H), 4.02 (m, 1 H), 4.10 (m, 1 H), 4.18 (m, 1 H), 4.59 (m, 1H), 4.74 (m, 1 H), 6.79 (s, 1 H), 7.04 (d, J = 8.60, 2H), 7.43 (br. t, 4H), 7.55 (m, 2H), 8.79 (m, 2H).

*N*-[(1*S*)-2-[(2*S*)-2-[5-[4'-(1,4-Dioxan-2-yl)[1,1'-biphenyl]-4-yl]-1*H*-imidazol-2-yl]-1-pyrrolidinyl]-2-oxo-1-phenylethyl]-cyclopropanecarboxamide, LCMS (M+1) 577 ¹H NMR (DMSO-D6, 400 MHz) δ 0.82 (m, 2H), 0.91 (m, 2H), 1.64 (m, 1 H), 1.76 (s, 1 H), 1.84 (m, 1 H), 1.99 (m, 1 H), 2.10 (m, 1 H), 3.16 (m, 1 H), 3.24 (m, 1 H), 3.43 (m, 1 H), 3.50 (m, 1 H), 3.88 (m, 1 H), 3.96 (m, 1 H), 4.10 (m, 1 H), 4.18 (m, 1H), 4.61 (m, 2H), 5.17 (m, 1 H), 6.79 (s, 1 H), 7.04 (t, J₁ = 8.60, J₂ = 0.71, 2H), 7.17 (t, J₁ = 7.60, J₂ = 0.71, 2H), 7.28 (d, J₁ = 7.32, 3H), 7.43 (t, J₁ = 8.27, J₂ = 0.71, 4H), 7.55 (d, J₁ = 1.95, 2H), 9.72 (m, 2H).

*N*-[(1*R*)-2-Oxo-1-phenyl-2-[(2*S*)-2-[5-(5-phenyl-1,4-dioxan-2-yl)-1*H*-imidazol-2-yl]-1-pyrrolidinyl]ethyl]-cyclopropanecarboxamide, LCMS (M+1) 501¹H NMR (DMSO-D6, 400 MHz) δ 0.84 (m, 2H), 0.91 (m, 2H), 1.64 (m, 1H), 1.75 (m, 1 H), 1.84 (m, 1H), 1.97 (m, 1H), 2.08 (m, 1H), 3.42 (m, 1 H), 3.49 (m, 1 H), 3.72 (m, 1 H), 3.90 (m, 1 H), 3.88 (m, 1H), 3.96 (m, 2H), 4.46 (m, 1H), 4.66 (m, 2H), 5.17 (m, 1 H), 6.82 (s, 1 H), 7.20 (t, J₁ = 7.03, J₂ = 0.71, 2H), 7.26 (d, J₁ = 7.32, 3H), 7.34 (t, J₁ = 7.13, J₂ = 0.69, 4H), 9.82 (m, 2H).

*N*-[(1*R*)-2-[(2*S*)-2-[5-(5-[1,1'-Biphenyl]-4-yl-1,4-dioxan-2-yl)-1*H*-imidazol-2-yl]-1-pyrrolidinyl]-2-oxo-1-phenylethyl]-cyclopropanecarboxamide, LCMS (M+1) 577 ¹H NMR (DMSO-D6, 400 MHz) δ 0.84 (m, 2H), 0.91 (m, 2H), 1.64 (m, 1 H), 1.76 (m, 1 H), 1.84 (m, 1 H), 1.96 (m, 1 H), 2.10 (m, 1 H), 3.42 (m, 1 H), 3.51 (m, 1H), 3.71 (m, 1H), 3.90 (m, 1 H), 3.98 (m, 2H), 4.46 (m, 1 H), 4.66 (m, 2H), 5.17 (d, J₁ = 0.72, 1 H), 6.82 (d, J = 0.97, 1 H), 7.17 (t, J₁ = 7.03, J₂ = 0.71, 2H), 7.26 (d, J = 7.32, 3H), 7.34 (t, J₁ = 7.13, J₂ = 0.62, 4H), 7.45 (d, J = 8.27, 4H), 7.52 (m, 2H), 9.82 (m, 2H).

*N*-[(1*R*)-2-[(2*R*)-2-[5-[6-(1,4-Dioxan-2-yl)-2-naphthalenyl]-1*H*-imidazol-2-yl]-1-pyrrolidinyl]-2-oxo-1-phenylethyl]-cyclopropanecarboxamide, LCMS (M+1) 551¹H NMR (DMSO-D6, 400 MHz) δ 0.84 (m, 2H), 0.91 (m, 2H), 1.63 (m, 1 H), 1.75 (m, 1 H), 1.84 (m, 1H), 1.98 (m, 1 H), 2.10 (m, 1 H), 3.16 (m, 1H), 3.24 (m, 1H), 3.43 (m, 1 H), 3.50 (m, 1 H), 3.89 (m, 1 H), 3.96 (m, 1 H), 4.10 (m, 1 H), 4.18 (m, 1 H), 4.55 (m, 1H), 4.68 (m, 1 H), 5.16 (d, J₁ = 0.42, 1 H), 6.94 (s, 1 H), 7.17 (t, J₁ = 7.03, J₂ = 0.71, 2H), 7.26 (d, J = 7.32, 3H), 7.84 (d, J = 0.16, 1 H), 7.89 (m, 1 H), 7.94 (d, J = 8.50, 2H), 8.14 (d, J = 0.42, 1 H), 8.60 (d, J = 1.65, 1H), 9.55 (m, 2H).

2-[5-[5-(2-Naphthalenyl)-1,4-dioxan-2-yl]-1*H*-imidazol-2-yl]-1-[(2*S*)-2-phenyl-2-(1-piperidinyl)acetyl]-, (2*S*)-pyrrolidine, LCMS (M+1) 551¹H NMR (DMSO-D6, 400 MHz) δ 1.54 (m, 1H), 1.67 (m, 4H), 1.75 (m, 3H), 1.84 (m, 1 H), 2.10 (m, 1H), 2.65 (m, 2H), 2.73 (m, 2H), 3.45 (m, 1 H), 3.53 (m, 1 H), 3.72 (m, 1 H), 3.90 (m, 4H), 4.47 (m, 1 H), 4.64 (m, 1 H), 4.64 (m, 1 H), 4.73 (m, 1 H), 6.82 (d, J = 0.97, 1 H), 7.25 (t, J₁ = 7.32, J₂ = 1.25, 2H), 7.38 (t, J₁ = 7.80, J₂ = 0.71, 2H), 7.46 (d, J = 0.70, 2H), 7.73 (d, J = 0.60, 2H), 7.80 (d, J = 1.72, 1 H), 7.86 (t, J₁ = 8.13, J₂ = 0.28, 1 H), 7.97 (d, J = 8.71, 1H), 8.10 (s, 1H), 11.73 (m, 1H).

2-[5-[5-(1,4-Dioxan-2-yl)-2-thienyl]-1H-imidazol-2-yl]-1-[(2R)-2-phenyl-2-(1-piperidinyl)acetyl]-, (2R)-pyrrolidine, LCMS (M+1) 507¹H NMR (DMSO-D6, 400 MHz) δ 1.54 (m, 1 H), 1.62 (m, 2H), 1.67 (m, 2H), 1.75 (m, 2H), 1.83 (m, 1 H), 1.99 (m, 1H), 2.10 (m, 1 H), 2.65 (m, 2H), 2.72 (m, 2H), 3.16 (m, 1 H), 3.24 (m, 1 H), 3.46 (m, 1H), 3.53 (m, 2H), 3.76 (m, 1H), 3.88 (m, 1H), 3.96 (m, 2H), 4.32 (m, 1H), 4.58 (m, 1 H), 6.64 (d, J = 0.25, 1 H), 6.71 (s, 1 H), 6.96 (t, J₁ = 5.99, J₂ = 1.40, 1 H), 7.24 (d, J = 7.32, 2H), 7.38 (t, J₁ = 7.03, J₂ = 0.71, 2H), 7.43 (m, 1 H), 11.18 (m, 1H).

1-[(2*R*)-2-Phenyl-2-(1-piperidinyl)acetyl]-2-[5-[5-(2-thienyl)-1,4-dioxan-2-yl]-1H-imidazol-2-yl]-, (2R)-pyrrolidine, LCMS (M+1) 507 ¹H NMR (DMSO-D6, 400 MHz) δ 1.54 (m, 1H), 1.62 (m, 2H), 1.65 (m, 6H), 1.75 (m, 2H), 1.83 (m, 1H), 1.99 (m, 1 H), 2.10 (m, 1H), 2.65 (m, 1 H), 2.73 (m, 2H), 3.46 (m, 1 H), 3.53 (m, 1H), 3.71 (m, 2H), 3.96 (m, 3H), 4.31 (m, 1H), 4.46 (m, 1H), 4.59 (m, 1H), 6.82 (d, J = 0.97, 1 H), 7.08 (t, J₁ = 5.99, J₂ = 3.41, 1 H), 7.16 (d, J = 1.40, 1 H), 7.24 (d, J = 7.32, 2H), 7.32 (d, J = 2.30, 3H), 7.43 (m, 1 H), 11.73 (m, 1H).

1-[(2*S*)-2-Phenyl-2-(1-piperidinyl)acetyl]-2-[5-[5-[4-(2-thienyl)phenyl]-1,4-dioxan-2-yl]-1*H*-imidazol-2-yl]-, (2R)-pyrrolidine, LCMS (M+1) 583 ¹H NMR (DMSO-D6, 400 MHz) δ 1.54 (m, 1H), 1.63 (m, 2H), 1.67 (m, 2H), 1.75 (m, 2H), 1.83 (m, 1 H), 1.99 (m, 1 H), 2.10 (m, 1 H), 2.65 (m, 2H), 2.73 (m, 2H), 3.46 (m, 1 H), 3.53 (m, 1 H), 3.71 (m, 1 H), 3.91 (m, 2H), 3.97 (m, 2H), 4.46 (m, 1 H), 4.59 (m, 1 H), 4.66 (m, 1 H), 6.82 (s, 1 H), 7.00 (t, J₁ = 4.76, J₂ = 3.29, 1 H), 7.22 (t, J₁ = 7.32, J₂ = 1.25, 2H), 7.32 (d, J = 1.36, 1 H), 7.38 (m, 5H), 7.43 (m, 1 H), 7.50 (d, J = 8.25, 2H),11.73 (m, 1 H).

2-[5-[5-[4-(1,4-Dioxan-2-yl)phenyl]-2-thienyl]-1*H*-imidazol-2-yl]-1-[(2*R*)-2-phenyl-2-(1-piperidinyl)acetyl]-, (2*S*)-pyrrolidine, LCMS (M+1) 583 ¹H NMR (DMSO-D6, 400 MHz) δ 1.54 (m, 1 H), 1.64 (m, 2H), 1.67 (m, 2H), 1.75 (m, 2H), 1.84 (m, 1 H), 1.99 (m, 1 H), 2.10 (m, 1H), 2.65 (m, 2H), 2.73 (m, 2H), 3.16 (m, 1 H), 3.24 (m, 1 H), 3.45 (m, 1H), 3.53 (m, 1 H), 3.88 (m, 1 H), 3.96 (m, 2H), 4.10 (m, 1 H), 4.18 (m, 1H), 4.59 (m, 1 H), 4.64 (m, 1H), 6.92 (d, J = 0.25, 1 H), 7.09 (d, J = 4.76, 1 H), 7.22 (d, J = 7.32, 2H), 7.29 (t, J₁ = 8.25, J₂ = 0.71, 2H), 7.38 (t, J₁ = 7.03, J₂ = 0.71, 2H), 7.43 (m, 1 H), 7.57 (t, J₁ = 2.42, J₂ = 0.71, 2H), 11.18 (m, 1 H).

*N*-[(1*S*)-1-[5-(5-[2,2'-bithiophen]-5-yl-1,4-dioxan-2-yl)-1*H*-imidazol-2-yl]ethyl]-*N-*methyl-□-phenyl-, (□*R*)- 1-piperidineacetamide, LCMS (M+1) 577 ¹H NMR (DMSO-D6, 400 MHz) δ 1.40 (d, J = 7.12, 3H), 1.54 (m, 1 H), 1.65 (m, 3H), 1.75 (m, 2H), 2.65 (m, 2H), 2.73 (m, 2H), 2.97 (t, J₁ = 14.23, J₂ = 1.50, 3H), 3.71 (m, 2H), 3.96 (m, 3H), 4.36 (M, 1H), 4.46 (m, 1H), 5.07 (d, J = 0.30, 1H), 6.74 (d, J₁ = 0.30, 1H), 7.06 (m, 2H), 7.12 (d, = 1.40, 1H), 7.22 (t, ₁ = 7.80, J₂ = 0.71, 2H), 7.27 (d, J = 0.25, 1 H), 7.35 (m, 3H), 7.43 (t, J₁ = 7.32, J₂ = 2.10, 1H), 11.73 (m, 1 H).

*N*-[(1*R*)-1-[5-[5'-(1,4-Dioxan-2-yl)[2,2'-bithiophen]-5-yl]-1H-imidazol-2-yl]ethyl]-N-methyl-□-phenyl-, (□*S*)-1-piperidineacetamide, LCMS (M+1) 577 ¹H NMR (DMSO-D6, 400 MHz) δ 1.40 (d, J = 1.12, 3H), 1.54 (m, 1H), 1.62 (m, 1H), 1.67 (m, 2H), 1.75 (m, 2H), 2.65 (m, 2H), 2.73 (m, 2H), 2.97 (t, J₁ = 14.23, J₂ = 1.50, 3H), 3.16 (m, 1 H), 3.24 (m, 1 H), 3.55 (m, 1 H), 3.76 (m, 1 H), 3.88 (m, 1 H), 3.97 (m, 2H), 4.30 (m, 1 H), 5.69 (d, J = 0.30, 1 H), 6.59 (d, J₁ = 0.25, 1H), 6.87 (d, J₁ = 0.30, 1 H), 6.93 (d, J₁ = 3.98, 1 H), 7.04 (d, J₁ = 4.00, 1 H), 7.23 (t, J₁ = 7.32, J₂ = 0.71, 2H), 7.37 (t, J₁ = 2.28, J₂ = 0.71, 3H), 7.43 (t, J₁ = 7.32, J₂ = 1.46, 1 H), 11.18 (m, 1H).

[(1*S*)-1-[[(2*S*)-2-(5-[1,1'-Bicyclohexyl]-4-yl-1*H*-imidazol-2-yl)-1-pyrrolidinyl]carbonyl]-2-methylpropyl]-carbamic acid methyl ester, compound 41, LCMS (M+1) 459. ¹H NMR (DMSO-D6, 400 MHz) δ 0.88 (t, J₁ = 6.50, J₂ = 0.93, 3H), 0.95 (t, J₁ = 6.75, J₂ = 0.93, 3H), 1.25 (m, 6H), 1.48 (m, 8H), 1.55 (m, 2H), 1.64 (m, 1 H), 1.76 (m, 2H), 1.85 (m, 1 H), 1.99 (m, 3H), 2.10 (m, 2H), 2.43 (m, 1 H), 3.54 (m, 1 H), 3.61 (d, J = 9.40, 3H), 3.63 (m, 1H), 4.03 (d, J = 0.42, 1H), 4.73 (m, 1 H), 6.37 (s, 1H), 9.06 (m, 2H).

[(1*S*)-1-[[(2*S*)-2-[5-(4-Cyclohexyl-1,3-butadiynyl)-1*H*-imidazol-2-yl]-1-pyrrolidinyl]carbonyl]-2-methylpropyl]-carbamic acid methyl ester, LCMS (M+1) 425.

[(1*S*)-1-[[(2*S*)-2-[5-(4-Bicyclo[2.2.2]oct-1-yl-1,3-butadiynyl)-1*H*-imidazol-2-yl]-1-pyrrolidinyl]carbonyl]-2-methylpropyl]-carbamic acid methyl ester, LCMS (M+1) 451.

### Example 14.

Preparation of pharmaceutical composition in the form of tablet. Starch (1600 mg), ground lactose (1600 mg), talk (400 mg) and bis-azole 14 (1000 mg) were mixed together. The resultant bar was comminuted into granules and sifted through sieve to collect granules of 14-16 mesh. The granules thus obtained were shaped into tablets of suitable form weighing 560 mg each.

### Example 15.

Preparation of pharmaceutical composition in the form of capsules. Bis-azole **14** and lactose powder were carefully mixed in ratio 2 : 1. The resultant powdery mixture was packed into gelatin capsules of suitable size by 300 mg to a capsule.

### Example 16.

Preparation of pharmaceutical composition in the form of injectable compositions for intramuscular, intraperitoneal or hypodermic injections. Bis-azole **14** (500 mg), chlorobutanol (300 mg), propylene glycol (2 ml), and injectable water (100 ml) were mixed together. The resultant solution was filtered and placed into 1 ml ampoules, which were sealed.

### Industrial applicability

The invention could be used in medicine, veterinary, biochemistry.

## Claims

1. Substituted azoles of the general formulas **1A** and **1B** and pharmaceutically acceptable salts thereof, 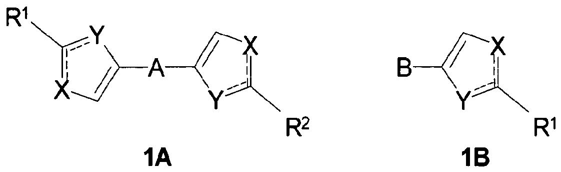 wherein:
solid lines with accompanying dotted lines (---) represent ordinary bond or double bond, provided that one of them is an ordinary bond, the other one is double bond;
**X** and **Y** optionally accept different meanings and represent nitrogen, oxygen, sulfur or NH group;
**R¹** and **R² -** represent optionally identical radicals **2.1-2.20,** where an asterisk (*) denotes the places of azole fragment attachment; 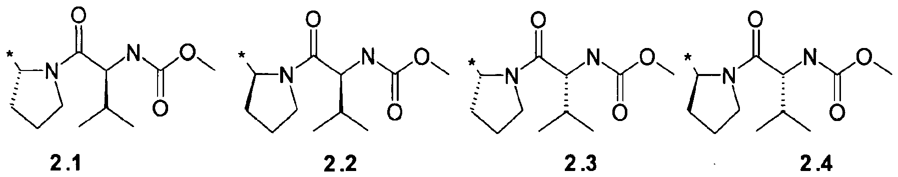 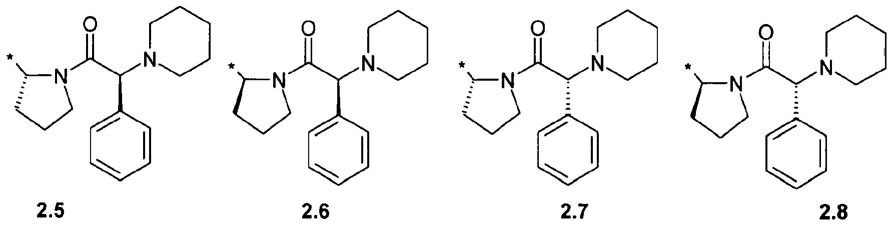 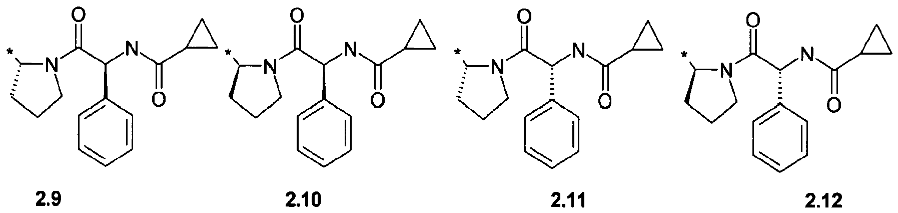 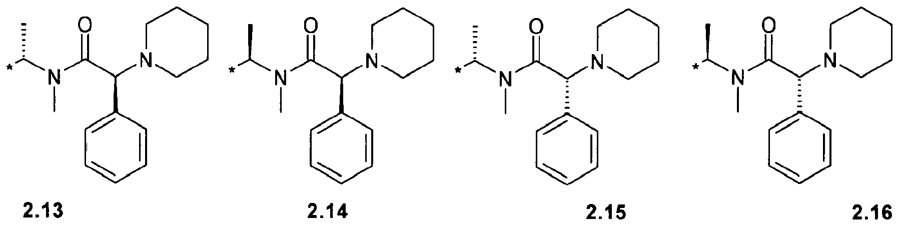 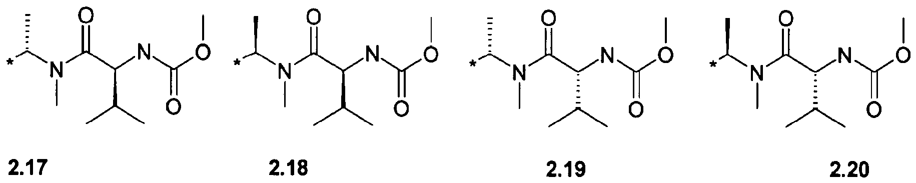
**A** represents:
- aliphatic C₂-C₈ biradical selected from biradicals **3.1-3.36,** where an asterisk (*) denotes the places of azole fragments attachment;
| | | |
|---|---|---|
| *-CH₂-CH₂-* | *-CH₂-CH₂-CH₂-* | *-CH₂-CH₂-CH₂-CH₂-* |
| **3.1** | **3.2** | **3.3** |
| *-CH₂-CH₂-CH₂-CH₂-CH₂-* | *-CH₂-CH₂-CH₂-CH₂-CH₂-CH₂* | *-CH=CH-* |
| **3.4** | **3.5** | **3.6** |
| *-CH₂-CH=CH-* | *-CH=CH-CH₂-CH₂-* | *-CH₂-CH=CH-CH₂-* |
| **3.7** | **3.8** | **3.9** |
| *-CH=CH-CH₂-CH₂-CH₂-* | *-CH₂-CH=CH-CH₂-CH₂-* | *-CH=CH-CH₂-CH=CH-* |
| **3.10** | **3.11** | **3.12** |
| *-CH₂-CH=C=CH-CH₂-* | *-CH=CH-CH₂-CH₂-CH₂-CH₂-* | *-CH₂-CH=CH-CH₂-CH₂-CH₂-* |
| **3.13** | **3.14** | **3.15** |
| *-CH=CH-CH=CH-CH₂-CH₂-* | *-CH₂-CH=CH-CH=CH-CH₂-* | *-CH₂-CH=C=CH-CH₂-CH₂-* |
| **3.16** | **3.17** | **3.18** |
| *-C≡C-* | *-C≡C-CH₂-* | *-C≡C-CH₂-CH₂-* |
| **3.19** | **3.20** | **3.21** |
| *-CH₂-C≡C-CH₂-* | *-C≡C-C≡C-* | *-C≡C-CH₂-CH₂-CH₂-* |
| **3.22** | **3.23** | **3.24** |
| *-CH₂-C≡C-CH₂-CH₂-* | *-C≡C-C≡C-CH₂-* | *-C≡C-CH₂-C≡C-* |
| **3.25** | **3.26** | **3.27** |
| *-C≡C-CH₂-CH₂-CH₂-CH₂-* | *-CH₂-C≡C-CH₂-CH₂-CH₂-* | *-CH₂-CH₂-C≡C-CH₂-CH₂-* |
| **3.28** | **3.29** | **3.30** |
| *-C≡C-C≡C-CH₂-CH₂-* | *-C≡C-CH₂-CH₂-C≡C-* | *-C≡C-CH₂-CH=CH-CH₂-* |
| **3.31** | **3.33** | **3.34** |
| *-C≡C-CH₂-CH₂-CH=CH-* | *-C≡C-C≡C-C≡C-* | |
| **3.35** | **3.36** | |
- dioxane, cyclo- and bicycloaliphatic biradical, selected from biradicals **3.37-3.47,** where an asterisk (*) denotes the places of azole fragment attachment; 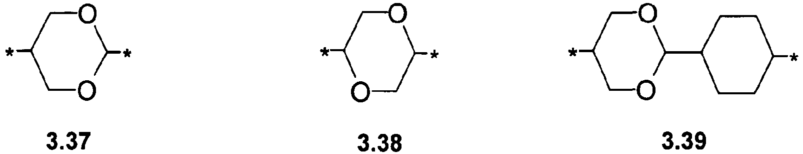 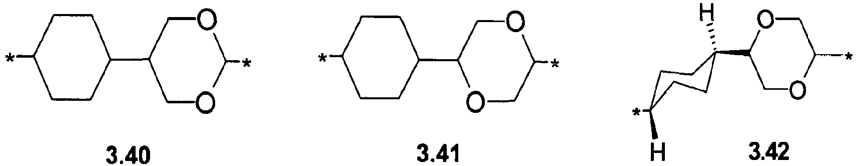 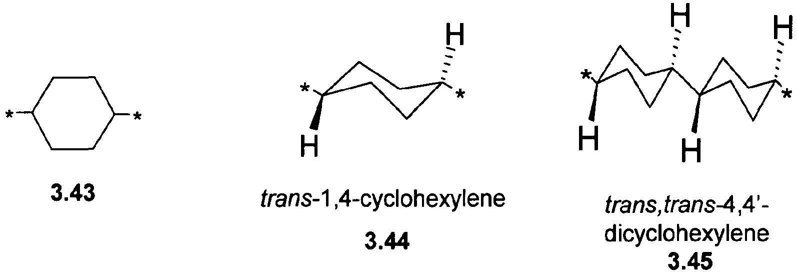 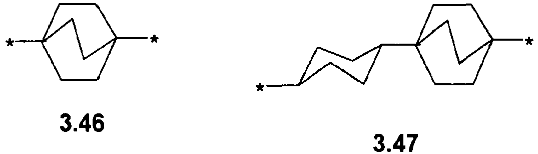
- alkyloxyalkyl, alkenyloxyalkyl, alkynyloxyalkyl biradicals and their thioanalogs selected from biradicals **3.48-3.56,** where an asterisk (*) denotes the places of azole fragment attachment;
| | | |
|---|---|---|
| *-CH₂-CH₂-O-CH₂-CH₂-* | *-CH₂-CH₂-S-CH₂-CH₂-* | *-CH=CH-O-CH₂-CH₂-* |
| **3.48** | **3.49** | **3.50** |
| *-CH₂-CH₂-O-CH₂-CH₂-O-CH₂-CH₂-* | *-CH=CH-CH₂-O-CH₂-CH=CH₂-* | *-C≡C-CH₂-O-CH₂-CH₂-* |
| **3.51** | **3.52** | **3.53** |
| *-C≡C-CH₂-O-CH₂-C≡C-* | *-CH=CH-CH₂-O-CH₂-C≡C-* | *-CH₂-CH₂-O-CH₂-C≡C-* |
| **3.54** | **3.55** | **3.56** |
- aryl and thiophene biradicals selected from biradicals **3.57-3.71,** where an asterisk (*) denotes the places of azole fragment attachment; 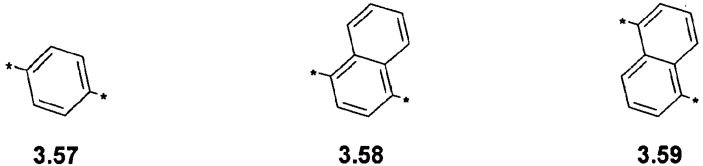 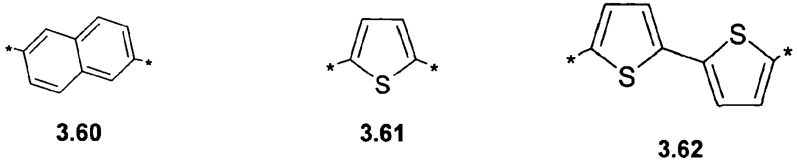 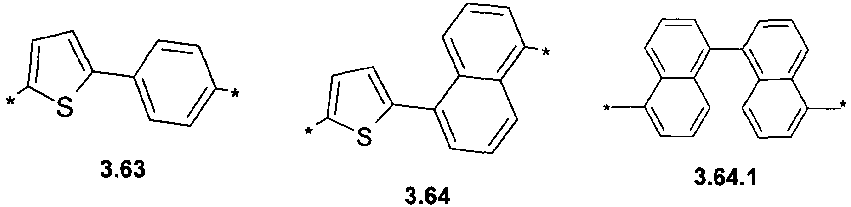 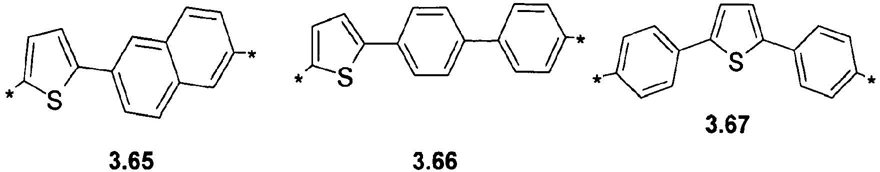 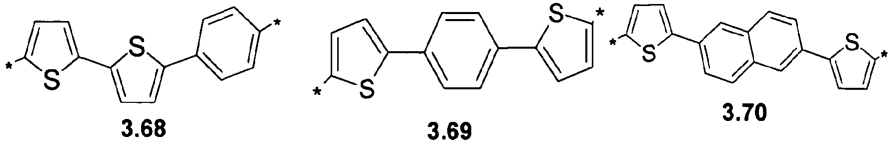 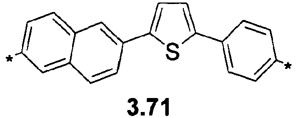
- alkynylcycloalkyl, alkynyldioxane, alkynylaryl, alkylthiophene, alkenylthiophene and alkynylthiophene biradicals selected from biradicals **3.72-3.129,** where an asterisk (*) denotes the places of azole fragment attachment; 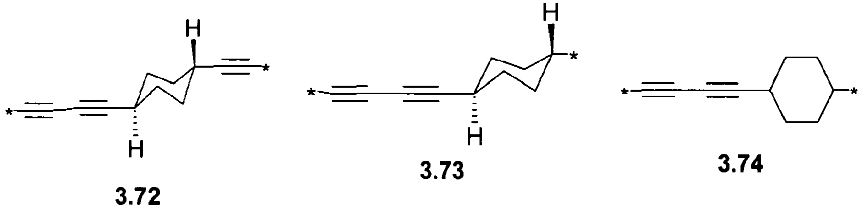 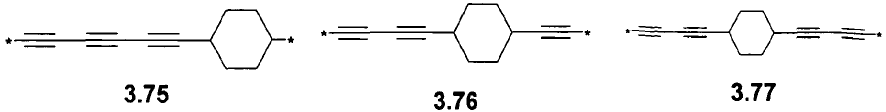 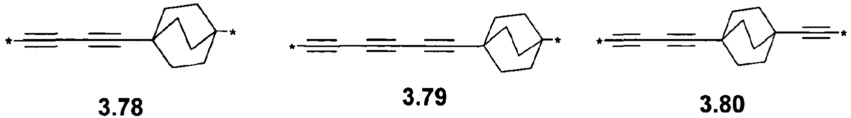 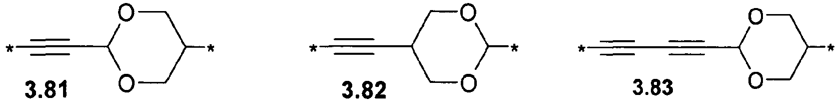 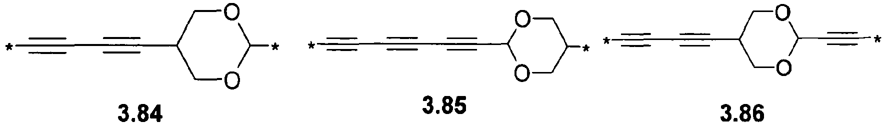 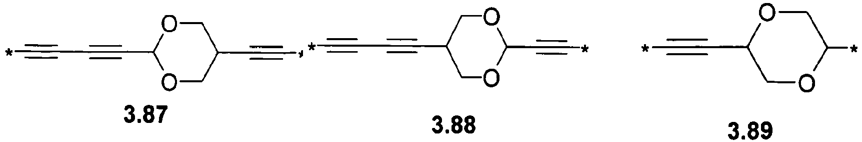 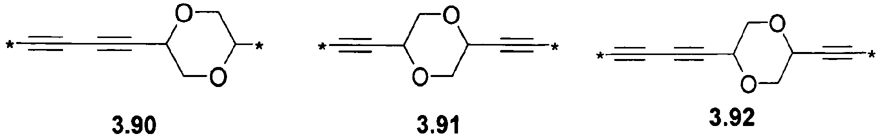 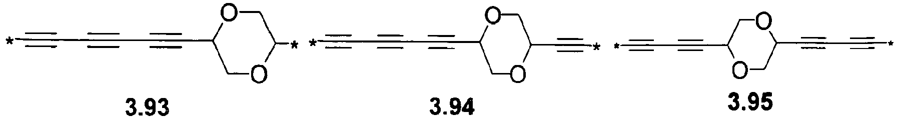 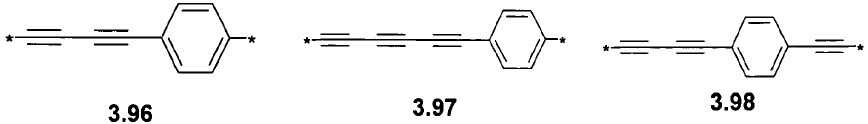 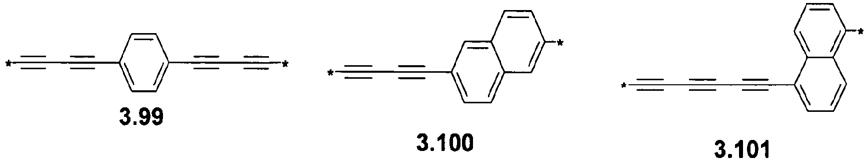 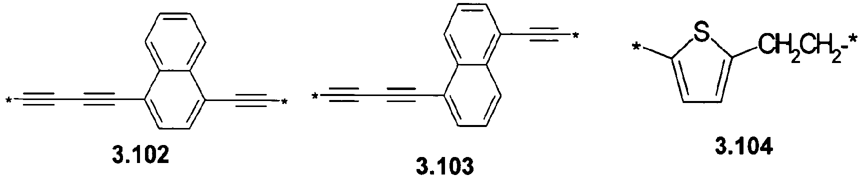 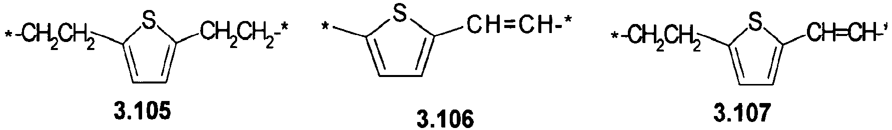 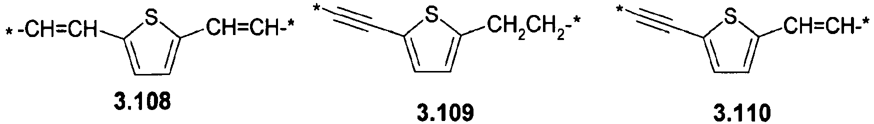 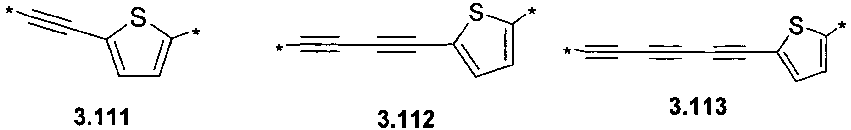 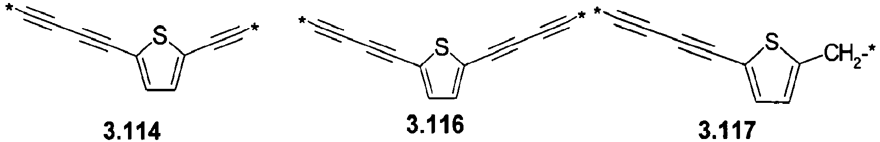 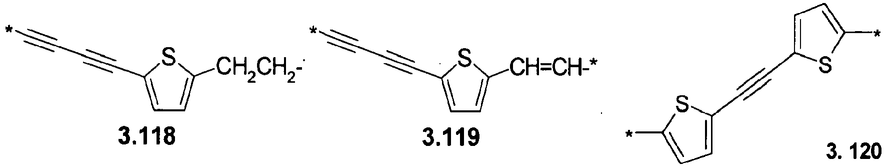 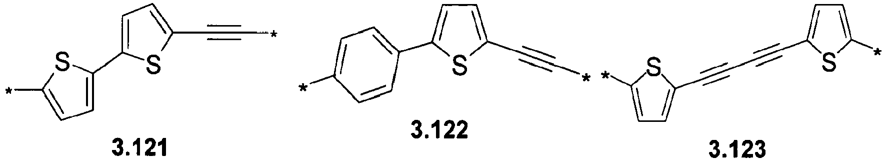 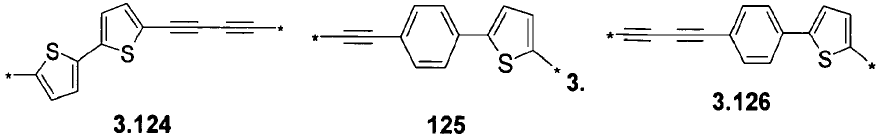 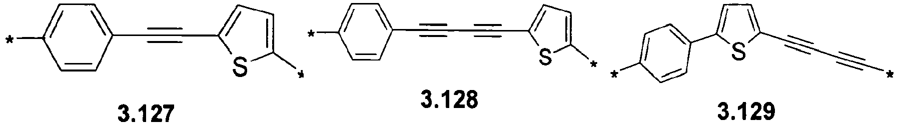
- alkyloxyaryl, alkenyloxyaryl, alkynyloxyaryl biradical selected from biradicals **3.130-3.136,** where an asterisk (*) denotes the places of azole fragment attachment; 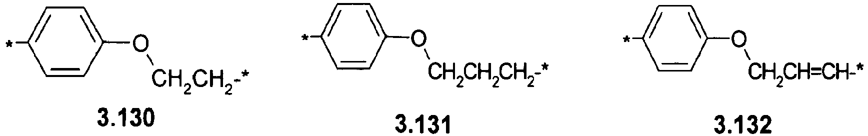 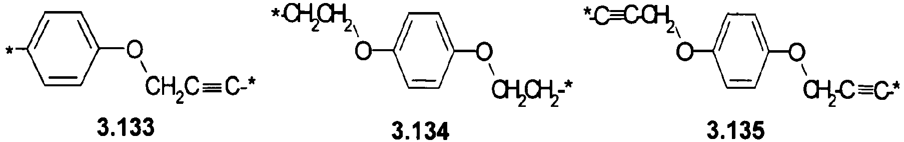 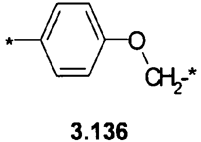
- cycloalkylthiophene, aryldioxane and thiophenedioxane biradical selected from biradicals **3.137-3.154,** where an asterisk (*) denotes the places of azole fragment attachment; 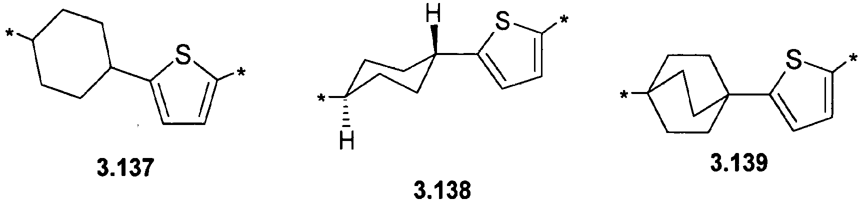 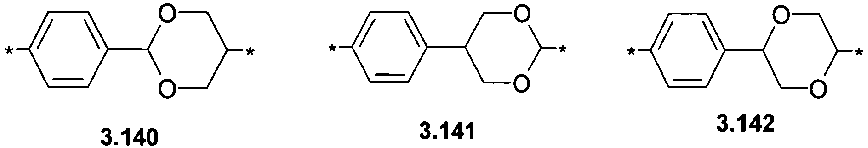 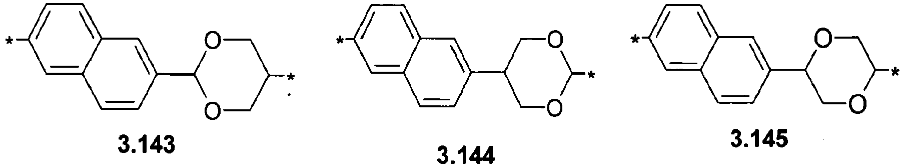 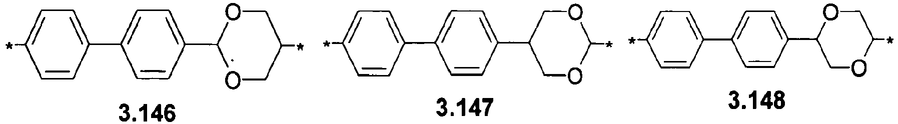 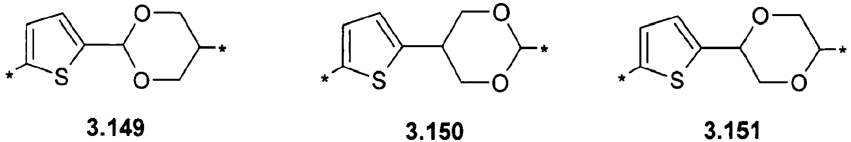 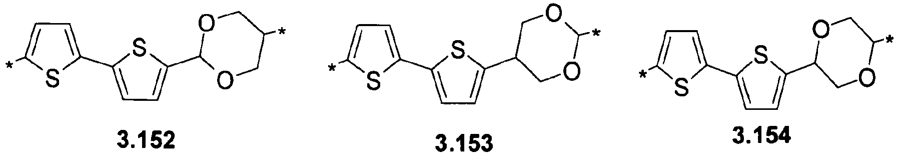 and also 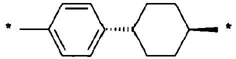 provided that Y= NH in one of the azole rings, and Y = O in the other azole rind, R¹ =R² = **2.3.**
**B** represents:
- aliphatic C₂-C₈ radical selected from radicals **4.1-4.12,** where an asterisk (*) denotes the place of azole fragment attachment;
| | | |
|---|---|---|
| *-C≡CH | HC≡C-CH₂-* | HC≡C-CH₂-CH₂-* |
| **4.1** | **4.2** | **4.3** |
| CH₃-C≡C-CH₂-* | HC≡C-C≡C-* | HC≡C-C≡C-CH₂-* |
| **4.4** | **4.5** | **4.6** |
| *-C≡C-C≡C-CH₃ | HC≡C-CH₂-C≡C-* | HC≡C-C≡C-CH₂-CH₂-* |
| **4.7** | **4.8** | **4.9** |
| *-C≡C-C≡C-CH₂-CH₃ | HC≡C-CH₂-CH₂-C≡C-* | HC≡C-C≡C-C≡C-* |
| **4.10** | **4.11** | **4.12** |
- aryl or thiophenyl radical selected from radicals **4.13-4.30,** where an asterisk (*) denotes the place of azole fragment attachment; 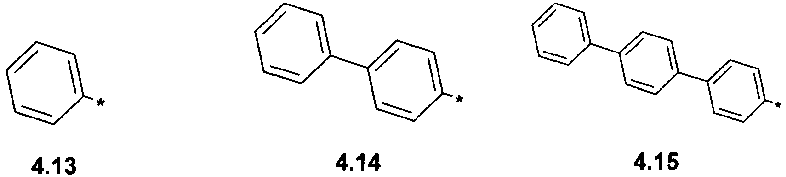 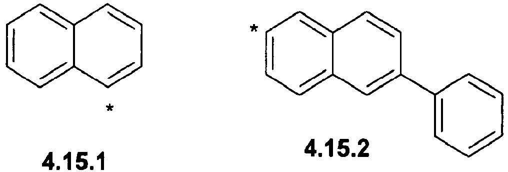 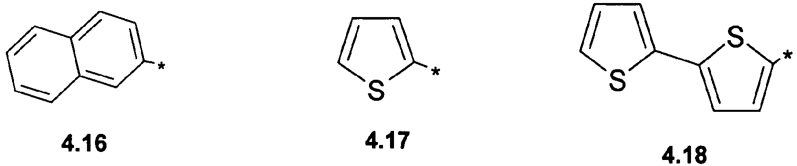 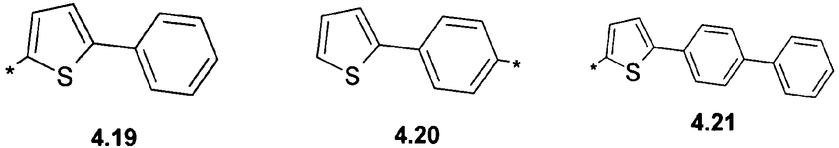 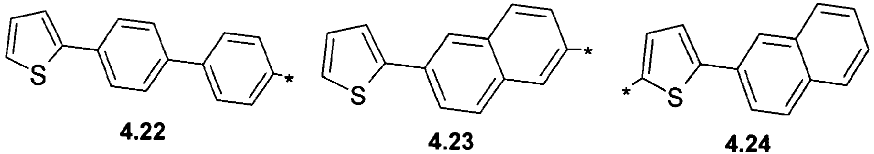 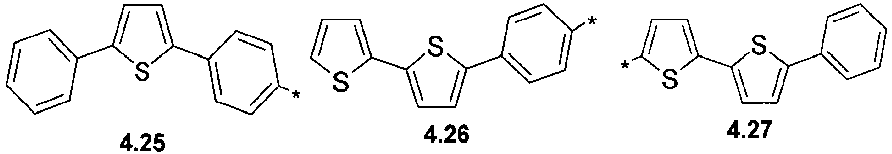 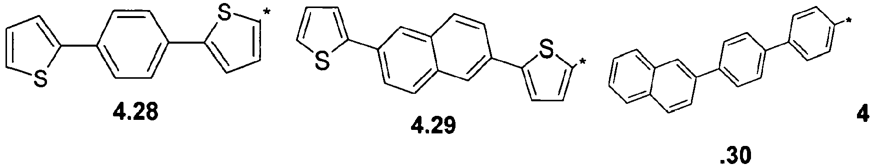
- alkynylcycloalkyl, alkynylaryl, alkylthiophene and alkynylthiophene radical, **4.31-4.47,** where an asterisk (*) denotes the place of azole fragment attachment; 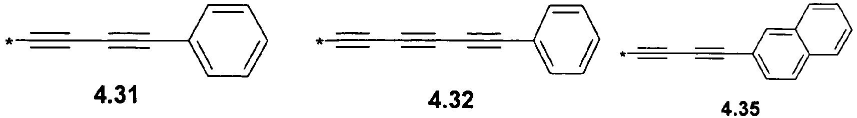 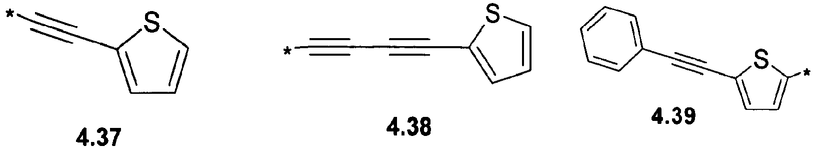 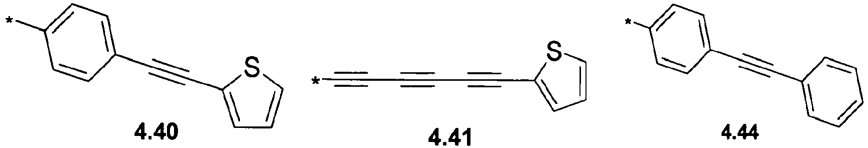 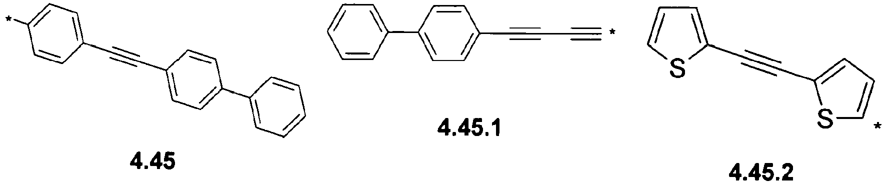 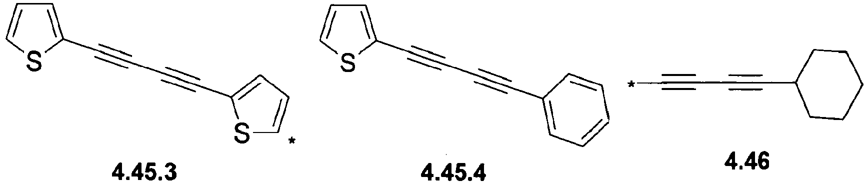 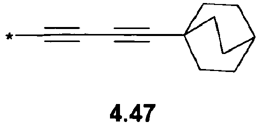
- cycloalkylbenzene, 4-cycloalkylbiphenyl, (bicyclooctane)benzene, 4-(bicyclooctane)biphenyl, aryldioxane and thiophenedioxane radical selected from radicals **4.48-4.72,** where an asterisk (*) denotes the place of azole fragment attachment; 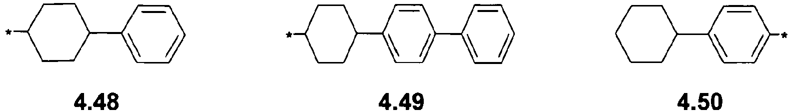 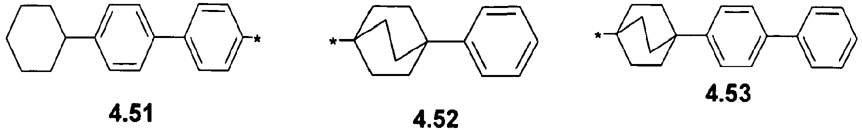 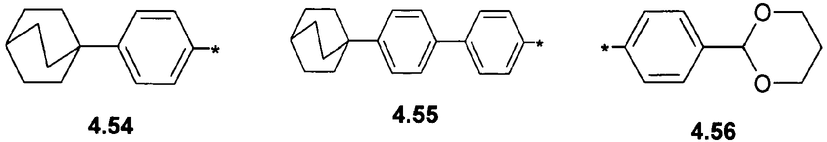 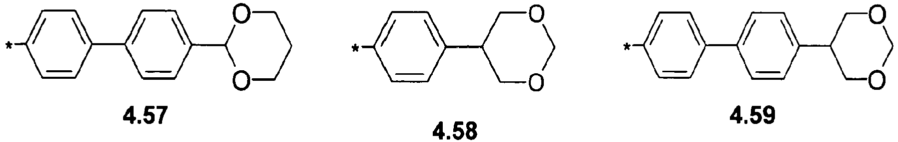 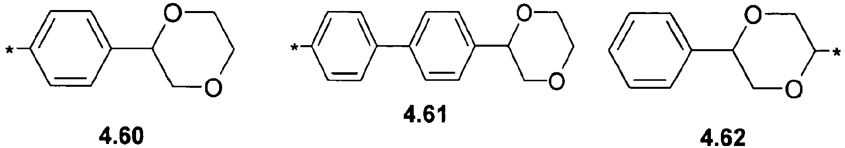 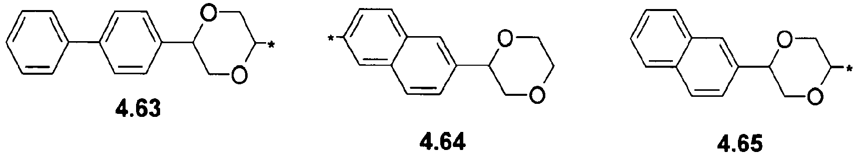 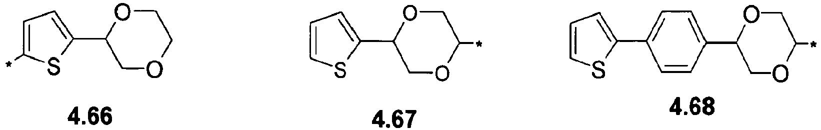 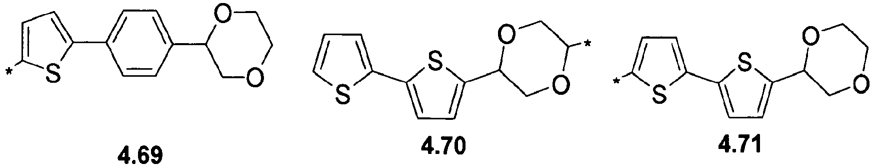 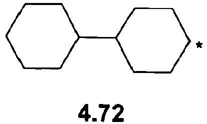
**with the exception of:**
((R)-1-{(R)-2-[5-(4-{2-[(R)-1-((R)-2-methoxycarbonylamino-3-methyl-butyryl)-pyrrolidin-2-yl]-3H-imidazol-4-yl}-buta-1,3-diynyl)-1H-imidazol-2-yl]-pyrrolidine-1-carbonyl}-2-methyl-propyl]-carbamic acid methyl ester;
((S)-1-{(S)-2-[5-(4-{2-[(S)-1-((S)-2-methoxycarbanylamino-3-methyl-butyryl)-pyrrolidin-2-yl]-3H-imidazol-4-yl}-phenyl)-1H-imidazol-2-yl]-pyrrolidine-1-carbonyl}-2-methyl-propyl)-carbamic acid methyl ester dihydrochloride;
((S)-1-{(S)-2-[5-(6-{2-[(S)-1-((S)-2-methoxycarbonylamino-3-methyl-butyryl)-pyrrolidin-2-yl]-3H-imidazol-4-yl}-naphthalen-2-yl)-1H-imidazol-2-yl]-pyrrolidine-1-carbonyl}-2-methyl-propyl)-carbamic acid methyl ester dihydrochloride;
[(S)-1-((S)-2-{5-[5-(4-{2-[(S)-1-((S)-2-methoxycarbonylamino-3-methyl-butyryl)-pyrrolidin-2-yl]-3H-imidazol-4-yl}-phenyl)-thiophen-2-yl]-1H-imidazol-2-yl}-pyrrolidine-1-carbonyl)-2-methyl-propyl]-carbamic acid methyl ester and dimethyl (2S,2'S)-1,1'-((2R,2'R)-2,2'-(5,5'-(4,4'-(thiophene-2,5-diyl)bis(4,1-phenylene))bis(1H-imidazole-5,2-diyl))bis(pyrrolidine-2,1-diyl))bis(3-methyl-1-oxobutane-2,1-diyl)dicarbamate.

2. Compounds according to claim 1, representing substituted azoles of the general formulas **5.1** - **5.70,** where X, Y, R¹, R² and solid lines with the accompanying dotted lines (---) have the above meanings.

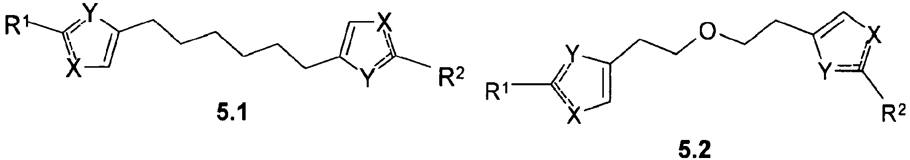

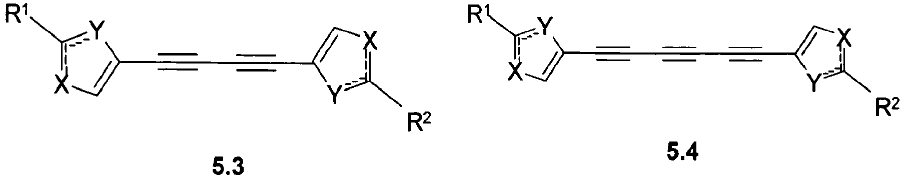

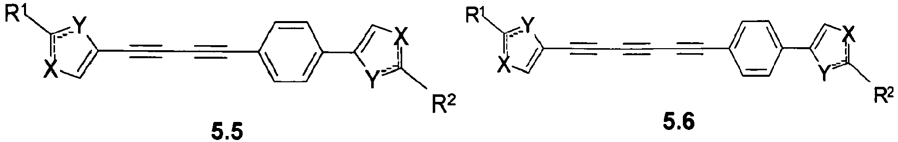

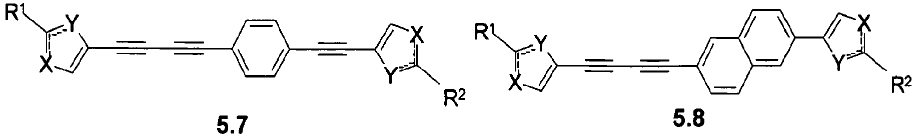

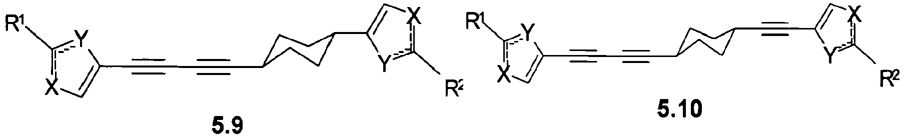

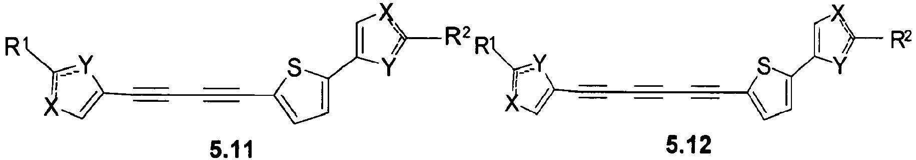

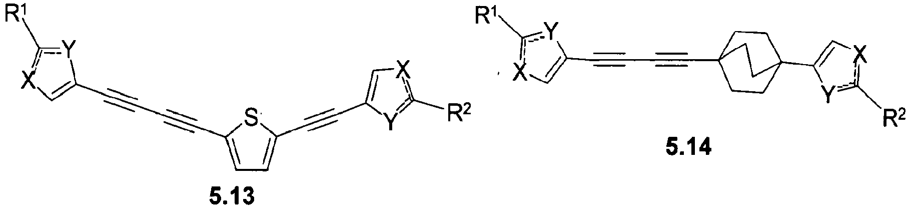

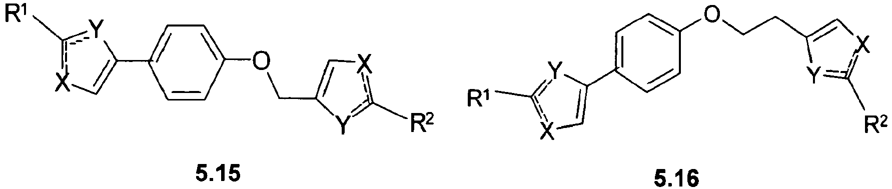

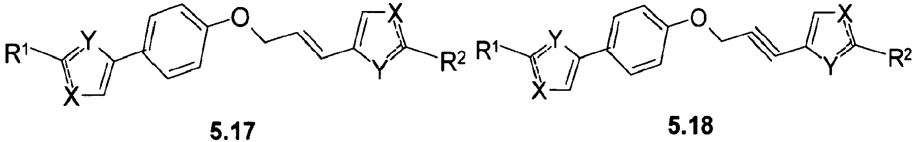

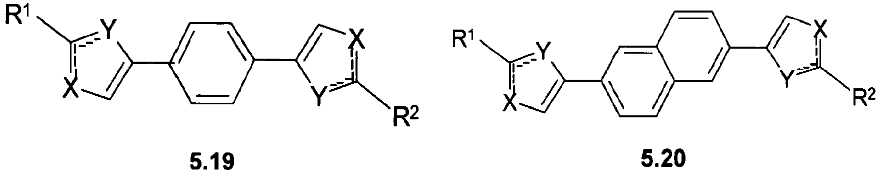

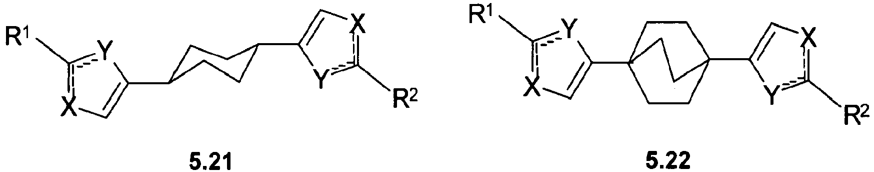

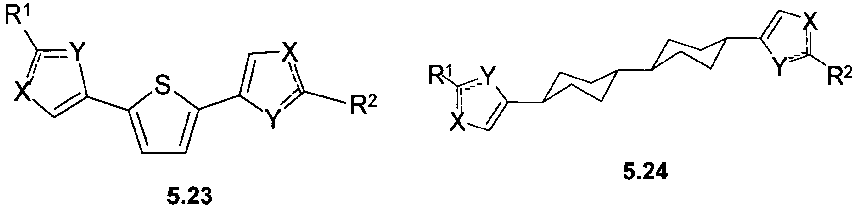

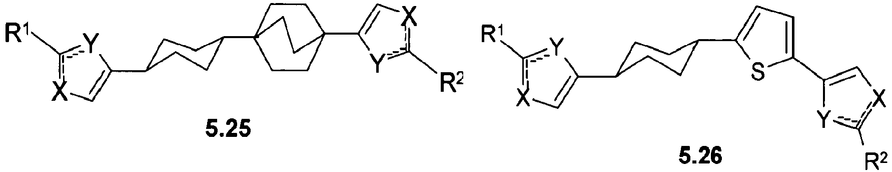

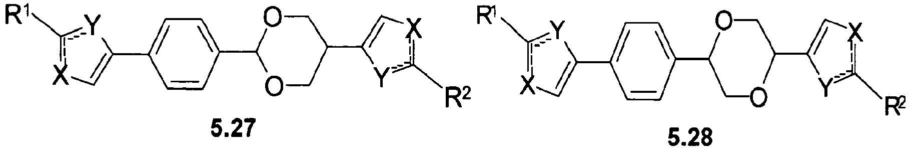

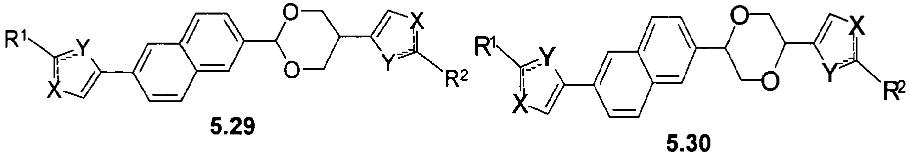

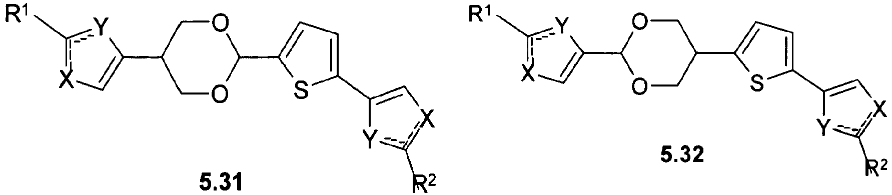

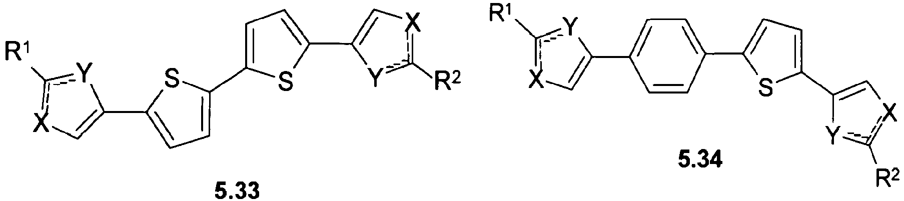

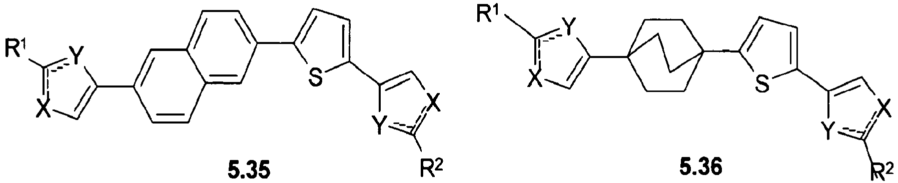

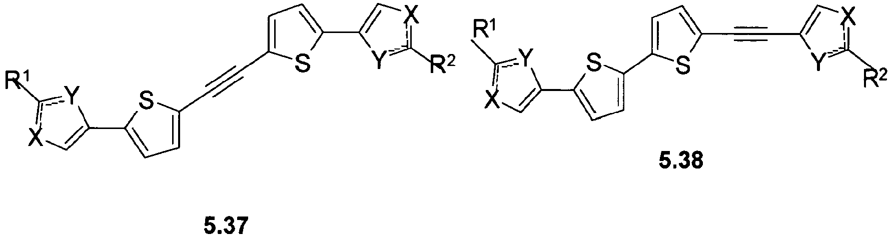

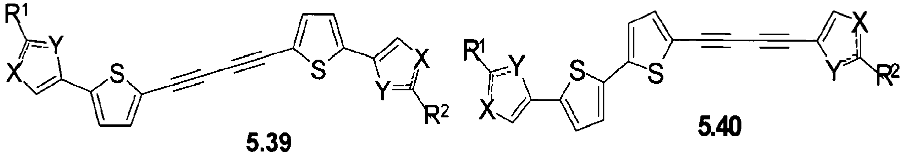

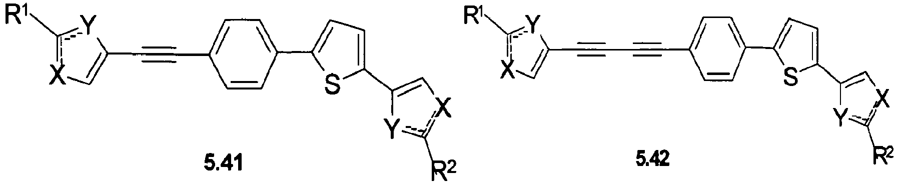

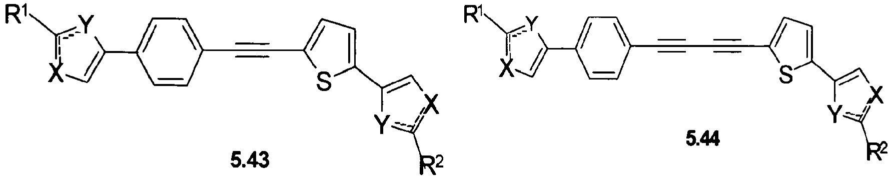

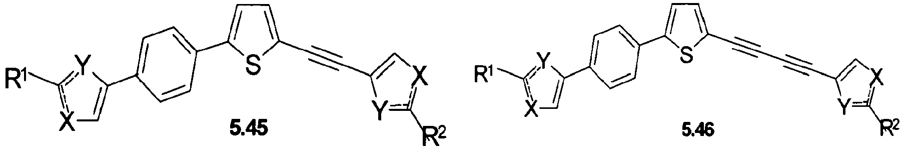

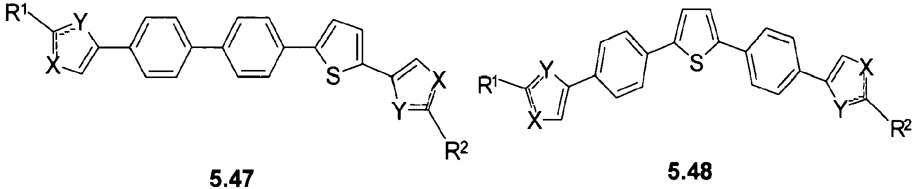

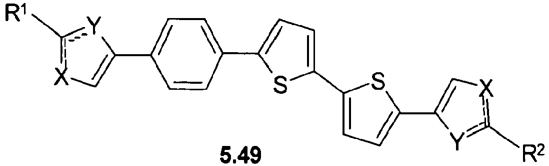

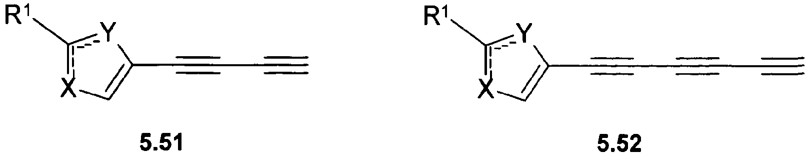

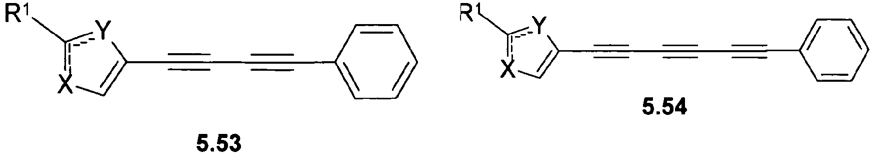

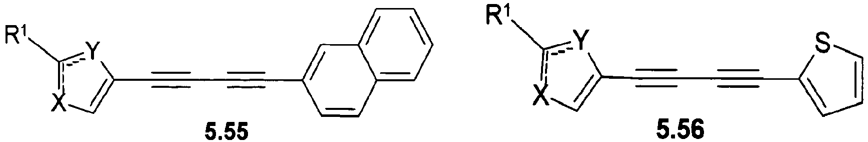

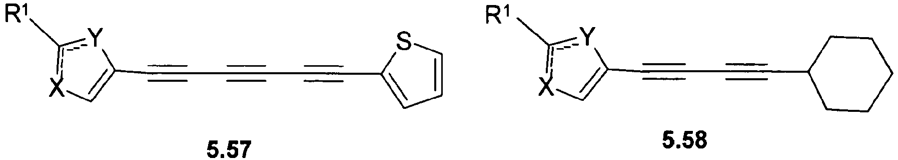

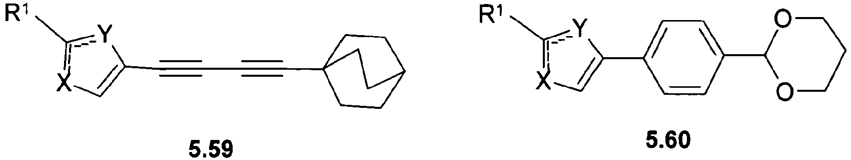

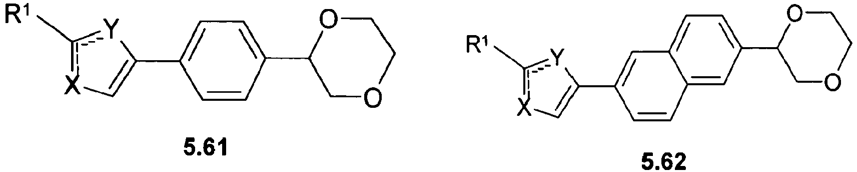

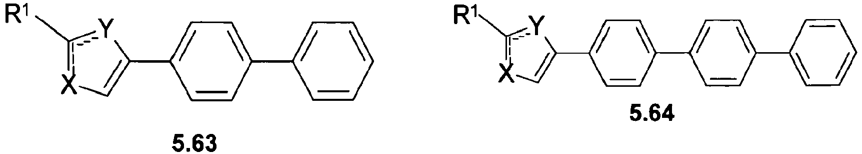

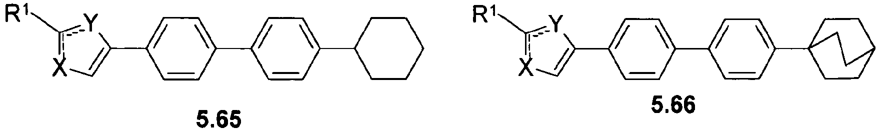

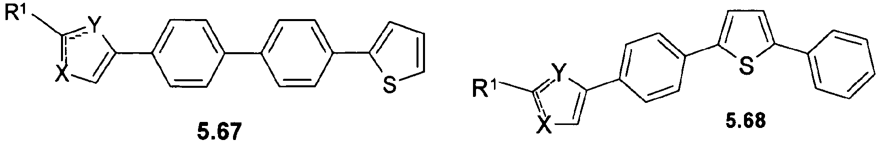

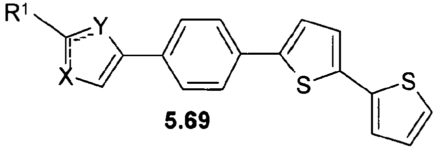

3. Compounds according to claim 1, representing substituted azoles of the general formulas **6.1** - **6.70,** where **A, X, Y,** and solid lines with accompanying dotted lines (---) have the above meanings.

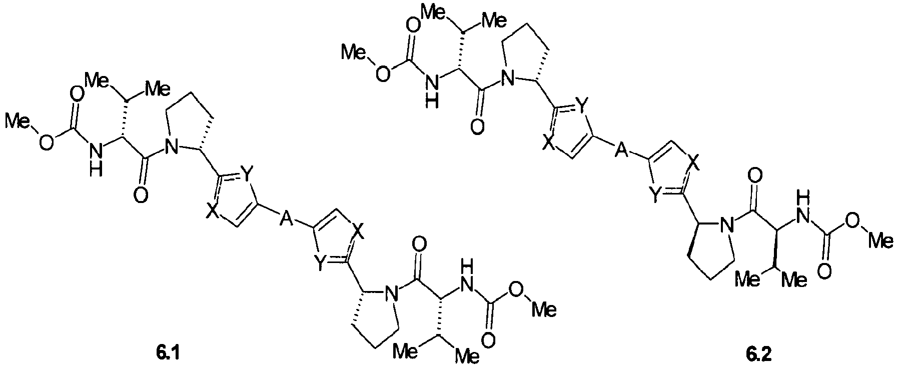

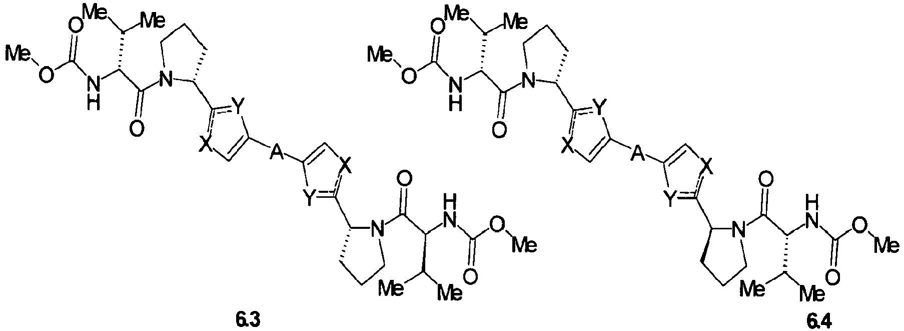

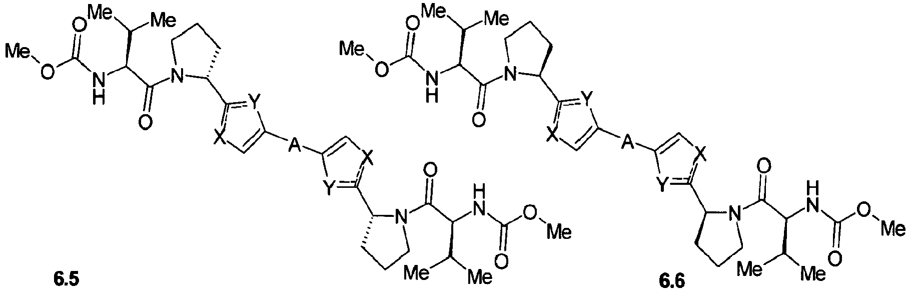

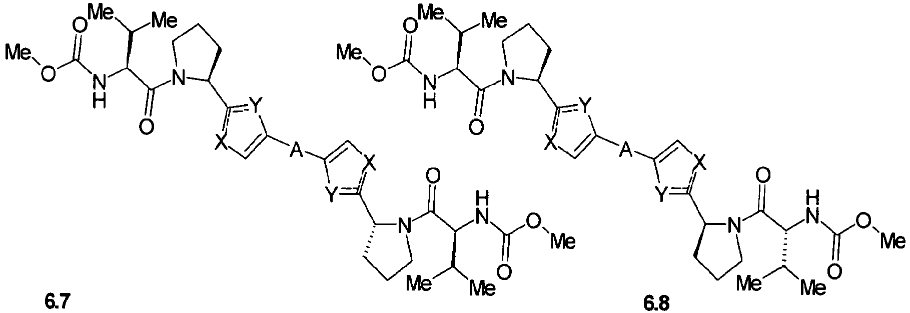

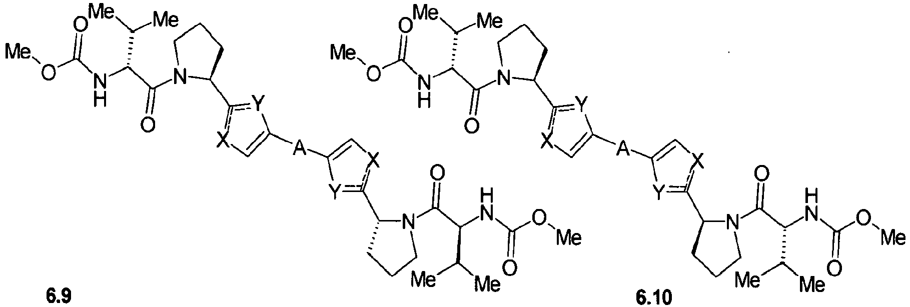

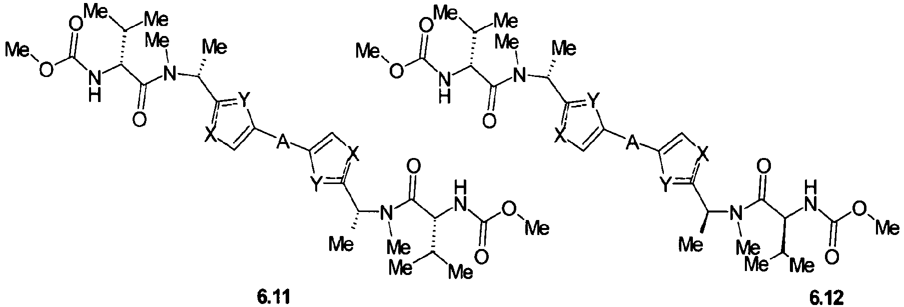

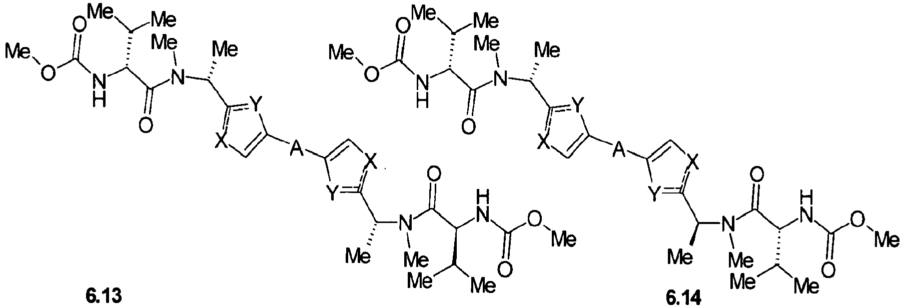

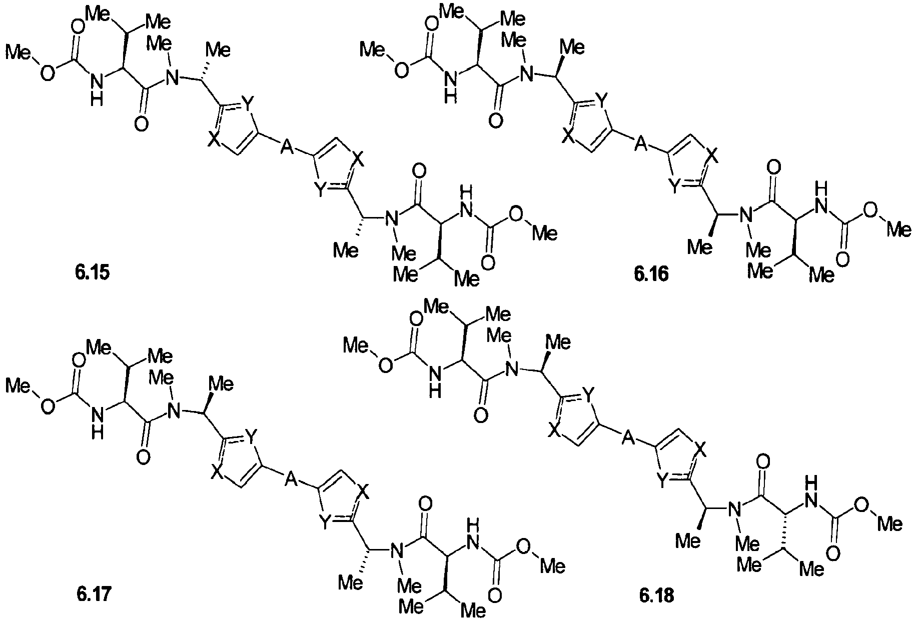

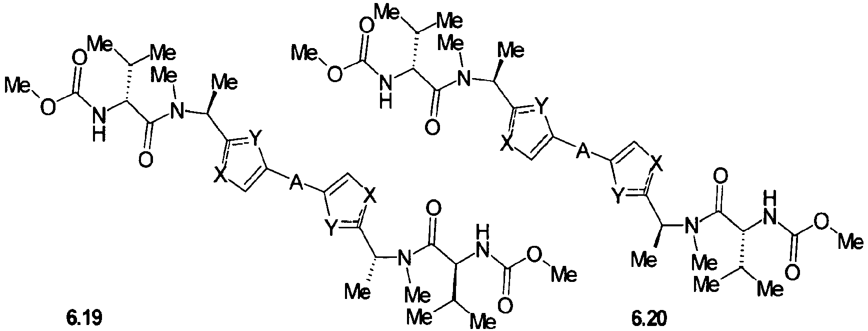

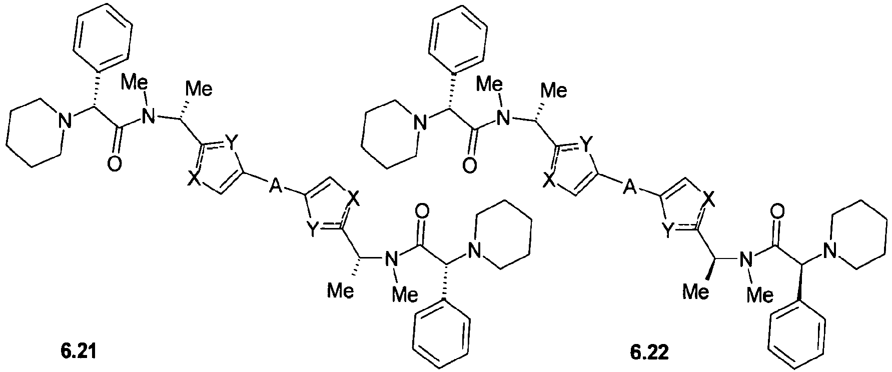

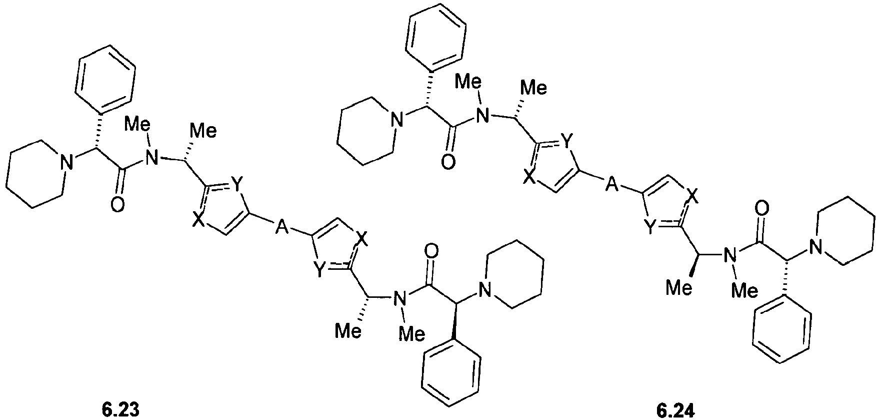

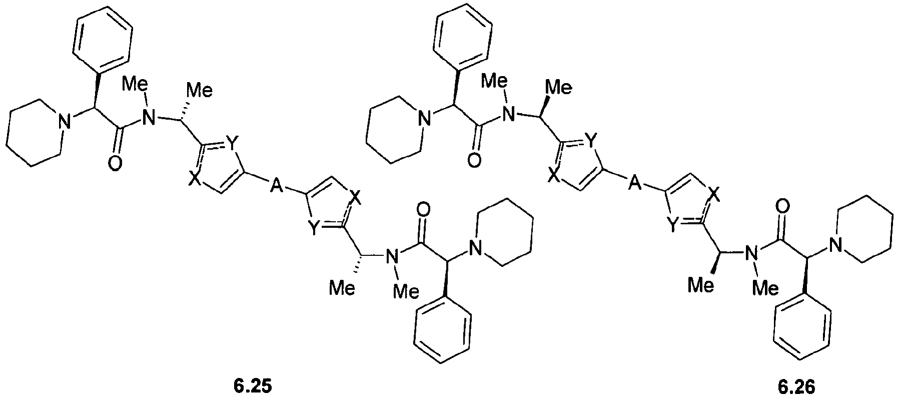

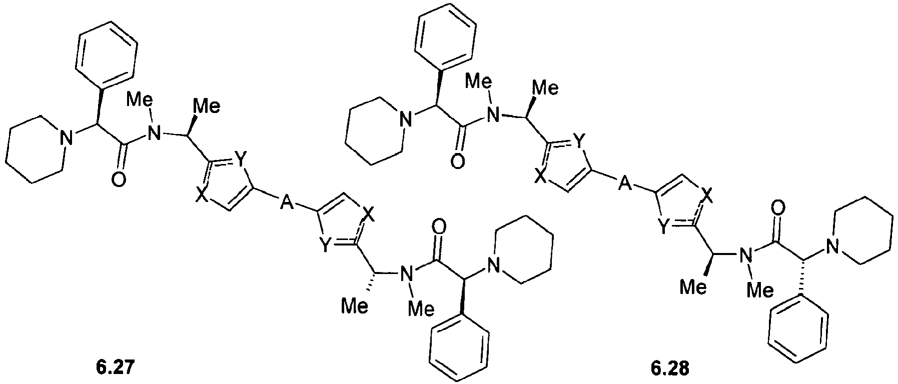

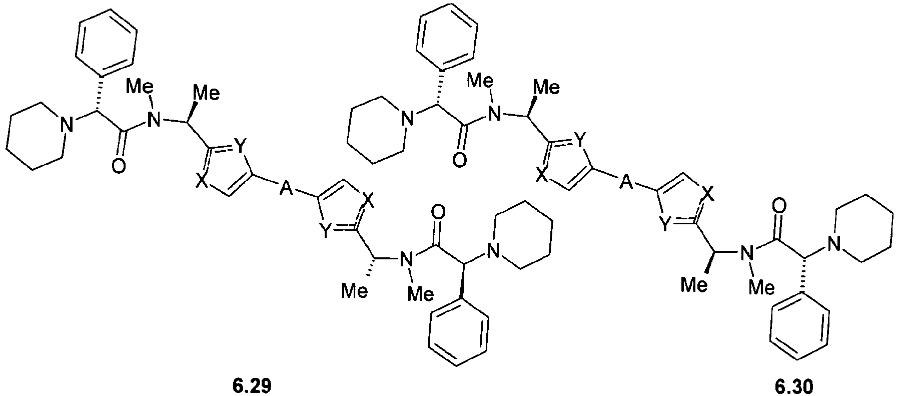

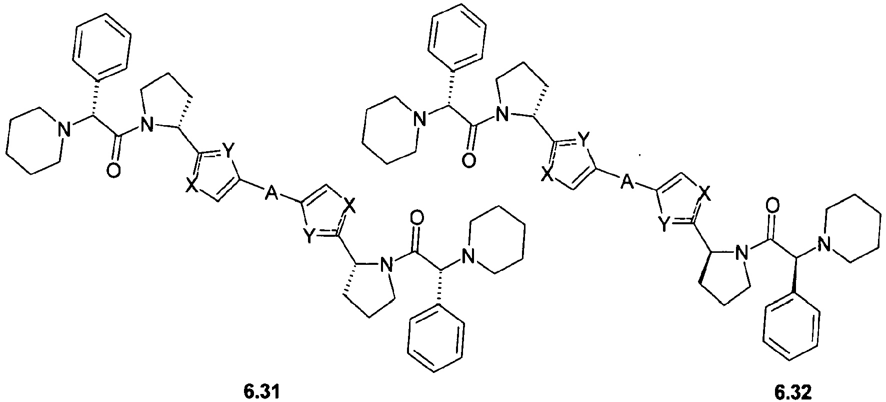

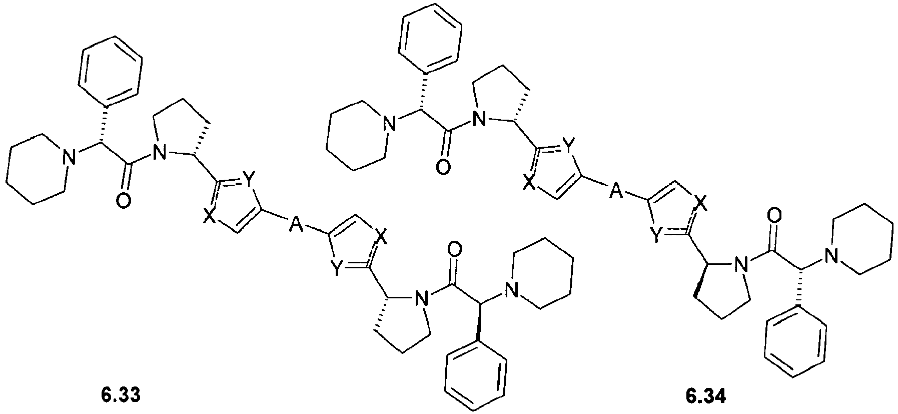

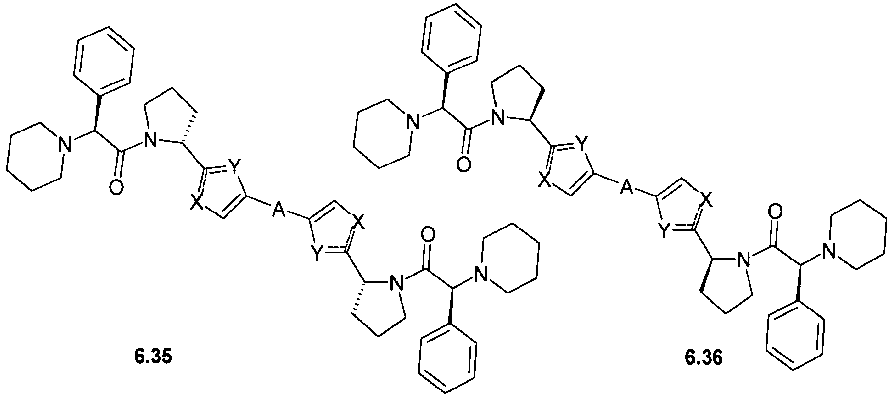

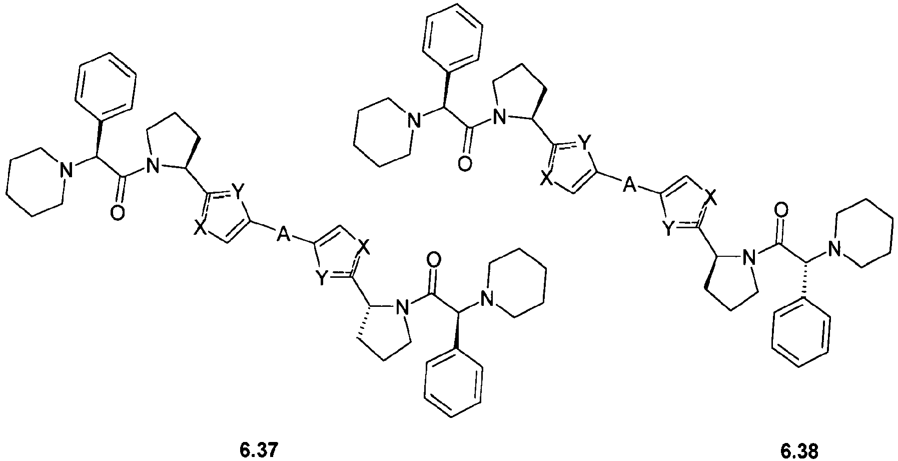

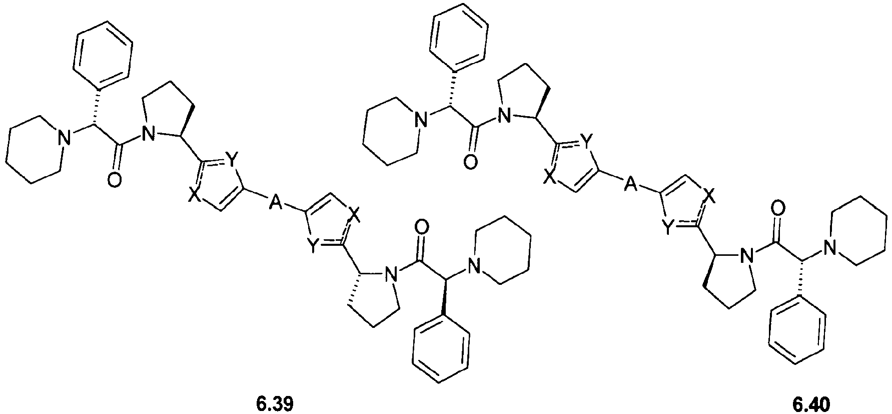

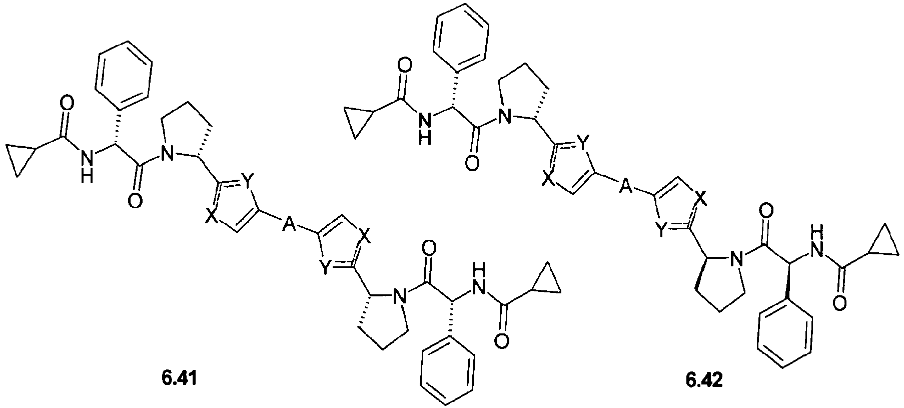

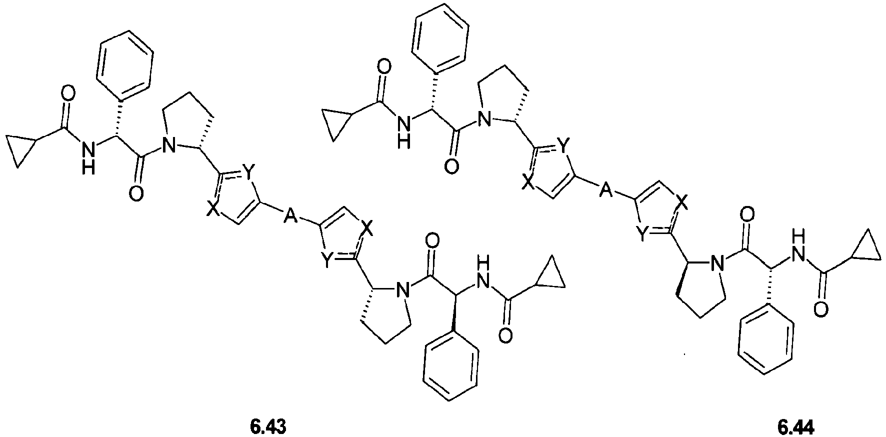

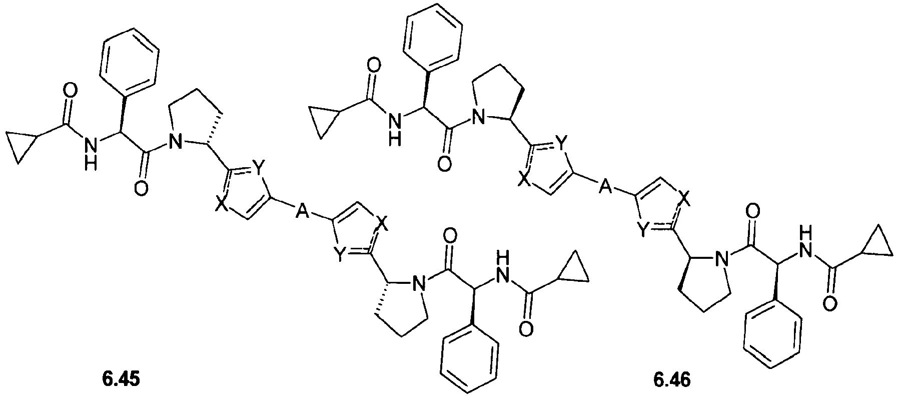

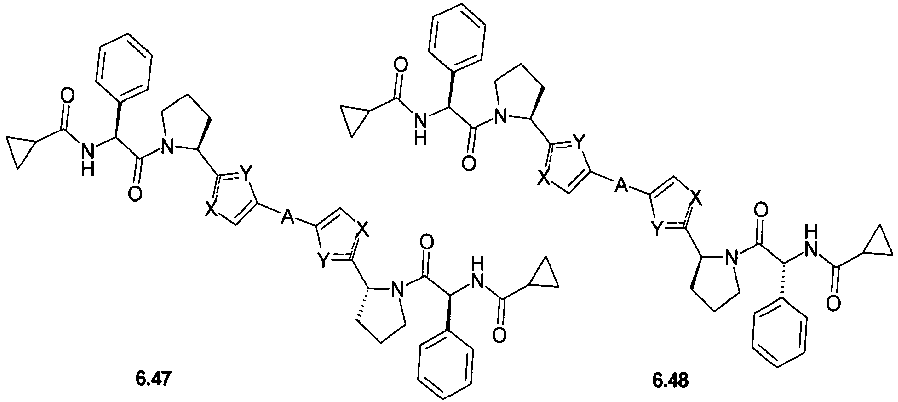

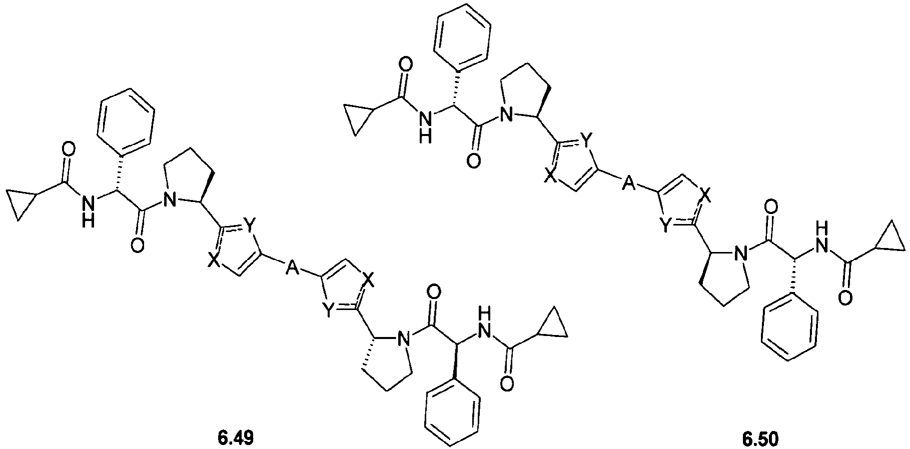

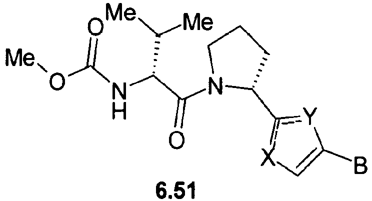

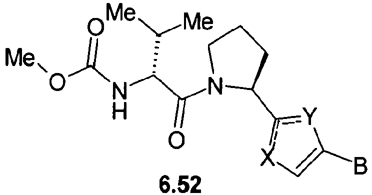

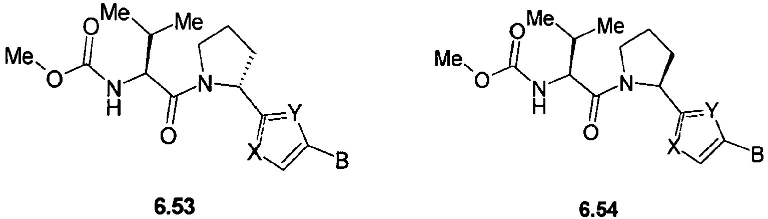

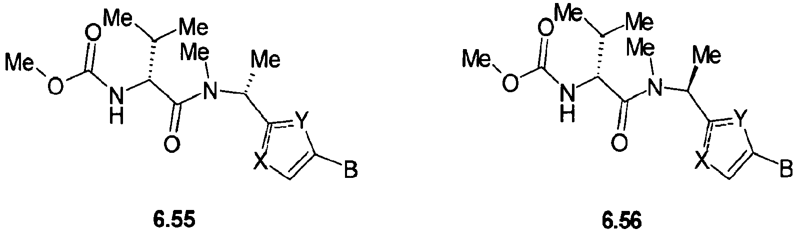

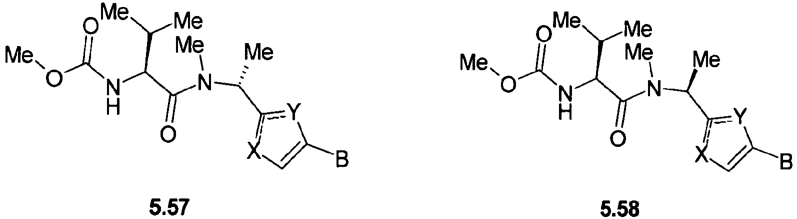

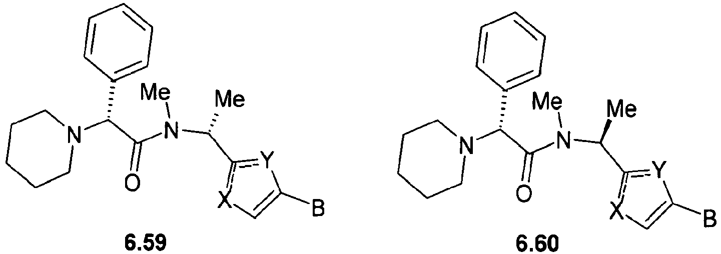

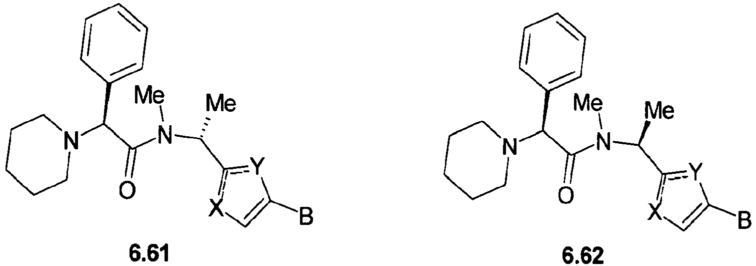

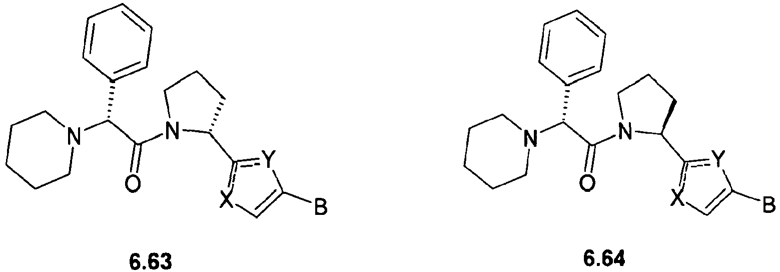

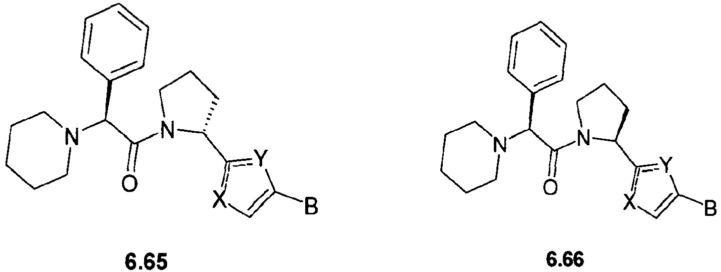

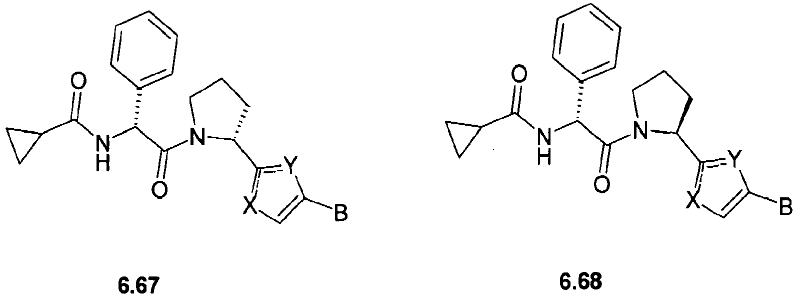

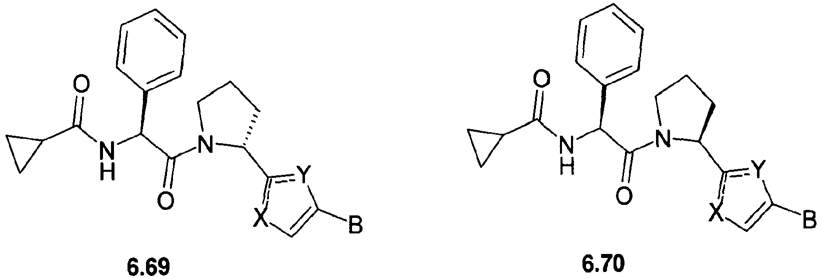

4. Compounds according to claims 1-3, representing substituted azoles of formulas **6 - 62 .**

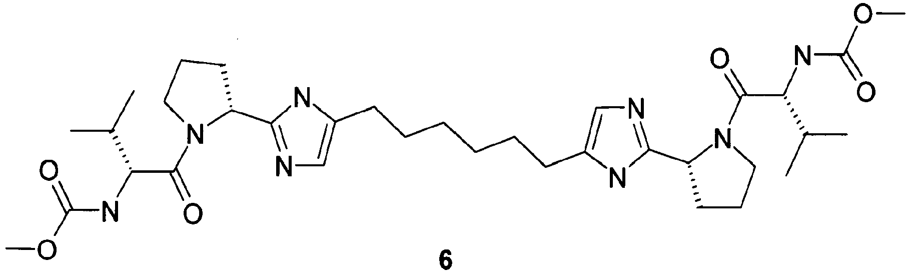

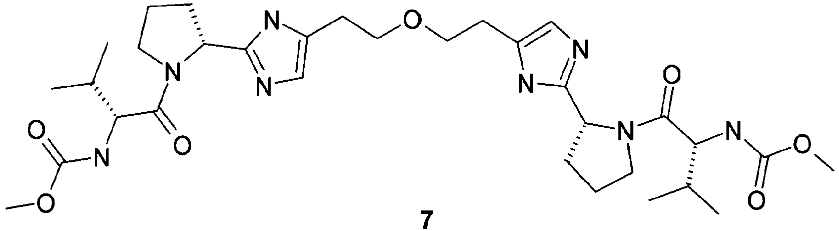

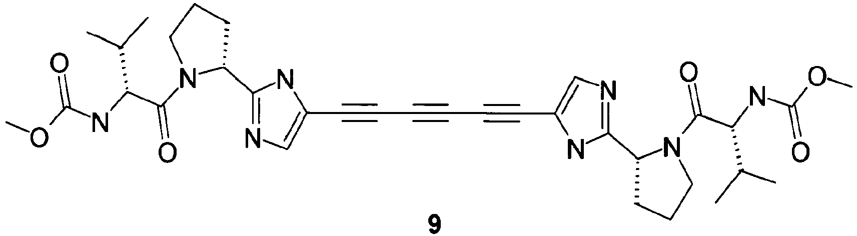

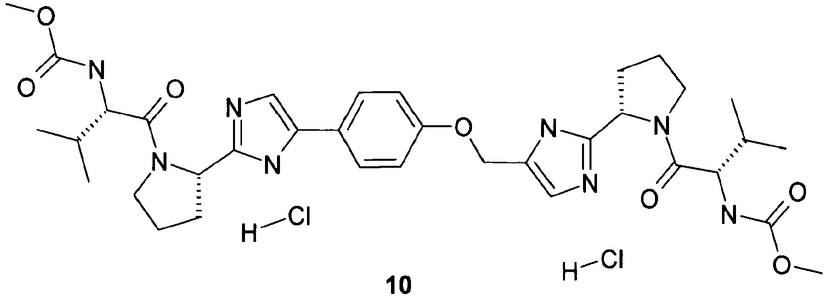

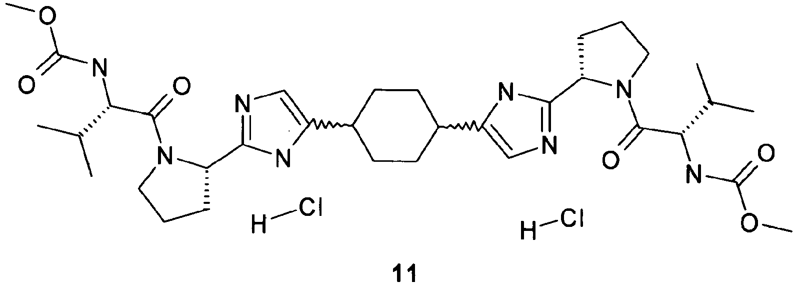

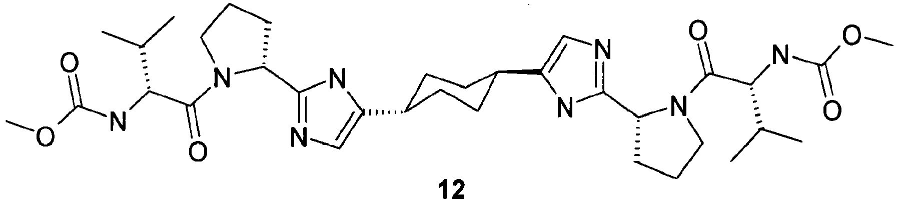

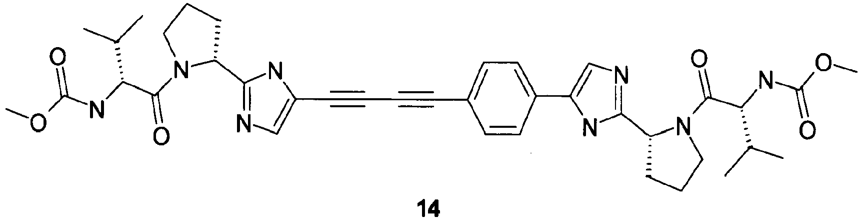

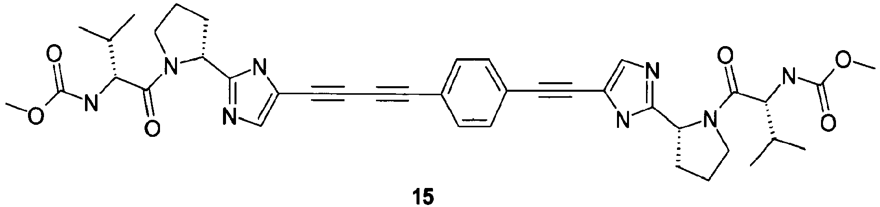

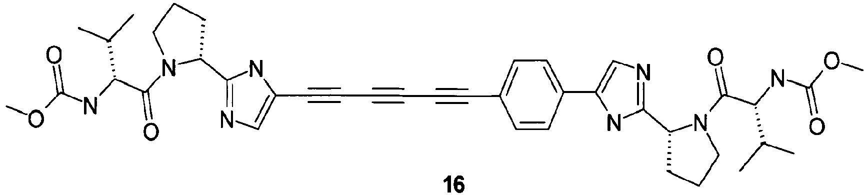

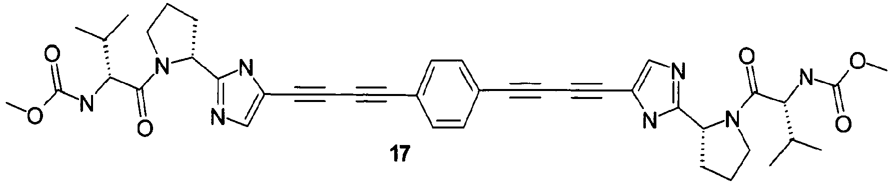

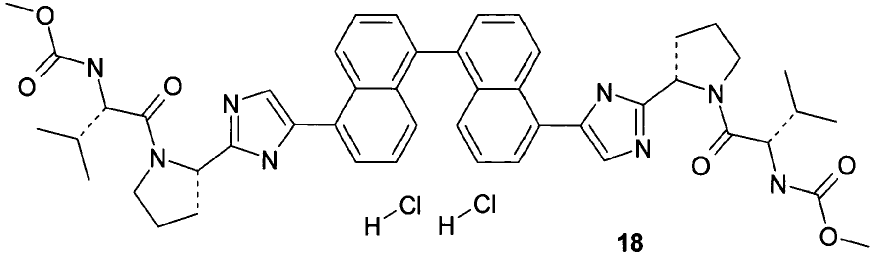

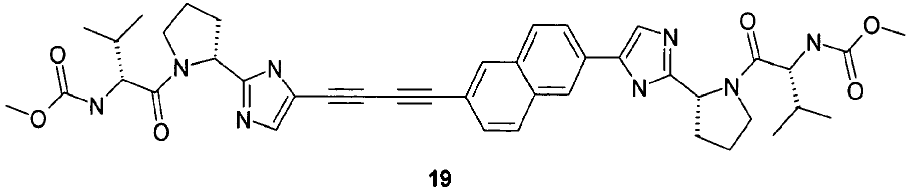

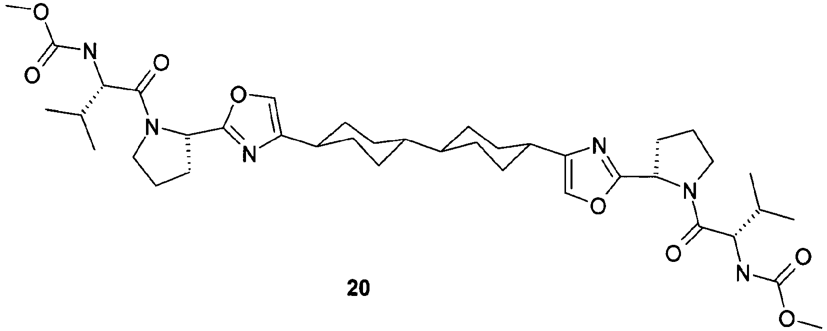

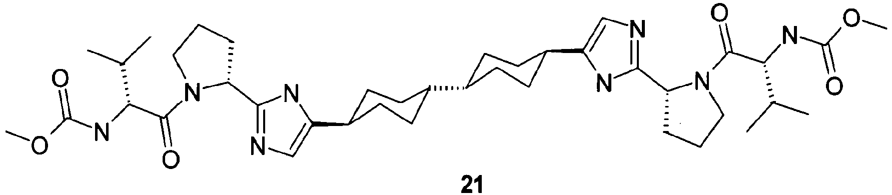

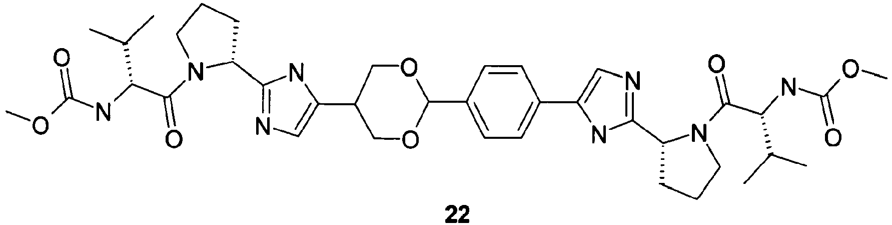

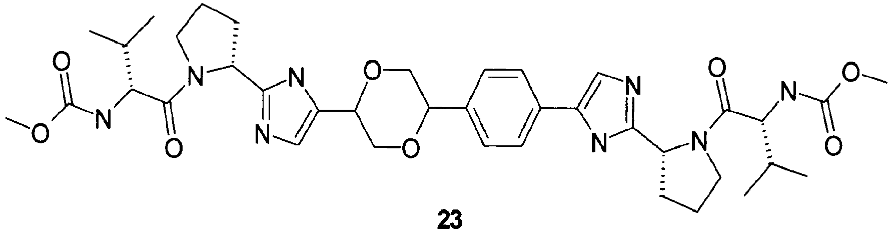

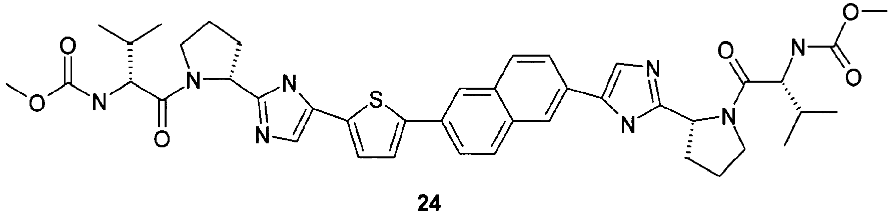

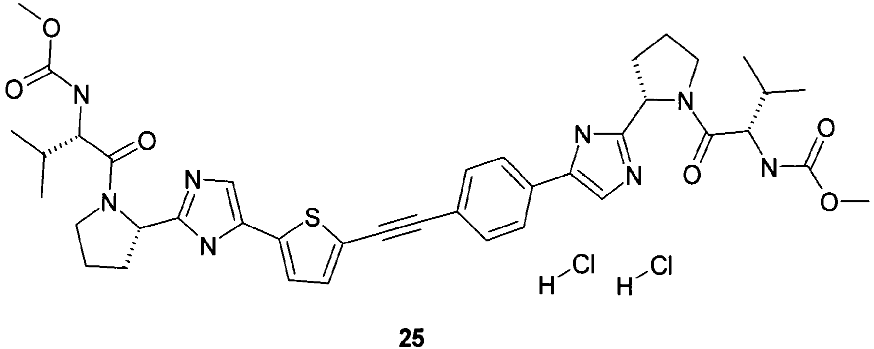

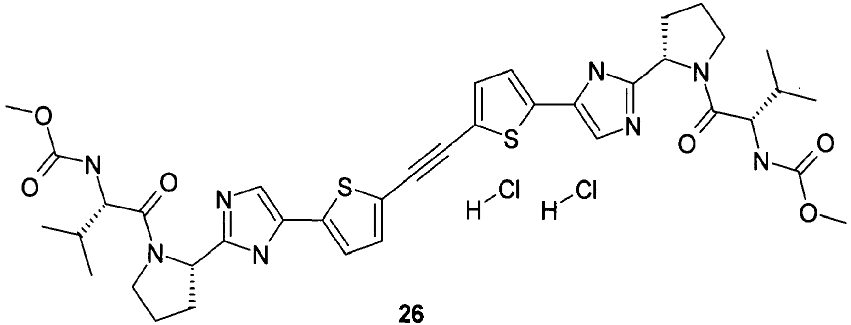

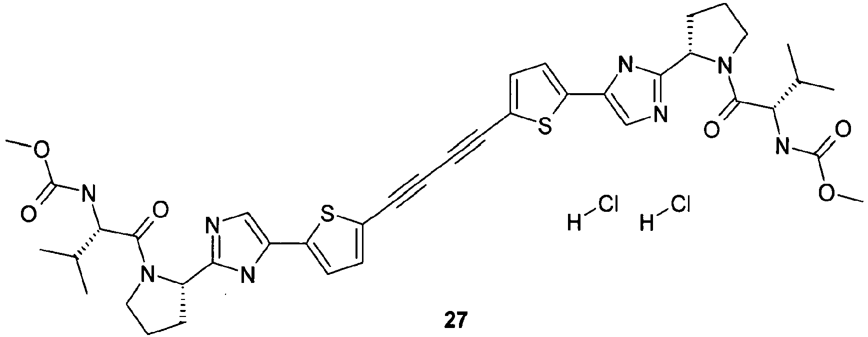

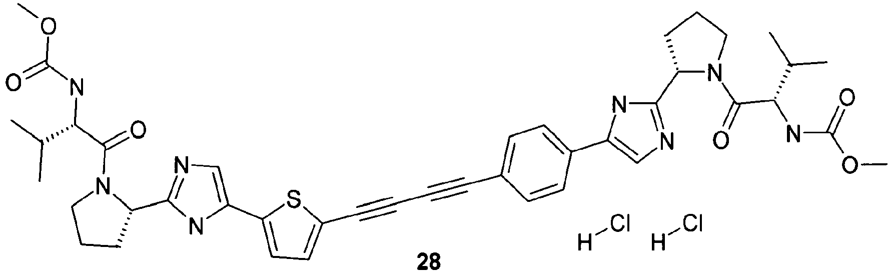

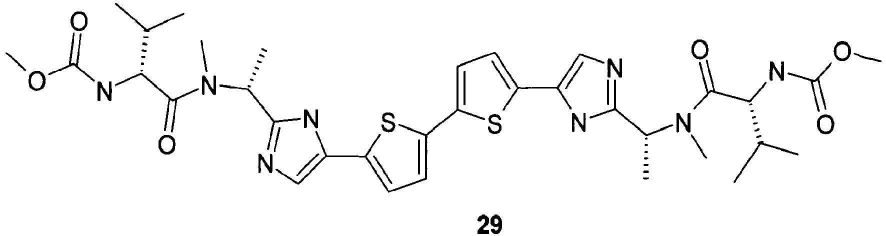

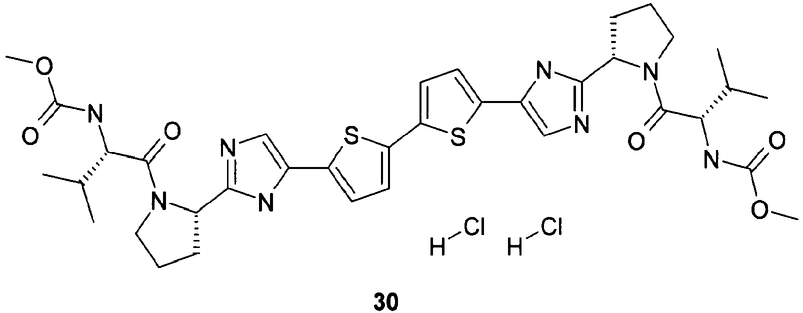

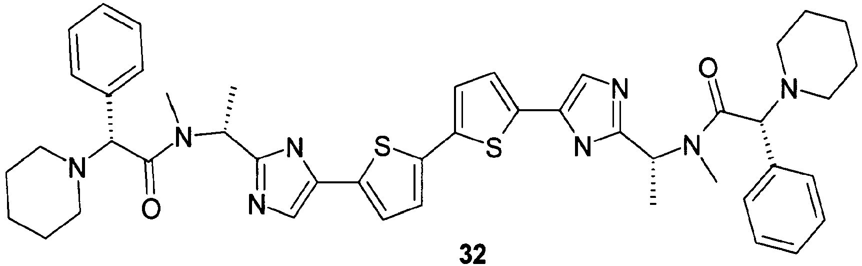

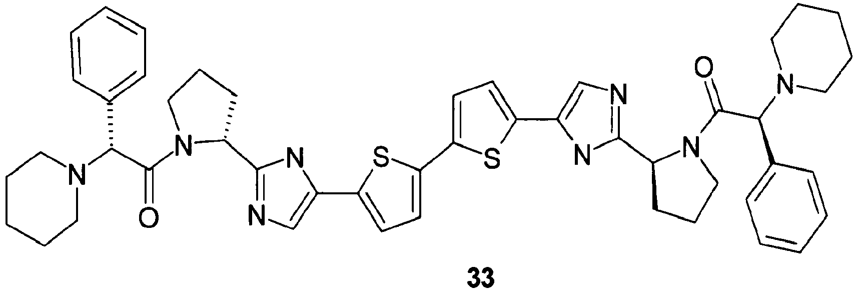

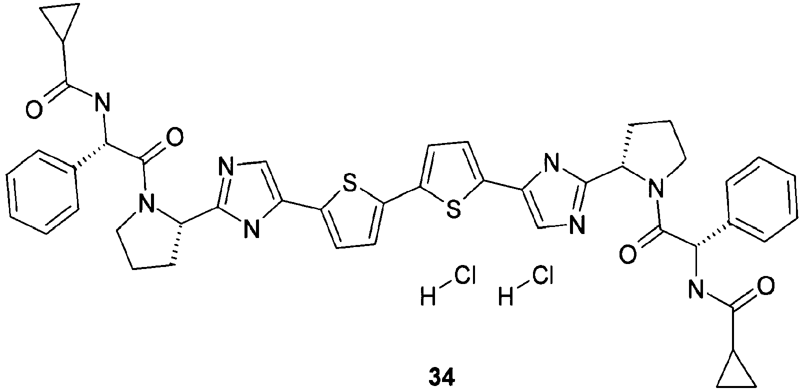

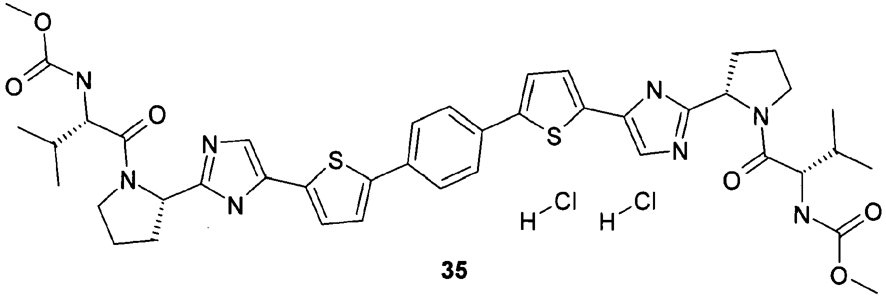

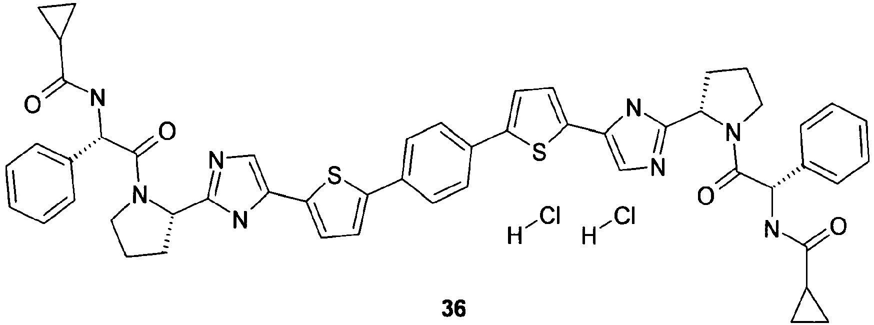

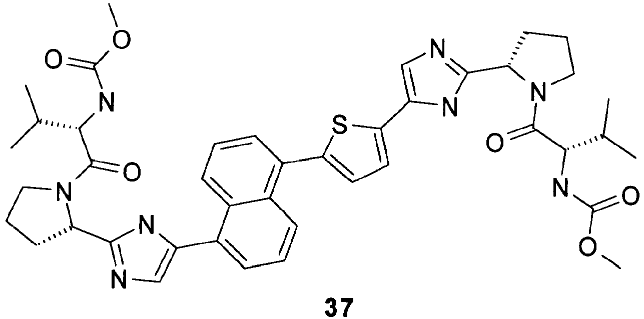

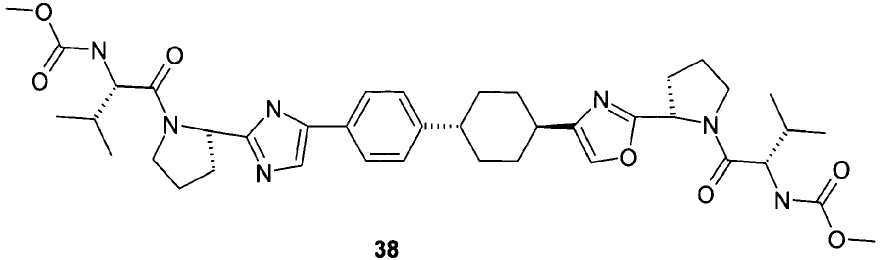

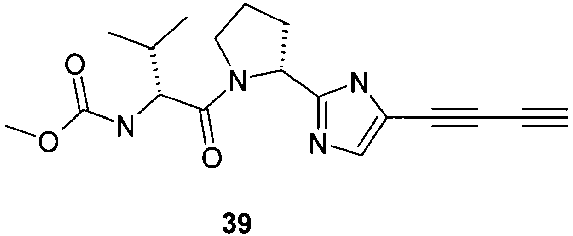

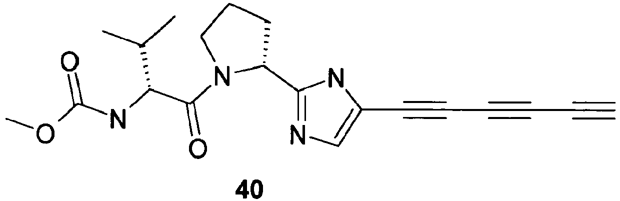

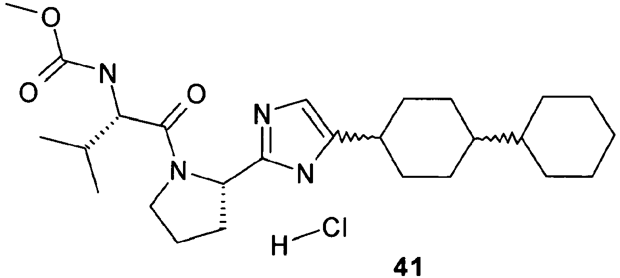

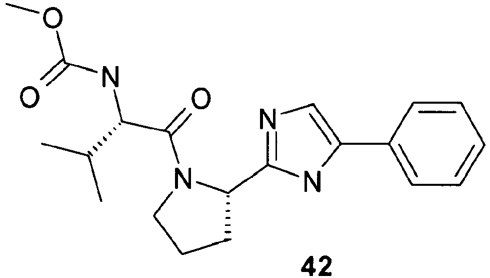

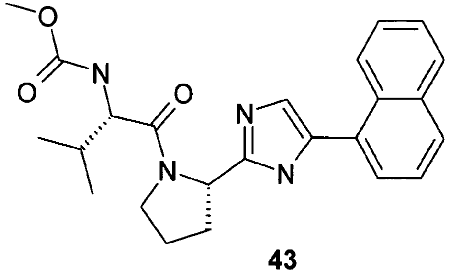

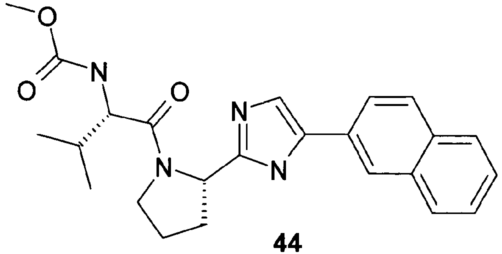

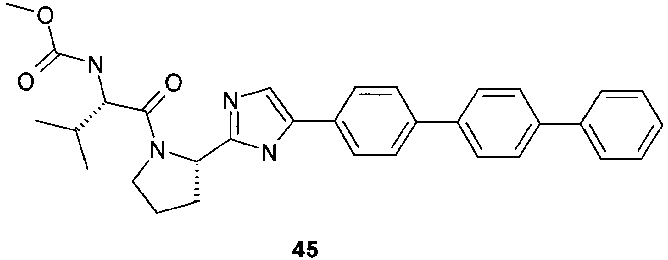

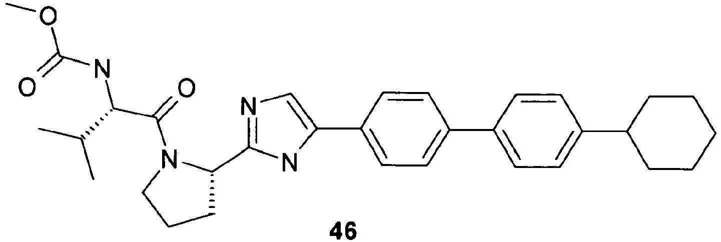

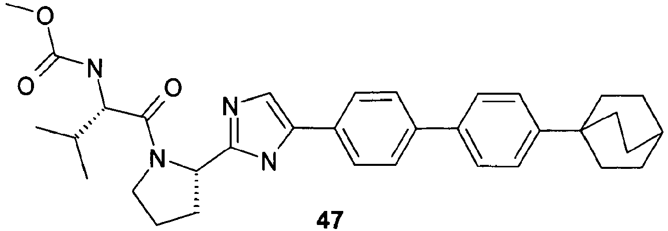

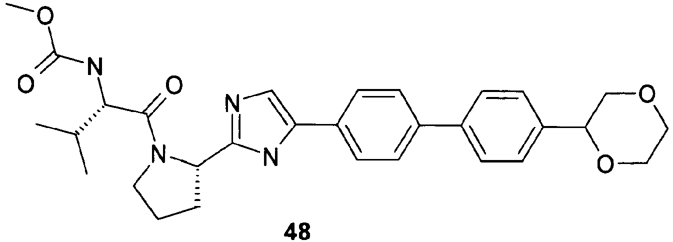

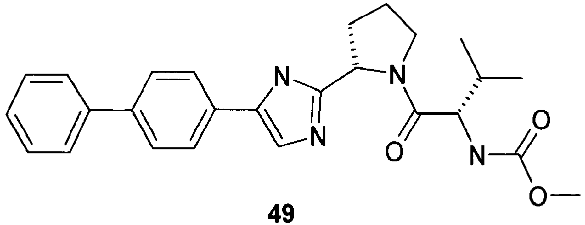

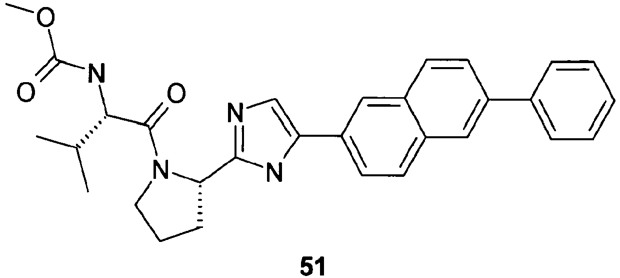

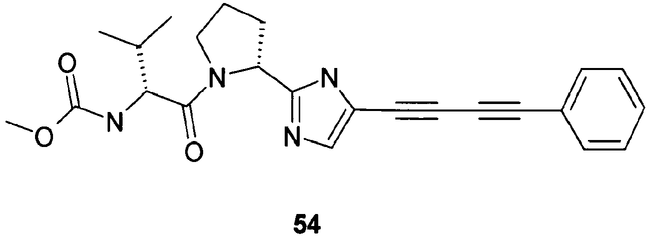

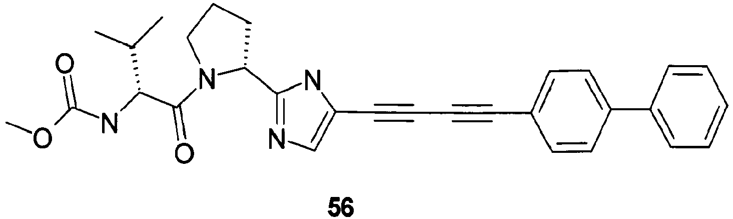

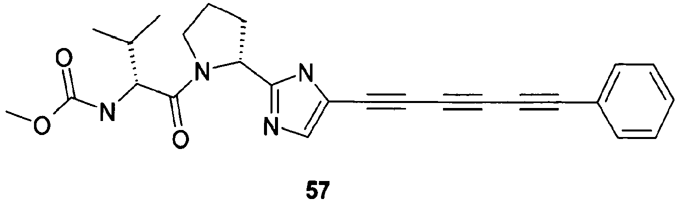

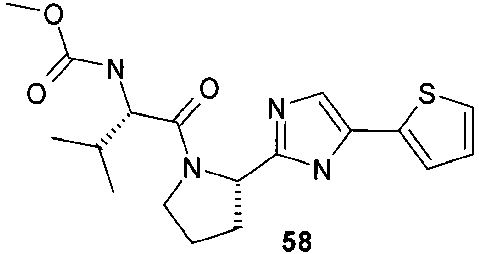

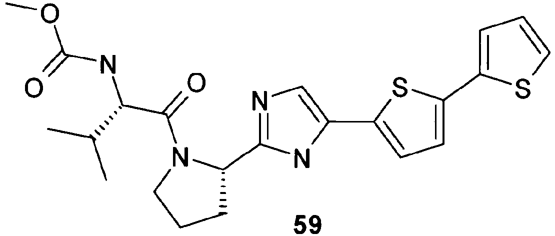

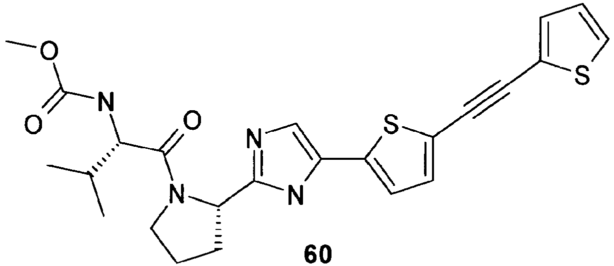

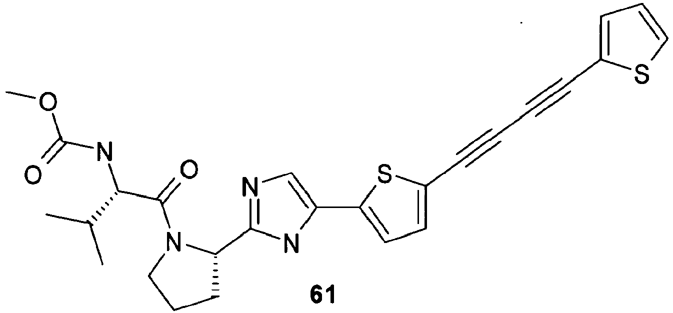

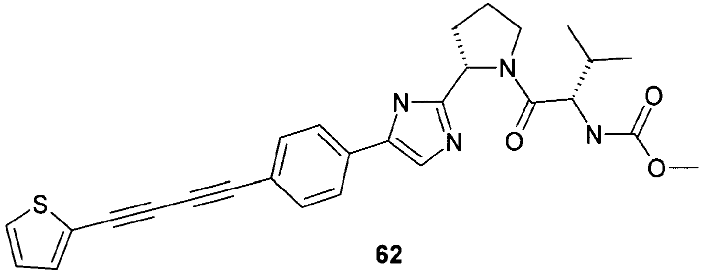

5. Ligands, the range of biological activity of which include viral NS5A protein representing substituted azoles of the general formulas **1A** and **1B** according to any of claims 1-4 and pharmaceutically acceptable salts thereof.

6. An active component for pharmaceutical compositions and medicaments intended for treatment and prophylaxis of flavivirus diseases caused by hepatisis C virus, GBV-C virus, yellow fever virus, West Nile virus, Dengue virus representing substituted azoles of the general formulas **1A** and **1B** according to any of claims 1-4 and pharmaceutically acceptable salts thereof.

7. A pharmaceutical composition for treatment and prophylaxis of diseases caused by hepatisis C virus, hepatitis GBV-C virus, yellow fever virus, West Nile virus, Dengue virus comprising the active component according to claim 6 in pharmaceutically effective amount.

8. The pharmaceutical composition according to claim 7 in the form of tablets, capsules, or injections placed in pharmaceutically acceptable packing.

9. A method for preparation of pharmaceutical composition by mixing of at least one of the active component of the general formulas **1A** and **1B** or its pharmaceutically acceptable salt with inert excipient and/or solvent.

10. A method for treatment of flavivirus diseases caused by hepatisis C virus, hepatitis GBV-C virus, yellow fever virus, West Nile virus, Dengue virus by introduction of pharmacologically effective amount of the active component according to claim 6 or pharmaceutical composition according to claims 7 or 8.

11. Therapeutical kit for prophylaxis and treatment of flavivirus diseases among them diseases caused by hepatisis C virus, hepatitis GBV-C virus, yellow fever virus, West Nile virus, Dengue virus comprising as one of the components substituted azole of the general formulas **1A** and **1B** according to any of claims 1-4 or its pharmaceutically acceptable salt or pharmaceutical composition according to claims 7 or 8.
